# EUROPEAN PATENT APPLICATION

(11) **EP 2 623 612 A1**
(43) Date of publication of application: **07.08.2013**
(21) Application number: 12153477.0
(22) Date of filing: 01.02.2012
(51) Int. Cl.: C12Q 1/68

(54) **Method for variant differentiation utilizing a pyrosequencing dispensation order**

(71) Applicant: Ruprecht-Karls-Universität Heidelberg, 69117 Heidelberg (DE)
(72) Inventor: Skorokhod, Alexander, 69124 Heidelberg (DE)
(74) Representative: Dick, Alexander

(57) **Abstract**

The present invention relates to a method for differentiating between at least two sequence variants of a polynucleotide by pyrosequencing, wherein at least one sequence variant comprises at least one mutation, said method comprising a) generating a dispensation order based on a nucleotide sequence encompassing the nucleotides affected by said variants, said dispensation order comprising dispensations corresponding to each nucleotide occurring in at least one of the variants, said nucleotide dispensations ordered in a sequence i) generating on average a pyrogram peak for at least every second nucleotide dispensation for each variant and ii) generating the maximal possible number of sequence pyrogram peaks for each variant; b) generating according to the dispensation order of a) a first pyrogram comprising expected intensity values for a first variant and at least a second pyrogram comprising expected intensity values for at least one further variant; c) comparing a sample pyrogram comprising measured intensity values, said sample pyrogram being derived from pyrosequencing analysis of a sample polynucleotide according to the nucleotide dispensation order of a), with the said first and at least the said second pyrogram generated in step b); and d) differentiating between said at least two sequence variants, wherein the sample polynucleotide corresponds to the first variant if the sample pyrogram is essentially identical with the first pyrogram and wherein the sample polynucleotide corresponds to a further variant if the sample pyrogram is essentially identical with a further pyrogram.

SEQUENCE LISTING

<110>  Ruprecht-Karls-Universität

<120>  Method for variant differentiation utilizing a pyrosequencing
       dispensation order

<130>  UH10783EP

<160>  43

<170>  PatentIn version 3.5

<210>  1
<211>  766
<212>  PRT
<213>  Homo sapiens

<400>  1

<210>  2
<211>  2949
<212>  DNA
<213>  Homo sapiens

<400>  2
cgcctccctt ccccctcccc gcccgacagc ggccgctcgg gccccggctc tcggttataa      60

gatggcggcg ctgagcggtg gcggtggtgg cggcgcggag ccgggccagg ctctgttcaa     120

cggggacatg gagcccgagg ccggcgccgg cgccggcgcc gcggcctctt cggctgcgga     180

ccctgccatt ccggaggagg tgtggaatat caaacaaatg attaagttga cacaggaaca     240

tatagaggcc ctattggaca aatttggtgg ggagcataat ccaccatcaa tatatctgga     300

ggcctatgaa gaatacacca gcaagctaga tgcactccaa caaagagaac aacagttatt     360

ggaatctctg gggaacggaa ctgatttttc tgtttctagc tctgcatcaa tggataccgt     420

tacatcttct tcctcttcta gcctttcagt gctaccttca tctctttcag tttttcaaaa     480

tcccacagat gtggcacgga gcaaccccaa gtcaccacaa aaacctatcg ttagagtctt     540

cctgcccaac aaacagagga cagtggtacc tgcaaggtgt ggagttacag tccgagacag     600

tctaaagaaa gcactgatga tgagaggtct aatcccagag tgctgtgctg tttacagaat     660

tcaggatgga gagaagaaac caattggttg ggacactgat atttcctggc ttactggaga     720

agaattgcat gtggaagtgt tggagaatgt tccacttaca acacacaact ttgtacgaaa     780

aacgtttttc accttagcat tttgtgactt ttgtcgaaag ctgcttttcc agggtttccg     840

ctgtcaaaca tgtggttata aatttcacca gcgttgtagt acagaagttc cactgatgtg     900

tgttaattat gaccaacttg atttgctgtt tgtctccaag ttctttgaac accacccaat     960

accacaggaa gaggcgtcct tagcagagac tgccctaaca tctggatcat ccccttccgc    1020

acccgcctcg gactctattg ggccccaaat tctcaccagt ccgtctcctt caaaatccat    1080

tccaattcca cagcccttcc gaccagcaga tgaagatcat cgaaatcaat ttgggcaacg    1140

agaccgatcc tcatcagctc ccaatgtgca tataaacaca atagaacctg tcaatattga    1200

tgacttgatt agagaccaag gatttcgtgg tgatggagga tcaaccacag gtttgtctgc    1260

taccccccct gcctcattac ctggctcact aactaacgtg aaagccttac agaaatctcc 1320

aggacctcag cgagaaagga agtcatcttc atcctcagaa gacaggaatc gaatgaaaac 1380

acttggtaga cgggactcga gtgatgattg ggagattcct gatgggcaga ttacagtggg 1440

acaaagaatt ggatctggat catttggaac agtctacaag ggaaagtggc atggtgatgt 1500

ggcagtgaaa atgttgaatg tgacagcacc tacacctcag cagttacaag ccttcaaaaa 1560

tgaagtagga gtactcagga aaacacgaca tgtgaatatc ctactcttca tgggctattc 1620

cacaaagcca caactggcta ttgttaccca gtggtgtgag ggctccagct tgtatcacca 1680

tctccatatc attgagacca aatttgagat gatcaaactt atagatattg cacgacagac 1740

tgcacagggc atggattact tacacgccaa gtcaatcatc cacagagacc tcaagagtaa 1800

taatatattt cttcatgaag acctcacagt aaaaataggt gattttggtc tagctacagt 1860

gaaatctcga tggagtgggt cccatcagtt tgaacagttg tctggatcca ttttgtggat 1920

ggcaccagaa gtcatcagaa tgcaagataa aaatccatac agctttcagt cagatgtata 1980

tgcatttgga attgttctgt atgaattgat gactggacag ttaccttatt caaacatcaa 2040

caacagggac cagataattt ttatggtggg acgaggatac ctgtctccag atctcagtaa 2100

ggtacggagt aactgtccaa aagccatgaa gagattaatg gcagagtgcc tcaaaaagaa 2160

aagagatgag agaccactct ttccccaaat tctcgcctct attgagctgc tggcccgctc 2220

attgccaaaa attcaccgca gtgcatcaga accctccttg aatcgggctg gtttccaaac 2280

agaggatttt agtctatatg cttgtgcttc tccaaaaaca cccatccagg cagggggata 2340

tggtgcgttt cctgtccact gaaacaaatg agtgagagag ttcaggagag tagcaacaaa 2400

aggaaaataa atgaacatat gtttgcttat atgttaaatt gaataaaata ctctcttttt 2460

ttttaaggtg aaccaaagaa cacttgtgtg gttaaagact agatataatt tttccccaaa 2520

ctaaaattta tacttaacat tggattttta acatccaagg gttaaaatac atagacattg 2580

ctaaaaattg gcagagcctc ttctagaggc tttactttct gttccgggtt tgtatcattc 2640

acttggttat tttaagtagt aaacttcagt ttctcatgca acttttgttg ccagctatca 2700

catgtccact agggactcca gaagaagacc ctacctatgc ctgtgtttgc aggtgagaag 2760

ttggcagtcg gttagcctgg gttagataag gcaaactgaa cagatctaat ttaggaagtc 2820

agtagaattt aataattcta ttattattct taataatttt tctataacta tttcttttta 2880

taacaatttg gaaaatgtgg atgtctttta tttccttgaa gcaataaact aagtttcttt 2940

ttataaaaa 2949

<210> 3
<211> 190753
<212> DNA
<213> Homo sapiens

<400> 3

cgcctccctt ccccctcccc gcccgacagc ggccgctcgg gccccggctc tcggttataa 60

gatggcggcg ctgagcggtg gcggtggtgg cggcgcggag ccgggccagg ctctgttcaa 120

cggggacatg gagcccgagg ccggcgccgg cgccggcgcc gcggcctctt cggctgcgga 180

ccctgccatt ccggaggagg tgagtgctgg cgccaccctg ccgccctccc gactccgggc 240

tcggcggctg gctggtgttt attttggaaa gaggcggcgg tgggggcttg atgccctcag 300

ccaccttctc gggccagctc cgcgggctgg gaggtgggca tcgcccccgt gtccctctcc 360

gtcatgcagc gccttcctac gtaaacacac acaatggccc ggggggtttc cctggccccc 420

accccagatg tggggattgg ggcagcggtg gttgagcggg aggctatcaa tagggggcga 480

aactcagggt tggtccgaga aggtcacgat tggctgaagt atccagctct gcatctctgt 540

ggggtggggg cggcggcggc ctcgacgtgg aggatatagg ttagttgctg gggctgagac 600

aacagcccga gttactgtcg cgtgtaattc ttacatggtc gtggggatga tggggctcat 660

catttcctct ctcctctccc ggactgcccc ccttctcagt ccgctgccct ttttcacttt 720

tctatttggg gatttctctt cacctgtttt acccagcaaa ttattttgat ttagtcttta 780

ctttttcaat cctaaatcgc agtttccgat gccttttctg gtctctggtc ctctgttcct 840

aatgtttgtc agcgctctgt cgctgattgg taacccccat tctattccca tctaccgccc 900

gctcattttc cagttgtcgg acctgcctgc cttctaaccc cagctcccac ttaagagcat 960

ttttgcactt ctcttaccct ggtcctcttg aggctctgta cttgatctca ccactcccta 1020

acattgttgt ctgttgttat cttcacaaat cctcctggac actttggagc tacttgtttt 1080

ctgagcccag aagctgtcaa gattccatca ggtttcactt ggctcttttc gcgcttgcac 1140

tactggcact ttttggctag tcgtccattg tgcattcaca cctctttatt cctacccatt 1200

tttataggtc tgattgattt cttagtgttg tcctcctttt tgtcctattt ttttcctttt 1260

cctttttcct ctccagtcct tgcttctctc agcctgtttt tgcattagtc agcctcttag 1320

cactgtgtca aattatttac gtttttttat tacataaaat ttattacaaa tatttggtat 1380

tttattacag aaaataatac tttattatgc tttacaaata agatatggta taataattgt 1440

ggtttacagt tattgattag gtaatgtgac ttactctgtt gactttgctc gaagttctct 1500

ttgctactta ctattaacat ctaatttctc aattctcata acatctcatt ctctctgcaa 1560

tttttttttt gcatcatcat ctttggaaat tcatccaata tgcttgcttt attcagcatc 1620

agcttgttta tgataatgtt tgttttctac tctttatatc atctttgtta catgcccaaa 1680

atgtgttctg taccatcatt tgatctgttc taaaatttct catttttaag tttcttaaaa 1740

tcattccact tttcagtatg catttttgct tagatcagtt tcctctcata tctgttcctt 1800

tcccccagct tcttgatttc taaggagaaa gctcttctct acttcaattt cctagtttat 1860

tctgtttccc ttgtttccag ttaccattca ttttgccttg tttcctggct tttggtactt 1920

aactttctga agcttcctct tttcttctcc acacctccac gttccttctt atttataaac 1980

atctttgttt cctttgacat ggaaatttat ttttaggata cattgttttt aatggataaa 2040

tactaggggt cacatctgct gtctgttttc tccaggaatc ggatatgcct ttgtcttaac 2100

caggcacagg tgcctctgga ttttatttta ctctgtaata gatgtgtagt tttgttgaat 2160

tgtatcttgt ttgaagacta ctacagagtg gaacaatgag tgaagtaata agtaggggtt 2220

atgaaattgt aattctctga tttataaatt gtttatcttg ggaactttgc tgcagagtta 2280

ttagaaccgt ttgcaattct gtaaagaagg cttttgtgaa gtaaaatctc taccctteta 2340

ttttatttga aagggccaga ttgtttggaa ctgtaccccc tgaagagtct gatttagtaa 2400

gtgagagcga gggccatgga tttctgtatt tggcacatgt cttgagcagt tcccatgtac 2460

caatccttga gaacctctag gctagctgaa tttaagtata aattgccagt aattggaaag 2520

catattcata tcttctgaaa ctataaggat actctcattt tacttggtta aaaaacaagt 2580

gtttcctact gtcctcttta cccaggtttt aatgtttagt ggtgaacagt agttttccct 2640

ctacattttt ttctgaactg ataataaatg tatttggctg ggagggtgac attgattaaa 2700

aaatgtatct cttgaatgta aatatcagta ttacagatga taaaataaat tcctccaaga 2760

aataatttta aatttgaagt tgatattcag tggaaactga aatgtgctgt ggtcttttat 2820

ttgaagtctt ccttacattc acttaaaggg atcttttact gcaaattaca tggaaagaat 2880

gaaaaggttt gcttgtgtgt aatgacacat tttattctga agatttattt tacctaacag 2940

taaaatgtag gttttttttt tttaaataaa agtttcccag agggaaattt catctaaaaa 3000

aaaagtctga tttcaaaggg aaagcaagtc attatcaaaa attagaaaac tataagtaca 3060

aaaagtaaaa aatcatcagt aattttgcca ctaagatatt attactatag acattttggt 3120

gtattccatc tgttcttttt taatgctttt ataacactat gtagttttgt attttaaaaa 3180

acttaaagca aaaatttcta cgtattatta gacatactgt gatttattta actaatcatt 3240

tttttggggt gttaggttgt ttttaatttt ttactgccat caaacatctt gaacatagga 3300

tgtagatttt agtctttaaa atatgttggg gaatgaacaa atttcacatc ctgtatttgt 3360

agtattaata ctttgtaggt gctcaaaata gaatattctg gtaaatgatt agtgcttatt 3420

aaatatttat caaatgaatg tacttgtact tttggcatta aacattaaca tctgaccatt 3480

tatatttacc tgattttttt tctatggcca tatggtatga aatagtgtat ggtataaatt 3540

aaccatatgg tataataaat acattttttt aagtgtgata ccagagtgat atttattaac 3600

tgttcttcct gtgctgtttc tgtagaaggg agcttctcac aattgcatta gaattacaat 3660

tttattatgt tctgttttca agatctctga tcgtcagtct taaactgttt aattataata 3720

atgtattgac tagggaatat tctgggatat aatctccttt ataatgaggt ccactgtatt 3780

aaaatacatc tttgcaagcc acaccaggtt ggattgcatc ataaccctga aaagtggtat 3840

tctcattaat gcaggtgctt gtgcagtttt ggctattgct gttaatactt atacagatat 3900

attcacaggt gcccttgtgg caaaaatcat aaaatagttg tttgtctttg gtatttctag 3960

tgttcacttt ctatattctt ttctctctcc ttatttactg aactcccttc tttaggcatc 4020

cactcactcc tttttctgtt tagaatatta tctgtcagtc attttatatg ttggccatta 4080

aaggaataaa ctgtcagtaa acagctaaga aaggaatgtt ggactgggtg cttgaatcct 4140

tgaatgtagt aaatgtgagt gcaaacttga tttaattgta catgtatttg gataataggc 4200

cagaaaaatt acattagggt aacaggctag aacagtctga cttttcttgt ttttctatcc 4260

cttgctttct tgattagaat gaataggagg tgggtctgga tatagcagct ggaaacctgt 4320

gttccatgag tgatggggaa gagagggagg gaataggttc ctctgatttt tggcattttc 4380

taagacctga tgcccacctt gtcagagaat gcgatgacta cttttgtgtt cttccttttc 4440

cctttttctc ccaattataa aattgttttc tctttcagaa ctgcagaagt gcattttgtt 4500

tctttgacac tttgatgttg ttaatttagc tgaataccta gtgaacattt tgtgtcataa 4560

tccccttgtt ttatgaaatc cagtatggtc tagtcacctt acatttctgc ctcatattgt 4620

ccttaagcct ttttttgtca gtagctctta ctagattttg tcttcatcag aagttaaagt 4680

gttttaagtc ctttactcat tctgtttctc tattttaact tacattggtt attctgtaaa 4740

gtcagatgtg gcagtagggc tggtcgtggt ggctcacacc tgtagtccca gctacttggg 4800

aagctgagat gggagcatca cttgagccct gaagttcgag gctgcagtga gccatgatcg 4860

caccactgca ctctagcctg gcaacagagt gagaccctgc ctcaaaacaa acaaacaaac 4920

aaacaaaaaa aaaacaaaaa aacttgcttg tagaacttct gaattcaaaa taggtgggcc 4980

tatttgggag cttttctgtt tttaaggtgt caagtactgc tttttaaaat cataaggtta 5040

tggataactt catgttagtg taagaagaaa aatatagcct catttgttcc atttctttct 5100

taaatttttt gttttcattg ccatgttttt atttttcgat ttcaattttt ccagcctaaa 5160

tcactaacat acttaattag catggtaatc agaagatact ctttaataca gtctccaccc 5220

taacattaag caattatttt tcccccctac cctctgagat tatttttgtg tccatgtttt 5280

ctcttgggct taaaaaaaaa aactattatt ctaattcctt cctgtatcaa gactatgcat 5340

atagagggaa ctcaatgccc agtaacttct ttttctgggc cctggtgatg tagaatataa 5400

aaattgcttt gaactcaatt aactttatat cttctggaag ctctgtaaca tcggataaag 5460

cgtcgttttc attcttgtaa tgtagctgca gttcctgaca gcacgtttgg gacaaatgta 5520

ctgtgggacg gtggttttca aagtacgcca gagctctagg agaatttttc gaaaacattc 5580

tatcattgta aataataatt tttttttttt ttttttttga gacggagtct cgctctgtct 5640

cgcagactgg agtgcagtgg cgcgatctca gctcactgca agctctgcct cccgggttca 5700

cgcctttctg cctttctcct gcctcagcct ccccagtagc tgggactaca ggcgcctgcc 5760

accacgccca gctaattttt tgtattttta gtagagacgg ggtttcaccg tgttggccag 5820

gatggtctca atctcctgac ctcgtgatct gcccgcctca gcctcccaaa gtgctgggat 5880

tacaggcgtg agccaccaca cctggccaga ataaaaattc caaattgcac taatgcatat 5940

gtgaaactgt ttttgtctgt tttgtagttt taaaatcttc atctataata gtacctggca 6000

cataggtact aaaatatttg gtgaaagaat tagtgaataa aaccttactg gatatgaggt 6060

gatctgattt tctgtaacat tctattcttt ttttctgccg gtcacaaaat caccttatga 6120

aattgacagc caaattggct gcaacaggca gtttgaaaaa cactgttttg gggtttcaag 6180

gaccttcttc agaggttacc ccagggctct gtttagtgcc tctataccag gggcccccaa 6240

cccctgggcc acagcctcgt atctaccagt ccgtgacctg ttaggaacct gactacaccg 6300

caggaggtga gtggcaggca agtgagcatt accgccaaag ctccacctcc tgtcagatca 6360

gcggcaggag cgtgaaccct atcagaaact gtgcatgtga gggatctaga ttgtgtgctt 6420

cttgtgagaa tctaatgcct gatgatctga ggtggaacaa tttcatcctg aaaccccctc 6480

tacccctgtc catggaaaaa ttgtcttcca tgaaattggt cactggtccc aaaaaggttg 6540

gggagtgctg ccctatacca taactattga agttcttcat ttatctgctt tacatgttag 6600

tttccttgaa aaaagggttt tatggctgta aaaattttag agctactatg tatggaagag 6660

aggtttgtgc tggcataaat cttctcaggt atcatctgtg tagaaaattt caacaacttg 6720

tgtcttaagg cagaggtcag caatcttaag ggtcagatgg caaatatttt aggcattgtg 6780

ggacatacag ttcctctcac aattcctcaa caatactgta gtagctcaga agtagctaat 6840

agacaatatt taaacaaatg agtttgactc tgttccagta gtcattttca ggacactgaa 6900

atttgaattt catatcattt tcatgtgtca tgaaaatctt cttttgattt tttttctacc 6960

acttaaacat gtaaaaagta ttcttagctt gtgagctata caaaagcaga tagtgggcca 7020

gttcatgggc tgaactgttc tgactcctgg tctaagggag actatatatg ttatatttga 7080

ggttctcaaa gtaagatgta ggttagaggt ctatagaaag ttcataattg cttttgtaaa 7140

aactagattg atttatttga gagggagaga gaggtggagt ttcactgtat tgcccaggct 7200

ggacttgaac tctgggctca agcaatcttc cagcctcagc ctcccaagta gctaggacta 7260

caggcatgca ccaagcccag ctcctagttc ctctttgcac tcagtttcat cttctaccct 7320

cagcccctgg caaacactgg tctgatctct gtgccctttc cagaaagtca tataaatgga 7380

gtcatataaa agtcatatca gtggggccgg gcatggtggc tcacacctgt aatttcagca 7440

ctttgggagg ctgaggaggg cagatcacct gaggtcagga gtttgagact ggcctggcca 7500

acatggtgaa accccctctc tactaaaaat acaaaaatta gccgagcatg gtggtgggca 7560

cctgcaatcc cagctacctg ggaggctgag gcaggagaaa cacttgaacc cgggagaggg 7620

aggttgtagt gagccgagat cgcgtcattg cactccagca attgtgaagc agtggttaag 7680

gttcattcat tattttacat atggatgtcc agttgtttca catttatcaa atttcttttg 7740

aatcaccttg gcacttttat tgaaatcaat tgcttatgtt tgtgagtttt ggattctact 7800

gttttattga tctgtgtgtg ttttcttagg ctaatacccc actaccttag ttatgatagc 7860

tttatagtta aatttaaaat caggtaggtc cagttgagaa gatttttaga actttgactt 7920

tttaatttct tgttaccata cttttggaag gagaagactc ataattttat tcaggttttc 7980

aaaggtgtct tctaccccca aatacttaag acttcagtat atagttaata tatctctgtc 8040

ttgaattatg tggtggtgag gttcatgaaa agtgacaggt aaaagttgca accaaaatta 8100

atttccttat aagaaaggac atattttagt aggttgcaca gaaaagtcaa tcacgtcctt 8160

tttaccgtgt tgcattatga ggtgtaatca aaatgtgtat aggggaaaat gaatatgtta 8220

agatgtttat tcaccaaaaa aagtcaccga aagtgtcaat taggttgtga aagatactag 8280

tttacaatgt gttattctcc atcttcatta gaagagttct tccatttgaa acctatgtca 8340

tgttctgtga tatttcagag ttagtatctt tatgaatctt aggggcatca ttaaatcatt 8400

atgtttcttc taaggaaagg catatggtag tagttggtca tatttctacc tttttcagtg 8460

gggcctatag ggccccttta ctttgtttta aactgcactg atagcttttt actggttagc 8520

ccagggaatt atccctacag tgtaaaatga tgtttctcat catgattttc atttaataat 8580

ttaaaaacaa aataacagga aacacatgtt tctagagagg aagtgtggaa tacctgacta 8640

gtagagggtc aggtgacaat tgcgaaggta gaaactgact aagttttgac catgttgcaa 8700

ggagtatcgt tccagttttg ttgggtcacc ttgcagagtt tttaaaaaaa atccttgggt 8760

tttttttgtt tgtttgtttg tttgttttgg tagagatgct gtgttgctca ggctagtctc 8820

gaaatcctgg gctcaagcag tccttcccag agtgctggga ttacaggcat gagccaccgc 8880

acccagcatc accttgccga ttttgaaact acacttccag ggagagagta atacaatatc 8940

ttggcagact atttttaact attgttccaa aattaatctg tttttaaaaa gtaaagaagt 9000

gtggatatct ttaaaagata ctttaattag gctggtcacg gtggctcatg cctgtaatcc 9060

cagcactttg ggaggccagg atgggtggat cacctgaggt caggagttcg agaccaacct 9120

gaccaacatg gagacactcc gtctctacta aaaatgcaaa aaattagccg ggcatggtgg 9180

cgcatgtctg taatcccagc tacttgggag gctgaggcag gagaattgct tgaacctggg 9240

aggcggaggt tgcagtgagc cgatattgcg ccattgcact ccagcctggg caacaagagt 9300

gagggaaact ccgtgtcaaa aaaaaaaaaa aagatacttt aattatattt aagttgggga 9360

atacttttgt tttatatttt ttcttgcact aaatatgtaa cctttaagtt aataacacca 9420

gaaaattttt actttatagt tttagaactg cttaattgaa ataaaatgtt aggatacttt 9480

gaattagtca cttattttgg catattttaa acagtttaat gtactgcttg gggttgtttc 9540

tcaattgact tgatattttt aaactgtatt ttttaccttg taccatttat tttgcaaaat 9600

aaatttctat tttggcttat agatgtattt ttaatattaa aggggattat tggtgtcagc 9660

cataaaattg taattaagtc ttaagtaggt atggtttttt tttttttttt tttttggaga 9720

cggagtctcg ctctatcatc catgctggag tgcagtggtg tgatctcggc tcattgcaac 9780

ctccgccccc cccccccccc gggttcaagt gattctcctg cctcagcctc cagagtagct 9840

ggcactacag gcgcatgcca ccatgcccgg ctaatttttt tgtatttttg gtagagatgg 9900

gatttcacca tgttagctag gatggtctcg atctcctgac ctcatgatcc gtccgcctca 9960

gcctcccaaa gtgctgggat tacaggcatg agccaccgtg cctggctgat tatttttata 10020

gagctcttgt tagcgtaatt tctggtaatg ttttatggag gtgacttaat tcccatcata 10080

aaaatatccc atcttttctg tgactagcaa gcagttattg cctttataac ttttttttac 10140

cataaaaaga taaagtacta ttgataatta ctcctaatta actcagaact tttttgtttt 10200

acacacatta atatatactt ccatgggaat agtgtcagag aacatcaaat agggaagaga 10260

ttatgattca gagtggtctt tatattccta ttctagagcc acagaaaatg ttcatctccc 10320

tttagttttt gcaggattgc ctctaacact gatgatattc cactcatatt cttcctgcac 10380

atgccttctc atactaacag taagtcacac aatctcaaat aagtttcatt atacatgaga 10440

actcagttga atgcttgcct ttcactggca tctcttgatc ctcctccccc tccttatggt 10500

atgcactgaa cttctagtag gccatatgtg ttaagtagat cataggagtg ctatgaaaat 10560

aaagtgaaat gatgaatatg taaaagcctt cacaaaatta tagtagtgtt ttctgttgat 10620

ttttaagaca aaagataaat actatgtatg taaaaatttc ctttcagaaa tctttgttct 10680

tttttttgtt ttgaatgttc aatatgctta gcacagggct ctagttaaca cttttggcag 10740

ttcttaatgt gggactgact gatgattgtc ctagaactgt ttcagtgtta actacattct 10800

attaatgtta ctttaaaaca ttatttaatt aaagaaacat gaagtggcaa catactgatt 10860

catgtgttca gtaagcaatt catgggaaag aggtaagctt tcttaataac agtagaaaga 10920

ctgttccatt tataggaaaa ctggtaatta tgacttgtgt tttggtattt aaaagctgtg 10980

gttggccggg cgcagtggct cacgcctgta atcccagcac tttgaaaggc cctggcgggt 11040

ggatcatgag gtcaggagat tgagaccatc ctggccaaca tggtgaaacc ccgtctctac 11100

taaatataca aaaaattagc tgggcctggt ggcatgtgcc tgtaatccca gctactctgg 11160

aggctgaggc aagagaatcg cttcaatcag ggagtcggag gttgcagtga gccaagatcg 11220

cgccactgca ctccagcctg gctacagagc gagactctgt ctcacaaaaa aaaaaaaaaa 11280

aaaaaaaaaa aaaaagctgt gattaacatt tgctttgtca ttcatccaaa actacattgg 11340

tgacttttgt attgagtcat ttcttagggc aacaggtatt catgtattca gtaaatattt 11400

gagtgcctac tatatgccag gtagtgatct aggtgcttag tagtacactt gaaaacaaaa 11460

caaaggtctc tacccttatg tagctgctgt ccagtggagg ggtgtgtgtg tattggggga 11520

tggggctgag aaaccttaga catacagaaa ggaaattatg tagtatgttc aaaggtaata 11580

agtgctgtgg agcaatgaaa gttaaacagt ttagggctgg gatgggggta ggtagcaatt 11640

taaataggga ggtcagggta ggcctcactt gagaaggggg tatttgaaca aaaatttgag 11700

aaaggaggag gaggcatttc agataaacca attagttcaa agattctgtg tcgggaatgt 11760

gccttgccta tttaagaaac agcaggaggc caaagttgct ggggcaaggt agagactagg 11820

ggattaggga aggatctctt tcagttatct aggccatatt ggtgatagca gaaatactga 11880

gaagtagtca gattttggat gttttgaaag tagattcatc ggggcttggt ggctcacgcc 11940

tgtaatccca gcactttggg aggccgaggc gggcagatca cccgaggtga ggagttcgag 12000

accagcctga ccaacatggt gaaaccctgt ctctactaaa aatacaaaaa ttaggtgaaa 12060

tattgaagga gatgttttga ttgaagtgat tttaagagag aagaggaggg gaagtaaaga 12120

tggtgaggaa ttatcctgta aaggggaaca gagaaatggg gccagagcta gtgaggaaag 12180

tggggtcaat aaatttttaa tgataagaaa aagaagagcg tatgatgata ggaatgagcc 12240

attagagagt aaaacgtttc aggagggaga gagaagaatt gctgaagcac tgtcttagaa 12300

gaggtaagag ggaaagggat ttagtgtata aataggaagg attggctttc tataggagca 12360

tctatacttt atgataatag gccattaagc agagtatgtg gttagaaatg ctgctaggaa 12420

ggtcgacgtg attggtggag tctgtacacg ttctgttgca gttgctttgg tttttttcag 12480

tgaagtaaga cttgaggtta tcagccgaga atgaggattg gggatatgtg aaagtagctc 12540

cgcagcagta tgggagcata aatgaactgg agacaaatag taaaattaat gggcaatttt 12600

ttttccttta agagatgggg gtctcactct gctacccagg ctggggtgca gtggcacagt 12660

catagctcac tgcagcctcc aactcctggg ctcaagtgat tttctcacct cagcttcccg 12720

agtagccagg actataggct aatggcaata ttaagttatt ttatgagttg tctagacagc 12780

attatgagtc tcctaacttt ttggtactga tcttcagatc agagttaaat gtaacttgcc 12840

caggcaattt aaacactcaa tatgagtcat tttcatttgg actcaaacat ggaatcattg 12900

ggaaatagaa catgaattta ttactcctta atgaagtacc tgccactatc ctgccatgaa 12960

tgtaggctaa atttggagtg gtctggtaac tgcttttctt ttaaaaaaat tttcttcatc 13020

ttttctgtat caaatactta ctggtttttc tatgtagaat aacataatct catcattact 13080

ttctttcaaa cactctccaa atttgacttg tctttgctca tgtttttcct accacctgaa 13140

atacagattt cctcccccat cccaccctcc aaaccttcca gacttacctc acctaccgtt 13200

tattgtagga agcttttctt aacctctttc caagtcctag tttgatgcct ctgctttgtg 13260

cttttgtaga atcccagagt ttaccttgtt ttactcacta tatcgtattg tggggttttt 13320

ttgttaatag gtatttcttt ttcctctaga cctgcactgt tcagtcatac tttctgtgat 13380

gatggaaatg ttcagtgtcg tcttaatatg gtagacagta gccgtatcta tgtggctact 13440

gtgcacttca aatttgcata gcaggactga ggaactaaat tttatttcat tttaattaat 13500

ttaaaaataa ctagcctgat ggctagtggc tgctttaatc agtgcagttc tagacccctt 13560

caaagtcaag agtgtgtggg attcatactt attctctcgt tgcttaacaa taatactgtc 13620

taatacttaa atgactgaat tctttttcgg tactcttagt ctgcatagtt ataactcacc 13680

tagaatatgg cagttttgtt ctctagattt ctgtctttaa ccatggcttt tcagtttgtt 13740

ttcaagatta tgttgattta cacagcactg agttcttcag tcctgcgaag ttagcgtttt 13800

ggttggatgt ggtggctggc atctgtaatc ccagcaatat gggaggctga ggcgggacga 13860

ctgcttgaag ccaggacttt gagatcagcc tgggcaacat agcgagcccc tgtctctaca 13920

aaaaattagc tgagtgtggt ggcatccaca tgtagtccca gctacttggg aggctgaggg 13980

cgggaggatt gcttgaaccc agttcacggc tgcagtgagc tatgatcaca ccactgcact 14040

cctgcctggg tgacagagca ggagtcaccc tctcaaaaaa aaagtgtatt ttttcccctt 14100

taggactgaa aaaattgggt gttacaagat tacctcaagg actggtctga gaactgggga 14160

tggtaaggaa gaaactcaag tggccagcct ctggtttgtg ggggtaggtg ggcaatttct 14220

gtttcaacca aagcagttct acttcataaa ttaatatatt ggaattgtgc ttgggatttc 14280

atttggaggg gaaaaaagtc ttctaaacaa taacactgtt aattgaagag acaaagcatg 14340

catatggcag cacgtgatta accaccaaag tggataacag atcaagaaga catgggaagt 14400

tgttatgggc tagtgaggtc ttgatggaag ttaaggttta atttaggtag gtagaaggaa 14460

gacaaaagga tgtaataggc agtgggaata gaatttgcaa agaattggag ttggaaatac 14520

atatgttagt tttggttaag aaacaatgag ctgattatag ttgagcaaat tgtatgaaat 14580

aaaatttgac aaaattagat gggactggat tgtaggaaaa agtgataggc tgacaaaaag 14640

tgcactttat attgcatgca atgagatgaa atactcggag gtattttggt tgttacaaag 14700

atggaggaag aggacactat tagcatttaa tgagaaagga tcagggaagt cagccatccc 14760

acaatgttca ggacagtttg cacatttaag aattgttcca aatcaccaca tgacctagaa 14820

tgacttgctg gactttgatg atgtaggtta aaaaaaaaaa gtgatcataa tgatgtgagc 14880

atagacagta actccatttt gtgtatgagg cacttttttg ttgttgttgt tgttgttgtt 14940

gttgttgttg ttgttttgag acggagtttc gctcttgttg cccaggctga aatgcagtgg 15000

caccatctag gctcactgta actgaggcac ctttaatata cactaagatt ttcaggaatt 15060

cagttaccac aatattggag gaatgttaaa gtttttgttc agaactttat caggagtact 15120

ccaccccttt agaaaatcac gtaattgatg gcagtgtcac ttgtgctgta ttggtgacgc 15180

aaatactctt cagtctgcag ttgtaactgc cacagtcaca gtgcttgttg tatatatgta 15240

caaagtacca tcagtgtgtt atttattata gtaatagcct aaatattata aaattgcact 15300

aaggtagatt atctctgaat tacatttatt ttttattttt atttttgtga cggagttttg 15360

ctcttattgc ccagggtgga gtacaatggc gtgctcacca aaaccttcgc ctcccgggtt 15420

caagcgattc tcctgcctca gcctcccaag tagctgggat tacaggcatg tgccaccatg 15480

cccggctaat tttgtatttt tattagagac ggggtttttc catgttggtc aggcttgtct 15540

tgaactcccg acctcaggtg atctgcccgc ctcggcctcc caaagtgctg ggattacagg 15600

catgaaccac tgtgcccagc ctctgaatta catttaaggg tagtatagag aatgtacaaa 15660

atagttgttg taaaaaggag aaattgattt ctgttttcag tattggaaga ctgggttatt 15720

cagatgaaaa caattaaaat tcttggaaac agtattaaaa aaaaaaaaaa aaaaagaaaa 15780

cgtagagcag ttgcagagct gaaaagatag tggggagctg ccaggccaaa ttctaggaat 15840

aaacaagaat ccaaataaat aagtggatga agtagctttt gccctaaagg cagttgccaa 15900

tctgtacaag ttgggctttg gttttggtgg accattgggg tgaagaggac agaaatcaag 15960

tcctagagtt catctaaagt gacagtcaaa aagaataacc tcagttttag atgggacccc 16020

agtgactcta tcgttaggtt aagggtgaaa caactgaact gtctacacac tcccatatcc 16080

atgtgattgc agggaaggtt gaatggagca ggaggaggaa aaggaaatta agaaaaataa 16140

acctttcaag ttgtgaccac agttttagcc ttcacagtga tttgccttga gggtttgctc 16200

tgtctgggtg gtccagggac cctccatgat aaatgaccct ccagccattt atcatggtta 16260

ctggtttcag actagtatta cttccatggc ctggcagatg gaaaggcaaa tcacgtgtgg 16320

agaaaggcct ttcattctag ggaattgctt ccagtaattt ttcaaggaca atgaacagta 16380

cattgtcaca agtaatcaaa catagtaaaa ataaaaaaat tagcaagcaa aattacctga 16440

gagatatata atatgtaata tatatgtaga attctttcag caatgtaatt aaaatttgtg 16500

tgggtttctt tatgtatata tacctaaaat tacgttttta cagaggaaaa ataataacca 16560

agcatgcatc caattggcac tgtgaataag aagagagcag aaacagatct ataaaatctt 16620

caaatgttgc aaatatcaga tggatttaaa acaatcactg ttaaatttaa agaattggag 16680

acaaacttga agaacaaaag actcttataa agtgacttag ggccaggcac ggtggctcac 16740

gcctgtaatc ccagcacttt gggaggccaa ggcaggcgga tcacctgagg tcaggagttc 16800

aagaccagcc tggccaacat ggtgaaactt tgtctctact aaaaatacaa agattagcca 16860

cgtgtggtga ggtgcacctg taatcccagc tgctcaggag gctgaggtgg gagaatcact 16920

tgaacctggg aggtggagat tgtagtgagc cgagatggca ccactgcact ccagcctggc 16980

ctataagagt gaaactgtgt ctcaaaaaaa aaaaaaagtt actttggttc atgcctgtaa 17040

tcccaatact ttgggagact gaggtgggag ggtcacttga ggccaggaat ttgggacccg 17100

cttgagccca ggagtttgag gctgcagtga tgtatgattg tgccacactg cactccagcc 17160

taggcaacag aatgagaaac tgtctcaaat aaaggaaaaa aaatggctgg gcacggtggc 17220

ttatgcctgt aatcccagca ctttgggagg ctgaggccga cagatcacga ggtcaggaga 17280

ttgagaccat cctggctaac acagtgaaac cccacctcta ctaaaagtac aaaaaattag 17340

ctgggcatgg tggcaggcac ctgtagtccc agctattcgg gaggctgagg caggagaatg 17400

gcgtgaaact gggaggtgga gcttgcagtg agctgagatc gcaccactgc actccagcct 17460

gggtggcagt gcaagactct gtctcaaaaa agaaaagggg gggaaaaacc caacttaata 17520

gatttgcaaa aaaccaaata gaaattccag aagtgaacac tttaccaaat atacctaaga 17580

gattatgcct agctgaagaa agagttcatt gcctgggaga caaggcagaa gaaactgttt 17640
agagtgtagc acagaataaa aaagaaaata ttgaagagag gtaaagagac atggaagaca 17700
gaataagatc taatttcttt aatcagagct ctggaaagag aggagaaaga atggtacaga 17760
agtaatattt caaaagatat ttctggctga aaattttata gatccaatga gaaaccagtt 17820
gattgattta agaaggttaa tgaatttcta gcaatataaa tagaaatcta cacccagaca 17880
aatcatagga aaactgcata aacccagata caaggagaaa agtcttgaaa gtagccagag 17940
agaaaaaaag atgtttttca aagaagcaac tatggactga tggttgactt ttcaatagaa 18000
aattacatat attctcaaaa taactgccaa tctagaattc tgtaattagc aaagaattat 18060
ccctctacaa tgagggtaaa atacttagtt gaacaaactc catcagctct ttctaaagga 18120
aattatgaag tatacattaa tacttaaggc agaaagattc tagataaaag tctgaggtgc 18180
aaaatggaat aaagagcaaa gagagtggca aatatgtgga tgtattaaaa gaaacgttga 18240
ctgtataaag tactagtaag accttaatta aaatatgtga caagaagctg ggcatggtac 18300
tttgagaggc tgaggcgggc agattgcctg agcccaggaa tttgagacca gcctgggcaa 18360
catagtgaaa tcccgtctct acaaataata taaaaattag ctgggtgtgg tggtgcatgc 18420
ccatagcccc agctactcag gtggctgagg tgagaggatc gcttgagccc aggaggttga 18480
ggctgcagtg atccttggtc gtgccactgc acaccagcct gggcaacaga ctgagaccct 18540
gtctcaaaaa aaatatatga caggcgaagg ccgggttcta agacctttgt attgtcagag 18600
agaaaggtag aaagtattaa ttgacttgac cttgataaat tatatgtttt aatttctttt 18660
tttttttttt tttttttgag acggagtctc gctctgttgc ccaggctgga gtgcagtggc 18720
gggatctcgg ctcactgcaa gctccgcctc ccgggttcac gccattctcc tgcctcagcc 18780
tcccaagtag ctgggactac aggcgcccgc cactacgccc ggctaatttt ttgtattttt 18840
agtagagacg gggtttcacc gttttagccg ggatggtctc gatctcctga cctcgtgatc 18900
cgcccgcctc ggcctcccaa agtgctggga ttacaggcgt gagccaccgc gcccggccta 18960
tgttttaatt tctaagttat cttctaaaaa tgtagaaacc agacttttaa cttctcaacc 19020
aacagaagat aacaaatgat taataaaaat taatcctgaa gaagtgaaga aaagaaagaa 19080
ccagtaggac aagtagcaca aagatgggta gatttaaatc taaacatatc accagctaca 19140
ttaaatacaa aatggattaa attattcagt taaaagccaa agattgttac actgaatttc 19200
caaaaaaatt cagttatatg gggtttataa ggaacatatc tgaaacctaa gaataaagaa 19260
gatcaaaagt aatcatcaca ataagacata ccatgcatat tctagcagac agtatggtac 19320
agttaatatc aaaggtggac agtaaggcag aaagcattat tggcagaaga gtcacctcaa 19380
atgataaaat gaccaattca ctgtgaagat ttaatagcct tagtaatata gtataacctg 19440
aaatatagct ttagaatatt tatagcaaaa gttaaacaaa actacaagaa atagacagat 19500

ttctcagtct taatggggta tttttaaaca gctctttaag taactggtat aagaagcaga 19560

caggttagtt aggatataaa atatttgtat aacacaatga acaagtttaa cccagtgggt 19620

gtatagaacc cattctaccc aacagtggca ggctacacat tcttttcaag catgtaggat 19680

tttgggggga aaattgactg agtaataatg ttgtaaaaca agtttcaaca aatttcaaag 19740

gattgaaacc aaaaaagcat ttttttctgt ccattttcat taaagatctc tatcaatagg 19800

gtaattttta aagcttcatg ttagaaattg agcaactatt aatacttgga aataatctgg 19860

tcaggcgcag tggctcacac ctgtaatccc agcactttgg gaggccgagg caggcggatc 19920

acgagggctg gagttcgaga ccagcctggc caacatggtg aaaccccgtc tctactaaaa 19980

atataaaaat tagctgggcg tggtggtgca tgcctgtagt cccagctact tgggaggctg 20040

agacagaaga attgcttgaa cccaggaggc agaggttgca gtgagccgag atcgtgccat 20100

tgcactccag tctgagagag cgagactccc tctaaaaaaa ataataatcc atgcatcaaa 20160

gaagaaatca caatggaagt tagaaaatac cttgaactag atgattaaaa atttttgatt 20220

gatcaaattc cacaacttga tatatcttag accattgaaa gtgagagaat caaatgttat 20280

gtctttagat acgaagtttc ttgacacccc tccctgcctc ccccaaaaaa gaaataccta 20340

ccacctatga aagattcttg attggaaaaa aaaaaaataa ctgagctgga gtttcattaa 20400

acttctagat ctactgggaa attcacagtc atttgatctt tcaatgagtc ataattgttt 20460

tgctgtggat ggtcttgcct cgatgttgat ggctgctggt tcatccctgg tggttactga 20520

aggataggat ggctatgcca gtttcttaag acaacagtga ggtttgcgac atcagttgac 20580

ttcttttcac aaaagatttc tctgtaccag gcgatgcttt ctggtagcat ttgaaccaca 20640

gtagaactgc ttttaaaatt agagtcagtc ctctcagatc tgccagtgct tcatcaacta 20700

attctatgta atattctaaa tcctttgctg tcattttaac agtgtttgct cacagcatct 20760

tcagcaagag tagagttcat gtcaagaaac cacttttttt actcatccat aagaagtaac 20820

ttcccatttg ttcaaatttc atcatgatat tgtagcaatt cagtcacatc tttaggttcc 20880

actcctaaat gtagttcttc tgctgcttct actacaccta cagttccttc ctccattgaa 20940

gttttgaact caaagtcatc catgagggtt agaatcaact tcttccaaac tcctgttaat 21000

gttgccagtt ttacctcctc ccatgaatta caaatgttct ttatggcatc gagaaaggtg 21060

aattctttgc agaaggcttt caattaatgt tgcccagatc catcagagta ataacgatat 21120

ctatggcggc tatagcccaa tgaaaggtac ttcttaaata aggaatgttg aaagtcacaa 21180

ttacttcttg atccatgggt tgcaagattt atgtgttagc aggtgagaaa acgttcatct 21240

tcttgtagct ctccatcaga gctcttgggt gaccaggcac attgtcaatg agtagtaatg 21300

tgttagaagg aatctttttt tctgagcggt gggtctcaac agtgggctta aaatattcag 21360

caaactatgt tgtaaaagaa tatgctgtca tccaggcttc attgttccat ttacagagca 21420

cagacagtaa attttgcaga attccaaagg ccctaggatt tttggaatga taaatgagca 21480

ttggcttcaa cttaaagtca ccagctgcat tagcctctaa taagagtcat cgtgtcctag 21540

gccaggcgtg gtggctcatg cctgtaatcc cagcactttg ggaggccgag gtgggcggat 21600

cacctgaggc cgggagttca agaccagcct ggccaacatg gagaaaccct gtctctacta 21660

aaaaaataca aaattagcca ggcatggtgg cacatgcctg taatcccagg tactcaggag 21720

gctgaggcag gagaatcgct tgaacccggg aggcagaggt tgcggtgagc cgagatcatg 21780

ccattgtact ccagcctagg caaaaagagc gaaactccat ctcaaaaaaa aaaaaaaaaa 21840

aaaagtcatc ctgtcctttg aagctttgaa gccaaccatt gacttaggga aatgttgtgg 21900

ctggtttgat cttctatcca gatcactaaa actttcttta tatcagcaac taggttgttt 21960

tgcttgcttg cttttcttaa actatttttc ttttaattct tagagggtct cactgtgttg 22020

gctaggctgg tctcaaactc ctggcctcaa gcaatcctcc caccttggcc tctgaaaatg 22080

ctgggattat aggcatgagc cgtagtgccc ggcctgtttt gctttcttat cattcatgta 22140

ttcactggag tagtgcttgt agtttccttc aaaaactctg cctttgcatt tacaatctgg 22200

ctgtttggta caagaggcct gcctttcaac atgccttctt cactaagctt aatcatttct 22260

agcttttgat ttcaagagag agacatgtga ctcttggttt cacttgaaca cttagaagtc 22320

attgtagggt ttttaattgg cctaatttca atattgaaat ctcagggaat aggaggccca 22380

agaaggggtt agggcagtag ctggtcagtg gagcagtgag aacacacaca acatttactg 22440

aagaagttca ctgccttata tgggtgtggt tcgtggcacc ccaaaagaat tacaatagta 22500

acatcgaaga tcatagaaca ctaaaataga cagcataata atgcagaagt ttgaaatact 22560

atgagaatta ctgaaatgtg acagagacat gaagtgagca tatgctgttg gaaaaatggt 22620

gccaacagat ttgctcgatg ctgggttgcc accaaccttc agtttgtaaa aaacaaacaa 22680

aaaccattat ccgctaagag cagtaaagca aagcacaata tgatgaagca tgcctgtaca 22740

gtttatactt cacagtcatt ggttaaattt actcattcct ttaagcataa aaaataaata 22800

tttagcacct ttgtgctgag ttgtatgcta ggcactggga tatagaaatg aatgatagtt 22860

tgtcttcaag gagcttacag ttaagtggta gaagtagata aataaagaat taaggacagt 22920

gtactgtgat gaaagtgtgc cccagatgca gaagaatcac agaaaaggag cacaattcaa 22980

tatgctttta ggggatggaa gacagatttc ctaggaaagg agatacttaa actgaatttt 23040

aaatacttca gatatacaaa atctttataa agaagagtaa ttaaacctta tttagagaca 23100

gaccacctaa ataaatggta cataccctgt actgagtgtg gattggaaga ctgagtatgt 23160

taaaaatgtt agtatttctc aaattgatag tggatttatt gaaatccaaa tgcaaattcc 23220

tgtaggtttt tctatggaac ctaacaacct gattcaacaa tttctgtgga agctttaaag 23280

aggcaagaat agccaaaata tactttaaaa acaataaggt aggaagactt ctttaattcc 23340

tttttccact cggcaaattt ttaatgctct atggtaggca ctgctcatcc tggttattac 23400

ctttgtgaat gtgagatctg gctcccaccc aaagctcaca ttcagatcca cttgtagtgg 23460

tatctagggt tttgaataga acacttcctg aaatacaggt taagtactgg gactaccaac 23520

aaacaaactt acaacactaa ttttattatc tcatatttgt atttacaatt tttcttgatc 23580

atattttttc atacaaactt taattagttt ctattaattt cttttaacat aggaagctta 23640

gaaatagaca cactttgctt ataatagatc tttaatatta tgtcagttgg actatggctt 23700

tgttagaaaa ggagaggaga atctactgct gagtgctgtc caaagagcag ttacccaact 23760

gagcagagag ggaggatgac cttgagggtc tcacaggcat gggtttttag gcccagcctg 23820

tggagcgtct gagggactta taaaaggaaa agggacccag gtgcagtggc tcatggctgt 23880

aatctcagca ctttgggagg ccaaggcggt tggatcactt gaggccagga gttcgagacc 23940

agcctggcca acatggtgaa accccgtctc tactaaaaat accaaaatta gctggctgtg 24000

atggtgcacg cctgtaatcc caggtactcg ggaggttgag gcccaagaat cacttgaatc 24060

cgggaggtga aggttgtggt gagctgagat tgtgccattg cattctagcc tgggcaacag 24120

agcaagactc agtcttaaaa acaaacaaac aagcaaacag aaaacaacag aaagaggaga 24180

tgaataacaa aatggcaggt aatactaaat aatatggatg aattttatat tcttacatat 24240

gggtaggtgg agggacacct gctcttgtgg cagattagtt gggaaaggat acacttgctg 24300

gaacaatgcc taccatatta aataaatgaa gctagacttc cccatctgtt atgcataaaa 24360

atcaatttct tgtaggttaa agattcaata caaaagggga aaccctgaaa gcttttagaa 24420

aaataaaggg acattattta aggcagagaa gaatttctta agactcaaaa aactagccat 24480

aaaaaatgga caaatttgac tacattgaaa atagaaactt ttttcattaa gccccattaa 24540

gacactcaaa aggcaaggca aggaatagaa gatacttata acacacacta ttagttatct 24600

attgttgtgt aattaccgaa gacttagcag tttgaaataa catttctctc acagttctgt 24660

gggtcaggaa actgagagtg gcttaattgg atgtttctgg cttagggcct tgaggctgta 24720

atcagtcaat ggctgcagtc atttgaaggc ttgactgctt tgtgctccct catgtggatg 24780

tagcaggcct cagagctggc tttctagaag ggagtaagag aaaacaccca agatggaaac 24840

cacagtattt tagtcttgga agtggcaccc catcacttct gccatattct ttttgttaga 24900

agcaaggcaa taactccagc ttacattcaa ggggagtttg tacaaggcac caccaggagg 24960

gtgaggatca ttgggagcca gtttagaggc tacctactac aacatgtaaa gaatgaactg 25020

gtatgaaaaa tatacataaa aaattcctat agatttctaa gacaaagaca gaacaccaaa 25080

ttggaaaaag ggcaaaaaat cctgagcagg catttgaatt aaaaaaaaat ttaaatgatt 25140

aataaacata tgaaatgacc cttaatctag ttaacaatta gggaaataga aattaaaacc 25200

acaatgagag accatttcat atagattggc agaaatgaaa aaatctgaca atattaaatg 25260

ttggtgaggc tatgaagcag tctgaactgt cttccactga tggatgggaa tgtaaattgg 25320

ggggaaaaat cctttggaaa ataataggat atcctataac cagcaatacc actcactctt 25380

aggcgtttgc cctagactaa tggttcttga gtatgcatca aagtcatctg gagggcttgt 25440

taaacccagc tgttggggct acccttagag tttctgatta agtaggaatt gggtggagcc 25500

tgatagtttg ctttgcattt ttaacaagtt cctgccggga gtgggctgag ggtggggggg 25560

gtgggatgtt ggtgctgctt gtccaggaat catactttga gagtcagcca ttgctttaga 25620

catagagaaa tgtgtgaata tatgaagtag gaagcatata tataagattg ctcataacag 25680

tattattcat attaacccca agatagaaaa aaagttcaag tatcctcagt agtgcagtag 25740

atatcattgt gtgtttactt atggaacaaa aaactagttc aaatagctaa ggaaaaaaac 25800

cacactacaa acagctcctg cctgcatcaa tatgtatgaa tctcacaaat acattagtca 25860

taccttagaa ccaagtcaca aaagattaag tgcaacatga ttctattcgt aaacagttta 25920

aaaacaaatg cagttaactg tattgtttag gaatacaaat ataggtacta gaattataag 25980

tagagatact agaattataa gaaaaatcaa gggaataatg atgataaaag tgagtgatag 26040

tgactacctc agggaggaga gaaagtggat gtgattaagg acaaatggga gacttttaag 26100

ataattggca atgctatttc ttaccttggg ttgtggggtt tgctttacag ttatgcttta 26160

atggaacgat gttttgcaaa cttctctaca tagctttata ataaagggaa aatttatctc 26220

atttaatttt catctctgat tactagtgag gttgagtatg ttttcatatg ttagcctttg 26280

ggttttttct taaattgttt atagacatgc catttgtaga ttatgctatt tgtttttaga 26340

acttattttt aggtattctg ttatggatag tcttctttat ccatcatatg tcaaatattt 26400

ccttctagtc cttttttatt tgtattttta tttattttta tttctttttt aagaggcagg 26460

cctcccactc tattgaggcc taggctatgg atagtcttct ttatccatca tgtcaaatat 26520

ttccttctag tcctttttta tttgtatttt tatttattta tttttttaag aggcaagcct 26580

cccactcttt tgagacccag gctggagtgc agtagtgtga tcatagctca ctgcagcctc 26640

ctgggctcaa gcagtcttct agcctcagcc taagtaactg ggacaaatgc acaccactgt 26700

gcccaactaa tttgaaaaag aaaaaatttg tagagatgga gtcttgctgt gttgcccagg 26760

ctggtctcga ctcctggctt caagtgattc tcccaccttg gcctcccaaa gtgctgggat 26820

tacagacgtg agacactgtt cctgccctag cctttcctta taaagacata tgttgttgtt 26880

actgtttatg tggtcagatt tagcagtctt ttcatttatt tatttatttt gattttgatt 26940

ttgatttttt taagaaatct ttcctggctg ggcgcggtgg ctcacacctg tcatcccagc 27000

actttgggag gccaaggtga gcagatcatg aggtcaagag attgagacca tcctggcatg 27060

gatgaaacat ggtgaaaccc catctctact aaaaatacaa aaattagctg gacgtggtgg 27120

tgtgtgcctg tagtcccaga tactcgatag cctgaggcag gagaattgct tgaacccggg 27180

aggcagaggt tgcatgagct gagattgtgc cactgcactc cagcctggcg acagagcaag 27240

actctgtctc aaaaaaaaaa aaaaaaaaaa agaaaaaaga aatctttcct atccagagtt 27300

aaaagcatat tctctatatt gtcttctgat aaattgaaaa ttttaaaaat tttgtttagg 27360

tatttaagca gtctagtatt tgttatatga agatgtcttg ggggatgtta ttttcccaac 27420

accatttgta catatgtgca cagtcttttt ctcacttatt tgtaaagcaa cctctcattt 27480

ctaccagatt cccatggacc gatttctggg ctacttctta acctgtttgt ctataacctg 27540

tttgtccaca ttactacttt atatctttgg ttttggtaat gccagtctct tctctttgtt 27600

tatttgcggc aaacatatgc tcattcactc ttccacgtga ccattagaag aattagattg 27660

tcaatgtcct ctaagtctag gtatttggtt aaaaaaaaaa aaaaagaatt aggttgtcaa 27720

attctgtaaa aagtactatt gggattttca ttgtgattgc attaagttta tatgttaatg 27780

tagggacata tatattagag atttattatg atactgagtt ttccaattca tgaacatggt 27840

ttttatcttc atttgtttat aggtctcttc cccccccacc cctttttttt tttttttttt 27900

tgagatggag tctcgctctg tcacccaggc tggagcacag tggcacaatc ttggcttatt 27960

gcagtctctg cctcccatgt tcaagcagtt ctcctctcag ccttccgagt agctgggact 28020

ataggtgcac accaccatgc ccggcttatt tttgtatttt cagtagagac ggagtttcac 28080

catattggtc aggctggtct cgaactcctg acctcaggtg atccacctgc ctcggcctcc 28140

caaagtgctg ggattacaga cgtgagccac cgcgcctggg ccatgtgttt ctatttttag 28200

tagagacagg atttcaccat gttggccagg ctggtctcaa actcttgacc tcaggtgatc 28260

cacccgcctc agcctcccaa cgtgttggga ttacaggtgt gagccactac tcccagctct 28320

gttttttttt ccttaaaagg tttatagttt tctacataat gttttcatcc ttttggatgt 28380

ggaggattgg tattatttgc gagaatcttt ttgtgctgtt gtgaatgaga tgtttagaaa 28440

aaaatatatt tttttactgg atattcatat acagaagaat gaaactagac ccccacctct 28500

caccctatac aaaaatcaac tcaaaatgga tcaaagacct acctgtaaga cccaaaacta 28560

caaggtgaaa ccacagtgtg gtatcatctg gccccaggta ggatggctgc tataaaactg 28620

tagaagactt gggaaatgca aatcaaaacc acagtgaggt attgtctcat cccagttagg 28680

gtggctgtta taaaaaagac aaaaaattaa aaatgctggt gaagatacag aggaaagaga 28740

actcttggac gctcttggtg ggaatgtaaa ctagtacaga cactgtatca gtatggaggt 28800

tcctgtgatc tagcaatccc actactggcc gtttacccaa aggaagggaa gtcagtacgt 28860

cgaagagaca tttgcatccc catgtttact gcagcacact attcacagta gccaagatat 28920

ggaatcagtt caacaacaga agattgggta aagacaatgt ggttgtatag catccgatgg 28980

aatgctattc agccgtaaaa aggaaaaatc ctgtcatttg cagcaacatg attggaactg 29040

gaggacatca cgttaagtga aataagccag caaaagaaag ttaaaccctg catgttctca 29100

ctcttatgtg gaagctaaaa agagttgatt tcatagaagt aaaaagtata acagaggata 29160

ctcgggctgg gaatagtagg ggtagagtgg aagctctggg gagatttatt catggataca 29220

aaattacagc tagataggag gaataagttc tagtgttctg tagtagtata gggtgactat 29280

agttaataat atatagtttc atatagctag gaggaggata ttgaatattc ccaacacaaa 29340
gaaatgacga atgtttctcc tgtctttagt gagaatgctt ctgttatgct tggtgactta 29400
cagacttttt atagataccc tatatgaagt ttaagaatgt ttcttccatt tctagtttgc 29460
tgacagttgg gcatttttaa aaatcctaaa tggttgttga attttgttag atttttttcg 29520
tacatttatt gaggtgatca tgtttttttc ctacattgat ctatttaggt aagtaattgt 29580
attggtagac ttcctagttt tgagtaagca actcttgtgt cactggatac attttattgt 29640
atattctttt aattgattgt tgaattaggt ttgcctaatt caggttttta tttagggatt 29700
ttacgactgt gtttacaagt gagctcagtc tatacctctc tgagctgttc catggttttg 29760
gtatcaagag ctgattcgtt ttctagagtt ttctgtgatt ttggccagtt tttatatcag 29820
accaaagaaa atgtttctcg aaagtaagtt cattaggctt taagtaacca ctgggcccat 29880
tctttgactt ttcaactttc tgagtgagtt ttagatgtgg atgttatata cagcatttgt 29940
tcagggcttt ggtttgtgag ctgagtttct ttttttattt ttttaataga tgagcttatc 30000
tcatttatat tttgttgata aggcagttct tccacattat taattatatt tttctttggg 30060
ggctttgtct cttatgggta tgtgtgtgtt tcccatctaa tagtttttga tgatttttta 30120
taaatcttat tctcttacta ccttcataga tttaatatct gtaagcctcc atttcttgat 30180
ttgtgaaaat aatcaggact attttaactc cttgctaata aaggtaagaa aatcagtaca 30240
cttatacctc cctttcccca gctaccatta aaaaaatttt tttccatctc ttcatatgag 30300
atataaacct tgtcattgaa aaaaatgcat ttatttttta ctttatcctt aagtctcaga 30360
gttacatgtt tgaatgggat taatattcac ctttaattct ttaaaaccat gacttcttga 30420
tttctaattc tgttttattt atcttttcat cagcttgatt ttcctatcaa gtagttctgt 30480
tttttctgcc aagaatggct tccttttttc ttcttcagcc atttctttct gtttccaccg 30540
tggccttcat ttctgaagtg gtctttttat taccttccat ttctttcttg aaccctgcta 30600
actcactttt catttcctat ttcttttttt tgagacaggg tctgggtctc tcactcaggc 30660
tggagtgcag tgttgtgatc ttggctcact gcaacctctg cctcctgggc tcaagccatc 30720
ctcccatctc ggcctctcaa ttagctggga ctacagacat gtgctaccac acctggctaa 30780
ttttttgcat ttttttgtag aggcagggtt tcatcatgtt gaccaggctg gtctcaaatt 30840
cgccttggcc tcccaaagtg ctgggattac aggagtgagc cactgcctcc tgcctcatct 30900
cccatttcat ctgttcttca attttgtttt ttcagtcatt tttttcccct aaaatgcatt 30960
tgatagtgat agatggggat atatcagaga cttcttttgt ttccttgact aacttctggt 31020
gtttatctgt ctttgcctat tatgtttctt ctctcatccc ccactccctg ttttattggt 31080
ggtgtggcgg gtggggttgt taacgtttct cctcatagac cctttgctgg attgtttctt 31140
tttattactc atccttgttg tctttgagta gagtacttct atttgagcct gctctttgct 31200

taagaatgtt atggggggct gggcacagtg gctcacacct gtaatcacag cactttggga 31260

ggccgaggtg ggcagatcac gaggtcaaga gatcgagacc atcctggcta acatggtgaa 31320

accccgtctc cactgaaaat acaaaaaatt agccaggcat ggtggcgggc gcctgtagcc 31380

ctagctactc aggaggctga ggcaggagaa tggtgtgaac cctgggggca gagcttgcag 31440

tgagctgaga tcgcgccact gcactccagc ctgggtgaca gagcaagact ctgtctcaaa 31500

aaaaaaaaaa aagaatgtta tggggaacag cagggagaat aagcctaagc aggcataact 31560

tttttctcag acatcttgtc tcagaaagct cgttcaccaa atctgttgtg ttctctaccc 31620

tgggaataca tcactcctcc atttttaatg tcttttttcc ccttcctgcc attttgggcc 31680

aagccaatca ttcagtcagg atgaagactc catccaggca ggagatgggt gaggctgata 31740

gtgaagttgg acatcaaata gattcctgtt cgatgatttg atttttaatt tgttctgctc 31800

agctgccttt agcttttaac cttttatatc ttatgttcaa tagttattct cacaaaggaa 31860

ctggttccca acataaattt aaaattaaaa gtgatcagtt ctctcctcta gcaaactgta 31920

actgctttta ttttatgtga cttgatctga tgtattgttt tgttccctgg atcatttagg 31980

ggtggggaag taaagacagg gaggtctgtg tttcacttta aaaacaaaat ctaaaccaga 32040

taaaaactcg taaaatgtca tatttcaggt cctctgtgat cttgcccttt cctacctctc 32100

tacccctgcc cacgtctatc agccatcctt gctgtgtact aaatgctcta gcacagtatt 32160

tcctaaagtt tatattgtgg atactgaata ttcaaagtgt tctcccaaag ataggttcca 32220

tagtttacaa ttttgggaaa ctttaaatgt tcctccttgt ctaacctgtt gagtttctaa 32280

gctcaaatga ggggggatac tgtgttatct aaatcttatt tgatttctgt attttacata 32340

ttaagggatt catctaaaaa gtatctgaat tgatttggca agcaggtgat accctgtact 32400

atattatata gctccccttt tgggatttca taattggata ttagcatagt aatggttgag 32460

aagaactgta gtaaagaagc ttacctttgt tgatttattt tttttttttg acacagattc 32520

tcgctctctc acccaggctg gagtgcagtg gcacaatctc ggctcactgc aacctccacc 32580

tcctgggctc aagcaattct gttacccttc caagtagttg ggactacagg tgtgtgccac 32640

cacacctggc taattgtttt taaatatttt tagtagagat ggggtttcac catgttggcc 32700

aggctggtct tgaactcctg acctcaaatg atccacctgc ctcggcctcc gaaagtgctg 32760

ggattacagc attagccact gcacctggcc ttacctttgt ctaaattaga attgtataaa 32820

cttccttgac tatggaacat tgcttgtgtg tagtatatgc taatattctt tgaaacattt 32880

tagggaaatg ctgtttaata ccaatcaaat ccttgcattt tttcagggtt gtttcctgtt 32940

gtttctggcc ctttgtccta gaatgctctc ttccttcatt ttctccatac cacccaattt 33000

tttttttcaa tggtttctga ggtgatactt accctgctgg gaggcatttg gaaatatttt 33060

gattgtcatg cctggaggat gctatttggg atttaatgcc ctcaaatcga ggaacacaat 33120

gcactaaaag tcctacacag caaagaattg ccccacccaa aatgccactt ctacagtggc 33180

cctccatatc tgcaagttct gcatcctagg attcacccaa ctgcagatca aaagtattag 33240

gaaaacagta aaaagtaata caataataca aatacaagat aatacagtat aacaactatt 33300

tatataacat ttacattata ttagtcatta taagtaatct aaagatgatt taaagtatat 33360

ggaaagtaag ttatatgcaa ataccctccc tttttatata agagacttcc gcatccaagg 33420

atcatggtgt tgtagggtag agtcctggaa ccaatccctt gtggatatgg aggaatgact 33480

gtatactcct ttggagaaca ctgaactcag caacttttac tcatccttta gaatttagtt 33540

ctggttatct tttccaaaag ctttctctaa gtacttttgt tttcccctct accccacacc 33600

ctctcatttt ctctgcgtgg tactctcaga gtattttagg cacatcttga ttatttgaag 33660

taaccacttt gtagtatagg agcctataac tgtatacctc tactgtactg tgagttcaaa 33720

tctttgaggg caaagacatt ttgtatcctt ggtatatgat atataattag tcttttaaaa 33780

agtgcatgtt acatgaatac attaataaat aagttgtact tttactttaa atcagaattg 33840

tggtatgact cttaaactgt tgcttagact gcattaataa tcttgtgcaa ttgtggaaaa 33900

taatctcatt ttactctcat aatagaaaat taaaatatgc ttccttttgc tcagatatgg 33960

gtgacagctg ttagagtagt gttttatttg ggttttgacc ttgcctgtac atcagaagca 34020

cctggagaac ttcagaaggt acagatgcct ggggccctgc accagaggtt ctggtttttg 34080

agggtgaggc cttggtatct ggatttttaa aagcttccaa ggtgattttt aatctgaagt 34140

taagattgaa aagtcattta gtgagacact gatatgtagc attttgggaa gtcagtaaac 34200

ttaagagtga gaagttacta aatcattgaa gaacagtgaa aggagtaggg agttttagct 34260

tagaaaaaaa aaatctagat ggagtatgtg agttctctga gggctatagg aatttgtccg 34320

tcttgctcac cactgtatcc tcaactccta gcatgagcct gagcacatac agggatttgg 34380

tagatattta ttggctgaca gaagaatatg tatcattagg tgtttaaaag atagtcattg 34440

tagaaccaca gagattatcc ccaaaaaagt tttatagtaa taatgtgcca cttaatttta 34500

tgattaaaaa taatgaactg gatatttaat tcatttatca tttcagttta ttatttactt 34560

tgtgccagtc actgaggatt ccatttgatt ctctggtcaa atctatgaag tttgtatcat 34620

tcaaataata gggctgtgtc aagaaactga cacttggggg ttaagtaatt tgcctaaggt 34680

tacaaagcta agtgacaaga gctggaattc ttaaagtctg tactcttaac tgctatgcct 34740

taccataagt cttcaccaca accctaagag ttagatatgg tttagaaagg ttatgcagct 34800

tgcctaaggg cacattgctt gagtggaaga tttgggactt ggacccagac attgtgacac 34860

tagagtccat gctgttaact gccatgctat tggaaccccc aaccccctcg cttcctattg 34920

tataatgtac aaccatctgt tttcatgaga ttattaacca cagtggacag cttgtgttgc 34980

tctccttata ccttttggca tcctcactga cttttgactg atgggttgaa tttggattat 35040

tgtcttgaat ctccttagta ccctgggctg tggtagtcct agaaaataaa acatttcttt 35100

actaggttct ttttcttcat ttctttttcc ttttagttag gattttaaat tagaatttta 35160

ataaacttgc ttgcattaat acactgatat ctgttagctt ctgttatttt aagtcggtag 35220

tctccagact taaaaatttt gttctctatc ataaaaaaaa tttgagcaca ttacccctag 35280

tagatatctg ttttatttat gctatatgtg tactactgaa gaaaatggta atattttaaa 35340

aaatatgaac ttgttagcat gaattttttt aaagctaagc taaaaatgaa gtgagtttaa 35400

aattatgaag ggtttttgct gatgtttcaa gtttagctaa tgtttcaagt tacaacatac 35460

cattaggcca aggttcgtta ttataatagt gtgtacaaat tcatatttta agtagcctgg 35520

ataatttttt taaaatagct agtttcttgt cagaaattaa gtaacctgga taatttttta 35580

aaagccagtt tcttgtcaga gattattaga ttagggtttc tcaacattgg cgctgttgat 35640

gttttgaaat ggatgtaatt cttgcttgta gggttatgat ctgcagttga gtgaatccta 35700

cgatgtagaa cttgcagata tggagggcca ctgtaaaagt ggcattttgg gtggggcaat 35760

aatactatgt attgtaggat gtttagcagc ttccgtggcc tctgctcaca agatgccagt 35820

agtaccccca agtagaaaca tcaaaaatgc cgggagacat ttccaaatgt cttgaggggc 35880

aaaattgctt ctggttaagg accactagat tagaattttt ttttttttta agacggagtt 35940

ttgcttttgt tgcccaggct atagtgcaat ggcgcaatct cggcccactg caacctccac 36000

ctcccagatt caagcgattc tcttgccaca gcctcctgag tagctaggat tacaggtgcc 36060

cgccaccgca cccttctaat tttgtacttt tagtagagac agggtttctc cacgttggtc 36120

aggctggtct cgaactccca acctcaggtg atctgcccgc ctcagcttcc agagtgctgg 36180

tattacaggt gtgagccact gcacccgcgc tagatcattg tttttatcct gtattatgga 36240

tgacaagcag cttgtagtag agtagggaaa gtgttaactt tgattttttc ccctctagca 36300

gcaataatgt tttcttcagt atgaagtttg agatctgttt gtaggaatta attttaagtc 36360

acttgtccat tctataaggt ttagttaaaa cttggtaaca taatccatac gtttacttaa 36420

atcaatatat gtgagtcata gtatgtcaca atgagataaa tgcaagagga gagccactgt 36480

caagtgttct gcagtatgga atgcccagcc ttcagcagac ctcttgacta tatgtgtcac 36540

atctctgata ctaaccctaa gttagggtgc ctgtgtaaat attaaatgct gaggccaggc 36600

acagtggctt acgcctgtga tcccagcact ttggaaggcc gaggtagacg gatcacaagg 36660

tcaggagatc aagaccatcc tgactaaaca cagtgaaacc ccgtctctat taaaaacgca 36720

aaaaattagc caggcatggt ggcatgtgcc tgtagtccca gctacttggg aggctgaggc 36780

aggagaatca cttgaaccca ggaggttgca gtgagctgaa atcatgccac tgcactctag 36840

cctggatgac aaagcgagac tctgtctcaa aaaaaaaaaa aaaattaaac gagcatggtg 36900

gcatgcatct gtagtcccag ctacttgggt ggccaaggtg ggaggattgc ttgaacccaa 36960

aagtttgagc ctgcggtgag ctgtgatcac actactgcac tccagcctgg gcacagagtg 37020

aaaccctaca tctcaaaaaa taaatattaa atgctgactt tttctaagtt tctagatgaa 37080

cacattaact aaataatgta gcctacctat atccccaaaa aagagtcttc ctatgggccc 37140

tcaggtgtat gtgcatacct attttgtaaa ccacttgttt gaccatcaca ttgtaactta 37200

ttgttcaatt tgtgtcactg cagccatatt acccaaaagg gaaaagtaat tttagctctc 37260

tgaactgacc tccacctaac caactcatca ggttaactga tgttctccat ttcaaaaaat 37320

atttgcggtc agattaggtg tgaatcatat ttaaaggaag attgttggtc ctatatcaaa 37380

gattagagaa tgaatgttca ttttacagtt ttaagttaaa atgtttaagg acagtgttta 37440

ccattgcaca tgattccctg atttaactga ccttttcagt taactagcca ctagacataa 37500

ttacattgac tataggggct tcttttgtgc cacaccagtg ttggaattgt gttgatttac 37560

ttgtggagtt ggaactacag tttctctcag cagcctgctt atctggttgt ttaaacttgt 37620

agctgaatta ttatacaaag ttttcaaact ttctgttttg gaatggtcag ttactcaaaa 37680

ctgtggcttc ctccttgcta ccttacaggg tagttgtgag agtattcctc aacaatagtt 37740

cagactctca ttacttcttg tctataattg tagcatgctt ttaatgggat tccctgcctc 37800

cagtacctct ctggcttgtt ggttaacata aacattattg ccagattaaa catcctaaag 37860

tataatgtgt aatcagatca aaagccattt ataacaataa aaactacatt aaaaaattac 37920

ttatgtcctg ggcttagatg tggtctctgc aatcagatgt tcattaaaca aattatttaa 37980

aaaatacata catacacaca cacgcaaatt ctgaattgtg atatgtgcca aaagaaaaaa 38040

tggggggtga tgtgagagct cagtgaggat caaatataga ttgggaagaa gggtgatgag 38100

gaaagacttc tttgagaaag tgagatttaa actatctgaa gaagctgtgc ttagtttaga 38160

aaagtgctag gcaaagagtg ttccaggaag aaggaatagc atgtgtgaag gctctgctct 38220

gctgctgaaa ttgtggttca gtttattgag tatggcatag aatgaatagt gcctgatgag 38280

gcaggaggta ggctgggatg agatcttttc agatcttgca ctctgtacta ggaagtttag 38340

gttgtattct gagttactgg ggagccatta aagggtttaa ggaaggggag tgttgtgaat 38400

cagttatgtg ttttgcagat tctactctga ctacagaatg gatgagagca gaatgggagt 38460

tagagcaaaa gaaatgagga aaccagttag aatgcctttg taagtagtag gtcagagaga 38520

aaggatgata attttgacta gagtcatgac agtaaacaag gtaacacatg aagtgattat 38580

gtcataaact attaccatta ccctgtgaag gaagtatgag cagaataaga tttagaataa 38640

acttattcca agcccatcta ataaagggtg gtgtgaggaa ctgaaacaat gggtgtttca 38700

ggacattcgg tccttgtctc taaaataaaa tccaaactct gttgaagatt tttcagccta 38760

ctcttattat ctttgtcttc tcctaaactc ttactcacct atgctataat cataccaaac 38820

tattacagtg agtatcatga acatttcctg taccttgatg ccactgatga ttgtttacta 38880

tggctggaat actcttactc ccacatacac ttaacaaaac cctactcatt gtttaggatc 38940

cagatcaaat atgaatgaag ccttccaaat ttgcctccag attgatattg cccatttatc 39000

ttcttttaac catagtagtt catattacta tcacattata gtatattatt caacagatgt 39060

ctatgtaatt gattatgtgc caggtactat gtgtagacat tatggatata tccacataca 39120

aaaatagaca cagttgccgc atttgtggaa actacattgt aacacagacc ttgaacaaaa 39180

tctgggcaca aatatgtaac taaatgttaa taagaggtat gaaggaatac ctgcaggttt 39240

ctgaaaccct aataaggccc ttgaggtcaa aattattttc ataatcctac taagatacta 39300

tttgcctttt cattctcgtt cttgtgagta tgtagtagtt ataggatgta tgatattcca 39360

acaaattgaa tacagaaggt gggagaatcc agatatcttt tgttaaggca gacattaaag 39420

aaattggcaa aaaaaaaatt ttttttttta aagccacttt ggggccaggt gcggtggctc 39480

acacctgtaa tcccagcact ctgggagtct gaggtgggca gatcacttga ggtcaggagt 39540

tcaagaccag cctggccaac atgatgaaac ctcgtcttta ccaaaaatat ggtagctggg 39600

tgtggtagca catgcctgta atccgtccct gtaatcggaa ggctgagagg cgggagaatc 39660

gcttgaacct gggaggccaa ggttgaagtg agctgagatc gtgccattgc actccagcct 39720

gggcgacaag agcaagactg tgtctcaaaa taaatgaata aataaacaaa caaataaata 39780

aataaataaa aatgaataag acactttgcc ctctcattgt tttttgttgt tttggaaaat 39840

gtggttattt ttttcattaa aaaatagatc acttttgtta attagttttt ttgtcatttt 39900

aaaatgaatt ttaaacattt cttggttata atttttaatg tggtaaatac tgatagcgat 39960

aaactacata gataaaagct ttctagggtc ctccataatt tttaagagta taaaggggcc 40020

aagtgtggtg gctaacacct gtaatcccaa cactttggga ggccaatgca ggaagatcca 40080

acatagcaag accctatctc tacaaaaaaa tttaaaaatt agccagacgt gatggtgtgt 40140

gcctgtagtc ctagttactt gggaggctga ggtgagagga tcacttgagc ccagagtttg 40200

agattgtggg gagctgtggt catgcactac attcctgcct gggctacaga gtgagaccct 40260

atctcaaaaa aaaaaattat atttttatac acacacacac acacacacac acacacacac 40320

acacacacac acacatatct tatatatata taagagatcc tgaaaccaaa atcagagtcc 40380

ctgtaatggg gagctgattt aaattggggg cttctggaga agtaaaagat atcttatgtc 40440

tctgttaaac acttatgttc ttctagaaat taggagtgtg gttttctccg tatttccaaa 40500

aactgcaagt ctttttatat aacgagtgtg ttggttgaat ctaattttaa aaaaaatttt 40560

tttaatttaa aaatttttta atttttaaaa atatggagtg cttcatgaat ttgcatgcca 40620

ttcttaggca agggccatgc ctatcttctc tgtattattc caattttagt atatgtgctg 40680

ctgaagcaag caccaaattt tgttatcaaa gtctactgta gatggccgtt ctttctttga 40740

tttgtggttt ggttgaaacc tttgttggaa ataccatctt atttcttatt actgtaggaa 40800

atagatctgg attcaaattc ctcttggtat gaagctggga agattactta acctgctaaa 40860

ccttaccatt atttatctgt aacatggaat acatattatc ttactgtggt gtaatgattt 40920

aatgaactaa tgtatgtgac ctgattcata agtgaccaga acagagacaa tccataaaat 40980

ggtaattctt tttattttat atacacagca catacatact ggggttagtt tcagttagtg 41040

agcagagagg tctgtggata aatcaggata taacaatcat cattcaagga tatattcacc 41100

attcaaacag tttatcagaa ggcagaaagc cagaatgatc tattgtgtat ggttattcat 41160

ctttagaaaa aggaacagaa agtgtgggct tagaatattt taaataagta gctctatgtt 41220

acaatgtaga tgatttaggg atggggtaga gttttatgtg tctggcacta cagcagtgcc 41280

tcacatttac taaatgtttg aataatatta attaactaga gattcttgaa aacacatttt 41340

tagaagcctt gaaaaagtta tatacgacag attaaaggcc aagcctgaga aagcttacat 41400

ggctaactgg aaaaataaat aaaggtacca tagaggaaaa acaaaattgc cctgtgggga 41460

gaacatgtgg tgtcatatgg tgtgactaaa taggatccag taagataaga caaggtagag 41520

catcttggga gtgattccat gtttcaaggt taaaatgtta actacattaa aggtagtaaa 41580

ccagtgaaag aatcctcaag atcccagtgc agaatgtttg ccaagagata agaagatcaa 41640

ctgttttggt attcatagca gaaagccata ggaaaattat ctttttgata ttcttttttg 41700

aagaagatag gtcctttatt tatttattta tttatttatt tatttattta ctttatttta 41760

ttttattttt tgagatggag tttcgctctt gttccccagc tggagtgcag tggtgcaatc 41820

ttggctcact gcagcctccg cctcccaggt tcaagcaatt cttctgcctc agcctcctga 41880

gtagctggga ttgcaggcat gcaccaccat gcccagttaa tttttatttt tagtagagac 41940

ggggtttctt ctccatgttg gtcaggttgg tctccaactc ctgacctcag gtgatccacc 42000

cgcctcggcc tcccaaagtg ctggcattac aagcgtgagt caccgcgccc agccaaagat 42060

aggtcctttt ttaagacaga tacttagggc tgggtgctgt ggctcatgcc tgtaatccca 42120

gcactttggg aggccgaggc aggtagatca cctaaggtct ggtgttcaag accagcctga 42180

ccaatatggt gaaacccaat ctctactaaa aatacaaaaa ttagctgggt gtggtggcgt 42240

gcgcctgtag tcccagctac ttgggtggca attcaaggag aattgcttga acccccggga 42300

ggcagaggtt gcagtgagcc gagatcgtgc ctctgccctc caacctgggt gacagagcaa 42360

gactccatct cacaaaaaaa aaaaaaaaaa aaagatactt tgataaagaa ataatagtta 42420

tttctcattt tatttctcat ttgagatgaa ctcaaagttg gctaaagtga cacacagttt 42480

tggacctata acttgcttac attttaaata ttaggttggt gcaaaagtaa ttgtggtttt 42540

tgccacccaa tagaaaggat tgtagacatt tttttatttg acaactgtaa agcattgcag 42600

gaattatatg tggaattata ggcttacttt gttttattgt gtttcatttt attgtactac 42660

acagataatg tggttttttt tacaaattga aggttggtgg cagccttaca tcaagcaagt 42720

ctgttagcgc catttttcca acagcacatg ctcactttgt gtctctgtgt cacattttag 42780

taattcttgc aatatttcaa actttgtctg ttttggtgtt ctgtgatctt gcatgttact 42840

attgtaattg ttttggggtg ccacaaacca cacccataat aaggcagtga acttaatcag 42900

taaatattgt gtgtgatcta actgctccac tgactggctg ttcccccaac tcttctccag 42960

cctccgcatg ccctgagaca caacaatatt gaagttaggc caattaataa ctctacaatg 43020

gcctctaagt attcaagtga aaccaagagt cacatgtctc ttgaaatcaa aagctagaaa 43080

tgattaagct tagtgaagaa ggcatgtcaa aaagctatgc cttttgtgcc agacagctag 43140

gttgtgaatg taaaggcaaa attttggaag gaaactacaa gtgctactcc agtgaataca 43200

cgaatgataa gaaagcaaaa caggctcatg cctgtaatcc cagcactttt aagaggccaa 43260

ggtgggagga tcacttgagg ctaggagttc gagaccagcc tggccatcat agtgagacgt 43320

cgtctccatt catttttaaa aagcaggcaa gcaaaacagc ctaattgctg agatggagaa 43380

agttttagtg atctggatag aatatcaaac cagccacaac atttccttaa gtcaatgaca 43440

ggctttaaag cttcaaagga caggctgact cttactagag gctaatgcag ctggtgactt 43500

taagttgaag tcagtgctca tttatcattc caaaaatcct agggcccttt ggaattctgc 43560

aaaatttact ctgtctgtgc tctgtaaatg gaacaatgaa gcctggatga cagcacattc 43620

ttttacagca tggtttactg aatattttaa gcccactgtt gagaccccct gctcagaaga 43680

aaaaaagatc ccttccaaca tattactaat cgttgataat atacctgatc acccaagagc 43740

tctgatgatg tacaaggaag attaatgttc tcttctgttt tctatgtgtg tgtgcttttt 43800

ttgttttgag acagggtctc attcttgccc aggctggtgt gatcatagct ctctgcagcc 43860

ttgaacccct gagctcaagc aatcctcctg cctcagctgc ctgagtaggt gggactacag 43920

gcatgcacca ccatgcccag ctaatttttt tttttttttt aatagagaca gggtctatgt 43980

ttcccaggct agtctcacaa actcctggtc tcaagtgatc ctcttgcctc agtaatgttt 44040

ttcttaacca ctaacgcagc attcattctg cagcccatgg atcaaggagt aattttgact 44100

ttcaattctt cttatttaag aagtatcttt tattaggcta tagctgccat agatattcct 44160

atgatggatc tgggcaacat taattgaaaa ccttctgcaa attattcacc tttctagatg 44220

ccattaagaa tgtttgtgat tcatgagagg aggtcaaaat agcaccatta acaggagttt 44280

ggaagaagtt cattctaatg cttgtggatg actttgaggg gtcaaaactt caatggagga 44340

aggaattgcg ggtgtggtag aaatagcaaa ataactagaa ttagaagtgg atcctgaaga 44400

tgtgaccgaa ttgctacaat gtcatggtaa aacttgaaga aatgagaagt tgcttcttat 44460

agatgagcaa agaaagtgat tttttgagat ggggtttact cctgctgaag atgctgtgaa 44520

cactgataaa atgacagcaa aagatttaga attttacata aaattagttg atgaagcact 44580

ggcagatttg agaggactga ctccaagttt gaaagtagtt ctactgtggt tcagatgcta 44640

tcagaccata ttgcctggtt cagagaaatc ttttgtgaaa aaaagtcagt cggcacagca 44700

gacttcattg ttgtcttgtt ttaagaaatt gccggccggg cacggtagct tacacctgta 44760

atctcagcac tttgggagac caaggcaggc agatcacctg aggtcaggag atcaagacca 44820

gcctggacaa catggtgaaa ccccatctct actaaaaata caaaacttag caagtcgtgg 44880

tggcacgcac ctgtaatccc agctacttgg gaggctgagg caggagaatt gcttgaaccc 44940

aggaggcaga ggttgcagtg agccaaaatc acgccactgt actcccaccc tgggcgacag 45000

agcaagactc catcttaaaa aaaaaaaaaa gaaagaaatt gccacagtca ccctaacctt 45060

tagcaaccac tgacttgatc cggcagcagc catcaacact gaggcaagac ccaccaacac 45120

aaaaatgatg actccttgaa ggcccaggta attgttagtg gtttttaaca ctattttaaa 45180

attaagatat gtgctttttt tttttttttt ttttagaata atgctattgg acattactag 45240

actacattat agctacactt ttttgttgtt aatttaaaaa ttgtgggccg ggctcagtgg 45300

ctcacgtctg taatcccagc actttgggag gctgaggcag gcagatcaca aggtcaagag 45360

atcgaaacca tcctggccaa catggtgaaa ccccatctct actaagaata caaaaattag 45420

ctgggcgtgg tggtgcacgc ctgtagtctc agctactcgg gaggctgagg caggagactc 45480

ttttgaaccc gggaggcaga ggttgcagtg agcagagatc ccgccattgt actccagcct 45540

ggcaacagag caaaacccta tctcaaaaat aaaataaaat aaattatggc caggcgtggt 45600

ggctcatgct tgtaatcctg gctctttggg aggccaaggc aggtggatca cttgaagtca 45660

ggagttcgag actagcctga ccaacatggt gaaaccacgt ctctactaaa aatacaaaaa 45720

aaattagctg ggcatggtgg tgcatgcctg taatcccaac tgcttgggag gctaaggcag 45780

gagaatcgct taaacctggg aggtggaggt tgcagtgagc tgagatttgc gccactgcac 45840

tccagcctgg gtgacagagc aagactccgt ctcaaaggaa aaaaaaatgg atacataatg 45900

attatatata tttctggggt acgtgtgata ttttgatata ggtatacagt gcgcaatgct 45960

gaagtcaagg tgattgggat acccatcacc ttaaacattt atctttgtgc tggaaacatt 46020

acagttctct tctagctatt ttgaaatata tgataaattg ttaactgtaa tttccctact 46080

atactgtgaa atactagaac ttactgtttc tgtccagttg tatgtttgta tccattaacc 46140

aacttccctt tatccctttc tccctccttt tcttcccaga ctctgataac cactactcta 46200

ctgtctacct ccatgagatc ctttatgtat tctggatata gatcctaatt aaattcatga 46260

cttgcagcta ttttcttgca ttctgtaggt ttttttcact ttcttgagaa tattcattgc 46320

acaaaaggtt ttaattttgt tgaagaatga tttgtcagtt tttttttgtt gctcgtactt 46380

ttggtgtcat atctaagaat ccattgctaa atccaaggtc attaagattt acccctatgt 46440

tttcttctga gagttttatt attttagctc ctatatcatt tattcatttt gagggttttt 46500

aaaatatggt gtgaggtagg ggtggacatt tattgctata aattgtcctt tgagcattgc 46560

ttttgctgta tgccatcagt tttggtatgt gtgttttttt tgttttcatt tgtctaaaag 46620

tattttctaa tttttcttgt gatttctttt tttgaccttg tatctatatt caagagggat 46680

attggtgtat aattttcttt tttgtacagt cttttgtatt agtgtaaagg tgatgctgga 46740

atcataaagt gagttggaaa ttacttactc cttttctgtt tcatggaagg tattttgtag 46800

aggtggtctt atgtcttctt taaatacctg atagaatttg ctactagaga tttattttta 46860

gaaaggtttt taactatgag tttaatttcc ttaatagtta caagagtgtt cagattatct 46920

gttttatctt gtgaatataa aagatatctt cagatatctt ttatcctgtg agagttttgt 46980

ttttctttgg tttttgagga attggttcat ttttttctaa cattttgaat ttatgtagag 47040

tttttcatag tattcttatt aacctttaaa tgtctgtgtg taggggagtc tgtagttcat 47100

tgttttttta ttactgatat tgttaatttg tgtcttccct ttttgtcagt cttgctagag 47160

atttgtaaat tgtattggtc ttttcaacaa acaagctttt gttttcaaca tttttttctg 47220

ttttcaatct catggatttc tgctttttat tatttttgtg ttgctttctt taggtttatt 47280

tagttcttca agtttcttaa gatagatttg caactgtttt tctttgctga tataatagta 47340

tcagtttaag agcagggctt ggtggcacat gcctgtaatt ccagctattt gggaggctga 47400

ggtggtagca tcacttgagc ctaggagttt gaaactagat tgcgcaacat gcaagaccca 47460

cccccccccc catctcttaa aaaaactaaa aaaaaaataa tggcacaagc ttaatgatat 47520

taattcactg tagcactact gtatttgcat cccacagatt tagtatgctg tattttcatt 47580

cagttgaata ttgaactagc cttgtggtcc tgggattaac cttattgtca tgtttattat 47640

gccttttaca attgctggat tctgtatgct aatattttac taatgattat gaggaatatt 47700

agtatgtaat ttggggcttc catttgactc aaagattatt tagaaatgtg ctgtttaatt 47760

tccaggtgtt tggagatttt cctattaact ttctatttct agtttaattc tattatggtc 47820

agagaacaaa cgtttgtggt ttttttttaa gcttgtgaaa gtttgtctta tgactcagaa 47880

tatggtctgt tttggtgagt gttccatgtg catttgacaa gaacatgtat tcagctgtta 47940

agtagaatgt tatataaata tcaatcagat caggtggatt gatgatgttc atttcttcca 48000

tattcttact gattttctgt ctactagttc tattactgaa aggagtgctg aagtcatcaa 48060

atataattaa gaatttgttt tcctatttgt aatgttctgt aagtttttac ttcatgttct 48120

ttgaagctcc attattaggt gcatatatat tagttatgct ttctattatg aaaattatat 48180

ttgaagtgaa ttactcgtag actacatata gttgggtcat tttaaaaatt cattctaaca 48240

atcttgtctt ttaatttgta tctatagact attcacattt aatgtaattt tggcatgttt 48300

agatttaggt ttaccagttt agtaatttgt tttctgttag ctgcttctgt tttccattac 48360

tctgtctttc ctgcattctt ttagattgtt tgaacaactt ttagccattc tgttttaatt 48420

tacctgttgt ggcttaaaaa ttcttaactc tccatatagt tttagtgatc actccagaga 48480

ttacattata aaaacttaac attttcacca cctgctttaa aataaattca tttctttaaa 48540

tggattatgg tccacttaaa gaaatgttaa aactacgtag gtctctttat cttctctcca 48600

ccttttctct tattgttggc tgtatgttac gtttctatta attgaaagct tcattgggca 48660

atgctattgt ttttactttc aaccattaaa catatttaag gaaactaaga ggagagggtt 48720

aatgtatttt tgtctgcatg tttaccattt tgcttactcc tcaacctact cttccaggtt 48780

tccttccggt attatttccc ttctgtgtga ggaatttctt ttagcaattc ttttagaaca 48840
ggtctgctgg tagcaaattc tcttagttcc catttatctg aaaatatctt tgttttatga 48900
ttgctctgaa agatacttta actggatata gaattctacc tttgatagtt tttttctctt 48960
tcagcacttt aaaatgtgta acttccttct ggccttcatg gtttctgctg agaaacctgt 49020
tgtcatttga agtggtgttc ccctatattt tatggattgt ttttctctgt tttcaagata 49080
atttttaggc tgggcgcagt ggctcccacc tgtaatccca gcactttgga aggccgaggc 49140
gggtggatca ccggaggtcg ggagttcgag accagcctgg ccaacatagt gaaaccctgt 49200
ctgtactaaa agtacaaaaa acttagccag gcgtgttcgc gggcacctat aatcccagct 49260
tctaggaagg ctgaggcagg aaaatcgctt gagcccggga ggcggaggtt gcattgagct 49320
gagatcacgc cattgtactc cagcctgggc gacagagcaa gatgctatct caaaaaataa 49380
tagtaatttt ttgtgtttca ttttcaacac tttatgatgt gtgagagtgt ggaattctac 49440
gtatttatcc atttttggat tcaccgacct ttttgaatct gtagttttat gtcatttgtc 49500
agatttggga aggcttttga cattatttct tcagatattt ttaatcatcc tattctttgc 49560
ttttagaagt tcactgacac agatgttaga tctttttgtt actgtcccat agatccctga 49620
tgtattactc tattcttgaa ttgctataaa gaaatgcctg agactaggta atttataaag 49680
aacagagatt cagttggctc atggttctgc ggactgtacg ggaaacatag cagattctgc 49740
ttctggggag gcctcaggaa acttaaaatc atggtgaaag gtgtaaaggg gaagcaggca 49800
tatctctcat ggctggagca gaagcaagag atggggaggg tgccacatac ttttaaatga 49860
ccagatctca taaaaactca ctatcacaat gacagcacta agggggatgg tgttaagcca 49920
tgggaaaccg cttccatgat ccaattttct cccatcaggc cccacctcca acattgggga 49980
ttacatttga acatgagatt tgggtgggga catagattca aaccatatca cctggattct 50040
gttcatttta ttttaatccc cacagctaca tcaattggag acctggttct ctccctgctc 50100
tcagaatgtt ggctcttttg aagatcctat tgttgccgct gccaccatgg ggttacgtga 50160
gaaaagtgag gggaaaaaac tgagacacta tctttgaatg ttatttcttt gccttttttt 50220
tttttttttt ttactttttt tcttttaaaa agtaaacttt ttttaggcca gtcgcggtgg 50280
ctcacgcctg taatcccagc actttgggag gccaaggcgg gcagatcaca aggtcaggag 50340
atcgaggcca tcctggccaa cgtgatgaaa ccccgtctct actgaaaata caaaaattag 50400
ctggacatgg tggcgcgtgc ctgtaatccc agctacccgg gaggctgagg caggagaatt 50460
gcttgaatca gggagttgga ggttgcagtg agttgaggtc gcgccactgc actccagtct 50520
ggcgacagtg agactccatt tcaaaaaaaa aaaaaaaaaa gtatgtaaaa tgtaaactca 50580
ctgatggttt catgatgatt cagatccttg tcctctatcc caatcgtcta ctgctattta 50640
cttccagtgt tgtcgattag ctgctctatg tgttctgtcc aagttttata gttggactaa 50700

atctttgaag tgtgttccca ccacccccgt aatgtgtgac tactaatatt tctgctcaat 50760

ttgttctttt tcccctcctt gtttttattt ttattcttgg cttcctaggg gttgctcctg 50820

tctttccata gcttaatgtc aagctaaaga tttgtcagag gttttgttca aatatttcaa 50880

ggccagtaag gtttctattc tttctttgtg tgtctatctg tgtatcaggg agtatattca 50940

aacttcaggc caccatgttg cctgacctgg cttttgcttt cctccgggct cctctgtgtc 51000

tcctgtgtgc atgaacatgc agaggctcag tcagtcaagg atgtgtggag gtgtgggccc 51060

tgtccagacc ctgtagcacg tgcttgcagt gtccgttcaa ctagtggagt gtggaaagtg 51120

tattaagccc ccaacttgca gtggaggtta tcacttaaat tcacagcact ccaaatcaat 51180

tgtcaacacc ctcacacaca cacagtctct agctgaacac atgccaacag agggagagat 51240

tggtcagtca tctgaagaag ggatggcagc agcctcaagc aaaaatgcca cagatggctg 51300

ggcatggtgg ctcacgcctc ttatttcagc actttgggag gccgaggcag atggatcacc 51360

tgaggtctgg agttcaagac cagcctggcc aacatggtga aaccctgtct gtactaaaga 51420

tacaaaaata gctaggcatg gtggtgcatg cctgtaattc cagctacctg gaaggctagg 51480

acaggagaat cgcttgaatc tgggaggcag aggttgcagt gagccgagat cgtgccattg 51540

cactccagcc tgggcgacga gcagaactct gtctcaaaaa aaaaaaaaaa aaaaaaaagc 51600

cacagactgt gatgttctta ctcaggttca gcctttagct gaagtccaga gcactgaaat 51660

ggttgttttg acggttttgt ccagctttat agttgctttt gggggagagg atttatcaat 51720

gtactcattt catcatgcca gaagtagtag tattgatatg attttttata tatatcttag 51780

aattttgaag atactgtttt cttattttct agcatacaac aatctgattc ttgtttttta 51840

ttccttagaa ttttattggt actttcttat ttccttttga gtttaactta gcaggatttg 51900

tttaattttg tatccctcca agcacccagg ctggagtgca gtggtacaat catggctcac 51960

cacagcctcc acctcatggg ctcaagtgat tcttcctctt tggcctcctg aatagctgga 52020

actacagaca catgccacca tacctggcta atttttattt ttttttattt ttggtagaga 52080

tgaggtctcg ctatgttgcc caggttggtc tcaagctcct gagctcaagc agtcctccca 52140

cctcaacctc ccaaagtgct gggcttacag gcataagcca ccatgcccag ctcttattct 52200

ttctttgtta aaaaaggaac tttggttctg aagactctta tttgcctttt ggctcaagga 52260

gaaattctta aattttcttt gttatttctt cttcttattt taattctcat aaaatttctc 52320

ttagatgtat gtaggataat ttaatctgta ttctgggata ttaactttac tctcataatt 52380

taagctttca ttttacttca tatttttttt ttaacttttt gttaaagtac catggacttt 52440

agctttgtcc attttgctag ttaactcctt ttttaggttt atttcaacag acattttaaa 52500

tttcaagctt gttctgtgca atttatttat ttatttattt atttattttg agatggagtt 52560

tcactcttgt tgcccaggct ggagtgcaat ggtgcgatct cggctcactg cagcctccac 52620

cttccagatt caaatgattc tcctgcttca gcctcccaag tagctgggac tgcaggcatg 52680

tgacaccaca accggctaat tttgtacttt tagtggaaac ggggttcacc atgttggtca 52740
ggctagtctc gaactcctga cttcaagtga tccacctgcc gcggcctccc aaagtgctag 52800
gattacaggc atgagccacc gtgtctggcc tctgtgcaat ttagtatttt tgttttcttt 52860
agtgagtgca gctcctccat aatagcacta tatacaatat aattttctgt gctatgcagt 52920
atattagcca gtagctgcag tggctgggaa tgtggcctgt gagagtgaga aactaaattt 52980
tatattttat ttaatgttag tttatataaa attaaatagc catacatgtc taatgattac 53040
tagattggac agcacagctc tacaatttaa gaatccttat actttttggc ttcaaaataa 53100
ttctgatgcc ttagttcatc tcctcattca attgcatgtc tgttttttat gctcttggtt 53160
ttttttaggt gtctaaaggt taataaaggt taaaaatatt ggaagttaga gtaggtttcc 53220
tcaacactta agtgagtttc ttctggagaa agtgaatatt gaatataaaa atccattaat 53280
ttctagttct actaagtgag ctaggcagtc cttgctttag agaatatggg tacttcttct 53340
caatatgtgt atgtgtgtag cctctgtccc tgtggtggcc agaatttact ataattctgt 53400
ctctggccat agtgtccaga cagaaatccc tgtaagctga ttaccctttt ccaccaggtt 53460
tagatcagat acaaatatat gggggcaaag actgagttac tctttttttt tttttttttt 53520
tttttgagac ggagtcttgc tctgtccccc aggctggagt agtggcgcga tctcggctca 53580
ctgcaagctc cgcctcccgg attcacgcca ttctcccgcg tcaccctccc gagtacctgg 53640
tactacaggc acgtgtcacc atgcccagcc aatttttttt tgtatttttt tttgtatttt 53700
tagtagagac agggcttcac cgtgttagcc aggatggtct cgatctcctg accttgtggt 53760
ctgcccgcct cagcctccca aagtgctggg attacgggcg tgagccaccg cgcccagcca 53820
gactgagtta ctcttgaaca tgtgatttgc atctaaacca aatttgttaa aagtcttttt 53880
tttttttttt tttaagtaag aaaaggcagt gattttgatt tgtaatgtct tttgttagga 53940
gaagtaaaag aaaaaaaatt cttgaagaaa agtgagccag aattactgcc tagggagtga 54000
tagtcatgta ggttcacaat gaagtttaga agtgctcttc gtggttccgt ttcctctttt 54060
gctttagtgt tcaggaaact aaaaactgac agtagactag ttggaagata gactaggttt 54120
tttttttttt tttctagtta acctaagtca gaagggtgat atcactagag acttggaagt 54180
aaaaagagct acctgcaaag acttgaagta ctaagattac ctagaatctg aatgccctgt 54240
gttactctgc agatagggca ttttactgtt atgaagagat aagatctatt gtaaatagtc 54300
taaactaata ataacgagta agtatggaat tggcgttttt cctttaaagt tttagcatat 54360
aattttgaaa tgttttaaga atatttttga aatgtttcta ttttttaatt tctcttttag 54420
aagtcttatc taaaataagc atgtatatct tacatgtaag gaattattaa cttcattttt 54480
tcataaaaat aatcagagta agactttcaa cagatgtcag gatagctgaa gttctccatc 54540
actgtcatac gtttcactta tgacaggttt gtaatttctg ttagggaatg tcatctaagt 54600

gctctgcatc tggtattttc tcctgcaccc tcatgacaag aaaaaagcct ttgattattt 54660

ctcttttctc tctatttaca tgttcaccac tgccttccat gttcagatgt gatctacatg 54720

caatgatgat tactctttcc ttcctttccc ccttgccctc ttttatcgtc tttctttctt 54780

cctttttttg gttgaccctg tttaaatggg atctatcaaa tataccaagt ttcagtgaaa 54840

gttacttcct tgtgttaaaa gtcatgactt tctcttttat ttatacactt aataacttga 54900

ttatatatac ttgaattttg ttttagtgca gaataatttt ataatctttc tgaaggtatt 54960

ttaaagtggc agttaattcc aacatttgta ataccaacaa tgcagataac tgagttgaat 55020

gacaacagca tgaatagcct tgattaagtt gtacatgtgc agatagctac tgtgtatatc 55080

atgcctgcca tctggacaac agtgattttt ttttcttttt ttttttttgt ttgagacaga 55140

gtctcagtct gctgcccaga ctggagtgca gtggcgtgat ttcggctcac cgcaacctct 55200

gtctcccagg ttcaagtgat tctcatgcct cagcctcctg agtagctgga attacgggtg 55260

tccgccacca tgcctggcta atttttgtat ttttagtaga gacggggttt cgccatgttg 55320

gccaggtagg tctcgaattc ctgacctcaa gtgatctgcc taccttggcc tcccaaagtg 55380

ctgggattgc agacatgagt cactgtgcct ggtcagaaaa cagtgatttt taagatgtca 55440

tcaattgtat ggtagaatct gaattcaaag atgttaagaa ggggatgggg gaaaggtgtc 55500

tcttagaaca tgtaaaagaa catgttattt ctgtcaactt ccaaactttt atctccagcc 55560

acatctctta cttgatttca gatttgtata cccagctcct gttttacatc tccactctgg 55620

ttaatgtgcc tcccgggata ttttaaatat aatttttaat tctgcatcct atgccttgtc 55680

ctcccaacct cagtgttcct tcttcattct ttttgatttt tggctcttac atcctttgac 55740

ttaggttata aactgtagag tcgaggatga ctccctttta tcatacctca catccaattt 55800

accagcaaat cctattgact gtacctttat aatatattta gcatctgaca catcttatca 55860

tctcttccac tatcagtctg ataaaagcca ccatcatctt tcacttggcc atcatttccc 55920

atctggtttc ttggcattta cactatagtt catttccagt atagcagcca gagtaatact 55980

ttaaaaatat aattgatcat attgtttcct ttttttcaaa agtaacttca ttgagttcac 56040

ctaccataca aatcactcat ttaaagtgta cagtgcggag gttttagtgt attcagtgtg 56100

cacaacaacc atcaccaaat ttaattttag aatgttttca ttagccctaa aagaaacccc 56160

acacccttag ccattctccc ccaatcctta tattcccccc atttctaggc aatctaattt 56220

ctgtctctgt ggatttttct attctggacc attttatata cgtggaatca tacactgtgt 56280

ggttttttgt gactgcttct tttacttagt gtaatgtttc caaggtttgg tcatgttata 56340

gaatgtatca gcatttgttt tcttttttatt accaagtaaa attccactga atggatatgc 56400

gacattttat ttattcatca gctgatgaat gtttggactg ttttaacttt ttggctattg 56460

tgaataatgc tgttatgaat attcacaagt ttttgtgtct cctcctgctg tggactactg 56520

tttccctaac agaacgtatt aatttctttt tgtacaaaag ccactccaaa gcatagtgct 56580

taaaacaacc actgcttggg tcaatgagca cactatattc tgcactgcac taactttggc 56640

tgtgctcatt catgtctgtg accagtggtg gttttaggta gatggctagt ctaggatggt 56700

cttgagtgga caacttggtt ctagtaaact agtccaggca tgttctcata gaaaggcaca 56760

ggtgagagag tgagcaagtt caattgtaca agaggacaag acttgcatat ttgcatttta 56820

agcttctctt tgggtcatgt ttgctaatac tacattacaa ggtatcacaa gagcaaggag 56880

aagcccaggg tcagaatgga aaggggattg cagagttacg ggtaaaagat gtgcctacag 56940

agaagccatt aattgggggt cattaatgct atgtctgttg cacataccaa ttttgcttct 57000

accttaatgc tttttgcatt tgtttccctt ctgtttgaac tagttttctc ttacatacct 57060

gcatgctgaa cttgtttcac ttccttccta tctctgttca tgtatcatct taccaatgag 57120

accttcaagt tttaccatac tacaaaatga atagccaccc accccgatcc tgctcacagt 57180

ctctgttttt ctctataact ttttatgacc atctgacatt gtctgttttt ttttttagcg 57240

tttatcttcc cttaactaga atgtatgctc tatgaggtca gggactttag tttaattaca 57300

ctgtcttcat tgcctagaac cctggcactc attaaatatt tgttgaatga aaaattaatc 57360

tacattgatc catttttacc taaattggaa ttatcttaaa gttctaacct gccttcatgc 57420

tatggcatgt ataaagtgtc agtcttttga ttagaatacc taatgatgga atatttcttc 57480

tacttcttat agagacatag accatgcaat atatgagtaa ttctaccatt tacttagctg 57540

tctgacttgg gccaagtttt ttttaacctc cctgtttcct catctgtaaa ataggggtaa 57600

taatagtccc agcctcatgg agcctggcac aaggtaggca ctgtataagg ttggtgcaat 57660

gtgtattgca taatattttt tatcacctac ttgttattgg agtatatggc acagggaaaa 57720

tggaattgaa agctggagca atcttatatt atggagaagt tgcaaaatct ttgaaaacta 57780

ttttttcaaa gggacaaagc acataaagca aatgaaatag gtgatttttt tttcattgaa 57840

tgcatcataa cattatatcc ttaaaacact gtcttacaaa gtgctctctg aaaaatactg 57900

ttctttattt cagggtttac gaaaaagtac ataaaactag tgagcataca tcttaactct 57960

ctgagagtct tataagtacc atcctagatg catggagaga agataattca ttacttacac 58020

taggtcatgc cttaggtcgt taagacttaa gtatctcatg gaaccccagt tgagaaagaa 58080

caaaaatata acatcttgac taaggtggct agtgcttctc tgatcttaac tattatttct 58140

ctagcctcag gtgagccttc ttgaatgtga tttcatcagt ctcctctaag cttacccaga 58200

atatagaatt tttaatattt tatatagcca gcacttataa actacctgct tcatgctagg 58260

tagtatactt aggtgctagg agtaaaactg atcaagtggg gtaagttcca aattcacaga 58320

gcgcacagtc ctaacaggct gtcatgcctc agttgtttat ttgtgacagt gtttcccaca 58380

gacttttaat tttggaatga taggatgtgg tgctctggaa tggtgtccca gggttttgga 58440

gaactgagag taacctggga aagcaggtta cagtgagaga aagttatgac caggagttgt 58500

accaaaacgc aggaaacatg atatgtatat tgttatggtg cataaaaata cttacaatta 58560

aaagtcctga acctacacta catctattgt ttcacatatt ttccttcaat ttaaatcctg 58620

ctccaaatac agctcctttc cgaaaattca gttttatgtg acagaaattt gatctgtttc 58680

actgaaaacc catttgaata gtaattgtga gcaattgttg aatttgtaaa tgttacgtaa 58740

atttagtgga tgactttgtg gacagtttga cgttatggac agttattctt gatactgttt 58800

cccctccttt cccctgccat ccctgaaact ttagggctta atctgctttt aattagccag 58860

aaaaaaatgt ttgatcctct tattcagttt tagattattt tagatgattt ctaagttaaa 58920

ctctaagtta gaatagtttc tgcttattga tttcatgaat atcacttctc tatttctact 58980

tctcaccttc tgccaggcca aaggaattgt ggaactatga tcatcaaact cctaaatcat 59040

cagccttttc cttaaaagac ttaaaagggt tctatctatg tagacacttg tctggctcct 59100

gattacagtt ttaccattct caagtgatac tatttgtttc atcacagtcc acataattca 59160

gggtcaagat actattatga aatgactgta aaaattacta atacctttgg ctctctaatt 59220

tttctctctt ttcatcatat gggcctgaca aaatgcccgc tacctgcctg cacttaggca 59280

attgaatgta gtgctttaaa tgtataccaa aaccctcaac agggcatcaa caatgtctgg 59340

cagtgcagct gcttcttcac tttcccaatt aaaaatgcgt ttgctggctg ggcgcagtgg 59400

ctcatgtgtg taatcccagc actttgggag gccgaggcag gtggatcact tgaggccagg 59460

agttcaagac caggctggcc aacatggtga aaccccgttt ctactaaaaa tacaaaaatt 59520

agctgggtgt ggtggcgtgt gcctgtaatc tcagctactt gggaggctga ggcaggagaa 59580

tcgtttgagc ccaggacgcg gagtctgcgg tgaaccaaca tcgcactact gctctctggc 59640

ctgggcaaca gaatgagact ctgtctcaga aaaaaaaaaa aaaattcatt tcctccactc 59700

attgcaaacc tctcacagct cccactccta tctgtggaat tcacttcaaa ctttactgag 59760

aaattaaatg cagctcttgt catcttttca ccaccaattc tacaaacttg tctgcattgg 59820

tcctcttgtt ctgtcttcct ttcatttgtt atcgaagacc attcccgaca gtctgatttc 59880

tctacttgtg actcagattt catctttcct gcatctctgt cttccatatc attcttctcc 59940

cctcgattaa attcccttta gccacaaaca tgctctatta tctcccagcc ccaatacatc 60000

tgcaaatgta tgtaaatagg aatgaccaaa tatgtacaga tatacaattc ctacctttcc 60060

taatgtcttc tagccactat tatgtgttcc tattcttatc taataaattt tcttaactga 60120

atattttttc cttctacctc agtgtctgtt tctttctttt cagccatttt tggtttctct 60180

tgtaaatgtt tatgatcccc agggctgagc cttcttggcc tatactctct tgtaggtctg 60240

cctcccagta tgctagtgac acccaaatat gtcttcagcc tcatctctcc tccagggttc 60300

taaacttgca taaccagctg ccttcttgat catttagtaa gcctctcctg ttggtgacat 60360

aatgcttgat ttctctccct catggcatca ctgtccaccc gttgcacaca tcagaatttc 60420

agaatcagcc ttaattctta gttttttctc actcttcctc catgtctaat ccagtagccc 60480

tatctccact gttttcacct tggtccaagc aatcatctct tgcttaacac aaccatagtt 60540

tcaactagtc ttcctccctg cattcactct tggcccaagc aatcatctct tgcttaacta 60600

tggttgctta acacaatcat agtttcaact agtcttcctc cctgcattca ttcttggcgc 60660

accgtaatcc attcttcact agagtgataa ttagaggtac tataatatat actgctctag 60720

agttatattg tctagaattt actagctgag tggccttgag caatatactt aacacttatg 60780

ccacaatgtg ctcgtctata aaatgggatg gtatatttat ctgttctctg attgctataa 60840

aggaatacct gagactgggt agtttataaa gaaaagaggt ttaattggct cacggttctg 60900

caggctctac agaaagcatg atactggcat ctgctcacct tctggggagg cctcaggaaa 60960

cttacagtca tggcagaagg caaagcagga gcaggtgcgt cttacatggc aggagcaagg 61020

ggtaggggga ggtgctacac acttgtaaac aatcagatct tttgagaact cactcatcaa 61080

aaggacagca tcaagaggat ggtgctaaac cattcatgaa agatccaccc ccatgatcta 61140

gtcacctcca accaggcccc acctccaaca ttggggatta caatttgaca tgagattggg 61200

tggggacaca gattcaaacc atatcagaag taaattgtta gtaaaatata aatcagatta 61260

tgtcacacat acctgttttt gaaactttag attctcattg ctcttagaat aaaattaaaa 61320

ctctacttac cgtggctttc aggatccttc ataacctggc atattgccta atttttctga 61380

tacccatctt gtttctactc tccccttgct taccatatag ccacagtcac tatctttaac 61440

tttctagttg gaaacatggc tttttgtggg gtgtatttcc ttttactttc tattgttttg 61500

ggaccaactt atttacgtca gttttgtgtt tgttttgtta tatgtaaaaa tagtgcctta 61560

aaaaatcagt tttttttttt aacctggtga atcatttgtt ttatctttat aaatgctggg 61620

acgagaggcc tactcctttt ttccaatttt gtgagagatt ggtaaaattc ttagaagtgg 61680

aattgctagg tcaaaagata gaaatgtttt cagatggatg attcctattg aagtaaattt 61740

tttgagaact taacatgtcc aaaaaggttt tcatttgccc tcatgcttga ctggtagttt 61800

gcctggatgt aaaattccag attcacagtc atttttgccc agacagtgaa aacattattc 61860

tactatcttt taaaaaataa cagttttatt gaggcataat tcatatacca tactatgtgc 61920

aatttagtgg tttttaatat attcatagag ttgtatagcc atcaccgcag tacattgtag 61980

aatattttta tcactcaaaa atgaaacccc tggatttatt agcagtcatt ctctgttccc 62040

accaatttgc ccacccccca gccttaagtc aacaagtaat ctactttccg tcttcattgt 62100

cttttaatgt tgctgatgaa aagtctgatg ccaaattaat tctagttata ggaagctttt 62160

agagttttcc gttttgaatt tctggaattt aattatgtaa actaggattt atgtagatat 62220

gagtctttta attcatccgc cttagcctca ctgagccctt ttactcagac cattggtgtt 62280

tttaactcca aatattttta gcataatctt tatttattct atttcttcca tttagaactc 62340

ttactaaatg tattttgaac ttcccaaaca tattctttat atctcttcaa ctttttactt 62400

gtatcaccca tttctttctt tcttgctctc tcttttttaa cctctctacc ttctgggaca 62460

tttctttgtg ttacatatca ctactatgat ctttatcaat actacttctg ttatttaccc 62520

cttctcctca agtgttttct tagtaataat atttcagttt tctccttgcc catttattct 62580

gtttgttcat tgtgatcttt ttctttcctc ccattggata ttcatagtgt cttgtgattc 62640

ttgtttattg aataaggact aaattgacaa aaactttgca gttacatagg tctaggattc 62700

ttttctaaat gggaaatgac tacaagtgtc atgtattcat tgactggctt tcctccaggg 62760

tgcagggatt ttaagaggca ggcttaaggc caccccagtt accaaagtaa ggacagcttt 62820

actatgttta ttcctggaag gagctagctt acttacttag tatcctcctc cttcagtatc 62880

ttccctcttt ccctctcgcc ctcccacttt tcctttctac ttcaagtata tagtaaagtg 62940

tagggaataa tataataact atgtacctat tactcagttt tgttgaattg tcttatatat 63000

gcttcagatt tttataaaca aaaaaattat agacacagat atagcacctt acagtcttac 63060

tttgatactt ttctctgccc tttctgcctt ttcagaagta accattatcc tgaatttact 63120

gtttatcatg cccatgcaca tttcagatac ttttgctgtg ggtgtagatc cacaaacaat 63180

ctagaacgtt gattgcatgt ttttgtgaac cctgaaagaa ccaacccttt aaggcagatt 63240

ctgagtggct aacagtccaa attcaaaata gacccacgcg atcctttgca gacatgtaga 63300

gatcatatgt gtactccgca ttcctggaaa acctatacac ccagtaactt taggactttc 63360

atagctgtct gttcctattt atgccacctg aattaacagc taccagaaaa taccatttgg 63420

ccttttgtac ctaacaaaca ctctgtgacc tgcctcagcc aatcagaact gaacaagttt 63480

gcacccctca tttgtatagt ggaccagagt gggaacctga ctgtgaactt tctctgtaaa 63540

tgacaacccc ttttctttgt tctctcagaa ggcgccttta ttttctacca aggtacatct 63600

ccacggtttg caaactgttt gctggaataa agcctgtttc ttttttaaga aagaaaatct 63660

ttttctgtag attgttgaca ttttaaaagt tgtatatatc ctgaaacttg ctttttcatc 63720

aatattatgt ttttgagatg tattcatgtt gataaatata gcgctagctt tttgttttga 63780

cttaatatgt ggtattttgt tatacagatg taccagtttg ccattttttc tctcttaggg 63840

aacatttgag ttggttctag ttttttgcta ttataaacat tgctgcagtg aaaatgtctt 63900

gtacatatgt gaatataagt agctacatga taggatatgc tcatccttag ctttactcta 63960

gatattgcca gtttactttc aaatatttat attataccac atgtagaata tgatagtgtt 64020

tgttgctaca tgtacatgat taccaactct tgtattatta ggcccaattt ttttgcagtc 64080

tcactgattt ttcatatgta tgtatgtatt ttttgagaca aggtcttgct ctgttgccca 64140

ggtgggagtg caataatgca ttcctgtctt attgtagcct caaacttctg ggctcaggca 64200

atcctcccac ctcagcctct cagcctctgt tagctgggag tacaagtatg tggtaccgca 64260

tccaactaat tttctttttt ttcttttttt tttttttttc tagagatggg gtcttgctat 64320

gttgcacagg tgctgggatt acagggatga gccattgtac caggcctggt ttttcattgc 64380

atttctttga ttactaatga ggtcatttat tttatgtgtt tattagatat tcgaattccc 64440

tatagtgaac tacctattca taagtctttt gcccattttc ttttggatta gttgaccttt 64500

tccaaaagga tggccactta ccctggaaat atttattata tagtccgttc tgttccccac 64560

taaattataa tgccaatgtg aaccatatgc tgtgtaaata aatacctgtg cctctgtttc 64620

tgagccctct attctgtctc tttgggcttt ctgtctctat gctagtatca tttctctgta 64680

agtcttgttt tcctggtaac accagtctta ccctccttat tattcaaaat agccaaaggc 64740

tactattata cagtgcttcc ataaaaattt tagaatcagg ctgggcgcag tggcttacgc 64800

ctgtaatccc agcactttgg gaggctgaag cggacggatc acgaggtcaa gagatcaaga 64860

ccatcctggc caatatggtg aaatcctgtc tctactaaaa atacagaaaa ttagctgggc 64920

atggtggtgt gcacctgtag tcccagctac ttgggaggct gaggcaggag aatcacttga 64980

acccaggagg cggaggttgc agtgagccga gatcatgcta ctgcactcga gcctggtgac 65040

aaagtgagac tccatctcaa aaaaaaaaaa aaaaaaaaat ttacaatcag tttttcaagt 65100

tccatggggg aaaaattatt tttggaattt tgtttattgc actacactga atttatttgt 65160

ggaaaattga catcattata atattgaatc ttcccataaa aggatattta tgtaggtctt 65220

tgtcaagtga aggggaacac atctgcatgc acacatacat gtcatttggt gtaatgtgaa 65280

ataaggttta gtggagaaaa ataaagaagt attaaggcac acacaattcc ctaaggtctg 65340

ttatccccat atagatgtgt gcactggcca gacacatcta gaactatgta ctgtggaaag 65400

ggctcctgtg taataattgg agctttgagg ttggtgaaat ggtcttacta gggaaagcca 65460

tcagtactct gtggctgatc agccttttct tgcatgaaac aaactcagac ctcctgagcc 65520

aactttttga taagctacca aaacaggggt ggaaacctct agagtcatct gactctgtct 65580

ctcttcttag tagccttttg tgcacctgca cattattttg ttgtttccgt cacaaaagtt 65640

ttgggaatct tttgttagac ttttcttcta gattctttat aatgttacta tcatgagaag 65700

catctttttt taattggctc tggctaatgt gttagaacac catgtaattt tatgtccttg 65760

attttatatc caacaaacct tgctaaactc ttctgttagt ttttatagtt aataggtatt 65820

agccctgatg gtttgtagat tctcttgggt ttttctgtag atagcatgtt tcctacaaat 65880

aagttttttg tctctataat tctaattctt atgcctcttt atataaatat atatttatat 65940

aaacacttat ataaacacta cctatactac caagattcaa tgattgttaa cattttactg 66000

tgtgtatatc tcctaagaaa aaggaaatta tttcatttaa ccaaaatatc atatgtagga 66060

aggttaaaat tctatcatct tacatctgat atgtgttcag atttccatgg ttgtcccaag 66120

aatgtctctt gcagctatct tccatcccta aactaagatc cactctgcat gcactgaatt 66180

atttgttgac ttttagtagt agttatcttt tagtcttaaa ctgaccacct actgttttgt 66240

tctcccatga aatgatttat taaagaatcc aggacagttg tcttagaatg tcccacattt 66300

tggattagtc caattgccaa ggctttgact ttcaaagaga agaaaaatgt cttagagaat 66360

gttccacaat ctatatttga ttatttcagt gtagcagtct tagcgtattt ctctattccc 66420

tgtgtttctt ctaaaccaga agttaagtat gaaggcttga ttagattcag tttaaatgtt 66480

ttgcatgtat acttcatggg tgatgtctca tgttgtatca catcaagagg catatgttgg 66540

gttgttccac tattaatgat tctaagccac ttgaaggtat gttttcccct tcatctttaa 66600

tctgtggcga aatactttgg cactatgtaa gtatcctgtt ctccatcagc ttttctccta 66660

atgattttta gcatcatttg atcctttcct gaaccaatta ttttatatta gctggcattc 66720

ttttgtaaag aagcatttcc ccatatcaag tgggaataac taatttttcc taaaaaggcg 66780

ggatacatgc ataatttttt tcatttaaag ttcagttttc taagggtgta ataatcccct 66840

acagtgtgag caactgctct cccacccttt taatttttat ggaattttga aggttttttg 66900

tttatttgtt tgtttttgca taccatctgt tatagtcaat tacagtactt attctttttg 66960

ctgaaaatct ccaaatttgg tcagtaggag tcccttcaag ctggcctctg atgttgttct 67020

gatatctggc acaagatgtt cctagatctc tcttcttgaa taataataga taggtttgaa 67080

atgctgtaat gctggccctt tcccccatta atgtagtcat tgtgttgttg ttactttgtg 67140

taatctaata atgcttattt tgttaagacc taatatgctt attaataaac tcattagtaa 67200

aacctaaatg cttatttagg tttactttca tgtatattag tctctagatt ctccatctgt 67260

gttcattttc tatggctgca ataataaata acaacaaact tggtcactta caacaccaca 67320

aatgtggtgt actctcttac atttctggag gtcggaaatc cagaatgagt ttgttttact 67380

gggcaaaaat caaggtatca gcaagacttt gctctctcta gagacaataa ggaaaaatct 67440

gatttcttgc cttttccagc tcctagagct gcactgcttg tatttcttgg ctcatggcca 67500

cctcctccat cttcaaggtc agcagtgtag catcttcaga tctttctctg ctgagttttt 67560

atatcaccta atcatctata gtaaaattcc cttttatttc cctcttataa gtatgcttgt 67620

cgattacatt tagggcttac ccagatatcc agaataatct ccctgtttca caatacttaa 67680

tcacatctgc aaagttcctt ttgccacata ggataacatt tacaggttct tctgggtctt 67740

ggtatatgtt tagttatctt ggtggggggc cactattcag ccctctcatg ccactctttc 67800

tttgatatta cttcttgatt cagtttcctt tttctaggtt gtcagttatt ttcttcttgc 67860

accttaacgt cagttcatta tcttcttgct tcttttactg atgttgagaa gtatgccatc 67920

agtctgactt gtctttactt tctggttgac tttaagaact gaactttttt attttcacct 67980

gttttggaaa atttatggcc agcttctgtt caaatatctc cccattccct gtttacctcc 68040

cagattaact cattttgtgc ttcgtgtctt ttcatatttc cattcattta tctctgcctt 68100

ttggctaatt ttctcagaat caccttctag tcctttaatt ctctcttcag cagtaatctg 68160

ttttacgtta cctgaggttt taatttgatg actgtatgat tcaaaactca tgtctttata 68220

gtttcctcat gcttcttctt tatatgtgtt gtctttaggc ttgctgttct tccagagtta 68280

tctcagtaga aatttacttt tgtgttttgg tctgcaattt attagctttg atttctttgt 68340

caaactgatt atattcgttt tctgtttttc aggaatgttc tgaatttttc caactaagtg 68400

gttgatggtc ctggcctcct tgctttcagt actgtgattc tttcaaccat tttcttgaaa 68460

ttttaagacc acttctgtct ctcccttccc tccctctgtt cctcccccaa ccccaccttt 68520

cgtaacaatt atttgtttct tggaatctct taggctagtc ctgtagtttt cccctttgac 68580

ctattaagta ttatgttact ggattcctaa ttaatgatta ttcttgcttt taatatatat 68640

atttatatat actattatat atgtttatat atcatatata ttaatttaag agtttgatgc 68700

ctttcttcac agtttggatt attttatgtt ttgtttgttt gtttgtttag agatagtctt 68760

gctctgttgc ccaagctgga gtatagtggc gtgatctcag ctcactgcac cctccatctc 68820

ccaagttcaa gagattctcc tgcctcagcc tcccaagtag ctgggactac aggcacgcac 68880

ccacacctag ctaatttttg tacttttagt agagaaaggg ttttgccttg ttggccaggc 68940

tggtctcaaa ctcctggcct caggttatcc acctgcctca gcctcccaaa gtgctgggat 69000

tacaggaatg agccattgta cgtggcctat ttttttttat gttatctttg ttgagttttg 69060

acatctaggt tttgctagct tcttaaagta atttagaaga tatataagta tatatatgaa 69120

tatatatata aaatcacctg ttactaattg atattttaat tacattttct ttaagggatg 69180

aggggagcca atggctgtag tttttttttt gtgccatagc tccccttcat gtccttccat 69240

agcagtttgt ctgcatttat attttaacat gggcatcttc taggattttt taaaaaaaat 69300

tctcagtctt ttttttggag aaggagtttt gccatttgta aagtcactaa aaatgatcag 69360

ttatttacta agcttgcttg gctgtaatag ctgtggtcta ctgaagagat taaaacagga 69420

gaggagatct tttagtaact ttcttgaagt ttttagtttt gtttttaatt ttttcatctc 69480

ccttttagga ttgtggtaac aggtttttct tttttttcca agaagatgtt ttaactatgt 69540

acctaccgtt agcttttcag tatcttattc tactttttaa tatatctcta ttttataaga 69600

gtcatcaaag ttctttctct gactcttctg gagattctga aagactttct ctcccaaaca 69660

cattctttaa aattccaaca agggtcattg tgactttttt ttattcttcg gatataaaat 69720

tactcttctt tggggagata aaaaacctga cttccagaca cttatggtct ggccccacta 69780

tacctagcca atattatcat tttcttttac tttttttttg agacgaagtc ttgctcttgt 69840

cacccaggct ggagtgcagt ggcgcagtct cggctcactg caacctccgc ctcctgggtt 69900

ccagtgattc tcctgcctca gcctcctggg tagctgggat tacaggcaca cgccaccacg 69960

cccggctaat tttgtatttt ttagtagaga cggggtttca ccgtgttggc cagctggtct 70020

cgaactcctg acctcaagtg atccacccgc ctcatcctcc caaagtgctg ggattacagg 70080

tgtgagccac cactcccagc ccaatcttaa ccgtttttta taccccagca agaaaatttt 70140

attctaatca gctcattctc ttcatttctc atctctgatt attttccccc actttctctc 70200

tcccatcctt tgagagaaat agttctttat gctaactgca tatgagtttt tatggaacat 70260

tgaaaaaaaa aagggagtgt gggtaatatt gtcctgaatt tactggacct aagaaagaat 70320

cactgaacta ggatgacctg ttttctttcc taccctattc tgaaaccagc ttaaatcttc 70380

agctttaatt gcatacctat tatgactaag acaccaaaat aaatcatgag gggacaaaaa 70440

aagaaaatgt ttctgatctt aaagtagcta atagacatgg aaaacataat caactttatg 70500

ttatcaaatt atgacttaga aaacagtttt gccgagttga tcagtgaaca catcatagag 70560

ggatgctcta aggaaagatc catcctggac aatgtggcaa aaccccatct ctactaaaaa 70620

tacaaaaatt agccaggtgt gctgctgcgt gcctgtagtc ccaactactt gggagtctgg 70680

ggtgggagga ttgatgccag ggaggctgaa gctgcagtaa accaagattg tgccactgca 70740

ctccagcctg ggagacggag accctgtttt aaaaataaat aaagtattca ttttttttta 70800

aacaaatata tgtttgctta ttgtatgcta ggcactgttc taggctctgg ggataaatca 70860

gaaaaaataa gacaaaaacc tatgccctcc tggacttcat tctgttgggg agtgggaaga 70920

cacacaccaa taacaaaatg aacacgtaaa atatatatag taaggtggtc acatatgcta 70980

cggaacaaat aaaattaaca aagggcatag aagctgattc tgtgccttta aggaatggga 71040

gggatagagc ctttctaaat gagaaggtaa gagaatgctt cattgataag gtgacattgg 71100

gataaagaga tgaatgaact tggtaagaca gcaaacttta tagaaatctt aggggaagag 71160

cattccaggc agaaggaata gtaaattcac aagtcctgca ctgggagtat acttggcacg 71220

ttcagttagg ccaaatgtga ttggaatggg atgagtgacg ggagaacaat aagaaatgaa 71280

gctagacaaa tagcagaggg ccaggtcatg gctttggtga tttttagtgg ggaagccagt 71340

ggagggtttt gagcaagaga atgacatgtg atctgcagtt atagttttgt ttttgagatg 71400

gggttcactc tgttgcctag gttggggtgg cgcgatcaca gctcattgca gcctcaactt 71460

cccaggctca agcaatcctc ccacctcagc ctctgggacc acaggcacac accaccacgt 71520

ctggctattt tttttttttc tttttttttt gtagatacag ggtcctacta tgttgcgatc 71580

ctcccgtctc agcctcccaa agtgctgaga ttacaggcgt gagccaccat gcatggcctg 71640

cagttacagt ttttgaagaa tagctactgt gccattctag gcatgaaata ggagaggcca 71700

tttcagaaac tcttgttaat aaactaacag agaaatgaag gtggcttaga ccagggtggt 71760

aaagttagag aaggtgacta atatctacat atattttgat ttggagatgg attgaaatgg 71820

attgtcagat gtgagaaaag agtcaaggat gatgctgaag tttttggttt actcaaagaa 71880

gggagcttat ttcctgaggt aaagagacta ggaaaaacaa gttttgtggg ggtgatgggg 71940

gagatcagaa gttagtcttt gaacatgtta agtttgaaat gcctattagg tatactagta 72000

gacatattca gcaggcagtt aggaatctgg tgttgagagg aaaaggtaga gttggatttt 72060

aaattttggg cttcatcagt tcatagttgg catataaaga tacaagattg gatgagatca 72120

cctagggagt aagtgtagct tgacaaaaga agccatctga cactttttgg agatatgaaa 72180

aattagcaaa ggcatcatga aagaaaacat tgaaatacct gactggaaaa acaatttgga 72240

acctctatca ctgcttctca gactttaata tgcatatgaa tcattttgtt aaagttcagt 72300

agatctgaga tggagcttga gcttctgctg tgtgttttgt ttgtttgttt ggtttttggt 72360

tttttttttt ttttggcagg ttgtggggtg gcggtagaaa ctgagtctca ctatgttgcc 72420

caggccggag tgtagtggtg cactcatggc tcaccgcagc ctcaacctcc tgggctcagg 72480

taatcctccc acatcagcct tctgagtacc tgggactaca ggcatgcacc accatgcccg 72540

gctaattttt gtatttttta tggagatggg gttttgctat gttgcccagg ctggttgtga 72600

actcctgggc tcaagcaatc cacctgcctc ggcctcccag agtgttggga ttacagatgt 72660

gagccactat gctaggccag cttctgctgt tctcacagtc tttcagaaga tgctgctgct 72720

tctggtctgt ggacccacac tttgagtagc aagactttat atgacaagag tcatgaaaaa 72780

caattgaaag accttaacat ttgtagaaga ataaagatta atatccagaa taaagactat 72840

ctataaatga atatggaaaa aacaaacaac tcaatagaat aaaagggagt gcagtatagg 72900

gaattttcca aaatctcaaa tggccagtaa gcataaaaag tgctcaactt tgtaagaaat 72960

acaaattaaa acatgattgc cttttccctt tcagactggc aaaagtttag aagtgtgatt 73020

gtgctgggtg ttggtgagat tgtggggaag agtggactta taccctgcag atagggactt 73080

gacttgatac atttcttctt ttggaagacc atttggcata tttattaaat tttaaaatag 73140

gtatatcctt caatcctgga atcctatacc tagataccaa aaaataaaac ttgcactttt 73200

actctgaaaa gcagttcaga actatttgtt ggagtattgt ttgatgttga ggaaatggaa 73260

taatctaaat gtcctttagt aaggaaatta ttaaatcaag agatttggaa ctcttttttt 73320

tcatttattt cccagtacat ttaaaaattg atatctgaca ttttttcatt ataactttga 73380

atagtttaaa aggccataat ttctactgtg tgttatattt atgttaaata cattttttgg 73440

acaagcctta aagctgcaga tttagatcat tcaacttaga aacagaatct tccgtataac 73500

ctaatagcca gttctcacta tcaaaccaga caaattggac tgtttttctt ttttattaag 73560

aaaaaaaacc tgattactta tttatcttaa aacagatata ctaatatatg ccttttaata 73620

accactaaac ttctggattc tagtctggct ggctggtgat gggtaaggct tggagccttg 73680

ccacaaattt gtttcattga taaaatatgg tcctgctctt aatttttccc ccttttctct 73740

caaggaattt ccctatttaa ttgtctattt gttaggtact ttggaattta tacgtctcag 73800

aatggtgcat cttagtagta tttgaggtgg aaaagaactt tgcctttctt ttataaagtg 73860

gaaaataatt attttaaaag aggaagtaga caaggagaac cagttcttaa gcagatcaat 73920

cagggagcat acagataaaa cttgaggatc tggaaattct cttaaaattg tctatgccca 73980

cctaacccct ggataccact gaagtttaga gactgttgaa ataagcagtg caatgctatg 74040

aaatgaatgc tatataacca ttagggagta aaatatatgc atattgacgt ggagagactt 74100

ctgagacatt actccatgaa aaaagcaagt gttaaaagta ttttatatat agtatttaat 74160

tatatgtgta catttcatat atgtatgtct taatgttttt tccatgagta aaagggagac 74220

atggaaggct gctcaccaaa ccataaacag aacttaacct ttttgaaagg gggcctggga 74280

atgtgaggaa gatgaagaga gatttcatgg tttattctga atattcatgt attctttgaa 74340

tcttttatag ttgagaatac gttgtattac tagtgtagaa aaaaatttaa agatgtttca 74400

taataaaaga gaaaaaccac tgtactaaat gatctctaag ctcaaaattt gaggaacact 74460

ggcagttact gtgatgtagt tgtctatgtt aaaatatttc taattcatat actaagtgat 74520

ttttatttaa attcttttga aatatttttt aggtgtggaa tatcaaacaa atgattaagt 74580

tgacacagga acatatagag gccctattgg acaaatttgg tggggagcat aatccaccat 74640

caatatatct ggaggtaagc ttttgagtat catatctagt aattttgaaa agaaaaaaaa 74700

tgaacttata aaaacatttg tactgataat cttgattatt ttaggaggtg ggattaggaa 74760

tagatttggg aagagagaat tagctttgcc tgtattatac ttcttaatct tgttttgtgt 74820

cacttgttaa aataaacatg tattttataa tttagaaaac atcagaacta agggggaagt 74880

gatataaatt tcaaggtatt actctgaatt aaatttttat ttttattttt attttattta 74940

tttatttttt tttgagacgg agtctcgctc tgtcgcccag gctggagtgc agtgacacaa 75000

tctcagctca ctacaacctc cgcctcccag cttcagacga ttgtcctgcc tcagcctcct 75060

gagcagctgg gactacaggc gcacattacc acgtccacct aattttttct atttttagta 75120

gagatggggt ttcaccatat tggccaggct ggtctcgaac tgactttgtg atccacccac 75180

ttggcctccc aaagtgctag gattacaggt gtgagccact gtgcctagcc aatttttttt 75240

tttttttttt aaagagaaag cacacagttt tggagctcag caaaccagtt accatattgg 75300

aaaatggggt aattttttaa aatatttttt ccagaatgag tttccccaaa attggtttct 75360

gcttccttat aggtaaaatt tgtaattttt aaatagctca acattgttta gctttactgt 75420

ttaataatgc cttgaggttt gtgtcaagcc ttgagcataa ttgtaaattt atattcatta 75480

atcctatgag ttagtaggtg ctgttataat cacagatgag ctttcttagc attagttttc 75540

ttaagtagtc agtcagactc cacagccaac aaggtgttac agaatctagg tggtaaggct 75600

ctacagccca cactgctcac tactacccta cacacccttt ctttataaaa gctggctcag 75660

ctgtcatcac tagtgaggca aggagatgga gaactctaaa ttagaaatga tcaggttgcc 75720

atttggctcc aaataccaga gatttttaag cacagattga aagacttctg aactaaattt 75780

gtaacttagt aatttaatta ctttttgaag ggccagttaa gtcttaagga ttttatgaga 75840

aagttaagtt tatatttctg aagtaaactt ggaagtttgg aaataacagc taatatgctg 75900

ttttctttct cctcttcttc atgtggctca tcccagatca cactcctagt taatatcaaa 75960

gctaggacta gaattcttat ttctcattac tagttagtgt acttcctaat agcctgtgct 76020

atcttctata catatattat taggaggctg atcaaaggtg tcataaggaa gcaagtactg 76080

ctaatgagaa tggcaatttt ttattaatca gaattagaca atataaccag cagtttttct 76140
tccaaccatt tctatgtctt ggagactttg aacccatgct gagatgtaat tcatttgcat 76200
caatagctat gtccccacta tagcagtgat tcactgcccc ctcctcacat aactcttcag 76260
gctttttact atttgaagac tgatcactaa caacaaacct tagagcttct tattctgtgc 76320
ccagtactgt gttaaatgta ttttattgct taactcattt cttatagtta ctttgagcaa 76380
gatactaggt actggatatt tttgttattc ccattttaca agtgaaaaaa cagagaggtt 76440
aagtaactta actaaggtga cactgctagt taggaaagaa ggcagatatc taagtcagaa 76500
atctgtgttc ttgatctcta tcttattcta cctttatcat gacattgtct cgctctttcc 76560
ccaccctcac cccccagaca gcttttctta tttgggctaa atatttctat tcccttcagc 76620
cattcttagt aacataattt atggaccttt tattagatac agtctttttg gttggttctt 76680
ttagaattag acacacaaaa ttgaactact tattaaaatt aaatctgagt aggacagcat 76740
acatcagacc ataactcaca ggttatatta ttagcttgtg gtaaaacaga ttcttatctt 76800
ggggacttgt ggtatacaat gtgtgtgtat tatgtgtatg tagtatgcaa aggtatgcat 76860
atacacatgt acatacacac acactgaagg cagcagtata atataataat tacgatgagc 76920
catggagtca tacagactcc atgtttgaat cccaactctg gcttagtttc tgtactcaag 76980
atatccagcc ttcttatacc ccacctcatt agtaaagtgg aaataatatc acatcttggg 77040
taagttaagg attaattgag tttataatta tgaaataaca tagttcctga ttcaaatatt 77100
aagaattcta taaatagttg atttatcttg tactgctcta ggccttgggg aatagcaatt 77160
gtctgtcttt ttttttttcc tcctgagatg gaatcttgct gtctcccggg ctggagtgca 77220
gtggcacgat cttggctcac tgcaacctcc acctcccagg ttcaagcaat tttcctacct 77280
cagcctccca agtagctggg attacaggca tgcaccacca tacccagcta atttttgtgt 77340
ttttagtaga gacaggtttt cagcatgttg gccagtctgg tctcaaactc ctgacctcag 77400
gtgatctgcc ccccttggcc tctcacagtg ctgggattac aggcgtgagc caccgtgcct 77460
ggccgggaat agcaattttt taaaaaatgg ccaacaatct ccgccttttt aagcttatat 77520
tttaatgagg ggatggatat atagacaatg aacatataaa tgagttttag aacattgcac 77580
ttatcaccat tttatttttt tattttttgt tgagatggag tctcgctctg ttgcccagtc 77640
tggcatgcag tagcacgatc tcagctcact gcagcctcct ctcctgggtt caagtgattc 77700
tcctgcgtca gcctctcaag tagctgggat tacaggtgtg cgttatgact cccgactaat 77760
ttttgtattt ttagtggaga caggtttcac catgttggcc aggcttgtct caaactcctg 77820
acctgaagtc atccacctgc ctcggcctcc ctaagtgcta ggattatagg cgtgagccac 77880
cgtgcctggc catcaccatt ttatatacta tgaatggact ctaaccagcc caagagaaaa 77940
gacctgtaca tactgatatt taaaggccca tcagtgaaga gctagatcta ctgattactc 78000

actgagaccc accgttctgc aggtttctca tctgccattt aagattcctt tacacttttt 78060

ttttttattg gggtaaaata tgcctaacaa aatttaccat tttaaccatt tttaagtata 78120

tggttctgtg gcattaagtc cattcatgtt tttgtgtatc cttcaccacc atccatttct 78180

agaaatttta tcatccaaaa ctgaaactct ctagccttta aacactaaat ctctatttcc 78240

ttctttctct agttcgtagc aaccaccatt ctactttcta tctctatgat ctatgaattg 78300

gactactcta ggaacttcat gttaatggaa tcatataata tttgtccttt tgtgactggc 78360

ttatttcatt tagcttaatg tctttgaggt ttatccatgt tgcaacaagg ataatgcata 78420

tatcaaaatt tttttccttt ttaaggctga ataataatcc attcattgta tgtatttacc 78480

atattttgat tatccattca tccatcagtg gacacttggg ttgcttctac gttttggcta 78540

ttatcaataa tgctgtgaac atgggtgtac gagtatctga gttcctttta cttctttggg 78600

gtatatacca ggagtagaat tgcaggatca catggtgatt ttatgattat tttttctgga 78660

gttgccacac ctttttctgt agcacctgcc ctgttttata ttcctactgg catcgcatgg 78720

ggttctaatt ttttacatcc ttaccaacac ttattatttt cggttgatta aaaaaaccat 78780

agcctaggct gagcgcggtg gctcacacct gtaatgccag cactttggga ggctaaggtg 78840

ggcggatcac gaggtcagga gtttgagacc agcctgacta acatgatgaa atcctgtctc 78900

tactaaaaaa tacaaaaatt tcaggtgtgt gtagtggtgc gcacctgtaa tcccagcaat 78960

tcaggaggct gaggcaggag aatcgcttga atccaggaga agaggttgca gtgagccgag 79020

atcatgccac tgcactccaa cctgggggac agaataagac tccgtctcaa acaaaacaaa 79080

acaaggcaaa acaaaaaaaa catagcctca tgtgcgtgaa gtgatacctt attatggtgc 79140

tgatttgcat tttcctgatg actaacaatg ttgagccttt tattatgtgc ttattggcca 79200

tttgtatatc ttttttgaga aatgtctgat ttaagttctt ggtccatttt ttaattggat 79260

tgtttgtttt gttcttgtta ttgagcgtag ttctttatat agtctagata tcagttctta 79320

ttggatattt aatttgcaaa tatttttccc attgtgtggg ttcccttttt actctgctta 79380

tagtttttta gtccaaatta tctatttttc tttttgttgc tcttgccttt gatatcatat 79440

ccaagaaatc atcaccaaat ccaatgccat gacattcttc tcccaaatgc tcttgcattt 79500

gggtctttga tccattttga gttaattttt atatgatgta aggtaagggt ccaacttcgt 79560

tcttttgcat atggataccc agttttctca gcatcatttg tggaaaagac tgaatggcct 79620

tggcaccctt gtcgaaaacc atttgactat atatgcaaga gtttatttct gggctttcta 79680

ttccattggt ctatatgtct gtctttatgc tgtcatcata ctgttttgat tacactagct 79740

ttgtgtagta agttttgaaa tcaggaaatg tgagttctcc aactttgttc tttttcaaaa 79800

ttgttttggc tattcaggat cccttgagat tccttttttt tttttgagat ggagtctcac 79860

tttgtcaccc aggctggagt gcagtggtgt gatcttggct cactgcagcc cacctcccag 79920

gttcaagtga ttctcgtgcc tcagcctcct gagtagctgg gattacaggc acctgccacc 79980

atgcctacct tttgtatttt tagtggagac ggagtttcac catgttgacc aggccaatct 80040

cgaactcctg acctcaagtg atccacctgc cttggcttcc caaagtggtg ggattacagg 80100

tgcaagccac tgtgctcagc ctccatgtga attttataaa ggatgtttct ctttctacaa 80160

gtaggtcatc aggattttga tagggattgg attgaatctg tagattgctt tggttaatac 80220

tgatatctta agaattctta actctgaaat atgagtggac agttaagaat aaccagatat 80280

ttaaagaaag ctgtcaacag gaaagacagc agcagaaatg gtcaacagaa actcagaaac 80340

agtgcagaaa acagaataaa acttaaaaat aactataatt agcatcagag ataagatcta 80400

ttagtcctgt cttgctttgt agcaatacta aagaagcaaa agtaacagag aacaagaaag 80460

acctcttgga aattaaaaat acaatagcca gaaatttatt tttattttta ttttttttta 80520

attttctttt taattctcag caaggcaagt tacgtctata gaagggtgcg cccttacaga 80580

tggagcaatg gtgagcgcac acttggacaa gggaggggaa ggggttctta tcccatatgc 80640

atgtggctct tgctgctgtg tcattcccct gttggctagg gttagactgc acaggctaaa 80700

ctaattccga ttaataacta gaatttttta atttttatta ttatttttct ttttttgaga 80760

tggagtcttg ctctgtcacc caggcgggag tgcagtggtg cgatctcggc tcacttcaag 80820

ctccacctcc cgggttcacg ccattctcct gcctcagcct cccgagtagc tgggactaca 80880

ggcgcccgcc accacgccca tctaattttg tttttgtatt tttagtagag actgggtttc 80940

accgtgttag ccaagatggt ctcgatctcc tgacctcgtg atccgcccgc ctcagcctcc 81000

caaagtgctg ggattatagg catgagccac tgtgcccagc ccagaaattt ttaaaaatat 81060

atgtatatac agacatatag gtatgtacat gtgtatatat gtatgtgttg tatatatata 81120

aataaatgtt gaaaaataaa tattggaaga taaatgttct agaaagcaga gtaaaagcag 81180

agagatggga aattttagag gaaacatgag aaaaattaag gaataaatcc caatccataa 81240

aaattataaa tgcgtttaac tgcaagaaac agaacccttc aagaataagt ttctggaaca 81300

aatagggatt ttcatttcct ttcacatagc aagaagcgta gaggtaaatt attgtaagac 81360

tagttctaca caggtcagtt gtcagatact tggttgaatc gctatagtcc ctttagcttt 81420

tctgtcaaga ccacagcaga tttttaagat ctttccctca tacaactcct caaagcagaa 81480

agcagagaac agagaagttc tcttgtatac taagctcttg ctaggaagaa aatctttctc 81540

aaaaatcctc tataactctt ccttttatat ctcattgact agaatgaggt cacatatcac 81600

ccctatacta gtgactatgc ttacatttcc tgaagtaaga gtctaataag tcaaaaattt 81660

ccagtgttca gtttttcttc agattagttt gtatgctgtc aagtagaagt tgtgctatga 81720

ttattttatt ctataacatt tcaacagaaa ccccttttaa gtttctatag gaagataaat 81780

cttcaactac ttattcttgc ttcttctctt cattgtagca aaacagagaa attggttaca 81840

ccaccatcag aaatcctctt gattttcttt ttcccacgta ctaatttatt taaaagccat 81900

cttttcagtt ctagatgttc tcctactggc agataacagt taacattcta ttgattccct 81960

tgatgataac tacagtgaaa tatgagatac agtactctaa agaatttcaa atttgtcttc 82020

tcagttgatc tttggttccc ttttttctac catcatccaa aggaagaaaa gataaattta 82080

ctctgaaaag ttgttcccca gagtttcttc atttcctctc agtgtttcca tattcattta 82140

tccaacaaat tattggaatg ccttctatgt gtaagatata atgctagatg cttttctagt 82200

agtagattct tctttctgta cttgatttat tggagcccag agatgaaggg atactgtctc 82260

tgtaatcacc ctacagctga gtcctaacta cttcagaatt tatggttgaa tcctacagtc 82320

ccctgtagac tgatgaagac agtaacagct cctgacattt actgagtatt tactatgtat 82380

caggtactaa tcatatatta gttcatttaa tcttcagcct accctttgag ttacacttaa 82440

ctattctcat tttcatttta taaatgaagc aattcaggca cagagggatt taataatatg 82500

gccaaggtta cacaacttgt aattggtagc caaggtttga atcccagaca ttctgaattt 82560

acagcccatg cgtttaatca ccgtatcatt cttacacttg gcagcctttc tgatttttag 82620

tctatataga acctagaata atacagaggc attgtgtcaa acccttcaat gaaattaata 82680

ctggaagctg gatgcttcct gtggaatgca gaacagtcca ttatatatca tttatgggca 82740

gtttgtaaga tttcattgta tcttgtgaga gtaagaataa ttagactaaa tttaattaac 82800

taaatgataa aaaataaaat ttttatttcg ctgctttttc tcattacatt gaaattaaat 82860

agatccttag atttttttcc cttctgtttt aattgagaaa taattcacat actatacgat 82920

taacttttta aaactgtgta attcagtggt ttttactata ttcaccacta tcttattcca 82980

aaacattttt atcactcccc aaaagaaacc ctttatgcat tagtagtcat tccctgcagc 83040

ccctgacaac cactgatcta ctttttgcct ttatggattt gctggttgaa atatttcata 83100

taaatagaat tatattatgt ggccttttgt gactggctgc ttttacttaa tgtaatgttt 83160

ccaaagtttg tttatgtagt agtatgtatc agtacttcat ttcttgttat ggctgaatat 83220

ttcatcatac acatatatca tgttttgttt atccattcat caactgatga acatttgggc 83280

tacttatatg tttttgccat tatgaataat gccaccatga acattcacgt gcaagttttt 83340

gtgtagatat attttcattt ttcctggctg tatgcctagg aatagaattg ctgggtcttg 83400

tggtaactct gttttacact ttgaggaact aactgccaga cagtttttca aagttgctgc 83460

actattttgt attcctaaca gcaatgtatg agggttacag tttctctcca tcctcatcaa 83520

cccttgttat tatctatttg tttttttaat taaagccatc ttagtaggtg tgaattggta 83580

tttcatttgg ttttgatttg cagtttccta atgactaata atatagaaca tcttttcatg 83640

ttcttgttag ccatttgtat atcttctttg gagaaatgtc tattcaaatc ctttgcccac 83700

ttaaaaaaac tgggtttgtc cttttattac tgagttgtaa gagttcttta ggccaggcat 83760

ggtggctcac acctgtaatc ccagcacttt gggaggctga ggtggatgga tcacttgagt 83820

ccaggagttc gagatcagcc taggcaacat ggcgaaaccc cgtctctaca aaaaatacaa 83880

aaattatcca ggcgtggtgg tgcatgcctg tagtcccacc tgcccgggag gctgagatgg 83940

gaggatcgct tgagcctggg aggcggaagt tgcagtgagc caagatggca gagtcaccca 84000

ggctggaatg caggggcacc atctcgactg actgcaacct ctacctccta ggctcaagcc 84060

atcctcccac ctcagcctcc cgggcagctg ggactacagg catgcgtcac tgtgcctgga 84120

taatttttgt attttttgta gagatgggga tttgccatgt tgcccaggct ggtctcgaac 84180

acctggactc aagcaatcct cccgccttgg cctcccaaag tgctgggatt acaggtatga 84240

gccaccgcac ctggccagga gttctttata tattctagat agtagatcca tgacttgcaa 84300

atattttctc ccattctgtg gtattttttc ccactctttc aattttgtct tttgaagtac 84360

aaaagtttaa atgtggatgg aattccaatt tatctatttt aaagtttaaa tgtggatgga 84420

attccaattt atctattttc tgtggttgct gtgcttttgg tgtatcatac atgagagacc 84480

attgcctaat caaaggtcag gatgatttac tcctgtgttt ttttcctaag agttttatag 84540

tattagttat atagccaaaa caggtttagt tgcttgctgc ctgcagagtc caattagtaa 84600

gagcaaagtc tagtataaag tgactttttt attccaaagt tagcttaaag gaagaagacg 84660

tacaggcttc ctgccttaag ggtactgctt ccctgttgga gcagaaagtg ggtgctttta 84720

aagaaggtgc ctacacgggg gcagaaatga gcgggtggaa gatctgcata ttcccttcgg 84780

tgccttcttt ctcaggcagt caagttggtg gcttcatggg caaaaatacc tcagaggtgg 84840

ctgaaaactc tagcagtctt acttttggtt gtagatcaac tattacctct tgaggcaact 84900

tcctgacggg tgagagttcc actcaggatt gtctaagcac ataattagat caacttgcct 84960

tgtagggaat gtctggtgaa aaggagataa aaggccataa ttgcatttct tttattcttt 85020

tatctttttc tttttgaaac agaatctcat tctgtcactc aggttggagt gtagtggcat 85080

gatctcggct cattgcagcc tctacctcct gggctcaggc gatcctcccg cttcagcctc 85140

ctgggttgct gggactatat gtgcatgcca ccatgcccag ccaagtttca tatttttttt 85200

agagatgggg tttcaccatg tttcccaggc tagcctcaaa ctcctgggct caagtgatct 85260

gcctgccttg gcctcccaaa gtactgggat tactggtgtg agccaccacc cctggcatat 85320

aattgcattt ctaaagagct aagtaggaag tggggaggag gaggaaagaa aaaaataatt 85380

aaactttttc ttagaaaaat gagggtgctc aattatataa tagatatgtg acccattttg 85440

ttttgttttt aatttttgta aagatggagt ctatgttgcc cagtcaggtc ttgaactcct 85500

ggcctcgagt gatcctccag cgttgacctc ccaaagagct gggattgcag gcgtgagcca 85560

acatgcctgg cctatttcga gttagttttt ggatatgttg tgaggtagta gcccaacttc 85620

attcttctgt gtgtggatat tcagttgtac cagcgccagt tgttgaagag accattcttt 85680

ctgcattgaa ttgtcttgct ggctttgtaa aaaaaaaaaa tcaattgact gtaaatgtaa 85740

ggttttattt cttgttctat tgagaaataa ttcacatact atattattca cttttttaaa 85800

gtgtgtaatt cagtggttct tagtgtattt acacaatgga caactgtcac cactatctga 85860

ttctaaaata tttttatcat tcccacaaag aaacccttca tgcattggaa gtcattccct 85920

ccagcccctg acaaccactg atctactttt tgcctttatg gatttactag ttgtaccttg 85980

ttctattctg ttctatatgt gtgttcttat tccaaaatta ttgcgctgtc ttgattacta 86040

tagttttata gttaagtttt ggaatcagga agtataagtc ctccaacttc tttttctttt 86100

tataagattg ttttgataat ttcaagtccg ttgtatttcc atatgaattt taagatcagc 86160

ttgtcagttt ctacaaaaaa aaattgagat tttgagaagg attgcattga atctgtagat 86220

caacttggga agcgttgtca ttttaatgat attaagtctt cttagattct tttttctttt 86280

ttgagatgga gtcttgctct cgttgccagg ctggagtgca gtggcgtgat ctcaactcac 86340

tgcaacctcc gcctcccagg ttatcaatta ttactaatac aaatatttat tggatttttg 86400

ctatgtggta gtctctacaa taatttgttt tacttgtgtt atctcattta atcctcacaa 86460

aattattttg aagttgagaa aaatttgcca tataaagaag agtatgataa aatattctaa 86520

aggagataaa gagaaaggat acctcttctg gattttgaca aagagagctt agaattagac 86580

tttttgaaaa atatttagag ttttgatgag aaggaatagg gtttttgaac aactgtctcc 86640

tgaagaagtg gtatctggta tctgcagcat cttgaataac caaaagaaat ggtggtggtg 86700

gttgtggttg aggtttgcac atatgttgga gtggcttgct catattagta gagtgagaaa 86760

acagaggaaa agggaaaagt agatgatatg agagaaaact gagggaatgg tagactaagg 86820

ccctaaacac ccaatattag cactgaagtg agagtagaag aagaatgggg aaagatgtgg 86880

agaattttgt agataaagaa gtagagtaaa cttttcattt gatgactttg atcttcagag 86940

tggatttgaa ggtaatgtta ttttcagagt gaattaaaag ttgaaggatg aagttaagat 87000

ccagagaaga attaggaaga ttggaataac tgttttgact gtaatatgat gttaaaaaca 87060

agcatgattt tcaggcagta gtgtgaagga ccaggcaaat ctaagttcca taaattgatc 87120

atagaactaa ttagaccagt tttattattt tctccaacag tgtgtggtaa cctgacagga 87180

aaaaatagaa caattgaatg gtcagactta cccaaaattt gtatctggca agctagttga 87240

taaaataaca agaattagcc tgaaaatctt aagatatcac caatggcatt gttgtagttg 87300

tgagccgcaa agtatgtgaa ggaagacaac aatggccaaa tcttagcaac tgtttgagag 87360

attaatgttg accttgaaca gattaatgta atgagataga catttaagaa gttgggataa 87420

gtatgtgtgc cccaggggtg cgtgaggttc taaatcgaga tcatttcatc atttttattg 87480

cttttgtaaa gactgtgatg cttctccggg tcttaaaggt agaatggaaa taaatgcttt 87540

tagaggtgag aggtttagag aaatctatgg cagtgttaaa agagtaattg aagttcatat 87600

tgaatgcagc aagggcagta ggtaaaatga gccaggggcc ttggtagatt gcagctttga 87660

ggatagggac agatggaata agtagatgtt ttgtacttaa caaaaggaaa agctattgat 87720

agatgtggtg aagtgatagt ttataatctg tcatggaggg atcagtatac tttcaaaaat 87780

taaagctaac attttggtaa ggaaatatgt ttggaaaatt gttcttttca ttacttttcc 87840

taaggtttat tttgtctagt aactgtactt gctcaggtga cagtaaataa gaatatttta 87900

tccatgcagg aggaaccttt gtgttccaga ctgcatttat gtgatcttca aagagtagga 87960

attgtgtttg tcttatgtgt ggttgtagtc tgagccctaa cagtgccaag cacatgtatc 88020

agtatatatt tactgagtga ttgaaggctc aataaataaa ttttattgag agaagtatgc 88080

ttaagtaatt aggccttggt actgaaagct aaaccaaatt gtagctcaga aaatttggaa 88140

tctatctgtt tataatgccc tcatctacta tgaaacaggg tctgggtagg gcaactagca 88200

ggctgtaaac aaagaagacc ttttgatgaa gtttaaatct cttcctcagt agctcttgtt 88260

actattcccc gcccccctta tttgttcgaa agtatgtggt aagatgcagc atagcagggt 88320

cctgtggccc ttattgaaat gaaatttctc tttcattctc ctctacattt tataattctc 88380

ccttcctccc ttacattcag acttgatagc atggcgtata tactctctac aatctattcc 88440

ctttcacctt ttcagcctta ttgtttcttt acctattttt catttaaatt tctttctttc 88500

ctttttaaga aaacagggat gctgctaaag cttcatttaa atttcacaaa catatattga 88560

tcttttgcta tgtcccaggt attgtatgac attgagctat acctatctat agtttttcat 88620

gtgtcttaaa ctttctcgcc tttgttacag aaaaaacaaa ctctttgtta cagaattttt 88680

cccatataga ggattccttc ctccctactt tcttcatcca tctaaatgcc aacctttcaa 88740

ggctgtcctc cttcctcatt attactttgt gtcaagaata ttgtttattt ctcatgtaca 88800

tcagttacac aaataaaaat acacccatcc tactccactg cccaacagta aactccttga 88860

gagacgggaa aggccatttt aaaatatttg cttctctctt tcataaatgt ttgtagtatt 88920

gcatctattt gagatggtag gtagaaatag tagagcatgg tgtgagtgag gggattacca 88980

atgccaaaaa gaactatgga cttgtatatt ccaattcgtt caatgtcatt ctgttctttt 89040

aaaatttgat atcttgtatg gttgcttgac tttatagcct tcatcaaatg aaacttctag 89100

gaagatactc tgatggatag tagccccata atttcttggc tgtagggtat aagccacatt 89160

ttcaaataca attctgtttt ttttttttct tttttcacaa ggcttacagc tttgcatact 89220

agttccaatc tctaagcagc attaggtggg gaaagaagtc atcaaacagc catactgaga 89280

agagtaaagt attttaactg ataactattc cagaaagaat gtggcagtta cacatcttct 89340

aaaccctatt aaggaggttt tggtactaac agtgagatct ggtactgtgt cctaaatttt 89400

ggggtttttt tggacaaatc cttaatacct catgtccttt tcaacttctt tttctgttga 89460

gagagagaga gaacgaatgg aattcatatg cctacaactt ttccgggaca aaataattct 89520

tttaatggga ggaacacttt tccttgatac agtgttttat cttacgttag acatgatttg 89580

gaaggtaaat ataagtggac cagaaaattg gaaatactgt gcttttttat tattcatgga 89640

ccatctagat atcacatatg tataggtcaa atttatcagg tgtaatatag tgtagtgtct 89700

aataacaaag aatttatcaa tttaaaggtg aaagtattca tttatagtat ttctggcact 89760
tattttgtaa tttgaaactt aaaaccctat caactggtga aaagattata atcaaaggct 89820
aaaattttat attctctttt gttaatgtca ggacaaagtc cggattgaat ataagtctgc 89880
tttattttat aggcctatga agaatacacc agcaagctag atgcactcca acaaagagaa 89940
caacagttat tggaatctct ggggaacgga actgattttt ctgtttctag ctctgcatca 90000
atggataccg ttacatcttc ttcctcttct agcctttcag tgctaccttc atctctttca 90060
gtttttcaaa atcccacaga tgtggcacgg agcaacccca agtcaccaca aaaacctatc 90120
gttagagtct tcctgcccaa caaacagagg acagtggtga gtcagtttta atatcaccat 90180
tttgctgttt ctttgtattt ttcagacaga tcagttgttg aaaattaata tattattact 90240
tcatactcag aggtcatgca aatagaggca tccatgtcat acaggaatga aaaatatgta 90300
attatttggt ggcaaagtct atattcctta actgaaataa atggtattgt ctctagtgtt 90360
ggatttgaca tttaaacctg tctgccaaac ttgaaccaca gtcatgtcta agcactagtt 90420
taaaataaat caggatttta agacttgagc ttgttcatta tctaatgtgt gtcaggtaag 90480
actcattctt cacccttgag aaatctcctt tctgagttat attatcagat gttgaacgtt 90540
cagtgtgatt gatatgtttc ccctcatcta cttcattttt ggttgttgaa tattagtatt 90600
aaccattgga acagcgtacc ctgtaagtaa catttgaagc atttaaagag aaatctgtgg 90660
aattattgtg ggtggtctat aatccttaag tgcaacgaat attgttagta gacttaataa 90720
gtaacccatc tgtatacatc actacttttt aaatgtctgt ggttactttt gacaataaaa 90780
attccaaata caactgaagt caaaattttt catttttttt ctctgacaac agaaatcaaa 90840
agtgcaattg gtcattgttt aatgttccaa aaattccttt ctgacttgaa aaaaaaatgt 90900
tattatagag gcattttact ttcagaagtt aagaattcct gcatatgagt ttagaaaact 90960
aatggagtta cgagttacca gcctgtaagt ttttatctta ggaaatatgg ctttctaaag 91020
gcatcattta ttgtcaggga ataaaaagta ataaaataaa aagtcatact ttttctgccc 91080
tttttccatg tacagacaaa agttggttgt aaaaaataga ctctaatttt tcattgtaca 91140
gaattacaaa tcaatttgta aaacaaatcc agttctaccc tttctcttta ttctgttggg 91200
tagaataaaa ttaaaattat ttttcccaca ttaagatggg gaattactaa gcatatcttt 91260
ccagggatgc cccacttaaa aggggaatat caacatatga attatctgta aatagataat 91320
cttcgaagca gcctaacaaa actggcaaac atcccatccc cagtagatct taaattaatt 91380
ctttattgtc tgtattcatg agcagaaagt aggaaatgtg ttcttcagtc ccaggcgttt 91440
cccttctctt catcacggta ttgttccttc caggttccac tcaacttagt aattttgtga 91500
gtttctgtat acaaatgtaa aattggggtt gttggagcct ttcaaatttc tcaacctaat 91560
gattaagttt aactagcctc cggccgggca cagtggctcg tgcctgtaat cccagcactt 91620
tggaaggctg aggcgggcag atcacgaggt caggaggtca ggagatcgag acagtcctgg 91680

ctaacacacg gtgaaacctt ggctctacta aaaatacaaa aagattagcg gggcgtggtg 91740

gtgggtgcct gtagtcccag ctaatttgga ggctgaggca ggatagtgct gtgaacccgg 91800

gaggcggagc ttgcagtgag ccgagatcgc gccactgcac tccagcctgg gcaacagagc 91860

gagactccat ctcaaaaaaa aaaaaaaaag gtttaactag cctcctcata tttatgagag 91920

aggccaagag aaatttacag gtatcactaa agcttataat ttccctttta aaaagaaata 91980

aaaacaactg agactcttca aagatgtaaa atgataaatg aataactatt aggcaattga 92040

ctaggcttac cttaattatc ctggctaggc ttagattaaa taagtaactt gttcttgtcc 92100

gtcatactcc cagtttaaca attcgtattc aatactcaga aaaactgtat atcgtgaact 92160

taaaaggcct tatgaatcat cagtaaatgg gtttgtggta ttaaactctt tcaagaacgt 92220

tattttgcca ttctctcttt tatctgaatc tgttaaccct tttcttaact acctcttttc 92280

ctctcctttt cttctctcaa ttcttattaa cattaacagt tactatttga gcatttgcca 92340

tgctgtgttc caagttctgt gctaaggact ttacatacat cttctcagcc aggataattc 92400

acattctaag taaaattgtt tgtggtagtg aatgacagtt cctctatcaa aatacatgtt 92460

tctgtttgtc ctgaacactc aggaacaaag ggcagtagaa cctactagca gcaaatgaaa 92520

tcaagtacca agaatgagat tatgtttttt aaagtggact tagatttgta gtccaagata 92580

gcacgctaaa cacttgcctt tatatctttg ttcctttcca gaaatttcat tgaaatgata 92640

gtaaagatat gtaaaaagaa taataaaaca ggataggatt ctgtcagaga actagaaatt 92700

tttaagaatt cctggaagat ggaaaatata tgaatggtta cgtaaataag ccaaaataga 92760

aaaccaaaca aaatactagg agctagattc ttacatgaag ggagccattc ttagcaaaat 92820

cctgatgaag cagatagcat agtcagcaga ttacaaagaa gtggatttgc ctcttgggta 92880

gtcgtcagag taattagtcg aaggtcttgc tctggcagga agacaaagtc atgtgtcttc 92940

ataaatcgag ctggtgatat aaatatgaat attttgaagg tgtgtgttag ttgtgagtcc 93000

tgtggagaac caaaacaaac ctcaactaaa aacattgaaa ttctgaattc aaaaaaaatt 93060

ttttaaaggt catggcctga tatttttaga catctgataa tgggttgctg agtcctgagt 93120

ccccagtata gccttacatt gcacatgtcc atagagatgt gaatgttgcc tcctggagtt 93180

gtacaacaag gggatcctgt tagttcctct tctgtatata tggaacaaat aatttgtagc 93240

atttattcca tgaaaaagct ctaaaagtcg ttttctaaac aaaatttctg cctggagagt 93300

tctatgtgac tgctgtgtgt gtttaaatgg tggaactgaa catcagatgt ccctgaacct 93360

cagagaaatc cagtggggta aaaagtaaac gttaatctac cgaggagtaa aataatactc 93420

tgcttccatc tggaatatac cagacttctt ctcacgtctg tacccattag taatctgttt 93480

ggaaaacaaa ctgctttccc ctactatact gtcaacacaa caaatttttg tgactagatg 93540

tgtgggggtg tttttcccca ccaagcaatc tccagttctc tgtagataat caactgggtt 93600

ctccacccaa ttgtctgcaa ttgttgttaa gttgccttca atttaactca attctgacac 93660

tatctaccca ggtatagcat agaccccaca ggttaagagc tcagtcccac aagactgtcc 93720

ccctcttgag atgctagtcc caagttccag gttgtgactt acacttctga ccagctggct 93780

ataaatcaag gattcccaca tcttctaatc tttgatcatt tgctagaata gctcacagaa 93840

cttcaagaaa cacttaacat ttactggttt gctataaagg atgttacaag aggtaaaaga 93900

cgaacagcca gatggaagaa atgcataggg aaaggtatgt gggaagggat gcagagcttc 93960

tgtgctctct ttaggagcat caccttccac tacctccaaa tgttcagcac cctggaagct 94020

ctaggaacat tgcatttcag tgatttttat ggaagcttca tcatataggt gtgatattta 94080

ctaggtcaat ctccacgacc tccccacttc tcagaggttg gtggatgggg ctgaaagttc 94140

acattatggc ttggtctttc ttttgaccac cttccattca ggaacccacc aaaactcacc 94200

ttgttggaac aaaagatgct cctaccacct aggaaattcc aagggaatca gagctctgtg 94260

tcaggaacca aggtcaaaga caaatatgag aacaaaagat gcacctggca ccctgatcac 94320

tcaggaaatt acaagagttt taggagctct gtgcctagaa ctggggttgg aggccaaagg 94380

gttgaagacc aaaatatata ttattataaa ccacaatatc agagtttgtc aacataataa 94440

accacttgcc atttgttgga cctatacaac tgtacaggag taagtccaca ccaaatcaac 94500

ctaattttta tttataaata tgactagaac accaacagca acagagaaaa ctcaagaaaa 94560

gaaagcaact agaagatgta aaaatattat agctcaggat gtacttcatt cttgaagcca 94620

aatcagtttg atataaaaat tgagtcattc agaggaccac agagtttgtg caaattgaaa 94680

gtatgattgt tgacttttgt taaacagaaa ggccaaacat taggatagct agagttaata 94740

agtattttgg aaagtaaagc taaagacata ccttttaaga tatgagataa agtgggcaaa 94800

tatgagaaaa aagataagag acatggagag agcaaaagtt ccaacttctg tttaataaag 94860

aagcaaacaa aaatagaggg aagatgtatt cacagacata atacaggaaa attttcctgt 94920

gttaaacaaa aaccagcctt cagatagata ggggcaactg actgtcaaca ggaaaacaaa 94980

aacagagatt gacctggata cttgctgtat aattcgaaag ttgaaagaga accttttcag 95040

aaagataaac tggtaatctg cgggagaatg aaaactggat cgttttctca ttggcaaact 95100

gagggctaga aggcagtgga tcagggccat taaagatctg atggaagtta atagccaagc 95160

taatattaaa agtaagacac tttgggtatc tagggactga aaattatttc agtggaatat 95220

aataggtatc tagaaataga tcccagaatt tcagaaaatg taatatgaaa aaaatggttc 95280

aatcagtaag aagaaggtga attatttaat ttttggtgct ggcatatgta gaagaaaata 95340

aaattgggcc cacatcttag actgcttaca aaagtaaatt ctatcagaat caagagatta 95400

aataccaaaa aaaaaaaaaa atgtaaatct ttcaagaaaa atctagtagg ctgcatgtaa 95460

aatctaggag ttgaggtcac tatcttaatc aaaacttaat tagctcaaaa gctgtaggag 95520

acagatggat taaatacaat tttgaaatta tgtatgcccc cccccacaaa aaaaaaacct 95580

taacagagtc aatagtctgt ataaatttgg aaacagcatt ggtgcccaga taccaagaag 95640

taaatagtct tctgaatata caaagaacac ttcaaaattg gcaagaaaaa gacaaaaccc 95700

agcagaaaaa tagggaaaag atataaatag gcagttcgta taagatgaaa tccaaatgaa 95760

cagtaaagca tgtgaacact gttcagatgc agtcagtcag ttgtgagtgt acaaattaaa 95820

actatgagat catttcatct gtctggcaaa atataaatta ttattggcag gggtgtagtg 95880

agaaaactgc tcccttagtt aaggatagaa atgtgaagta tcacagctta ttggaaagca 95940

ctttgataat atctgtcaaa atttacaaga actgtatctt ccaagccagt actccatcta 96000

ttgggaattt agcacataaa agcaccaata tttatacaag gatatttgtt aaatcatcgt 96060

aataagaaaa aaatatagaa tgtcatcagt agagaaatgg ttgtataaat catggtacag 96120

ccacaccatg tagtacaatg cagcctgtga aaagacttgt acttgaaggt ttttctgaga 96180

ggtattataa ttgagaaaag gaaagctgtt ggaaactctg atattactta cttattcttt 96240

atgtatatca cattgcaata ttctagtagg tctagaaata tctctctcaa ctagaaatgc 96300

tgaataaaat atgtttattt aaatgtatag ctgagctcaa tagaaaaacc tatagagggt 96360

gaaaacaaag acggagttga aaaaaccaga aaagcatagg agctgatatg ttaaggtatc 96420

gctgaactat aaccctcaac aaatctaacc caccatctat ttttgtatgg cctgcgaact 96480

aagaatggat tttacatttt taaatgcttg aaaaaagtta aaggaataat agtatttcat 96540

aatctatgaa aatcacacga aatacaaatt ttggccaggt acggtagctc atgcctgtaa 96600

tctcagcatg ggaggctgag gggagtggat cacctgaggt caggagttct agaccagcct 96660

ggccaacatg gtgaaaccca catctctact ataaatacaa aattggccgg gtgtggtgcc 96720

gcatgcctgt aacccagcta tttgggaggc tgaggcagga gaatcgcttg aacctgggag 96780

gtggaggttg cggtgagccg agattgcgcc gttgcactcc agccggggca acaagagcaa 96840

aactccatct caaaaaaaaa taaataaata aaatttcagt gtccatcagt aaaatgttat 96900

tggaacacaa caatgctaat ctgtttatat gttgtctgtg gctggtttca tgctttagca 96960

gtggtgttga gcagttgtac atcacagtgt aatctacaga acctctggtt ctttatggaa 97020

gaagtttgct gacctctgcg gtcgtggcct caggtgggcc agtttgtcac tctggaacaa 97080

aggatgtgaa tatcaatgtg tgggaataca acactgggcc ttcagcctgt gtcacaaagg 97140

tagttggaac ccagattctc acataaagct gaccctttga aggacattac cttcagtgaa 97200

aggctgaaat ctattaccag aatacataaa gataacaata aacttgactg tctttgacag 97260

tactctaaat aaagtttccc ctgtgatttt ttaactatgg acctattctc atcattgtgt 97320

gggattcaaa tttatactgc ctgcattgtc caggaaccct caagccatga agtggacata 97380

acgactggtc ttggagtggt aatagcactc aggcactgca cagaagcaat ggaaaccctc 97440

cctcagcccg tctaggtctc atagaattca cagaaataat gctttactga ctatctcaag 97500

atctcaagtt gtaagacatg caaggaaaca gtctacaatc agtgagttag cagacttaaa 97560

acagcagggt tagactccta agaaagaacc aagtagaact tttaggtagt cttgatttaa 97620

acctcatcat ctatgaagta ttcttgcttg gattgggacc ttctatttta taagaaacac 97680

aggggataga ggaaaaaggt aagcaccatg aggaaacaat tagacaattc agaatgttga 97740

cttcctgtaa gacaatagcc ctagactcct tgataagtta atgtcttgga caataaaaag 97800

gttggggatt attccacact gtacaagacc aagtgcacat aacaaccaaa tgcagtgtgt 97860

aatcctgagg ggggaagaat cattcttggg acttgggaaa tttttaacat tgattgaata 97920

ctacatattg ttatggaatt aacgttctta aatgtgagga tggtaacgta attatatagg 97980

agaatgtcct tgttcttagg aattacatgt ataagtattt aggagtaaag tgttaggatg 98040

actgcaactt cccccgcaaa cccacgcttt tttgagatgg aacttataca cagtagaggg 98100

tacaaatctg aagtatatcg ctcagagttt ttacatgtgt aactaccatg tagattaaga 98160

tgtaggacat ttcctgatgc tttagagggt tctcctgtgc ccctcccaat tcactaaaaa 98220

taaccactat ttcatttcta tctctgtcag ttacttggaa acttaacttc aaatggtgtg 98280

atggaaatca acatgcagag aaagagaaaa gcaaatacga aaagataatc ttagaatcta 98340

agaaaatttc gatgttaatt gtaccatttg ttcagatttt ctgtctgaaa ttttttgtaa 98400

tagagttgga aaaattgaaa acatagaaaa aaagaaatgt agaaagttca aaagaaacag 98460

ttatctctag gaaaaaagag tgaacatact gatactcatt tgaggatgta gcaagataac 98520

actttaagta aaatttcagt atgtcaagtt agttacagca aattacatag gaaatgttta 98580

aatgtcacat agaaattcaa atatttattt aaaaaactca gtgaataagg taaagctaga 98640

ttaaacccag ccaaagaatg aattagtgaa ctctggaaag tgaagaaaga gagttataga 98700

aaatataaaa gagaggttaa gcagtatgaa aaatggaatg agcattatat gcctcaaaga 98760

gttccaaaag gtaagaatag aaataaatga gtagagaaaa attcaaataa attagggcta 98820

acatttacaa atgacataaa tctaacgatt tgggaagcac aatctctgag ttggacaaac 98880

aatttgaatc caggatagac ataataaaac caaagaatat tgttttaaaa gcactcaggg 98940

aaaaaattat atagaaaata attaaaatta gactaggaaa atttgttatg gcagttgtag 99000

aaatcagaaa acaatagaat aatatactca gagtactgag aggtacctgt aaatctctca 99060

gctaagttaa gctataaatt tgattaggtt gacttgggca aatgcaccat aaagtccttt 99120

ccagagaagg agttcacttt tcataggttc tacttgaagg aattgtccaa gaaggtttac 99180

tgtataaagg aaattgaaca cagaaggaat gaaaagtaaa aattaaacct aaaatttgca 99240

gaagaaaaca ctacagaaaa tttttgtgac ctggggtttg gcaaagattt cttaaatatg 99300

acaccagaag cacagtctat aatgaacgta ttagtttatt gggttaatca aaatgtaaaa 99360

cttctgctct tcaaaagaca gtattaagag aataaaaaga gaaaccacag attgataatc 99420

tttgtaagcc atgtatctga tcaaggacat gtagaatata taaagaactc tgaaagctca 99480

atagtaagaa aacaaatggc tgggtgcgga ggctcatgcc tgtaatccca gcactttggg 99540

aggctgaggc aggtggatca tgaggtcggg agttcaagac cagcctggcc aagatggtga 99600

aaccccatct ttactaaaaa tgcaaaaatt agctgggcgt ggtggcaggc gcctgtaacc 99660

ccagctgctc gggaggctga ggcagaaaat tgcttgagct cgggaggcag aggttgcagt 99720

gagccgagat tgcgccactg cactccagcc tgggcaacag agtgagactc catctcaaaa 99780

aaaaaaaaaa agaaaaaaca acccaattaa aaacggacaa aggatttgaa cagtttcatc 99840

aaagatatat ggatggtaat aaacgcatga gaagacgctc aacattagtc attaggaaaa 99900

tgcaaattaa ataggcacgc gataccctta catgcctcct agaatggctg aaattagagt 99960

gaccatacca agtattagtg gggatgtgaa agaactagaa tttccgtaca ctgctgatag 100020

gagtgtttaa ttggtacaac cactttggaa taaaatttgg cagtttattt aatgaaacct 100080

tttcaaaatc ccaacgggct tttttttttt ttcctcagaa atagaaagtc cgtcctaaaa 100140

ttcataccgt atctcaagga atcccgtata gccaaagcag tcaaaaaaag tacagagttg 100200

ggggcatcat gcttcctgat ctcaaaactt actacaaagt tagagtaatc aaaactaagt 100260

ggtactggca tgcagacaga tgagagtcct gaactaaatc ttcacattta tggtcaaatg 100320

atctttgaca agggtaccag gaccactcca tgaagtaaag agaatctgtt cagcaaatga 100380

tgctggcaac tgaatatcca catgcaaaag aatgaagtgg tacccttccc ttataccatg 100440

tacaaagaat aacccaaaat ggatcaaaga cttgaacata acagttaaag ccataaaact 100500

tttactacaa gaaaacatag gagaaaagca tcataacttt ggatttgtca atgatttctt 100560

ggccgtgatg ccaaaagccc agggaacaca agaaaaaatg gaaaaattgt actattatca 100620

gaatttaaaa cttctgatca tcaaaggata taatcaacag agtgaaaagc caacctggaa 100680

tgggaaaaat atttgcaaat cttgtatctg ataaggggtt aatatccaga atataaagaa 100740

ctcctgtaat agcatacaac cttgttaaaa aatgggcaga ggacttgaac agacatttct 100800

ttaatgaaga tatccagatg gccacgagca tatgaaaaga tgcccaacat tgctaattat 100860

taggaacatg caaatcaaaa ccacaaggag ataccacctc acacccatta ggatgtcggc 100920

tatcagacaa aaaagaagat aacaagagtt ggcaagggtg tggagaaatt ggacccctgt 100980

tgcactgttg gtggaaatgt aaaatggagc aactgctatg gaaaacagta tagaggttcc 101040

tcaaaaaact aaaaatagaa ttaccatata atccatcaat tctccttctg tgtatatacc 101100

caaaataatt gaaagcagga tctcaaagag atatttgtac actcatgttc atagcagcat 101160

tattcactat agtagccaaa ggcagaagca acccagattt ctgttgatag aggagtggat 101220

gaacaaaata tggtatgtat atacattgga atattattca gccttaggac attctaacac 101280

atactacaac atgagtaaat ctcaaggaca ttatgttaag tgaaatgagc cagtcataaa 101340

acgacaaata ctgtatgatt ccacttattg aagtacctag agtgttcaga ctcacagaga 101400

cacagagtag aatggtgctt gccaggggct gggggaaggg gtgctgggga gttgtttaat 101460

ggttatagag ttttagtttt gcaggatgaa aagagttcag gagattggtt gcacaacatt 101520

gtgaatgtac ttaacactac tgaattgtac acttaaaaat ggttgaaatg ttaagcttta 101580

tgttagatat attttaccat aattttgtga aataatagta tggtagttat gtaaaacgtt 101640

aggctggaca cggtggctca cacctgtaat ctcagtgctt tgggaagtca aggcgagaga 101700

atcacttgag accaggagtt ccataccagc cagggcaaca tagtgagacc ccatgtctac 101760

aaaaaaaatt tttaattagc caggagtggt ggtgtacatc tgtagtccta gctacctgag 101820

aggctgaagc aggaggatca cttgtgccca ggagttaaaa gctacattgt gctatgtgca 101880

ccattgcact ccagcctggg tgaaagagca agaccctgtc cacccccaca aaaagtcaga 101940

tgtacactta gcatatcaca cagcccttct actcctcggt atttacccaa gagaaaaggg 102000

agcatatgtt cgtagaaaga tttgtatacg aatgttctta gttgctttgt catagcccca 102060

aactggaaat aacccaaatg tccataaaca ggtgaatggt tggattgtgg tgtatctata 102120

aaagggaata ctactcagta gaaaggaatg aactgctaat gcacacaaca tagatgaatc 102180

tcaaagtaat tatgctgagt gaaagaagtc agacaaaaaa tgattacacg ttttaaaatt 102240

cagtttacat aaaattctag aaaatgcaaa ctcttctata gtgacaacag atttgtattt 102300

gcctgggcac aggactatgg caggatggaa gatttacata ggagcacaag gaacctttca 102360

gaaagtgatg ggtatgttca ttatcttgaa tgtgatgatg gttgcatggg tatatacgta 102420

atctcaaaat gtatcaaatt gtgtactcta aatcattgca gtttattgta tgtcagttgt 102480

acctagataa atctgtttgt ttgtttttta atagccttta agaaagctga tagtactgtg 102540

gaaatattat atgaaataga ctttgggaga aagaattatt attaaaattt ttttttcaac 102600

ttttatttta gattcaggag gtacatgcac agctttgtta tctgggtata ttgcatgatg 102660

ctgaggtttg atgtacaaat gatcccatca tccaagtact gagcatagta gccaatagtt 102720

tttcaacctt tgccctcccc gctctagtag cctccggttt ctgttattgc tgtctttatg 102780

tccatgagta cccaaagttt agctcctact tagaaatgag aacatttgat atttggtttt 102840

ctcttcctgt gttaatttgc ttaggataat ggcttccagc tgcatccagg ttgctgcaaa 102900

ggacatgatt tcattcattt ttgtggctgc atagtattcc atggtgcata tgcctttgct 102960

attgtgaata gtgctgtgtt aaatatgtgg ttttttcgta gaatgattca ttttcttttg 103020

gatgtatata taccgagcaa tgagactgct gggtcgaatg gtagttctat tttaagttct 103080

ttgagaaatt tccaaactgc tttccacagt gcctaaccaa tttacattcc caccaacagt 103140

gtacaagcct tccctttctt ctgcagcttt gccagcatct gttgtttttt gccagcactt 103200

tgggaggttg aggcgggagg atcacttgag gtcaggagtt cgagaccagc ctggccaaca 103260

tggtgaaacc tcatctctac taaaaataca aaaaaaaaaa ttagccagcc atggtggtgc 103320

acacctgtaa tcccagttac ttggagctga ggcaggagaa ttgcttgacc ctgggaggca 103380

caggttgcaa tgagctgaga tcccaccact gcactccagc ctgggtgaca gagcgagact 103440
ctgtctcaaa aaaaaaaaaa aaaaaaaaaa tgtaattcag acgctgggtg tagtggcttg 103500
cgcctgtaat cccaacactt tgggaggcca aggcagaagg attgcttgag cccaggagtt 103560
caagaccagc ctgggcaatg taaccagacc ctgtctctac aaaaaattag ccaggtgtgc 103620
tagcatgagt ttgtagcccc agctacttgg gagactgagg cgggataatc caggattttg 103680
aggcttcagt aaggtatgat tgtgctgctg cactccagcc tgagcaactg agtgagtccc 103740
tgtcttaaaa aaagactttc tagatcctgg agtatgtgca ataactaaaa agaataagca 103800
tatatgttcc ttgagatttt tatttttaat tttatttttt gagagtgagt cttgcccaga 103860
atggagtgca gtggtataat cacagctaac tgcagccttg acttctcagg ctcaggtgat 103920
cctcccatct caccctccaa gtagctgaga ccacaggcgt gtgccatgcc taatttttaa 103980
atttttttgt agaggtaggg ccttgctatg ttgcccaggc tggtctcaaa ctcttgggct 104040
caagcaatcc tccctctgtg gcctcccata gtgctgggat tataggcatg aaccactgca 104100
cctggccctg agatatattt gaataattgt aaaaatctgt taattggggt tattggaggg 104160
atggggatat tccctgggac aagaaaaagg gactgacctt ttacttttat acttgatacc 104220
cttatttcac ataatgccaa ggtgtctgtt ttttatgatt gttcagggtg attatatatt 104280
tattcatgta ttgtttagag tagttgtatc tcatttagct atttctttct tgatggataa 104340
ttaggtaatg ttgcagtata tatttaagta catatatcct gaaacactgt gggaatactt 104400
ttattgggga agataaatac cttgaaataa ttattgggtc atagaatata cacactgaaa 104460
actttgcgat aacttaatat cagaaaagat tttgattctc aacaagtgta tgaaacttcc 104520
ttttcccaca agatcctggc caacatggaa tgttaacaga tttttaaaag tattaccaat 104580
ctaataaaat ttaaagtata tttgacttga atcctgtgtg tattgtttgt ctgtatgaca 104640
gttgtttgta ttatatgaca aaaaattaac caaaaaatat tagactcacc tgattacccc 104700
aaaagatggc ttgtgttcta agcctgttta ggcttataag tcaaaagaaa atttctttaa 104760
aaaaatttta tttgatggtt tattgagata taattcaaaa gccatacaat ttacccattt 104820
aaagtataaa atgatttttg tatattcaca aagttctgtg gccgtgataa ttcatttaaa 104880
aatatttttt catcccaaaa ataaacactg tacccgttag cagtcacttc ccattcctcc 104940
catccctatg taaccaccca tttgctccct atctctatag atttgcctat tctctggcat 105000
ttcatataaa tgaaatcata caatatgtgt ttgtgactga cttcttttac ttagcataat 105060
gttttcaaag tccatccaca ttgtaccctg tgtcagtcct tcatgccttc ttatggctca 105120
gtaatattcc attggctgta tataccacat tttgtttaca catttatcag ctgatggaca 105180
tctgtgtcgt ttctactttt tagctgttat gagcaatgct gctgtgaaca ttcgtattca 105240
aggttttgtg tggacatatg atttcatttg tgcctctagt aaccttttaa gagactggca 105300

gaatgttttt caaaatggct acaccatttt actttccctt gagcatgagg gttctgtttt 105360

tttcacatca ctgccaaact tgtttattat ttgtcttttt tattatagcc atcctaatgg 105420

gtatgaaatg gtatctcatt ttgggtttta tttgcatttc ccttatgact agtaatgtga 105480

agcatctttt tgtgtgattg ttggctttta tatatctttg tggagaaagg atttgctttt 105540

atatagatag ttttacatgc aattcataga taaataccaa tgacctgaga cgagaagaat 105600

tacaaaggtt tttgaacaat gctctaataa tagaaatcgg acagaaaaga gaaagtagcc 105660

aagattattt cctctagaga tgggaaaatg tacaagttct ccaaggatct atgacacaac 105720

aagagaaaga agtggagata acaagaataa aactgaatgc tctagagaca caaaagggaa 105780

atactgaaga taggtaagct ctcttcagta tcttctttgt ttttaagttt attcctaggt 105840

aagctatctt cagtgtaagg tagactacgt aagataggta agctgtcttc agtataaggt 105900

agactagata agctatcttc aaaataaggt agactaagtc aaaggattgt aatatcaaga 105960

aatgtgatag accctggatg tggtctttta catcactaaa tgaaaaccat acttaataag 106020

ttgtatatag tattgtaaag ataattgcct gttttaggta tttcaaaatt ggtgactttg 106080

gtttagcatt tagaaaatat tgttcataag gttttttaac tttttattta gaaaatttaa 106140

aacacttggc cggtgctggc gtgttggctc atgcctgtaa tcctagcact ttgggaggcc 106200

aaggcgggcg gatcacctga ggtcaggagt tcgagaccag cctggccaac atgatgaaat 106260

cctgtttcta ttaaaagtat aaaaattagc caggcattgt ggcgcacacc tgtaatccca 106320

gctactcggg aggctgaggc gggagaattg cttgaacctg ggaggcagag gttgcagtga 106380

gccaatatcg tgccactgca ctccaggctg ggcaacagag caagactcca tctcaaaaaa 106440

aaaaaaaaaa ttaaaatact tgtatggaaa aatataaagg acaatatatt aaacagctgt 106500

ttagtcacct acaatgaaca aatactaatt ttttgttata tttgcttcat attatacata 106560

gaagtacaaa tttaggttcc actaccagtc ccattcctct gctggacacc catcgtccca 106620

tcttgtcccc aaaaagacag tcactatgat aaatgtgagg taccttcaat catatttttg 106680

tattttatta tatatatgtg tagccataag ccaatgtttt tgtttttttt ttaatttgta 106740

aggtgatacg atactatatg tatgtaatgg tcagcatctt gtccttttta ttcaaaatta 106800

tgatttcaag gtgtatgtat ttatatgtta acacatacag atctatttca ttcaatgcta 106860

ttatcaagta ttctagcttc ttaatatacc acagtttatc tttttctcca ttgatgagca 106920

tttaggttat atttggattt ttttgttatg acaaacagtg ctgtaataag aacattcttg 106980

tgcttgtctc cttgtacaca tataacaagg tgtcacaaac ctgaaagtgg aattcctggg 107040

acagggagta tcatttgaag agactgtcct ttccccagtg tatgttcttg gcacctttgt 107100

caaaaatgag ttcactgtag atgtatggaa ttatttcatt gttctctatt ctgttccatt 107160

ggtctatatg tctgttttta tgccagtacc atgctgtttt ggttactata gctttgtagt 107220

ataatttgaa gtgaggtaat gtgattcttc cagttttgtt ctttttgctt tggctattct 107280

gggcctttgg tagttccata taaattttag gattattttt ctatttctgt gaagaatgtc 107340

tttggtgttt tcatagggat tacattgaat ctgtagatta ctttgggtag tatggacact 107400

ttagcaatat tgattcttcc agtccatgaa catggccggt ctttccattt tttcatgtgt 107460

tctctccagt ttcttgcatc agtgttttat agttttcatt gtagaaatct ttcacttctt 107520

tgtttttaag tttattccta ggtattttat ttgtagctat tgtaaatgga gttactttct 107580

tgatttttgt ttcggattgt ttgctgttgg catgtagaaa tgctgctgac ttttgtatgt 107640

taattttgta tcctgcaact ttactgaatt tgtcagttat aattgttttt ttggtggagt 107700

ctttaggttt ttccaaatat aagatcatat tatccacaaa caaggataat ttgacttatt 107760

ctttccaatt tggataccct tttttttttc tcttatccaa ttgctctagc taggacttcc 107820

aatagtgtgt cgaataacag tggtgaaagt gggcatcctt gtcttgttcc aggtcttaga 107880

agaatacttt caggtttttt tcattaatta tgattctagc tgtgggtctg ttgtctgatg 107940

aaaagtctga aacggtacct aaaactacta aattaatttt aacatctttg agttgttttt 108000

agttctgtga gaagagcata tctattttgg tttactcagt gagtagagta gtgtctaata 108060

ctgagtaaat gtactgtaag tatttttgaa agaatgagtc tttgggttta cataccctgg 108120

ggtttgtaaa caaatatctg ttgattggca ttaatcctga tggtatccaa ggtacaggaa 108180

tggcaaaggg aaaagatagg gcaatactga ctgatgcttc aaaatcatgc cctagttatg 108240

ctataatcaa gcaggaaatg tttatggaat ggaaagatta aggaaaaggt atgttcttat 108300

tttagcaata aaacgaatac cagaagcttt aacattcacc agtacaaata aatagtttca 108360

atggaatagg tcgaaagtaa agggacatca ctagagtaaa tgctagacct tccctctcct 108420

tttattttta gcaacagcaa agcagaaact aagatctaca agtgatcaaa gagggtgatc 108480

cattcagttt ctgtgtagac aggaataata ataatacctt ttacatattg gtacagtttg 108540

taaaaacact ttcacttact catttaatct tcatagcaac ttgatgaggt agaatactat 108600

aggaagcagt attagctcag gttggtacgt aaattactgt gtttaaattt caataaaaca 108660

gctatggaat ccaagacatt cttggcgcct aataaactgt attctttgcc aacagtgaaa 108720

gtgcttctct gttgcttggt aagttttttc cccttagaat actaataaag taattgatta 108780

actttcattt ttattttgat ttgattggga cagcaattta gcagtaaaaa atgtcacctt 108840

tataaatcct gtggtttctg gttcttggcc agttaaattc aacctgacca ggaggcacgc 108900

ttaattctaa aattgctttt accttctgaa gtttttgtgg tatagacatc ctcctttttc 108960

tactttaatg aaagcatgtt ataagcagat cataacaatt tttttttctt taaaacaata 109020

ttgtaattag gccagttgca gtggctcaca cctgtaatcc cagcactttg ggaggctgag 109080

gcaggcgggt cacttgaggt aaggagttcg agaccagcct gaggaacata ctaaaacccc 109140

gtctttacta acaatacaaa aaaattagcc gggcttgctg gcacatgcct gtaatcccag 109200

ctgctctgga agctgaggca tgagaatccc ttgaacctgt gaagtggagt ttgcagtgat 109260

tctaggtcgc accattgcac aagcctgggt ggcagagcaa gaccctgtct caaaaaaaaa 109320

aaaaaaaaaa aatggctcac acctgtaatc ctagcacttt gggaggctga ggcgggcgaa 109380

tcatgaggtc aggagatcga gaccatcttg gctaacaagg agaaaccccg tctctactaa 109440

aaatacaaaa aaaaacaaaa ttagccaggc gtggtggcag acacctgtag tcccagctac 109500

tcaggaggct gaggcaggag aatggcgtga gcccgggagg tggagcttgc agtgagctga 109560

gatcacgcca ctgcactgca gcctgggcga cagagcgaga ctctgtctca aaaaaaaaaa 109620

aaaaatgtaa ttgatgtaat agtcccaaaa aagaacttgg cattaagtta aattataaaa 109680

tcagaaagct atgtaattta aatttgtatt caaaatctgt atattggcat gtatattctg 109740

tgccagttta tttaagatgt tactgtatca tgaagcttac ttaaggcata taatcgtctg 109800

cactgtaaaa caaactacca aattaatgta ctatctcaaa gaattaaaca tataacaatt 109860

ttgatgacca cctaaatttt agaacaactg tttttttaaa aaacttttta ctatggaaac 109920

ttttatatat ataatatata taaattatat atgttatata taatatatat atgttatata 109980

taatatatat gttatatata atatatatgt tatatattgt atataaatta tgttatatat 110040

aaattatata cattatatat aaagtataaa ttatatgaat tttatatata tatatttttt 110100

gagatggagt ctcactctgt cacccaggct ggagtgcagt ggcacagtct tggctcacta 110160

caacctccgc ctcctaagtt caagtgattc tcctgcctca gcctccctag tagccaggat 110220

gacaggagcc tgccaccatg cctggctaat ttttgtgttt ttaatagaga tggggtttca 110280

ccatgttggc caggctggtc ttgaactcca gacctcaggt gatctgcctg ccttggcctc 110340

ccaaaatgct gggattacag gcatgagcca ccatgtctgg ccaactatgg aaaattttaa 110400

acatacataa aagtagagta gtatatgaat cattgtgagc tcattaacaa aagataattt 110460

cagttcactt taaaaattga cgtgtgaaag tttggactca tacattttgt tttggtctaa 110520

agtttccttg gcaaatattc acatggttcc attgactctg gctccttttt aagataggat 110580

gcttttttat ttttttaaga gaaaaattat tgctctgaca agctaatcaa agatatttaa 110640

ttttggaatt tggaggaaag gcataaacct agtttattac aaaaatacct ctttttagtt 110700

ttcctagcct taaagtagga gacaacctcc ctccctttac acaggttata ctttcatatg 110760

gtatttgcat aaagaaatct tgttttcacc ctggcctaaa tatacatcca attttctctg 110820

caccttttag gtaactcttt tttaaggagg gtgtgtgtgt gtgtgtgtgt gtgtgtatgt 110880

atgcgtccgc atgcatgtgc atgtgtgtac acgcatgtat ggattttcat ggtttttaaa 110940

atatagacat aagtacaaat acatttattt ccccactctt catacataag atacataata 111000

ctgtatatat cattctgtat tttgttttta acgtttatgt aggccattct ggaaattttg 111060

tttcatacat aattttttat atatatacaa ctacagttcc attgtataga tgtactatag 111120

tgtatttaac cagtgatgtg tgtatggtca ttcaggttct ttctagactt ttgatataca 111180

gtcagtactg tagtgaataa tgttgcacat acattattct tatgtattgc aggcatatct 111240

gtgagataga ttcccagaag tatgcttgac taggtcaaag agaaatttgc atttgtgact 111300

ttgatagata caaatttcgc ttcatggatc ttgcttaatt atgagatgtc tgtttatagc 111360

ttcataccag tagaatatgt tatcaatttt tttttggatt tttgccaatc tgataaatga 111420

aaaataactt cactagtttg ggtttgcttt cctctgagtg agactgaaca tgttttcata 111480

tgtgtgaagg ccatttctac ttctgtttct gggacctgtc tctcatatat ttttcccttt 111540

ttctcttggg ttattggtct tcatctcaat ttttcaggag ctctttgtgt atcaggaagc 111600

taacacatct gctaaatgag ttgcaaatat atttttcacc atttgtactg tctttttact 111660

tcacttttaa tgtgtttgtt ttgccatcca aaagtttttt tttagcttta tgtaggtgaa 111720

tataatattt ttttccttta tgacttgtag attttgcatc agagttataa aagccttttt 111780

catttgaaga tttgaaggtg ccatgctttc ttctagttct tttatggttt gtttcactct 111840

ccttcccttt cttccttcca gcatttaaat atttgatcca tttgtagttt attctggtat 111900

acaatatgaa gtatggatga accttttttt ctagattagt tccagttgtc ccagcatcag 111960

ttatttaaaa gttcatcttt accccatttc agatgttgcc tttatcttat tcgcactttc 112020

tttatgtgtt agggtctatt ttggtctttc tgttttgttc cattgatttc ttcatctgtt 112080

gtacaggtac tatcatgaaa gaacaaatag tataaaaatt cagcctacaa aagtggaatt 112140

aagaaggact gatagatggc ttgccttggt aatagagcag aaaaccaaat ttttgcctgt 112200

tgatgaaata tatatgcaac ttaggatcag catatgtaga gaagcatgag ggagcatagc 112260

catatttgaa gtgcattact ggagtgccta agggaaatta ccaaagaggc tgaaggatag 112320

atagctccaa gtcattggtg gccttttatg acacattatg gaattggaat tttatcttac 112380

aaataatggg aaaattttaa gcaaactagt gaaatttaag attatatatt agaatgggtt 112440

aaatgctgag ttcagtgaac tcagcgtggg cttcagattc cacaaaccct atgaaatttt 112500

gtttcagaga gcacatttga ggctttcatt agattctcag aagtctttct ctctctcccc 112560

ctcccaccct cacacacaca cacacacaca cacacacaca cacaggtgtg catgcacatg 112620

catgcccaaa agttaagaat accttcttaa aagtaaaaaa ttaaaaacaa agataaaaaa 112680

gaaaaaaaga atgccttctt tagaaagatt tctatggtaa cataggatag gatacattgg 112740

atgtggttta aaactaaaga tcggtcacca gtcaggaaat tttggcatta gtataggcgg 112800

aagacgaatg gcacagggaa aagtgagctt tcacttaagt ttattatttc tgaaaggtta 112860

caaccagttc tcattgttcg tagtagttat ggtctataaa ttcactacaa acaattagtg 112920

aacactgaac cttgctccta ggggaagtac aaggattatt attatttaaa tgagccaaaa 112980

aacccctctg tatattggcc tctaggttgt ttcttccatt acaacaggtt gagacctatt 113040

ggcttaaaag tgtgcttgca ccaaactaaa ttttttgtac atctaattgt tttaaatata 113100

atcctgataa gcagattttt agccactgag agcctgccta ctttgcagtc cccacaaaac 113160
tgctttgtac tcaacatcta ttaggtataa ataagatcaa atcccaaggc tataaagatt 113220
ccaagctgct gttgcccttt ggagctcttt gatacataga ctccctgctg ggttgtctaa 113280
cattgtcagc tagacatata agctccctct cctactcccc tctcccctga gagtccccta 113340
gccctcctct tctgggtgat agccactcca cctcagcttc tggccagtcc tgtttgggta 113400
gtggccactc tgccttagcc tctggacagt atgctttaag gacatcctct gcctgcagat 113460
ctgtcaatgt cacccaataa agctatgcat gctactgcca cctcgtggtc atatcttttt 113520
ccttgatcag accccagatc cttcacattt aaaatacagt taggttccta ccagcctcta 113580
gtcacatttt catcagtcag tcgatacgta accttgtttt gtgtgtgttt ctgtttaaag 113640
acatattgtt gattcattaa cattgaactc acggctgaca ttactataac tcatgcctga 113700
aggaagctgg cctaacacat gtacttactc tgccagggac attatagcct tcttatgctt 113760
accagcccta aacagcacgt gagcactatg cttagggatt attttaaaca gaagaatcat 113820
caacaagaag cacaaaagtg agaaaaaagt ggcaccaaat aggctctgcc caggatactt 113880
gtttatagta tgaaaattga aacaagaagg caagagaaag cttcactgtt caacctcagt 113940
tggagaggtg catgcattag gcgactcaaa tatctcattg ctctgcgcat gtctatgaaa 114000
gcactaagag tattgacttt agagttacaa ataaatttta gcaagtagag agatttcaag 114060
tatggactcc acaagtaatg aggatcaact gtaccatcca gtctctttaa tacttccagt 114120
gatgagccca gtctctcatg acaaactttt accttgctgg gtaggtaaaa gttcagggtt 114180
tttctttgtt tgtttgtgac agtatctcgc tctgtcaccc aggctggaat gcagtggtac 114240
gattttggct tgccgcaacc tctgccttct gggttcaagt gattatcgtg cctcagccac 114300
ctgagtagct gggattatag gcgcccgccg ctctgcccgg ctactttttg tgttttttag 114360
tagagatggg gtttcaccat gttggctaag ctggtctcga actcctgacc tcaaatgatc 114420
cacccacctt ggcctcccaa agtgttggga ttacaggtgt tagccactgc acccaacctg 114480
gatagtttta atggttatat tgcactaccc tctgcgtcct gttatttctg gtcatagatc 114540
ctaattaggc tttctggact ctttccccct tcaatatatt atttgaattc agctgtctta 114600
tatttacctt tacccccttt ttttcccctc tagcataaat attcccaatt tctttggaaa 114660
cttttggttt gctaagcacc tcaatttaaa tgaatggaac ttattgttac aaactaacat 114720
ttccccttct tcttacacct cccaataaaa actgtgtcct ctgtattcac actgcctcac 114780
tgtagtctac accttcagtt ccaagtaact cagatttgcc tgtgggggat gaggcaagga 114840
aaaggctaaa gatagataac tttaaatata cagagagatt actttttttt tccctttccc 114900
tttccctcct tctctgtctc tctcttccca tgtcatcttt gctgcttcgt atgttagctt 114960
cattctataa cagattttct cagtgtggtg gagaatatgg ccattgaggg ctccagattt 115020
gtatcttctg caaatctaca gaactacagt agaaacaagg acttttctct gtcagtgtcc 115080

atgtgtcaat ttagggaaga actcaggtac tgtttgggtc atatatatag ccctggacca 115140

gttgctcttg ccgggggtat gggaaactga ttagccagct tctgtcatct gtagtgacat 115200

aaaagtagtg acccttgata gcttttctag taccatgtgg atttctagag aagggaatat 115260

tcccagagga aacaggggca ccaaacaaca aatatcaagt atacatgtta agacaggttt 115320

ttttcttccc gctatgttta gggccagtaa gaggtctctt aaggacagtc agtgtgattg 115380

aagggttata cagttttcag ctttgaacag tattggatca aaattgattt tgcttttaat 115440

attgacatct attattgctc agtgatggat atactgcgtt ggtgggtata ttgtagcaga 115500

tactgttact tcttcttttt atatgtttaa agtatttcat aattttaata aaatagaaaa 115560

ttaactttgc tttgatttaa gttggtgaat aataacaaat atttgggtta taatttccct 115620

ttagtattaa gttagctgta gaaatggtgt tgtatctgac ctagtaaccc atttgacttt 115680

ttaaagatga attactaaat ttttttaatg atatgaaaaa atgtaatttg ctccctttac 115740

ctcttatcaa tatatttatg ataccatagg tacctgcaag gtgtggagtt acagtccgag 115800

acagtctaaa gaaagcactg atgatgagag gtctaatccc agagtgctgt gctgtttaca 115860

gaattcagga tgggtatggt ttgtatgtga cgtgaaattt tgtttaaaaa gaaaatcaca 115920

cattaaactt tgaagttttc ttaggatctt taccaaaacc tagggaattg aaagtgtact 115980

ttaggaaaaa gtattaaaat aatactaagt tagcctgaag aaatactgta ggccatatga 116040

ggagttaaat aattgtatat gactgtaggg tttgttactt tgatcaaatg attttatttg 116100

gaatttgaga ttcttacaat ttttgaacca ttcagagtgt gatttatttg gataatagac 116160

tcttaccccc ctcccatttt taatacaaac tcatagtttc acaaaaggta tatcaaaatt 116220

aacattttat attgacctac ttttctttca gaaagtgtct aacattgttc caagaccctc 116280

acattttgaa tcctctttaa aaaaaaaaaa attattttgg gggcatgttg tccctgtccc 116340

ttgagtactc tttttccttg aatggataga taagtccgta cctgtgattt tttttttttt 116400

tttttttttt tttggacccc aggaacaatc cattttctgc tgttgtaggt cttttctgga 116460

gctgacttga agaaaagagt acatctcttt accctgctgt ttgtccaaga gtgatacatt 116520

tatttggggt aaacttaaaa ttaatttatt gccatttaaa tttctaacga tggaatatta 116580

gggagccaaa cctccctcac tgttactagc ccctcgataa ccaattttca tatcttcagc 116640

atgaggtata tgaatatttt taggtgtaat aaccaagaaa ggcttgtgtc tacatttttc 116700

agagagaaga aaccaattgg ttgggacact gatatttcct ggcttactgg agaagaattg 116760

catgtggaag tgttggagaa tgttccactt acaacacaca actttgtatg tatctttaca 116820

tttttttttg aaatgtcaaa aatgtttaga ttttaatgaa tgaattttta tttagggaat 116880

gtgaaatatg gatgagtaat tttggaactg acattttacc tgagttgaaa tcagttgttt 116940

tctttaaaaa cttgtattta aacaagagtt taattttaat ctttatactt tctttttaat 117000

ttaaaaaagt aaaatgtatg cattgttaaa agataatttt gaatagtgca gaagtatgtc 117060

aagtaaaaaa agtgaaaata ccctcctaga ggccaacatt tgtttagatt aacagattat 117120

tgttctgtct tccaaacttt tttctgtgta cacaaacgtg tgcttgtacc tgtaagccta 117180

aagttttttc ctttcttttt ttctctcttt ccttcattac tttcttttct tttccctttc 117240

ttaaatcaaa gtagagccat gctatgtgat attctttgcc ttattttttt ttaattcaac 117300

aggatgtcac ggacatcttt tcatgtcagt atacctggct ttattttagt atgactatgt 117360

aataattcgt aagaatagga attcattata tttaaccatt tctctattga tagacattta 117420

agtttttgta ttataaaaaa tctgttacat acagggctaa acaaggtctt tctgcatata 117480

tcttgacaca cttgtacaca cccttgtgtt tctgaaggat aggttaatgg aaatagaatg 117540

gctggatatt aaactctcca tgaggctttt ttctttgtct ggtttttgca tttgctagaa 117600

cctagcataa gcctaagggt caccagcata aggcccggaa tgtgggacct ttccacctta 117660

gagatgagga tctacataga acttgagaac ttatccccta aaatggcagg cagaagccaa 117720

aagttgtctc tgaatcagga atacatttct tacttttctc tatgtatgga attttggcca 117780

gagttttttc taggtggata attactgcta ccctttaggc gtcaagtgtt ttcccatgtg 117840

tcttggtgat taatccagct gggctcttaa agcagatgat tgattagatt atttcctttg 117900

gggtttatgc tttcaagcct ccattagtag tgaataatga aatcatgttt gtgtttctgt 117960

aaggcatttt ttgggagaag tgtaagatac acctaatgtc aatcaaattt tactgtgcag 118020

caaataattt tttaacatgt tgaattttaa gtggataaat cttaacattt ttatttaagt 118080

tcttaagaat acaaatacag ttgagcatac atttcatagt gattactact ctgtttctta 118140

ataattcttc ttagctagaa attgaatatg aggagataaa agaaagacct aagttgctaa 118200

gtgtgagaaa atagtattaa aatgatgcct caggaaggtc cttgccacag aaggattgtg 118260

aaagcaaact gcagattgat tccagagtat ggaaattgtc accactcagt tggatacctg 118320

ccagtaaatt tttattgaga actttccatt tgccaatacc atatcttacc tagggatatg 118380

acaaatgaat aagacagtgt cactgccata gagtagagag aagatttaat aaattttcat 118440

aaacattcat aattactata tatgtataaa gttctataga tgcacagaga ttcctaacct 118500

ggcttttgag gagttaggaa ggtttctcag agacagaaat atctaagctt aggcctgatg 118560

gatgaggaag taagtatcaa gtaaagagta ccctaaacag agggaagagc aagaaagcat 118620

gttacctttc aaagttttga aagaagttca gaatggatga tacaaggttg aagagtaggt 118680

tgttaacagt attgtgggta gaataagagt tgtgggtaga ataagcagag tccagctcat 118740

gaaaatcctt gttagccata ttaaagattt ggattttctt gtaaaataat tagaaatgta 118800

atagttgtaa gcttggggag aaatgtgatt agatttgtgt gttagaatga ttgctctgag 118860

tgccatatag aggcaagatc aaaaacctat gttctgctta gaaaggaaaa gtgttcttgg 118920

ctgtcttggg ttttgcttgg ctattcaagg agggctgctt atgcctcatg gtttcatatt 118980

ataaaagcaa tcccttcagt atttctctat atcccaagag tccttgggaa ctgggaagtg 119040

ggaaaacaag atttgaaact tttatatcca aaccttctcc tttttctgca gacactcagt 119100

gtcttccttc acacagcccc acaccttaca aattaatgca tgcaaattac cttgactgtg 119160

cctctcacta atttgccata catatttatg tatactcaga tactagatta agtgtaagct 119220

gtgacccaaa agaaagatat atcttcctgt gctcatcttt attgacaaag gtatacttac 119280

agatacaggc atatattgct taaaatttat gatcaaatgc atatccacat gttttctttc 119340

cttcagctgt tttggtcacc tacctacttg gtttggtgaa taatggccac ataaaaaatt 119400

ttaaagattt taaaatttct tgtatatcca gagacaaatg gaaaaagaac acaattagaa 119460

atagacattt acctgtttta tatcccctag aaagtgatac ataggaaaaa aggtgaagaa 119520

aataagagtc acttttaaaa ctaaatgtcc tcaaaaagcc agaatgtatt atatatcagg 119580

atgtaatttt cttgaaatat tttcaataac tttctattct taatggaaca gaatgtgtaa 119640

ataaatgtgt attgaaaatg gacttttggc tgggcacagt ggctcatgcc tctaatccct 119700

tgagaggccg aggcaggcag atcacttgag cccaagagtt caagaccagc ctgggtaaca 119760

tgacaaaacc tcatctctcc aaaaaaatac aaaaagtagg tgggcatggt ggtgtgcacc 119820

aatagccttg gctattcagc ctgaggtggg aggataactt gagcctggga ggcagacttt 119880

gcagtgagtc atgattgtgc caccatactc cagcctgggc aacagagcaa gaccctatca 119940

aaaaaaaaag aaaagaaaaa gaaaagtaga cttttgatgt tgaaatctat ttaatgtatc 120000

ataaaaaaat ttacatgtag cagaatagat taggaagttc taattcatgt tgtatatagt 120060

cagggtaagt agtgttgtat gaatacagtt atatatggag tcataatgta aaatatcatt 120120

atttgtgatt aaaactctga aaaactgggc acagtggctc acgcctgtaa tcccagcact 120180

ttgggaggct aaggtgggca gctcacgagg tcaggagttc aagaccagcc tggccagcct 120240

ggtaaaatcc tgtctcattt gaaaatcttg tgagttgtaa ctggttttat acaaaatatt 120300

gaagagtgga aattgtataa ttacaatcat gtaattaaaa gtattaacca ccccccccaa 120360

aaaaaaaacc tgtctctact aaaaatacaa aaattagcca ggtgtgatgg tgtgcgccta 120420

tagtcctagc tgctcgggag gctgaggcag gaaaatcgct tgaatccagg aggtggaggt 120480

tgtagtgagc cgagatcgtg ccactgcact ccggcctggg tgacagtgag actctgtctc 120540

aaaaaaaaaa tctctgaaaa actgaaatga attaagaata tagaggccga gtgtggtggc 120600

tcatgtctgt aacactctgg gaagacgagg caggcggatc acttgaggtc aggagtttga 120660

gaccagcctg gccaacatgg tgaaactcca tctccaccaa aaaatacaaa cgttagctag 120720

gcatggtggt gcatgcctgt agtccccagc tacttgggag gctgaggcag gagaatcact 120780

tgaaaccagg aggcagaggt tgcagtgggc cgagatcctg ccactgtact ccaacctggg 120840

cgacagagcg agactccatc tcaagaatac agagcaaaga acaaataatg aaatagaagt 120900

cacccatgct ctcgccactc tgaagtagcc actcacattt tgatatttat tcttatattt 120960

tcttattatt atatacacta aataaatata ttttaagcaa tttctggctt tagtgggata 121020

gattcttcct agtgcagttc tgttacgtga ctcatgttct acatcatttg cctttgacat 121080

ggaattctta acatgttgcc ttctaagttt cacctagaga agtgttcaca aataagttta 121140

tgtggcccaa acattctaat cctctaagaa attgatcatt tgttagaaaa aatagatctt 121200

attgtctttt aggtgatttt tctgtttctt atttttttta gtaagattag gaagagctgt 121260

ttcaattttc atatgattac ttactagttt tataaataat tgtttttaca tttttatcca 121320

aagttaacca ttatgttttt ggaccataga tcaggggttc tgattctgtc agctattttg 121380

tttttgtttt tgttttagct attatgtaga ttgtatttat agtctctctc cctcccaccc 121440

ccaattccac tctggaggaa ttcactgtta atttttaatg gtttctgttt taagctcttt 121500

tggtgattat tttcatctta ctaaatacag ttacacattg cctgatgact gggatatgtt 121560

ctgagaaatg catcaatggt gattttgtta tgtgcttaca caagcctaca tggtatagcc 121620

taactataca actaggctat atggtagagc ctattgcttc taagctaata acctgcacgg 121680

aatgttactg aactgaatgc tgtaggtaac cttaacacag tggcaagttt gtgtatctaa 121740

acgtagaaaa ggtacagtaa aaatacagta taaaagatca aaaatggtct acctgtgtag 121800

gatacctaat atgaacagag cttttaggac tggaaattgc tctggtgagt gagtggtgag 121860

tgaatgtgaa ggcgtaggac attactattt gttactgtag acttttatga taaacattgt 121920

acacttaggc tacagtacag ttttaaaatt tttcttttta aaaagtttat aaagtaaaaa 121980

atttacatta aactaaggtt taattttttt taactttttg actcttgaaa taacagtttc 122040

aaaagtttaa aaacattata tagctataca aaaatatttt atgtgcttat tctataaact 122100

tctattttaa aaatttttaa tttttctttt ttacttttta aacttttctg ttaaaaatga 122160

agacacaagc tgcacacggt ggctcatgtt tgtaatccca gcacttgagg aggcctaggc 122220

agacgcatca cctgaggtca ggagttcaag accagcctgg tcaacatggt gaaacccttc 122280

ctctaataaa aaataggaaa attagccagg cctagtggca tgtgcctgtg gtcccagctg 122340

ctcgggaggc tgaagcagga gaatcacttg aaccaggagg tggaggttgc agtgagccga 122400

gatcgtgcca cagcactcca gcctgggcga cagagccaga ctctgtctca aaaaaaaaaa 122460

aaaaaaaaaa aagacacaaa catacacatt agcctaggtc tacacaaggt caggatcttc 122520

aaggtatcac taggcaatag gaattattca actcctttat aatcttatgg gaccactgtg 122580

gtatgaagtc catgattaac tgaagtgtca ttatgtgaca catgactgca attatctttt 122640

agccacaatt tcttgcttta ttaactttag atatcatata ctgattactg attgtataag 122700

gaattagctc atttatagtt cttcctctcc ctcctctccc ccaatatttt tattagtagt 122760

tttttggttc ttctattggg tgcctttgta actttaatat atgcctttct tgttccgtca 122820

acaccagtcg gcatttctta acctcccttc tttgtatgat aattaaagta taggcttctt 122880

cctttcacct ctgtttcctc ctccttttca tgtctacttt tacttttatg ttgtcaagct 122940

tgaaatcagt tgccagcctt ttactcattt ctcatttttt aacttctggg tttttaagtt 123000

agatagtggg gtgggaaata aatatgtgta gtccatctat tatcttgaac cccggttttt 123060

cattttataa tagtacttta gtatgtgagt ttcatgatat aaatatatta caactttgtt 123120

attttaaaca agagagtaga tacgtcagtt tctagaaagt tttcttgtga gtttttgaaa 123180

tctctgtgat tttttacttt gcaggtacga aaaacgtttt tcaccttagc attttgtgac 123240

ttttgtcgaa agctgctttt ccagggtttc cgctgtcaaa catgtggtta taaatttcac 123300

cagcgttgta gtacagaagt tccactgatg tgtgttaatt atgaccaact tgagtaagta 123360

atccaaaaat atctcttttc tacctaccat tttacactta aattttctta atgtgaagct 123420

acgatgtcta aaagtctgtg agggtttttc ttccatacga ttgttataga gaattttttt 123480

taagtgtagt tagagaataa tatgtggaat ggacagtatt tctctcccaa attgtaatgc 123540

tggttcagct atacagttaa tttatatttt atattatcgt ttaattaatc aagaccccta 123600

acccatagaa accatttttg gatagtttct aggaggagag ggagagttgt ttcaattaaa 123660

ttaagcatta tgattttgta ccacagatca ggtagtctga ttctgttagc tattttgtaa 123720

attacgttta tattcttcct ctctctcctt catccccatt cagctccctt cccccaattt 123780

atttgagtaa gatgtaaaat ttttgtatcc agtatatatc tctttctaaa atttctcttt 123840

gctgtatgcc agtttttcta atagattaga ctgagtctat tatctctttt tgtgtcattg 123900

gtgctgctgc tgttaaagtc ttacttttct tgatcacctg agcaaataaa acttaactct 123960

gtactttaag taaattataa tgtcacctaa tttacagtag atacttttta ttctcattct 124020

ttaaccataa agcatgattt tcatcttgta gtatgtagaa atttgctgaa ctgaatgatt 124080

agttttaagt tataagcatg cctttgaaag tgcaatacaa ttttttttaa aataagcctt 124140

tagacaaaaa tacaactaat tgaattttaa cagttgtttc tgagaatgga atttgatctc 124200

agtttttttg gttaactatg tattttggta tatgaagctt ctgggttttg cacaagttag 124260

gtttgttttg ttttgcctca cagtttgctg tttgtctcca agttctttga acaccaccca 124320

ataccacagg aagaggcgtc cttagcagag actgccctaa catctggatc atccccttcc 124380

gcacccgcct cggactctat tgggtatggt ttgacttctg ctcttgggcg acatgctact 124440

tgaaccgctt tcttttggat ctcctggtta attagaaacc tttccaatgt ttaaattgtt 124500

aaattaagga ctttttcccc aaataactta tcataccact cagatattta catgcatttg 124560

atacaaataa atgggattaa agctgacata gactatttca gaatcagtcc tgaaaaaatc 124620

aatattgggt atgatttgac ttctgctctt cagtgacatg cttcttgaac tgctttcttt 124680

tggatctcct ggttaattag aaacatttcc aacatttaaa tgcttaaatt aaggatcttt 124740

tccccaaata acttatttta tcacaccagt cagatattta cttgcatttg gtacaaataa 124800

gtgggattaa agctgacata gactatttca gaaccagtcc tgaaaaaatc atgcaacaga 124860

tcattttgag tctacacctt gagttcatct tttattaggt atagaagtat atgacttcca 124920

cttatgaaga agcattgata tgtgagacaa tggcaaacaa tgtaaaaata gtatataatt 124980

ataatctaca atttatgatg gagtatattg aagtatgtga tgaggacata aatgtattca 125040

tgtttacaga aggaagaata gtgaggaaaa agagagtgct caggaaaact taatgaagaa 125100

ggtggtattt gaactagact ttaaagaatt actacaatct gaacgggcct agggaataga 125160

agcatggtga aaggggaatg gagaaacaac agatataaag ggaataaaca gatataaagg 125220

gaatgaagat gttaggttta gaagctagtg aagaaaggtt tatctaactt aagaactacc 125280

atgtgtaaaa ccagattatg gagagtcttg gaattgaggc cagaatttag acttaaaggt 125340

ctttaagcag attactaact tgatgaaaat ggctttaaag aaaaaatcaa ttagcagtga 125400

aatacagatg gattgacaga aaatttaggg tgaagaaggc caacctagga tgttgttggt 125460

agtgaaaact gagagaggca gtgaagacaa gttcaagtgc tagaagtatg gaaaagggat 125520

agatattcat aaagcgtaaa agaaaaaaat gaacagtatt attaatcagt tgaggataaa 125580

gctgagaagt gactttaaaa ataatgcaaa ggcagccggg tgcggtggtt cacgcatgta 125640

atcccagcac tttgggaggc tgaggcgggc agatcacgag gtcaggagtt cgagaccagc 125700

ctggccaaca tggtgaaacc ctgtctctac taaagataca aaaaaaaaaa aattagctgg 125760

gcatgatggc acacacctgt aatcccagct actcaggagg ctgaggcagg agaatcactt 125820

gaacctggga ggtggaggtt gcagtgagct gagatcgcac cattgcactc cagcctgggt 125880

gacaggtgag attctgtctc aaaaaaataa aaataatgca aaggcgtcat ttaagcttca 125940

tagtaggaaa taaaaaggaa gacacaataa agatgagtta agtgggtatc agtttacttt 126000

ggaacatttc tcgaactcct ggcctcaggt gatcttcctg cctcagcctc ccaaactgct 126060

agaattacag gcgtgagaca ctgcacctaa ttagctttgg aacatttctg acacaggtct 126120

gtgtactctt tcacattgaa tttggggcag cgttatttag gctgcgtctg gaagcacatg 126180

ctttaaaaaa aaaaaaaaaa aaaaaggccg ggcgcggtgg ctcactcctg taatcccagc 126240

actttgggag gccgaggcag gcggatcacg aggtcgggag atcatgacca tcctggctaa 126300

cacagtgaaa ccccgtctct actaaaaata caaaaacaaa attagccggg cgtggtggcg 126360

ggtgcctgta gtcccagcta ctcaggagtc aggaggatgg cgtgaaccca ggaggtggag 126420

cttgtagcgg gccgagatca cgtcactgca ttccagcctg ggtgacagag caagactccg 126480

tctcaaaaaa aagaaaaaaa aagtccccat acagcattct gtcctagaat attcctgaga 126540

tgttaagatt agaaatattt attgtcagtt ttaaccctac ttcctcccac tcttcacagt 126600

gtaatgccat ttcttcagat ttctacctag agaaatcttt tcaaaatcag ttgacatttt 126660

cagtacaact ttgtaatttt tacaaagcca acctttctgg tgatctcatg ggttagaaaa 126720

ttttgcaaaa gaattttgtc ttaagcagtg agtatactta cctatttgga acactggaaa 126780

atgcagccgt tgtgttctct tgtcaggttg tagcagttat ctcttctagt ggagtcattt 126840

tattcccgtt ggctctatca ctacttgtgt gcagctgtgt tttttttaga tggtcaaatc 126900

aaaatgttat tatttggttg gtttttaatt aatcttttgg ttataatatt ttctcctgac 126960

agattctgta gaccatgatg cctttctaat ggcaggtccc agttctgtga gcagttataa 127020

aacaccctaa tgaaattagc ttggcctctc tgaaggttta taatggtaat gatccaggac 127080

atttgccttc cagtgaagga atgctatctt aaaattaaga aaccatttgc ctgtgccatg 127140

aatattttat tggtaactga actgaaatat attctgatct tgagcaaatg ataagatgtt 127200

caaacttgtc tgtaagtcat tttcctgatg tttttatgac ataactccat atggttgtct 127260

ctgtaaagat agaagccaat cttgttcagt atcaaaactc ttttgcagtt tgttactagt 127320

cctgataaca ataataatgg tcatgaaaca agtgtaataa tggatgtaat caagaaatat 127380

tggagaagca atacattgcc cagttttgaa tacaaagtga gagatacctc cttttctact 127440

ttttaaaaat gctgtagaat gtataaattt gtgagaggtc aacatttaaa atgtacgtat 127500

caccagacca aagttttcaa aacttttaaa tattctagat taagaatgag atctaaacta 127560

actggtaatt tgctgaaggg tattataatt aaaattcctt ttgtctttaa tttggtactg 127620

ctttaaagag gtttactgca agataggtac aggtatcatt gggagaaagc cagtttgccg 127680

actgccaaga tacttcatag ggtataagtt accttggtta gtggaaaaat ttcccagtat 127740

ggtaatgtaa ttttatcgat tcccagaaat gatgagattg ggagttatac tggtttgtat 127800

taactgaaat cagaaaaaaa attgaatcag gataccaggt aatatttttc agtgaaaaat 127860

atacctgtta tgtaaattag acttcttgtg ttgtgtgcgc caccactcac tcttcttttg 127920

taagagattt atgggaaatc aaattataat cagaagactg ttttcgttaa gcatagaatt 127980

aggacatggc tgagatattc aatgacatca gattatgatc acttcaagtg ttcccttgta 128040

cttgccctga aagctagaga agttgacttg gtggaccaag acacaactat tagataccaa 128100

ctaccaaatt gagtttctct gattttgtat aatacgcaga tatcatcatt ttctaatata 128160

tacattcttt agatatgata gtgaagtgtc ttagattaaa tctggttttt gtttttcttc 128220

tggaccaatg tcttttgcat acttaactct gatttgttat ctactacatg tttctgtcat 128280

attcctaact tggtgagttt cagaagtgac ttactgccat ctctgcctat cccagatcaa 128340

ttattacagt agactatctt atgcaattct agttattcat actttttcca attttaagcc 128400

tttttttttt ttttttaaga tggagtctca ctttgtcacc caggcgagtg cagagacgtg 128460

atcttggctc actgcagtct ctgcctccct ggttcaagtg attctcctgc ctcagccccc 128520

tgagtagctg ggattacagg cccttaacac cacacccagc taatttttgt atttttagta 128580

gagacagggt tttactatgt tggccaggct ggtcttgaac tcctgacctc aggtgatcca 128640

cctgctttgg cctccaaaag tgctgggatt acaggcatga gccactgcac ctggcctgcc 128700

ttgagacttt aaatcagcct gtaaatggtt gtcagtcagt cagtgccctt tctaaaactt 128760

tattgactaa tgtcattttt gcattctttt tcctgctcct aaaatttctt agctatagac 128820

atatatttgg ctacctaaag caaaaataaa gacagctctg tcagaaacca aaagtttctc 128880

aataatcaga aaaaataaaa aggacctaga tggaacatgc taattttcct aaaggcttgt 128940

ttcttaccta taattctcat tgagccgata ccaatttttt tttagtacat aatattttat 129000

tatttgcata tcaattctaa gtggattcat ttcattaata taaacacatg aagtcaaaac 129060

ttctttcctt atctttaata atatgcttca aagaagtaaa attgtgaact ggtgtggttc 129120

agattctgac atgttttatt cagagactga ctttcactgt taggcttcct tggctcttca 129180

aacctttatt cattcctttc ctactatatt tttttcccat tcctcacgtc tcacaaaagt 129240

gtctttttat tccctcaaca ttgtctttct agctgtgtct tagtaaccac taataattag 129300

tttgcataaa ataggtggaa atgataacca atatgtgaag agagcttatt ggcacttagc 129360

cattcattgg tcctgatgga gttaagtgag acagcttacc tcatctatca agtgacactc 129420

atttccccac tcctaggata ccctttctga ggggctacat ccttccaagt gtttacaatc 129480

tagtctcaaa actttagtgt tctctgtgag tgccaggttc attttagggt gagatatcat 129540

agactatgtt atttagctac cataccgaaa taggtatgta acatattttg gtgattttcc 129600

aaatagcata caaatgtaac attttggtgg ttttccaaat agcagttttc aaaaatattt 129660

gctttagtgg ttaatatatg attctcttgt gtctctgtta tcaataatgg gcatgataaa 129720

aaatccagaa tatgagagat attggcactc tgaggatcat cttctgaatt tgaaaaggat 129780

ttttcaatat tgttctggat tttcattcaa ctcctgtaaa ggaacaagta catcattcag 129840

gtcctgaaat atgcatttgt attctcaaaa tatttataat ttcttaatat gtaaaatttt 129900

cattttagta aattcagatg tcaagacaat gttagaaaaa aatggcaaat tatattcagt 129960

cattctcaga gcatttttat ataacttcaa aggttgaact tcttcagttg atggccacag 130020

gtaatttcta gccataagta aatttcccta gtgttttcca ggtaagaatc agtggtctta 130080

tcattgatag ttcctggagg gcctacttga gcaaagcagc tttggcagta ttggattttt 130140

aaattaatac ttttaaaagt cattactgct aggtttttaa tgctttaatg attttgagaa 130200

tataaaaaca agaaaatcct tttatcttcc tttttaaata ttattacctt tatatcgtta 130260

ctctgaatct tatcttccaa tgacttcatt tttccaggcc ccaaattctc accagtccgt 130320

ctccttcaaa atccattcca attccacagc ccttccgacc agcagatgaa gatcatcgaa 130380

atcaatttgg gcaacgagac cgatcctcat cagctcccaa tgtgcatata aacacaatag 130440

aacctgtcaa tattgatgta agtatccagc attgctagaa ctaaaaaaaa accaagtatg 130500

tatctttatt tttctgctat aattataact tagatcagaa ataagtgcca tttttcattt 130560

atcacagtta ttttaagtga taagcttctt gtgaatcaca aatcagaaaa gcttctggtt 130620

tctctctgat gacattaaat atttcactga ctccaggtta tacagtcact ctgatttttt 130680

tcccttatga taccatctct ataaaagtca tcttcaaatg aaaatggttt aaatatcaaa 130740

ggactgatag aagcccttga cagaattaag ttctttaaaa cttttataaa aatgattatg 130800

attgtgctat aagaggtgga tatgaaatta agaatttcag gccaggcata gtggctcatg 130860

cctgtaatcc caacactttg ggaggccaag atgggtggaa cgcttgaccc agaagttaga 130920

gaccagccta ggcaacatag tgagacccca tctctaccaa aagtaaaaca aattagccat 130980

gcatggtgag gcatgcctgt agtcccagct actctggagg gtgaggtggg aggattgctt 131040

gagcccagga ggtcaaggct gcagtgagct atgtttgcac cactgcactc cagcctgagc 131100

aacagagtga gaccctgtct caaaagaaaa gaatttcaat ttgtgctatc ataagcttgg 131160

cattatgacc aacaaaaact tgattttttc tgtgtttatt ttaaaattag catataattg 131220

aaactataaa ttttattaaa tattaatata aaagaaaaac ttataaattt taattttgta 131280

attttaggta aatttgcaaa tcagactttc ttcccacttt ttactaagaa attttctcta 131340

tttttattgg gttcatttta agtgactttt ttctagtact agttttcctt aactagcaag 131400

gttcacctct atctagcaag acctaaaaac aaaggaagaa aggggaaaag gagaatgtga 131460

tataagaaat caaaccatat gtccaggtta gggttgttct cagtctgtcc aaaattgcaa 131520

ccttcatctt tactttgaaa actatcatcc ttttagacta ttcccttttt ctctgattgt 131580

taccactgtt ccctggtatg ttgggcttca tttagagtgt catctttgta tcttgccttt 131640

tcatcctctc tcgtaaccag tcacagggta tcaccttttc atgtgcatct caaactggtt 131700

tttacttatt accgctgcca tttgggccca tattttttca cttgaattat atactacatt 131760

aatctcctaa atagattttc tgccattgat ttctctctat ctatttccat tccttctgca 131820

actgtcagaa tttttttaca cttctagcat catctgttct cttgttccaa aacttttagt 131880

gacttactgt tgattacaag gtaaagtgca aactctttag catttttatt catgttcaag 131940

cacttatgat tccattaaaa agatttacta ataactgtgg gctatgcatt gtgttaggca 132000

attgggaatt atttatgaat tagcacatgg cctttgccct caaggaagtc acagtctaat 132060

aggtgaatca aacatttaaa tagataatta caaaatatat aatggtagtt tagagaaggg 132120

ggtaataaac tccatctggt tggatctagg aacattgagc agagaaggga caacctttaa 132180

gccagattat ggtgaataag taggagtaca ctacataagg gggttaggaa agtcatttgt 132240

taaggaaatg aaaggcatag aagtgccttt acatggaatg taattaatag ttgatattga 132300

agtttagggt ccaggagaag gcttggggaa tggtggaagg tgaaactagg caggtaatat 132360

ctacagtgaa aggctttgtg tattactctg aaatctaaag cagtgctagg gaatctgaag 132420

aattttgatt gggagaggaa gtcatcagct ctatatttta gaaaaatctt tgatggtaga 132480

gtggaggata gatgaaatgg gaaacacata gaggcaggac tgtcaataat gtggttttta 132540

cagtatttca gacaagaaat gatatttaaa ctcaagtaat agcattggtg ctgagaaaga 132600

gtgtgttttg gggagggaga ctatgaatta gtgaattagt ggtaagagtc ttaggaatca 132660

tgttgaaaat gactactatt tatgaatact tattacatac aggtactatg ctaagtgctt 132720

tacgtagact ttcttatttc atcctcataa aaactcatag gttatgtact atgattatct 132780

ttattttact gttaagaaaa ttagatttac agaggttaag aaacataccc agattacact 132840

gctgataaat tactgaagtg ggttcaaacc tggcctttct ctcttacact taaccactat 132900

actgttttgt agtagaggag aggagtgaaa aatatgagaa gtagaggata atgccaggtt 132960

tctggcttat agatacttag cttatagacc gagtttctgg taaatagcac agtttgttaa 133020

ataccaaagg aaaacaaggt ttgcagaagc agcaatttta gctttttggg ggcatatatt 133080

gactttaaga tgcctgtggg actttcaggt ttagaaatcc agtagcagtt ggatataagg 133140

accttgagta gagatacaga tttaggagta attagcatat ttatgtcagt taaagccatg 133200

gatgtaaatt gctcaaagag catatgtaaa ttgaaaaggg gagaaaatgt aaccctgata 133260

aacattaaca ttggaggtgc aggcagagac tctctgtctc ctttatgggc tggctcttcc 133320

taagtctagc cccagataac taagaacaag tgttgcagaa gccaaaggaa gaaaaggggt 133380

ttcaagaata ccaaagtagt tggtgtcagt tgccattacg attcaaatga gataaagact 133440

gaaaggacta tcaattttgg caattgaaat gtcatcttta cttcattgag agctgtttta 133500

gagggccagt aagaggagaa ggcagcaggt gaggaagtag gattaagtgt taattttgag 133560

aagcttggct gtgaaggaat gacaaaagag gatagcttga ttcagggttg agggacaatt 133620

ttttttggtt tttcgggttt tgtttttgtt ttgttttgtt ttttgagatg gagtcttgct 133680

ctgtcaccca ggctggagtg cagtggtgtg atcgctgctc actgcagcct ccacctcccg 133740

ggttcaagca attctcctgc ctcagcctct cgagtagctg cgattacagg ctcccgccat 133800

catgcccagc taatttttgt atttttagta gggttttgcc atgttggcca agctggtcct 133860

gaactcctga cctcaagtga tccgcccacc tccgcctccc agagtgttgg gattacaggc 133920

gtgagccact gcacccagcc aagggacaat tgtttttaat gtaagagaca ttagtatatt 133980

tgtaactgga gaggaagaag gcagtgaaga gaaattgaag ataacaagag aggaaatgat 134040

tgatgtagta aaattccttg agcttggaaa ttatgtctct cagagactgt gaagaattaa 134100

agatggacat agccagctct gtaactcttt acagactgtg tgatgttggg caaattattt 134160

aatcctgggg ctagtagtgt ttgccttcca tagtggtttg aattgcttat tatgcctggt 134220

acataataag aattcagaaa ttatagctaa tattaatatg caaatagtta tagatattag 134280

agcagaaaag ttgtttgatg gcttttgttt tctctattat gatgaaggga agggatgtaa 134340

gtaagagaag gaactacaaa agagtgggaa aaaagttgaa atatccagtt ttcaaatgct 134400

agaagaacct ttgtaaccta gaatgagtag aaaagattgt caaggagctt taagaacaca 134460

ttcggaattt aaaaatctaa gtttatgttg ttactagcag taacttgtaa gagtggagaa 134520

agcaaaattt ggttaatcca tagttgtgga gagtttcaga gctgatgcaa cagaaaagaa 134580

agggatatgg cctttgtctt gtagttcctt ccacctgaaa gactctttgc ttttctacat 134640
gcctatctct gaaaccccaa ctcagagtaa ttccttgact gctttatcag tgaccaagtc 134700
ctatagttat catacacagc actaaaaatc ttatcggctg ggtgcagtgg ctcacacctg 134760
taatcccagc actttgggag gccaaggcag gcggatcgcc tgaggtcagg agttcaagac 134820
cagcctggcc aacatgatga aaccccatct ctaccaaaaa taaaaaatta gccgggcatg 134880
gtggcaggcg cctgtaatcc tagctactcg ggaggccgag gcaggagaat cgctggaacc 134940
caggaggcga aggttgcagt gagctaagat cgtgccattg cactccagcc tgggtgacaa 135000
gagcaagact tcatctcaaa atcttatcac ctgtatcact tagttggcaa tcaattgagc 135060
agcaaacttt ggcatctctt ttattatatt cttatgcaat tattcttaaa ttatttgatt 135120
ttcacttact tccaatgtgt gcatcttact tcccatgaga ttgtaagctc tcaagaatgg 135180
aaagttaatg acatcactag gatttttata tttgttagta gccatataac tcctgtcacc 135240
ttcttttcag gtacgtattt gatttttctg tagaaaatgt tgaagacttt atatgataca 135300
ttaaacatga tagaaataca tctttaaaga atttactttg ttttagcctg taaacaaaaa 135360
gttgtctatt tgcagagact attcagagat atttggggcc attcaatccc tcatatttaa 135420
gttaaactaa ataaacagac taatgcaagt tctacccatc aaggcccaaa ttgcattacc 135480
agtagcgact gtccccacta ccatcgttgt tataaagagc taaatatata tatagttttt 135540
tttttgtttt ttttctgtga tggagtctca ctctgtcacc caggctggag tgcagtggtg 135600
caatctcagc tcactgcaac ctccgcctca caggttcaag caattctcct gcctcagcct 135660
cctgagtatc taggattaca gtcgcgtgcc accatgcctg tctaattttt gtatttttag 135720
tagagatggt gtttcaccat gttgaccagg ctggtctcaa actcctaacc tcgtgatcca 135780
ccagcctcag cctcccaaag tgctggtatt acaggcttga gccaccgcac ccggcgcata 135840
aagagctata ttttaataat aaagacaaat tttagtggcc ggttgcggcg gcttatgcat 135900
gtaatcccag cactttggga ggctgaggtg gacggatcac ctgaggtcag gagttcaaga 135960
ccagcctggc caacatggtg aaaccccgtc tctactaaaa atacaaaaat tagccaggca 136020
tggtggtgcg tgcttgtagt cccaggtatt caggaggctg aggcaggagg atcacttgaa 136080
cccaagaggc agaggttgca ataagccaag atcacgccac tgcactccag cctgggcgac 136140
agagcaactg agtctcaaaa aaaaggacaa attttaacaa aacctttcta tgagccactt 136200
tgtttctttc ctcttctagt gtgcccttat ccatccatat ttttatgatt gtaaccagtg 136260
tacttttaat tttatatttt taaattatac tataaacatg tttcatgatt caagcttcat 136320
aattattttg gtagctgcat aatacttcat taaattgata caccataatt ttcttaacca 136380
aaatatgtca aaatgcctat aatagagaaa taattattta taacttttta gtatgatgga 136440
taatgttgca ctaaacatct ttgtgcatat cactttttct tctgaattat ttccttaaga 136500

aaagttccca gaagtagaat tacagaatca aaggatatga acatttttat ctctcttaat 136560

gtgcaccagt ataatttttt ttaaggattg atgaagccat tttttaaaaa tttatttatt 136620

tccaaagttc aggggtacat gtgcaggatg tgcagggttg ttacgtaggt caacatgtgc 136680

cacagcggtt tgccacacag attatcccat tacctcggta tgaagcacag catccattag 136740

ttattctttc tgttgctctt cctcctttta ccatccaccc tccaacaggc cccagtatgt 136800

gttgtttccc ccatgtgtcc acttgttctt atcattcagc tcccacttat aagtgagaaa 136860

acacagtatt tggttttctg ctcctgcatt agattgttga ggataatgga agccattggt 136920

tttgaatggc ctgaaatgga catcaacatt tgattaggac taataattgt ttcattatag 136980

gtttacattg gcaagtgctt caaaatttag attgtattat gttcactaga taattccaaa 137040

ttgttttgtg taatagttat aagatgtatt gttttaatta ataaaataat tcttttaacg 137100

ttagtggaaa attcagtgtt atcgctactc tctgattata tgcttgcttg gaataaatat 137160

acattactat ttatttgtag gacttgatta gagaccaagg atttcgtggt gatggaggta 137220

agtagtgatt tcaggttttt ttaaaaactc aaggaaactg caattgcttt gctgcttatt 137280

tcctttatac ttgcctcttt caagtaacag acacagagaa aaatgtgtag agaaacccaa 137340

aatttttttg tttttctgta gtgtttgtca tttacctcta ataaaatgtt aactagttta 137400

taacatgagt agaaaagatg actggacata aaaggaagtc tttttttttt tttttttttt 137460

ttttgagacg gagtctcgct ctgtcgccca ggttggagtg cagtggcgcg atctcggctc 137520

actgcaagct ccgcctcccg ggttcacgcc attctcctgc ctcagcctcc cgagtagctg 137580

ggactacagg cgcctgctac cacgcccggc taattttttg tatttttagt agagacgggg 137640

tttcaccgtg ttagccagga tggtctcgat ctcctgacct cgtgatccgc ccgcctcggc 137700

ctcccaaagt gctgggatta caggcgtgag ccaccgcgcc cggccaaaag gaagtcttaa 137760

aatgtattat ctacagtttt aaaatttctt ccaggatcag acaagatcgg acacgttcag 137820

gatggtatgg ccgtagacta cagttttaaa atatcttacc aaggaaagat ccttaatttt 137880

tatacccgct ttattaattt ctaaccatct tgaaagctat tgttgataaa tttcctttgt 137940

ggggctccac tgatacttaa agattgacct tagaatcaga taaaacttaa ctttgctaaa 138000

tcattctgaa gagggggttt gtcagacatt atcaaccact tccttcaact ttctggaagt 138060

gttttaaatg tacattttat agaacagacc cataatggca aagcccattt gtcctcttct 138120

taggtcagta aatacacaaa tgagaaactg aattgagatt tccaactgaa ttttcatcta 138180

gtattcactc tagcacataa gacaacattg cttaagaaaa tactttttgt aagcattacc 138240

ctatataatg ttttataaga ggtgatattt gagactgtct tgaagtgttc ttccaggagg 138300

tcctttacac ttaccttccc tgttgtcttc tgcctagtaa ggaagacctg taataactgc 138360

ttatcatgct tagagttgac ctcttcactg tgaccttctt tatcttcaaa atatctaagc 138420

ccagactcaa caatatttta cattgagtaa acattgttat aaaccttctt ttgttatgtt 138480

tctgtatacc catgaagcaa ccaaaataat aataagcctg cattctatac tctggacttg 138540

gtattgatgt tagcacatag ttacacaagc tttttttttc ctgtttgtta tttcatgaac 138600

ctgccaatta atgttgctgc cagtttgact ttcgtatgtc ttaatagctg tggcttttga 138660

taattttgcc taatacatcc agcatttaaa tgttgccatc atgttagcat cacaaaatta 138720

acttagtcat aaacacagcc tgcttagtac ctaaaagcaa gtggcatttc ttgtcctttt 138780

catgagtcac tttttaaaaa atcattggga ttttatgaaa ataagcagat ttttggtcca 138840

gaattatttt atgaaacagg cttcaattca tcttgtttat tccccatgac ttctttcatt 138900

tcttctgtgt gtctgtcttc ctgtgtttgc ctgcccctct ctttctcttc taacagcccc 138960

tttgaaccag ctgatgcgct gtcttcggaa ataccaatcc cggactccca gtcccctcct 139020

acattctgtc cccagtgaaa tagtgtttga ttttgagcct ggcccagtgt tcagaggtag 139080

ttgggctctt ctttcttgtt ttcacccaaa gcaaactaaa tataaaacta cagatgctgt 139140

ttgtgcctca ccctcacagc gtgtgtttgt aagtgtgaaa gttttcagta ctaaatttct 139200

gtttggcctg gctggaatgc tttgaatgta cgtctcacac gtactcactg ccacaagctt 139260

tctgtatgct gtctgtcata aatttttaaa agcaagaaaa tcctgacctg agatttccat 139320

cttgtttttt cgttatttta ttacttcttg gtcttgataa tttcttaaac ttagtgggtg 139380

ggaataaata aggtgggtgg ggaagagctt actggattcc tttgatttta atgcatttaa 139440

gtgattattc ttgatgactt aatatttgtt aattttgtgg tttttaagaa aattaaagtg 139500

tcaatggaaa cttctattat gagattttat taggcttttg gccttttttc agattctgta 139560

atactagcag tgttttttgg gtttttcttt cccccaatat gggatgtgta tatttttgtc 139620

aaaggtaggg agctgttaaa aaagacaaaa aaaagattta taacatattt tagatatttc 139680

agtgtacttc agaaatttga gaatttatcc ttttaattat gtcctaatag aagaaagttt 139740

acagtataat ttcattctcc catttcatct tgccatgttt tatttagtag ttaaactgat 139800

ttgtaaaaac ttaagtcggg ccaggcgcgg tggctcacgc ctgtaatccc agcactttgg 139860

gaggccgagg tggatggatc acctgaggtc aggagtttga gaccagcctg gtcaatgtgg 139920

tgaaaccccg tctctacaaa aatacaaaaa aaattagcta ggcatgatgg cggatgcctg 139980

taatcccagc tacttgggag gctgaggctg gagaatcgct tgaacccagg aggcggaggt 140040

tgcagtgagc cgagatcaca ccattgtact ccagcctggg tgacagagca agactccatc 140100

tcaaaaaaaa aaaagtaaaa attgggacag atgtctttct ctaaatattt ttaaagattt 140160

atatttactg actcttgcta gttagtatct gttatatatt ctgaatgtag taatggtgct 140220

ttagattttt gctctctcag ccctgctgtt tctcagaaaa tccatagaat gggatggaag 140280

tcatacagta gtgagtaata caactaaatt aagtgataca ataaaactac ttagtagatc 140340

ataaccgtga agcctggtca agcagtcgag gctttataat gttgaaaatt atcaatggaa 140400

ggtagaaaat ggattgtgct ctacttaata gacattgtgg ataccatttt attttagaaa 140460

attgcatatg agataatgaa aattctacat ggtgatataa tatgatgtaa taatggtaaa 140520

cattttctac agattaaaca tttaaatgtg gttattggta tccttgttat ctgaaagata 140580

gtggctcttt ttttccttaa gagtagcagt cattttttaa aaagaatcta ttttcttgag 140640

gtcattttgt tgttctgtat atagaactat tgcctggaca tctgagttct actcagctgt 140700

attcaggccc cagtaagatt cactgccctg aactcttctg aaccaggtgc tactgtacct 140760

taactcagga tgtttgccat gagaaaggta tgcaaccctg ccaacagaga tcacttccaa 140820

agagtatact cctcaggctc acttgaccta tagaatattt gtatttatag taacttggct 140880

gagaggccat agcgcttact taacaaagct ctcacttaca aaggcagaga tttttcagaa 140940

agtcttgaga aatatgcccg gctttattta cattaacttt gttttgtagg taacaaataa 141000

tctttgttta ataatgtaag cctccaggaa ccaatgatac tgaccaatat ctcttaaata 141060

gtagagcatg tagtttagga ttatatttga gtttagtgat taatatgaat aagtcagata 141120

ttttcaacat tatggccatt attagaaaat gtttccatct ggggatttcc ttttttttaa 141180

tattgattgg ctgttgaggt aatattaaat aattaattaa aaatgtattt gttatatagg 141240

cttttacatt tattttgctt tttgattttt ttcatcaaag aaacagaaac ttgggagtat 141300

ttttagtatt tctgtcttgt tttagagaga ttgtttttct cctagatttt gcaccagtaa 141360

ataaagtatg tgtctatgtc tatcatcaga tatcttaaag gtcattaaat tggccagaaa 141420

actaaaagaa attatagttg taatcaccaa atgaggcccc tttttggccc atcctttcca 141480

aaaggtctat atttaaacat gcactacatt tttaaattaa gtctaaatat cccccaacct 141540

tctacccctg ataaattaac atacttgctc ctccttaatg tatacatttt tcttttcact 141600

aatttaggat caaccacagg tttgtctgct accccccctg cctcattacc tggctcacta 141660

actaacgtga aagccttaca gaaatctcca ggacctcagc gagaaaggaa gtcatcttca 141720

tcctcagaag acaggaatcg aatggtaaga gtatatgata tctttttttc tctgaattct 141780

ttcttcttag aagtcacagc caaatgtaat attatccttt agatatatta tgtccatatg 141840

tgacacagaa ttcccataat taaataaatt taagaactga tagttttttg cttaaagcat 141900

atttctacgg cactgctttt tgctgtcatc tataatataa tttagtaaaa ggcagttttg 141960

gaagagtaac agtattctgt tctaaagtaa ggaaaaagag agaaagctaa tattagaagg 142020

cacgaaaagg ctggtccaga attcagatat ttcagatatc tactgaagga cattcttccc 142080

tatttaaaaa atcaactttc ttctgcaaaa tgaatccacc atggcacatg tatacgtatg 142140

taacaaacct gcacattctg cacatgtatc ccagaactta aagtaaaatt taaaaataaa 142200

aaacgaatac tgtttagccg tagtattgct actaattgtt gaataagagg atcttttacc 142260

ctaccaaagt aattttatat gttgattttt tttttttttt tggaaagacc gaattagata 142320

agatacatga agaaatttag cactgattga aaaagactac ctagatgaat tgtcagtagt 142380

taccacaggt taacttaaaa ttttttgtga tttagagcca aaactattca caaatatagc 142440

agcacttatc ttgctcctta aagtcttcca gatgataaaa acattttact tatttcagta 142500

atatacattc ctgctcatac cccataaata atttatattt tttaataaat tgtttccatc 142560

ctaaccatcc ttctgagcaa agtatcacaa ggacagaaaa ccaaacacca catgttctca 142620

ctcataggtg ggaattgaac agtgagaaca cttggacaca gggcagggaa catcacacac 142680

tgggacctgt catggggtag ggggagggga gagggacagc attaagagaa atacctaatg 142740

taaatgacaa gttaatgggt gcagcacacc aacatggcac atgtatacat aagtaacaaa 142800

cctgcaagtt gtgcacatgt accctagaac ttaaagtata ataaaataaa aaataaaaat 142860

aaattttttc catcctaata ttgacttcag tcttaaattt aagttttgta ttttaagagt 142920

catactttta actactattc ttccagagaa tttttcttaa ggggatctct tcctgtatcc 142980

ctctcaggca taaggtaatg tacttagggt gaaacataag gttttctttt tctgtttggc 143040

ttgacttgac ttttttactg tttttatcaa gaaaacactt ggtagacggg actcgagtga 143100

tgattgggag attcctgatg ggcagattac agtgggacaa agaattggat ctggatcatt 143160

tggaacagtc tacaagggaa agtggcatgg taagtatgta atgtggtgac attgtgacaa 143220

gtcataatag gatatgttta acaactttta ttttgtaaaa aatatcatca aaggaaatat 143280

tcactgttcg catcaataaa ctattttgat tagtttcagg actcctccaa aagtttctaa 143340

caaaaattat gggaaataaa aactgttcac agcagtcggg actcctacca ttttattaca 143400

gtaataattt tttaaaggga attcctccag gttaactagt cctcaaaagg attttatttt 143460

cttttagagt ctttcagctg ataattttat ttgtattata agtcacaagt aaacatatta 143520

aaaatgtact taatggctgg gcgcagtggc ttatgcctgt aatcccagca ctttgggaag 143580

ctgaggctgg ctgatcacga ggtcaggaga tcaagaccat actggccaac atggtgaaac 143640

cccatctcta ctaaaaatac aaaaattagc tgggtgtgga agcacgtgcc tgtagtccca 143700

gctacttggg aggctgaggc aggagaatca ctggaaccca ggaggcggag gttgcagtga 143760

gctgagatta cgccactgca ctccaccctg gtgacagtga gactccgtct caaaaaaaaa 143820

aaattaacaa agaaatataa gtggccagta aacatataca aaatgttcag ccttactagt 143880

tatcaaagaa ttgcaaattc aaaaaataga catcattatt tgcctcttag ttggacaaaa 143940

tctttttaaa ttggattata ttaagagtag tggatgtatt ttcatcaaag gtttaatatc 144000

aatgaaaagt gaaagtgaac atgtatccaa ctaatagaga attggataaa tttataccat 144060

catatgtgat tatataggag ttaaaatggc atggtagagg tacatttatt gatgtagaaa 144120

ggtgtctttg gtatatgaaa tttttcaaag cagtatgtgt aagataccat attatggagc 144180

tcatagaaat atataacata attttttata tgacagtatt ttaggccagg cacagtggct 144240

cacgcctgta atcccagcac tttgggaggc cgaggcaggt ggatcaccca aggtcaggag 144300

ttcgagacca gcttggccaa catagtgaaa cctcatctct actaaaaata caaaaaatta 144360

gccaggcttg gtggtgggcg cctgtaatct cagctactca ggaggctgag gcaggagaat 144420

tgcttgaacc taggaggtgg aggttgcagt gagccgagat cccgccattg cactccaacc 144480

tggataacag cgagactgtc ttaaaaaaaa aaaaaaaaaa gactgtgttt tagtttttat 144540

ctccttaatc tatcttttca caggtgttca taaatattca cactaaattc atgtaaaagc 144600

ctaataacat ataatgtcac ttttgagtga cataattaag ggaatttttt tataccttca 144660

aaatgtcttt aaacttttct taagtgctgt acagtatttt atgatacaaa cagtagaata 144720

agcactgtat tactttgata attgaggaaa atcaatgttg atttaactta ttaaaatata 144780

catacaggtt gagtatcttt atttatttat ttttgtttgt tttgttttgt tttgagacaa 144840

ggtctcgctc tgtcgcccag gctggagtgc agtggcacaa tctcaactca ctacaacctc 144900

tgcctcccag attcaagcag ttctcctacc tcagcctcct gagtagctag gattataggc 144960

gcgtaccacc acccctggct aatttttgta ttttgagtag agacgagttt tgccatgttg 145020

gccaggctgt tctcaaactc ctgacctcag gtgagccacc caccttggcc ttccaaagtg 145080

ctgggattac aggtgtgagg cagcacacct ggccaggttg ggtatcttta atccaaaatc 145140

ccaaacccga aatgctccaa aatccaaaac tttctgagtg ctgacatgat gctcaaagga 145200

aatgcttatt ggaggatttc atatgtttgg attagggatg ttaaactggt aagtataatc 145260

aaaatattcc aaaatcagaa aaaatttgaa atttgagaca cttctggtcc caggcatttt 145320

ggataaggga tactcagcct gtgtataaaa gtgcacataa attagccagg catggtggca 145380

tgtgcctgta ctcccagcta ttgaggaggc tgaggtggga gaatggcttg agcccaggag 145440

ttcaaggctg cagtgagcca tgatcacacc actgcactcc atccagccta ggtgacagag 145500

caagactctg tctctaaaaa aattaaataa acagaacatt actagcactc tagaaacacc 145560

ctcccatgtc ctcttctagc caatcacctc tctcccaagg gtaaccacca ctgtgattac 145620

aacaggaagt gcatagtgtg tactcttttg tgtcggccct tttcactcaa cattgtttat 145680

aagattcatc tatattgttg tgtgaagttg gaggtcattc attctcttta cagtatttca 145740

ttgtgtgact ataacatgat ttcttctttc atctgttgca attggatcgt ttccagtttg 145800

gggctttgat tgatgctggt gctgtaaaca tttttagtgt atgtcttttg gtgaacatgt 145860

aaccattgat gggtatatat acctaggaca gaattgtgag accacagggt atgcatatgt 145920

ccagttttag taatgctgcc aacaatgtta caaagtagtt gtaccaattt aaacacctac 145980

tggcagtgtt gacgttacag ctgtttcaca taaagttttt tttttttgat gattttaata 146040

aaatatcatt ttcttttttt attattatta tacttttaag ttttagggta catgtgcaaa 146100

gtgtgcaggt tagttacata tatatacatg tgccatgctg gtgtgctgca cccattaact 146160

cacatgaagt tttttttaaa ttttagtgac agttttagtc attttcctaa ttgaaagtat 146220

cataagtaat ccataaattt gaaaaaaatg ttaactactc tgataaaaaa gttttatagt 146280

ttcctacttt taagcaaaat tccatagggc ctggtaattg tagtttcaac attacttgca 146340

gtttcagtta gtaaataaat attaagccta gtaagtataa tttaatattg tcaaataatt 146400

tggaaaatac catgggtact taattgattt taccaaattt ccatggaaca aacaaggttg 146460

gctatttttt ggattgatat tttgaaatac tagtacagga atatcattgt tagttgaatt 146520

tttagcctta gaaaacaaat ggagtttaga tagctaaagt ataatttatt tgtgatttaa 146580

taatggtatg gagttagggc tatgataatt agtgaaaaca cccaagaatg ttttatactt 146640

ttaaatttta aaaattgaaa tgacacttgg agtaacaatt gccttttagg tgatgtggca 146700

gtgaaaatgt tgaatgtgac agcacctaca cctcagcagt tacaagcctt caaaaatgaa 146760

gtaggagtac tcaggtgagc ttgtgtgaat tactcttttc cagagaaaga agttattttt 146820

attagctcct ggttcccagt ggtagcaact attagcttta cagatttact caaaatgaat 146880

aaatttgtag aaacagagta tgtctgagta tatttttgtc tttaaccaca ttcttttaag 146940

tagtatgcaa tgttatatgg tatggctgat agaatactta gtcctagact gaattaatgg 147000

aagtatagta ttctgataat ataaagtaat agttctactt atgaaaagaa tactctccag 147060

ttttaagctt atcagaatac atttagaggt ggtatttagt cctgggctct ggaattttag 147120

aaacattgac aaactaggat atgcctagtg aggaccacct aaatagggaa gattctagag 147180

gtgtaacggg ggaaaataat caacagaact gaggatattt agttcacaga aggctgttat 147240

gttcaagaga gtgcacagtt attccagagt gcggaaaaaa aagttattcc agagagcaga 147300

ccagggaagc aagccagagg tgaaagttgt aagaaaatga ttttgtctca acacttggaa 147360

actttataat accagaacca cttaaataaa gatatgagag tcagctacaa ctgagtgatg 147420

aacttcccat agttgaaggt atttaagcaa cctctagttg cctgtcagat atatttaaaa 147480

agatatctct gcataaagta ggaggttaga cttggcaatt gccagtctct tctaaatgta 147540

tccttttgtt gcctttttta aaaaaaaaaa agctttttct gacaacattt taccgacaga 147600

ctactttggt tctcttttgt aagaattgct aaagtttgtc gacatttaat gtttactgtc 147660

acatttcttt gtacaggaaa acacgacatg tgaatatcct actcttcatg ggctattcca 147720

caaagccaca actggctatt gttacccagt ggtgtgaggg ctccagcttg tatcaccatc 147780

tccatatcat tgagaccaaa tttgagatga tcaaacttat agatattgca cgacagactg 147840

cacagggcat ggagtaagtt ccattcgtta aatgtcttgt aaattatttt tgaagaccat 147900

tgaggatgtt ttaaaggttt tggctgctat tcttttggat tgcattttaa attactgtcc 147960

aggaacataa ggatgctaac taatggctgg taaataatat gatactaaaa aataaatgtc 148020

tctgtctagt gcagccttca gaacatatat caagtatttg ataataaata catgactgca 148080

aacttaggct tagcactcag tgattgagct aagcaagaga ggttcagaag atagaaacag 148140

caaaaacctg ctaaaaagtt gttagcagtt gtgcaagtaa acagaatggt tgttagttac 148200

tttttcaaat cagtttctct gagtgcccgt atttttggtt gcaaaatggt cagttaataa 148260

agttaaagtg aaaaatctgc attctgaccc tttttgagga tttcagagtg agttcctatc 148320

tgttgaattt tgctatgcaa tttaaggagt tattttataa agtttaccat aagctaatat 148380

ggggaactga ctttgaagga taaattttaa attttgcaac tcttaagtgc aaatgaatag 148440

gtaaattaaa aggtaaaatt aaacaaattt tgaaagcact taggtgaaaa ttataaactc 148500

agtaaaatat gaatttgaaa gctctgtgag aagtttaaaa atagatatga tctgaatttt 148560

gttttttaca aattgctttc acttacatag attattatgt catttaatct ttataatgtt 148620

atgaaggaga tcttttttct ttttacagat aaggaaattg aggcttttaa gttccttgtc 148680

taagggcaca catttaataa gtggcaccaa aggtgtttaa ctcaggattt ctgactccca 148740

atccagtatt ctttccccat aaccactatg ctacttttac attacaaaat tagaataaaa 148800

gagtaaaagg gtatatatgt actaacacct acaactctaa ctgagtattg ctcctagcaa 148860

gtaagtatag agccaagact ctaaaccaga tctggctctt agatcttcca actataccac 148920

cttctctttc tcaaaactag gcaatatatc tataatttag attgtttaca agcctatatt 148980

cggccaaaat acttattaca gcaaattatt accttattca gtaacacccc cacttacccc 149040

tagacttgaa acaatctcaa cgtttcagat aagttagaat ctctgaatct gttcgaatct 149100

aaaggctttt aaagaattaa aatcttggcc aggcacagtg gctcacactt gtaatcccag 149160

aactatggga ggccgaggca ggcggatcac ctgaggtcgg gagttcaaga ctagcctgac 149220

caacatggag aaaccccgtc tctactaaaa atacaaaatg agccaggcac aagcctgtaa 149280

tcccagctac ttgggaggct aaggcagaag aatcacttga acccgggagg cagaggttgc 149340

agtgggccaa gatcacacca ttgcactcca gcctgggcaa caagagcaaa attccgtctc 149400

aaaaaaaaaa aaaaagaatt aaaatcttgt gaagagtaaa ccatggcaaa gattgtgaac 149460

attcagtgag aaacaaaggc ttattctctt cccacaccac tggtctcttt gcaatttctt 149520

cagcaggcca agcgtgttcc ttctttagga cctttatgtt tgttatattc tctttaggat 149580

acacatgcca caaatatcct tgtgtctcgg ttcatatgtc cccttttaat agaaatcctt 149640

gctcacctta tataactaac atgtccccac gtcactctgt cactctctat acccataggc 149700

ttgattttct ttataacgca tagctccatc tgacttgttt cttgtctttt atccccacta 149760

gaatgcaggc tgtatgagag caggggcttt ttttcattat tttatgccta atgcctagaa 149820

tgggacctgg catactcagt acataactgt taaatgaaaa tgattacaca caactgcata 149880

ttattgatag atttaccttt cagaagaaag agatgccaaa tccttctcac atcacagctg 149940

agaaatgtgg ctgggcatgt tggctcacac ctgtaatccc agcactttgg aaggccaagg 150000

tgggaggatt gcttgagcct aagagttcaa gaccagccag gaaacatagg gagaccctcg 150060

tctctgtaaa aaataaaaaa tttagcaagg tgtggtggca cttgcctgta gtcctagcta 150120

ctcactaggc tgagttggga ggatcacttg agcccaggag tgtgaggatg caaggagtcg 150180

tgattgtgcc actgcatcca gcctgggtaa cagagcgaga ccctgtccca aaacaaacag 150240

gctgggtgta gtggctcatg cctgtaaacc caacacttta ggaggccgag gtgggtggat 150300

taagacaaga agtaacagta aagaagaaca ttatcaaatt ggaatagtgc tgcagtctga 150360

agaacagtca gtgaagaggt gatatatttt caaaatatca ctttacagtt tgggattatc 150420

agtgtaaact ttagtcatct actctgaaac actttggttg tgtttttaaa tagatcttac 150480

catttaacat gatgcaatga gtgtacgata gtatgaacat agatcattcc attcagttta 150540

tcctagattt tagtaactga aaaagtatta attccaagtt ttaagccctc cagcagagta 150600

tactttttag taccagttta aaattaacca aggaggatat agtttctttt ctctttttgt 150660

tttgtcttgt aagatcataa catacaactg gttaaatgta ccgacacatc ttcagtttct 150720

gaaggatttg gcaggttgaa actcctctta ttaacagggc tatgagtttc agattaaggt 150780

gacagatttt tgctccttcc tggaactcca ctaaaactgt aataaaggaa ttttttttaa 150840

agcatggaca cataaggatg gggataacag agaggaatga gacaatatca gcaacatttt 150900

gaaagctgga gagcaggtgg aaaagtgatc atagacttag accccaaaag gctaaatgat 150960

cagtcagcag tggggaaatg aaagccaacc tggtttatac cgtagaatcc tcaattctca 151020

ggaattggca atatcagcta tctcagggga tgaaagggtt aaaatgaaag gcctgtttga 151080

aaagctgtta tttctctaaa tctgttctct tactcaccag gtaactgctc catccctatc 151140

ctagcagtag actggaagtt tcttctctag agaggggaaa ataaatatct ctggactggg 151200

agaccctaat ctatgtctag gacatgtata tctttcccaa aacatgggga tttgatgact 151260

gtgtgcttac taaatgatga agggagattt ccccagccct ctctttttat ttgattcctg 151320

acatgctagc agccaaaccc tactcttccg aaatgcagaa gattcgaaga gtcttgggtg 151380

aattttacca gctcaagagg aaagacccaa agaaagtgac atcagggatt ccacctagat 151440

tactgtatag ttcaaaaata gcaagcccat ctgtatgctt aaagcctcca ggcagcttgt 151500

aagtccctca cttagtctaa gtaagagtat cgctggataa ctagatattg gtggggaagt 151560

cttatgcaaa taagagagac tgtaaaacac atagagaaaa ataggcattg ggagaaacac 151620

atacaatgca agattatctc tcccctgcct caccccacct ccaaaaaacc tatcagtttt 151680

ctcagaggga gaaaagataa ctttatgtcc actaaacagg aacagaggtt ttctaaggaa 151740

cattcatgta acagcagcaa caaaatgcta aaatgttgat atttacagta cactggaaat 151800

tatgtccttt gcaattattt aaatgtaact tttaaatgtt aatttaaaag gagttaacgt 151860

agtttctcag aattctttta aggggtgttc agcaaaaaaa gttttaaaac tattatgtta 151920

aacactatat ggtttaatat taaattccta tattatgcaa cataattcgg aagggacact 151980

tagataaatt ttttacaaac caagttacat aaaatatgta taattaattg gaacacataa 152040

ccagattgca tcagtgagtc ttgaagtgga tattcctgtt ttcttctcta gtgtcaatga 152100

ctaaagcaca ctattttcac tacttttttt aaattttgag acagagtctc actcctctgt 152160

cacccaggct ggacaggctg gagcgcagcg acactctctc agctcgctgc aacctctgcc 152220

tcaagtgatc ctcccacctc agtctcccta gtagctggga ctacaagggc gcaccatcac 152280

acctggctca tgtttttgta ttttttgtag agacagagtt tcgccatgtc acccaggctg 152340

gtctcaaact cctgggctca agcgagccac tgcctccgcc tcccacagtg ctgggactac 152400

agatgtaagc caccaggccc ggcctatttt cagtacattt gattgaactt tgttgttgtt 152460

gttgttgttg ttgttgttgt tgttgttgtt gtagttaaac aatctgtttt aatcaggctg 152520

acagatgtta gaacaaacag gatggtccta ggggtcatga actggttctt caaatcctaa 152580

ctgttgctgc tgattttgta acagatttta acatatcttt tgacttctgg tcactcattt 152640

cagcctcatg tttggttctc tgttttaatt tccccctatc tattcccatg gatcaaatgg 152700

cacagatgga attccttaca tacctaattt ttcctcccta tttccaatac tgttcatctt 152760

gaatgccaac catagtttca tcttgagggc tttgttttgg aattatctcc agaagctatt 152820

cccagttaga agcctgcttg gataattaat attgcttctt agctccctcc agctctcttt 152880

ccttctgtcc cctggctcta ccctgagcac aaatgataat tctctgtgac catcacagcc 152940

accatcttac tgtgcctcct ttcaactctt catgtctata tcctcttccc tgaccagcat 153000

catctcacaa aggactgacc aagccagata tagtctatag ctagacaata aggctatagg 153060

gtatttaaat agcctcttaa tatgtacttt tgcaaagcct ttatttcaga atagcctgcc 153120

ctatgttgaa agtgatcttt tctgactgct caccaaaatt cattctaaag tctctggata 153180

taagcagaat aggaacaaac ggattcattt tataaagtgc taaggttgca gttaactcat 153240

aagataaaac ctttaataat tagaaaagtt agtctggaca cggtggctca cacctgtaat 153300

cccagcactt tgtgaggcca aggcaggagt attgcccgag ccaaggagtt caagaccagc 153360

ctgggcaacg gcaaaacccc gtctctacaa aaaatacaaa aattagctgg atgtgatggt 153420

gtgcacctgt gttctcacct acttgggagg ctgaggtggg aggatcactt gagcccagga 153480

agtcgaggct gcagtgagcc atgatcgtgc cactacactc cgccctggat gacagagcaa 153540

gaccctgtct caaaaataaa taagaataat tagaaaagtt gaatcattag gactttcaaa 153600

tgtgtcacct ttattggatt acagaatata agcaaaaaat ggataggtaa catttttcct 153660

gtgtggttat atcttccatt tgtacctcag tgaaaaacta tttctgattc ctaggtttac 153720

ttgaaaagga gcagagctgt tctaatggta gataattata aactcactct gaggaatcag 153780

ggttggtaaa gtatgtttta tcatcttctt ttttggtttt tttttttttt ttttgagatg 153840

gaatctcact gtcacccagg ctggagtaca gtggcacgac cttggctcac tgaaacctcc 153900

gcctctcagg ttcaagcgat tctcctgcct cagcctcctg agtaactggg attacaggca 153960

cccaccacca cacctggcta attttgtatt tttagtaaac gggttttcac catgatggcc 154020

aggctggtct tgaactcctg acctcaagtg atctgcccgc ctcggcctcc caaagtgctg 154080

ggattacagg cgtgagtcac tgcacctggc ctgttttatc atcttttcac ctgccagtca 154140

ttgattcatc ccaaggaccc agatatctta agaatactgt tactaaagaa attccaggaa 154200

tggtcagtac attgtgcctt tttttttttt tttttttggc aggccttata atttcagtat 154260

aatatttatg gtatgatttt gaatttaact ttatcaaaaa attaaatcac agaggcacat 154320

agaaaaagtt acagcctatc gatatattta cagaagcatt atattctcaa aataagatga 154380

ttaaaaataa tttggagata aatccttaca atttactttg ttttaaacaa tgatgagcat 154440

gcctctttta ctcataagtg aacccagttg aagatagaag gactaattaa agctgaaaaa 154500

atggtgaaca tgtattagtg attgataata attctaagtg gccgaagaat atttaattat 154560

agtgaacata attttctggt cggtaaaaat aataataggg tgctgataat aataataatc 154620

agaaaatgca aggtaaaaca aaaaggtacc actttccacc cactggaatt ggcaaaatgc 154680

ctgagttctg ataagatcaa atgttcatag gattagagga attgcttcct gggtcatttc 154740

tgatgcaacc agccacctta acagcattct ggaagtagct gttaaaatag gaaaatgctt 154800

attctaaccc caagaaacat tagcttttgt tccaagtcgt gtatacagaa agagatgtat 154860

tataggaaac aatataatag tgaaaaattg gtctggatgc agtggctcat gcctctaatc 154920

ccagcacttt ggaagactag ggtaatagga tcacttgagg ccgggagttt gagaccagcc 154980

tggatgacac tgtgcgaccc tgactctatg taaaacttaa aacattaagg acattttttt 155040

aaaaaaagaa aaacttattg aaaaattgga aacatgttca tcaggagaag acttgataaa 155100

taacatattg gcacttacat acagtagaat cgtatatagt agttaaaagt ggattatata 155160

tgtatcaaca taaagcttta aaatattaat gttaagtgag aaaagcaagc tgcagcatga 155220

gaccacttaa aaatttttaa gcagaacatt ttttacattt gggctttaaa aagtggtgtg 155280

tatgtatata tgtaaaagta ctgaaataag gattagaaag caaagatcaa gtaacatagt 155340

gattatctcc aggaatcaag tacaaacttt gaaaaaagac tggaggtggc caagcacggt 155400

ggctcatgcc tgtaattcca gcaccttgga aggccaaggc aggtggatca cttgaggaca 155460

ggagttcgag actagcctgg ccaacatggt gaaacaccat atctactaaa aatacaaaaa 155520

atcagctggg catggtggcc ggtgcctgta atcccaatta cttgggaggc tgaggcaaca 155580

gaatcgcttg aacccaggag acggaggttg cagtgagcca agatggcacc actgcactcc 155640

agcctaggtg acagagcgag attctgtctc aaaaaaaaaa aagactggag gtgttttagt 155700

ccattttctt tactataaca gaatacctga ggctgggtaa tttgttgttt tctgcaaaaa 155760

gaaacttatt tctcatagtt ctagaggctg ggaagtccaa gggcattggt gctaacatct 155820

gctgggcttc tggtgagggc tttcctactg catggtaaca tggtggagaa gcagaagagg 155880

gagtgggcac acacaaaagg ggcagaacac aagggacagc ctcactctat agcaaccccc 155940

tttcacagta actgtagaag tcactcctgg ccaggtgtgg tggctcatgc ctgtactact 156000

agcatattgg caggctgagg agggaggatt gcttgagccc aggagtttga gaccagcctg 156060

gacaacatag taagacctca tctctacaaa aaaatttttt ttaattagcc aagcatggta 156120

gcacactcct gtagccccag atactctgga ggctgaggca ggaggatcac ttgagcccag 156180

aagtttgagg ctacagtgag gcatgattga gtccactgca ctccagcctg ggtgacaaag 156240

tgagaccctg actcaaagaa aaaaagaagt cagtgactcc tgctttcatg agggcattcc 156300

tcatgaccca gacccaaatg cctcttaaag gtcccaccaa ctctcaacac cattacactg 156360

gggccaagcc tccacatgag ttttgtgggg acaagccata ttcaaactgt agcaggaggc 156420

aaatgtataa aagttttaat gggtctgatt atggtaagaa tatgagtgac attatccttt 156480

gctactgcag tttttttaaa atttcaaaat catgttacga ggaaatatgt aatcattata 156540

ggaaattcag aaaatgtaaa cataaataaa aagcaccagt aatccaccat caagagataa 156600

ctgtcattaa tattttgatg tgtattgtgt atcctttcag aatgatgtgt tttaatacta 156660

tacacaatgg tttcttgctg ttgttttaaa tcatactggg ttttcctttt tactcttaaa 156720

tatctctact ggtcaataaa tatctgatac cagctctgct acatactgtt aactcactgt 156780

ctcccctttt tctttatgcc aactatgttc tgtggggttt cttaaaatat actgtaatgt 156840

atttagcaat gatatgcact tactatgtgt cagacattct gtgtacttta tatacattac 156900

ctcatttact cctcaacaac cctgtgaggt tgcagctttt attatatcct tgttttatgg 156960

atgaagaaac ctggttatgg aaggggcaag taacttgccc caggtacttc agctccaggg 157020

tgtattcctt taaccattaa agcatgttgc ttccccaact tattcagtcc cttcgaaaac 157080

ttcaagtaga cccttaatgt tatagggcag ttacactttc agtttcctag ttcattcact 157140

gtcctctttt agacactgtc ataccgcctc aaaacctcat atccttaagc ctctaaaacc 157200

tcttcttcaa gaaatcagcg atgacattgc ttgccatctc tttaagacag ttggaagtaa 157260

ccattatgac atctacccac ctgcctgcat tcataccagg ttcttctgcc ttatcactag 157320

taaaagccaa tccctccatt tgtacactag attccatccc cttaccccta cccagagaca 157380

tcttttgagt aaatctccca tcttgtttat catcagtttt tcctttccta ctgaattttc 157440

tcctatttta aaacatcttt tggctgggca cggtggttca tgcctgtaat cccaacactt 157500

cgggaagctg aggcgggtgg attgcttgag ctcaggagtt caagatcaga ctgagcaaca 157560

tagtggaacc catctctacc aaaaaataca aaaattagcc aggcgtggtg gtctgtgcct 157620

gtggtcccag ctacttggga agctgagaca agtggatctc tagagcctgg gaagatgagg 157680

ctgcagagag tcaagatcgc gccactgcat tccagcctgg gtgacagagc aagactctgt 157740

ctcaaaaata agtaaataaa taaaacatct ttcactgagt gcagtggttc acacctgtaa 157800

tcccagccct ttgggaagct aacgtgggaa gatcacttga gctcaggagt tggagaccag 157860

cttgggtaac agagtgagtc cttgtctcag aaaactaaag taaaatttaa aagtagggca 157920

ggtgtggtgg ctcacacttg taatccaagc actttaggag gctgaggctg gtggatcact 157980

tgagcccagg agtttgagac caccctaggc aacatggcaa aacccgtctc tacaaaaaat 158040

acaaaaatta tccagatgtg gtggtgtatg tctgtggtcc cagctactcg ggaggctgag 158100

gttgcagtga gtggagattg caccactgca ctccagccag ggcgacagag tgaaaccctg 158160

tctcaaaaaa aaaattaaga agtaacagta ataatgaaac atctttctta tgcacatttg 158220

gcagaatgtt gacatttgtg gaatctatgt ggaaggtgtg tgggtattct ttccattttt 158280

ctgtatgttt attttctttt ttgttgttgt tttgtttttt ttattattat actttcagtt 158340

ttagggtaca tgtgcacaat gtgcaggtta gtaacatatg tatacatgtg ccatgcttgt 158400

gtgctgcacc cattaactcg tcatttagca ttaggtatat cttctaatgc tatccctccc 158460

cctgccccca ccccacaata gtccccagag tgtgatgttc cccttcctgt gtccatgtgt 158520

tctcattgtt caattcccat ctatgagtga gaacatgcgg tgtttggttt tttgtccttg 158580

cgatagttta ctgagaatga tgatttccaa tttcatccat gtccctacaa aggacatgaa 158640

ctcatcattt tttatggctg catggtgtat atgtgccaca ttttcttaat ccagtctatc 158700

attgttggac atttgggttg gttccaagtc tttgctattg tgaatagtgc cgcaataaac 158760

atacgtgtgc atgtgtcttt atagcagcat gatttataat cctttgggtg tatacccagt 158820

aatgggatgg ctgggtcaaa tggtatttct agttctagat ccctgaggaa tcgccacact 158880

gacttccaca aaggttgaac tagtttacag tcccaccaac agtgtaaaag tgttcctatt 158940

tctccatatc ctctccagca cctgttgttt cctgactttt taatgattgc cattctaatt 159000

ggtgtgagat ggtatctcat tatggttttg atttgcattt ctctgatggc cagtgatgat 159060

gagcattttt tcatgtgtct tttggctgca taaatgtctt cttttgagaa gtgtctgttc 159120

atgtcctttg cccacttttt gatagggttg tttgtttttt tcttgtaaat ttgtttgagt 159180

tctttgtaga ttctggatat tagccctttg tcagatgagt aggttgcgaa aattttctcc 159240

cattttgtag gttgcctgtt cactctgatg gtagtttctt ttgctgtgca gaagctcttt 159300

agtttagtta gatcccattt gtccattttg gcttttgttg ccattgcttt tggtgtttta 159360

gacatgaagt ccttgcccat gcctatgtcc tgaatggtaa tgcctaggtt ttcttctagg 159420

gtttttatgg ttttaggtct aacgtttaag tctttaatcc atcttgaatt aatttttgta 159480

taaggtgtaa ggaagggatc cagtttcagc tttctacata tggctagcca gttttcccag 159540

caccgtttat taagtaggga atcctttccc cattgcttgt ttttctcagg tttgtcaaag 159600

atcagatagt tgtagatatg tggcgttatt tctgagggct ctgttctgtt ccattgatct 159660

atatctctgt tttggtacca gtaccatgct gttttggtta ctgtagcctt gtagtatagt 159720

ttgcagtcag gtagtgtgat gcctccagct ttgtgctttt ggcttaggat tgccttggtg 159780

atgcgggctc ttttttggtt ccatatgaac tttaaagtag ttttttccag ttctgtgaag 159840

aaagtcattg gtagcttgat ggggatggca ttgaatctgt aaattacctt gggcaatatg 159900

gccattttca tgataactga ttcttctacc catgagcatg gaatgttctt ccatttgttt 159960

gtatcctctt ttatttcatt gagcagtggt ttgtagttct ccttgaagag gtccttcaca 160020

tcccttgtaa gttgggttcc taagtatttt attctctttg aagcaattgt gaatggaagt 160080

tcactcatga tttggctctc tgtttgtctg ttattggtgt ataagaatgc ttgtggtttt 160140

tgtacattga ttttgtatcc caagactttg ctgaagttgc ttatcagctt aaggagattt 160200

tgggctgaga cagtggggtt ttctagatat acaatcatgt cgtctgcaaa cagggacaat 160260

ttgacttcct cttttcctaa ttgaataccc tttatttcct tctcctgcct gattgccctg 160320

gccagaactt ccaacactat gttgaatagg agtggtgaga gagggcatcc ctgtcttgtg 160380

ccagttttca aagggaatgc ttccagtttt tgcccattca gtatgatatt ggctgtgggt 160440

ttgtcataga tagctcttaa tattttgaga tacgtcccat caatacctaa tttattgaga 160500

gtttttagca tgaagggttg ttgaattttg tcaaaggcct tttctgcatc tattgagata 160560

atcatgtggt ttttgtcttt ggttctgttt atatgctgga ttacatttat tgatttgcgt 160620

atgttgaacc agccttgcat cccagggatg aagcccactt gatcatggtg aataagcttt 160680

ttgatgtgct gctggattcg gtttgccagt actttattga ggatttttgc atcaatgttc 160740

atcaaggata tcggtctaaa attctctttt ttggttgtgt ctctgcctgg ctttggtatc 160800

aggatgattc tggcctcata aaatgagtta gggaggattc cctctttttc tattgattgg 160860

aatagtttca gaaggaatgg taccagttcc tccttgtatc tctggtagaa ttcagctttg 160920

aatccgtctg gtcctggact ctttttggtt ggtaagctat tgattactgc cacaatttga 160980

gatcctgtta ttggtctatt cagagattca acttcttcct ggtttagtct tgggagagtg 161040

tatgtgtcga ggaatttatc catttcttct agattttcta gtttatttgc atagaggtgt 161100

ttgtagtatt ctctgatggt agtttgtatt tctgtgggat tggtggtgat atccccttta 161160

tcattttttt attgcatcta tttgattctt ctctcttttc ttctttatta gtcttgctag 161220

tggtctatca attttgttga tcctttcaaa aaaccagctc ctgggccagc cgccccgtcc 161280

gggaaggagg tgggggggtc agccccccgc ccagccagct gcctcgtccg ggaggtgagg 161340

ggcgcctctg cccggccgcc cctactggga agtgaggagc ccctctgccc agccagctgc 161400

cccgtccggg agggaggtgg gggggtcagc cccctgcccg gccagccgcc ctgtccagga 161460

gggaggtggg gggggtcagc cccccgcccg gccagccacc ccgtccggga gggaggttgg 161520

ggggtcagcc ccccgcccgg ccagccgcct cgtccgggag gtgaggggcg cctctccccg 161580

gccgccccta ctgggaagtg aggagcccct ctgcccggcc accaccccgt ctgggaggtg 161640

tacccaacag ctcattgaga acgggccatg atgacaatgg cggttttgtg gaatagaagg 161700

aggggaaaag cggggaaaag attgagaaat cggatggttg ccgtgtctgt gtagaaagag 161760

gtagacatgg gagacttttc attttgttct gtactaagaa aaattcttct gccttgtgat 161820

cctgttgatc tgtgacctta cccccaaccc tgtgccctct gaaacatgtg ctgtgtccac 161880

tcagggttaa atggattaag ggcggtgcaa gatgtgcttt gttaaacaga tgcttgaagg 161940

cagcatgctc cttaagagtc atcaccactc cctaatctca agtacccagg aacacaaaca 162000

ctgcggaagg ccgcagggtc ctctgcctag gaaaaccaga gacctttgtt cacttgttta 162060

tctgctgacc ttccctccac tattgtccta tgaccctgcc aaatacccct ctgcgagaaa 162120

cacccaagaa tgatcaatta aaaaaaaaaa aaaaaaaaaa acagctcctg gattcattaa 162180

ttttttgaag ggtttttttg tctctatttc cttcagttct gctctgattt cagttaattc 162240

ttgccttctg ctagcttttg aatgtgtttg ctcttgcttt tctagttctt ttaattgtga 162300

tgttagggtg gcaattttgg atctttcctg ctttctcttg tgggcattta gtgctataag 162360

tgtccctcta cacactgctt tgaatgtgtc ccagagattc tggtatgttg tgtctttgtt 162420

ctcattggtt tcaaagaaca tctttatttc tgccttcatt tcgttatgta cccagtagtc 162480

attcaggagc agcttgttca gtttccatgt agttgagtgg ttttgagtga gtttcttaat 162540

cctgagttct agttagattg cactgtggtc tgagacacag tttgttataa tttctgttct 162600

tttacatttg ctgaggagag ctttacttcc aagtatgtgg tcagttttgg aataggtgtg 162660

gtgtggtgct gaaaaaaatg tatattctgt tgatttgggg tggagagttc tgtagatgtc 162720

tattaggtcc ccttggtgca gagctgagtt caattcctgg gtgtccttat taactttctg 162780

tcgcgttgat ctgtctaatg ttgacagtgg gatgttaaag tctcccatta ttattgtgtg 162840

ggagtctaag tctctttgta ggtcactaag gacttgcttt atgaatctgg gtgctcctgt 162900

attgggtgca tatatattta ggatagttag ctcttcttgt tgaattggtc cctttaccat 162960

tatgtaatgg ccttcttggt ctcttttgat ctttgttggt ttaaagtctg ttttatcaga 163020

gactaggatt gcaacccctg cctttttttg ttttccactt gcttggtaga tcttcctcca 163080

tccttttatt ttgagcctat gtgtgtctct gcatgtgaga tgggtttcct gtatgcagca 163140

cactgatggg tcttgactct ttatccagtt tgccagtctg tgtcttttaa ttggagcatt 163200

cagtccattt acgtttaaag ttaatattgt tatgtgtgaa tttgatcctg tcattatgat 163260

attagctggt tattttgctc gttagttgat gcagtttctt cctagtctcg atggtcttta 163320

cattttggca tgattttgca gtggctggta ccacttgttc ctttccatgt ttagtgcttc 163380

cttcaggagt tcttttaggg caggcctggg ggtggcaaaa tctctcagca tttgcttgtc 163440

tgtaaagtat tttatttctc cttcacttat gaagcttagt ttggctggat atgaaattct 163500

gggttgaaaa ttgttttctt taagaatgtt gaatattggc ccccactctc ttctggcttg 163560

tagagtttct gctgagagat ccgctgttag tctgatgggc ttccctttgt gggtaacccg 163620

acctttctgt ctggctgccc ttaacatttt ttccttcttt caactttggt gaatctgaca 163680

gttatgtgtc ttggagttgc tcttctcaag gagtatcttt gtggcgttct ctgtatttcg 163740

tgaatctgaa cattggcctg ccttgctaga ttggggaagt tctcctggat aatatcctgc 163800

agagtgtttt ccaacttggt tccattctcc ccgtcacttt caggtacact aatcagacgt 163860

agatttggtc ttttcacata gtcccatatt tcttggaggc tttgttcgtt tctttttatt 163920

cttttttctc taaacttccc ttctcgcttc atttcattca tttcatcttc catcactgat 163980

accctttctt ccagttgatc gcatcggctc ctgaggcttc tgcattcttc acatagttct 164040

cgagccttgg ctttcagccc catcagctcc tttaagcact tctctacact ggttattcta 164100

gttatacatt cgtctaaatt ttttattatt ttcattaaaa aaaacttttg gggtccctcc 164160

cttaatcctg cttctctctt cagttattac ctcattcctc ttcttccttc gacagtaaaa 164220

cttctcaaaa gacttgttgg tattcactgt ccagttgctc tccttccttt ttatcttgga 164280

ccccactcaa accaggcttt tgcccctgct gctccagaaa acatcttttt tttttttttt 164340

tttctctctc tcaaggaaga gtctcactct gtccctcagg ctggagtaca atggcatgat 164400

ctcagctcac tacaacctct gtctccaggg ttcaagtgat tctcctgcct tagtctccca 164460

agtagctggg attacaggtg cacaccacca cacccagcta atttttgtat gtttagtaga 164520

gacagggttt cactatgttg gccaggctgg tctggaactc ctgacctcaa gtggtctgcc 164580

catctcggca tcccaaagtg ctggaattac aggcatgatc cactgtgcct agccaggaaa 164640

cagcgtctta atatgatagt tactggtggc ctccatgttg ctagatccag cagtaaaatt 164700

ctcagtccac agtttatttg acctgtcaga aacatctgat acagttgatc acacacttct 164760

tcatgaaaca ggttcttaat cccttgttta attgtgttct tagtttcttg ctgctcagaa 164820

tgtggtctga gtagcatctg taccacctgg gagcctttta gaaatgctaa gttttagata 164880

ccaccccaga cctgaatcaa agactgcatt ttaacaagat ccccaaatga ttcgtgtcca 164940

tattaaagtg tgagaaacac tgctttcagg catattctcc tgattaccct tctactcact 165000

gtctactcct cagctggttc ctcctctcct tctttggtgt ttcgtggttg gacaagtcat 165060

ttaaaatacc tgtaatcagg gccgggtgtg gtggctcacg cctctaatcc cagcagtttg 165120

ggaggccaag gcagatggat cacttgaggt cgggagttca agaccagctt gaccaaaatg 165180

gtgaaacccc atctctacta aaaatacaaa attagctgga cgtggtggta cttgcctgta 165240

atcccagcta cttgggaggc tgaggcggga gaatcacttg aacccgggag gcagaggttg 165300

cagtgagcca aaatcgtgcc attgcactcc agcctgggtg acaagggcaa aactccatct 165360

taaataaata aataacctat aatcagaacc agcaatacaa agtattatca aggataaaga 165420

acaactggaa ctctcataca ttgctggtaa agattttaaa tgatacagcc atttagggga 165480

tcaggttggc agtttctttt aaagttaaat ttaccatttg tgcaattcta atcctaccta 165540

attacccaaa ataaatgaaa ccatcagtca acaacaaaga cttgtatatg aatacagcat 165600

cttcttcata tttgccacaa actaggaaca acacagtgtc caataagtaa atagataagt 165660

tgtgatatac ccataccccg taacactgca tgcagccagt gcgtgaatct cacaattgtt 165720

ttgctgcgca aaagaagcca ggtgattctg tttatatgaa atttgagaaa aggcaaaatt 165780

aatctgtagt gacagaagtc attatcaatg gtttcccaag cctaaagggg gtgttactaa 165840

tcgcaaaaat gtatgaagaa actttctggg gtgacggata ttctttatat tgattgtgac 165900

tggtcattac acaagtgtat gtgtgtgttg aaacttttaa aactgtccat gtaaaatggg 165960

tgcatcttgt gtgtaaactg tacctcagta aatttcagtt tttaaaagtg aaaaatacct 166020

gatggcttac acctataatt ctagcacttc gggaggccga ggtgggagga tggcttgagc 166080

ccaggagttc aagaccagcc tgggcaacat agtgagaccc atctatacaa agcagttttt 166140

taattatcca ggcatggtgg cgcccacctg tagtcccagc tacacaagat gctgagttgg 166200

gtggatctct tgagcccagg aggttgaggc tgcagtgagc tgtgatcatg ccactgtact 166260

ccagcctggg caagagagca acaccctgtc tccaaaaaaa aaagtgaagg aaactaaaaa 166320

atcaaggcaa aaatagaatt taggccacta tggagcataa ctttaaaata tgtgaacttt 166380

acctattcga tattttaatt atttttaaag tgataaatga ttactgacta cagagaagta 166440

acagaatgcc attctcgtta atcttacttt ccagggttgt aataagggag cagagggcat 166500

caaagtataa ggaaggctgt aatttggcac tgtcaccact atcataccta ccagtccagg 166560

tggtcaccca aggagagact catcttgctg cctaaagtct gatgaacctt tgtaaaattg 166620

tgcagtatta ggtgaaagag ggctgacatg caaatgtcta agtaggtcag ttctgtgttt 166680

taccaagagt ttaaaatact tgaaaatggc agcagactta agtgagagat gctagtggct 166740

tttacaaata agctaagttg ataaattagc caaatggcat agcaatatta tcaataggca 166800

ttttaatgat aatctctcat tgttttctta aggataagat caagtgatgt aacatgaatg 166860

acaggattta actgggttca tactggatga atgatcatac ctgaagacag caaactataa 166920

tgagattcct atcctcagtc ctctcttgaa tataattacg tatttagaag taacttatgt 166980

gaggaattat tagcatgccg atctttaaaa atctcctttt ttcagctctc acgtaagact 167040

cttgaaacaa gaaaataaaa ataacctatg ttttattaaa gaaaaatgaa gcaggcaaga 167100

aaaaaccgac ttttttagtg aaagctaaag ttttaatctg aaattataga ccatgtctac 167160

aaaatttaaa atcctaaggc tgggcgtggt ggctcacgcc tgtaatccca gcactttgga 167220

aggctgaggc aggcagatca cgaggtcaag agactgagcc catcctagcc aacatggtga 167280

aaccccatct ctactaaaaa cacaaaaatt agctgggcat ggtggcgcgc gcctgtagtc 167340

ccagctactc aggaggctga ggcaggagaa tcacttgaac ctaggaggtg gaggttgcag 167400

tgagccgata tcgcaccacc gcactccagg ctggtgacag agtgagactc tgtctcaaaa 167460

aaaaaatcct agtcaagtcc aaaaaaagag aaatatacaa gtataagatg gggaaaatgt 167520

tttataggag caattcaaga ataattattc taggattgta attgaatata atcctttttg 167580

tgcccttttt taaaaattct atttattgtc ataaattttg acccagcagc aggaagcatg 167640

taattaaata taattttaat tggactcagt agcatgactt tggctgccat aacaatcaca 167700

ataatgtctg atttaatgtt gtatgcccac catgtgggag atgaagatga ggatctcacc 167760

atagtttgtg ttggttagat ccgatctgaa gtattgcttt taatttgggg caccacatta 167820

tgctctgtaa gaatgtggaa aagctgaatc ttggataaga gcttgaagta gtgtgagaag 167880

aaattttacc agatgatttt gtagctctaa gattaaatgg ttctataggt ggacactttt 167940

tcttcaagga atgagactgt taagagaaac ctatcaagag aatgtgttgt tcctttggtt 168000

gatagcatat ttgttctttc ctagtacttc tgtaaaactg taatgctctc cttaacatct 168060

taccaaggaa agagggaggt ccaatctaat tatcctaatt aatggattga cttctgtgtc 168120

atatatggca aagtaaagtt gatatattct tgttcccttc cctactccca aacagttatg 168180

tgagttattt gttttctgtt atttttgttt gggttttttt tttggcattt tccattgtaa 168240

ctgggtggtg cttaggaaat tacgttttag tgtcccccta tatagaatta tttagggtag 168300

tgattactgt gaaacattat cttatgtagg gattcggttg tgtctcatcc catttctcta 168360

gctgggggat tagaccccct tttccttcca gcctttcatt ctacagacct ggacttctac 168420

tctttgctgc ttttgttaca gaaggattac agaaagagaa ttattcaatg agataaaaat 168480

taacaaagca gtttgtaagt aataaaggat tacacaggta taaagtgttg tttcaggttg 168540

tatttttatg caactcggcc tataacaaat ttatgctata taacttctat gctgggtata 168600

tcactggttc cccaattaca tttatatctt gttaattatc tagtgctttc ctggtcagaa 168660

aatttctctc cccactctcc ccagttacat ttctgtctac tctattcttg ctatattcct 168720

ctgcatcaaa tttgttgaaa ggattaacac catcccatgg agaacacctt tatcttcccc 168780

tcttttatct tcagcatgcc tctgtatttt gatatggttg aagcccattt gtttttgttt 168840

ttaagtcttg caggaaaaag aacctccttt caaagaccaa caccttggct gtatactaga 168900

tcccaggtct ctctgagtgc aggacctagc tccaccaacc aacaccatca tcatttatcc 168960

ccctttcttt ctaatggaga caagatctca ccatcttaaa gctttgattg ggcccatttt 169020

atttccacca gtttttaact ttgtatgtca ctgcagaact ttgtaagcag tgagacctat 169080

aaccgaaata acattttctt tcagtgaaag agtgacaata tgatgtagaa cactgagcct 169140

aaagtcatga caagataatt ttcaattgta gtaaatgcta tgaagaacat aaactgtgat 169200

atgaatagta attggagaag ggattacttt agataaatag taatttgaaa tgaaaatttt 169260

atctagaaaa tactttaaaa tttttgatat tgggaagaat gaggacgttt aaatgaagat 169320

gagacagcat gaataatctt ggttatttgc aaatcttcca cttttaacag tgtttattgc 169380

atgattttga ttataccatt tggattttaa attctcattt taaggtaaaa ctgtctaatg 169440

atcattgtca gatttcagaa gtgtcatata gctattctct ccggtattca attaagaaaa 169500

ataaccttga ctagcacatt atatgcatct tattgtagtt gattcagtag acatgtattg 169560

aatacttacc acacatacca agcactgttc tcggcactgg gtatacaaag aaataaacaa 169620

gacacagccc ttttgagaca ggaggatctc ttgagcccag gaggtggagg ttgcagtgag 169680

ccaaggtcgt gccactacac tccagcctgg gtgatagagt aagactttgt ctccccaaaa 169740
acaacaacaa caatgctgcc ttttacttca atgaatgtag ctaaagcagt ttttaggggt 169800
aacatagatg attttaaagt tatcgttgac tttaaaatag acctactatg gccattttag 169860
agaaagtttt ctcctcacta gttttaagaa aatattttaa gaatatatga caactacctt 169920
taaaaatagt tttaattacc aaactgcatg ttctagtagt ttacattcca aatagaagtg 169980
aacataggca cgttgaaaaa acacatgaat caagccatta ttatgataat tactacataa 170040
gttgagcagt gaaagagcac tttcagttcg gttagtcatg ggaaagcttc actcaggagt 170100
tagaatttgt attcagtttt aaaggatgga tatgaatagg tggaaaatag aacgaaagct 170160
aacttggagg gttgggtgta ggaggggagc attgaacaca gtggtgggat taaatatcaa 170220
ggtatattta agggacatgg ataaataggc ttgactggag tgaaaggttt gtattggtaa 170280
ctagtaaaag ataaagcaaa gtcttttaat agaaaacaag attttgactg taccattata 170340
gtgatatgtt cctataatct taaattcatt tacagtctat tttaatattc tgtgaagggt 170400
ttattacaat gtactatttt cagttgtatc atgattctaa ataagtcttt acacccccaa 170460
gtatgttctg tagatttcga ggccagagtc ctttagccct actcaggtta aaatgatgtt 170520
ttgtttttca gttacttaca cgccaagtca atcatccaca gagacctcaa gagtaatagt 170580
atccttcctg aaatttgtct gcgaagtttg aaaacatcct gactttttct tctgcatttt 170640
gtcttcacat tatgtaaaaa cagttttcat gctaagttcg atatactgta aagagaatta 170700
ataaaggatt gtgcatgcat gtataggaga gcaggatacc acagcctgct tttggtttct 170760
cgacaactga acattacaag aaaatctatc agaagtcttt acaatagtag gagtttttga 170820
ttgcttgctt acattttatc agcactataa aactgatagt tttgtagcta tctattagtc 170880
cctttcagac ctctgacctt gctcagtggt agttgagata taactgaaga ctctaaatta 170940
tataacaatg aggtgagaaa aacataatat ttctcttccc taagtgcaga ctaagatact 171000
atctgcagca tcttcattcc aatgaagagc ctttactgct cgcccaggag tgccaagaga 171060
atatctgggc ctacattgct aaaatctaat gggaaagttt taggttctcc tataaactta 171120
ggaaagcatc tcacctcatc ctaacacatt tcaagcccca aaaatcttaa aagcaggtta 171180
tataggctaa atagaactaa tcattgtttt agacatactt attgactcta agaggaaaga 171240
tgaagtacta tgttttaaag aatattatat tacagaatta tagaaattag atctcttacc 171300
taaactcttc ataatgcttg ctctgatagg aaaatgagat ctactgtttt cctttactta 171360
ctacacctca gatatatttc ttcatgaaga cctcacagta aaaataggtg attttggtct 171420
agctacagtg aaatctcgat ggagtgggtc ccatcagttt gaacagttgt ctggatccat 171480
tttgtggatg gtaagaattg aggctatttt tccactgatt aaatttttgg ccctgagatg 171540
ctgctgagtt actagaaagt cattgaaggt ctcaactata gtattttcat agttcccagt 171600

attcacaaaa atcagtgttc ttatttttta tgtaaataga ttttttaact tttttcttta 171660

cccttaaaac gaatattttg aaaccagttt cagtgtattt caaacaaaaa tatatgtctt 171720

ataaacagtg tttcatattt tattcttaaa taaatatgaa cccttaaaac gaatattttg 171780

aaaccagttt cagtgtattt caaacaaaaa tatatgtctt ataaacagtg tttcatattt 171840

tattctaaat tgtttaaagt attttgtgtt caaaatgttc tgtgtaccct gttgaaaaaa 171900

aaaacaggta tgcaatttaa ggcaggtgtg atccacagcc attattatgg ttttgctaag 171960

agaactactc cttttaacag agaagctgtt tcgcaatctt atttaagcct aaattggaaa 172020

gttacttcct ttagactaga aagtatctca taattatggg gcagctggaa gaggaaagac 172080

aaaaaaaaat gagaggtaga ttaacagcct tgtgctgtct tgcatagctc tttctttctt 172140

cttgtttttt gctttgtgga aaagaagaaa gagaagttct aaaagaaggg aacaaaaact 172200

tgtgtgcatt gcagcaagct gtggaaagct cagtcatatg aatcattccc taaaacagca 172260

ttcttaaaag ggtccctcac accgttttag agggtccaca agatcttccc tttgtgagac 172320

aagattttct ttatatcctt caaccaaaac aacacattgc aacagactga gtgcaaaagc 172380

aaatatgaga atccagctgg ctgctgttaa gccagacatt gaggagaatc acaggccact 172440

catggtggct ggagtccata gttccagcta cctgggaggc tagggcagga ggatcacttg 172500

atcccaggag ttgactggcc tgctcaacat agtgagaccc catctctaaa ccataaaagg 172560

aggataattg tagtactatt cttcttacta aacttttttt tgataatagt tatttttcat 172620

taaaaatgaa tgatctgtgt taacatctac ttgttattat tttagtagtt aaatgaatta 172680

ctagtttaat ttctccatta aattttaatg gtaaacatcc acagatataa tctacctaaa 172740

caaaagttct ttatcatcct caataatttt taagagtgaa aaagagtcct gagaccaaaa 172800

agtttgaaaa acacagctct aagctgaata cagcctttcc aaaagtctta gtgcaattct 172860

aagctttaaa taacttaatc tgcactaaga ctttcgggca ccctgctgga aacagaaaag 172920

ttgtaagggc tttcaaagcc acaaacttta tgtagcagtc tccagaaagg gaagtccaag 172980

ataggactcc caagtttgtc aaaataaacc tagagttaaa ttgagctgtg atttcttatg 173040

acagtaagtg gaattagggc aggtggcaag gtggaagagg ggacattgga cttagaagat 173100

ctgtattctg gtaccctatt tggccattaa cctgcaagtt tcttaacctc ctctaagtct 173160

acatctgaaa tgttgagttg gactagtaga tttccaatgc cccttttttc taagattcgg 173220

tgactggagt tagctagatt ttttccatta tttaacatat gtttaacttt taattaataa 173280

ttataagtga tagaaaaatt aataactata atctgatgtc aggcaccttt attaaggcat 173340

acacaccttg tgaaaaagat ggcagtgtat cctaaaaaga tgagggaaaa cagattttaa 173400

gtgctgaaat tgcaaaacct aaaattataa acaattgtca cgtgctttta aagtatgtta 173460

atttttgact atgtgggaga gttaggctca atcaagtctc cagttttgtc cttacttttt 173520

caaaaacctt agtttataca gtttgtagat tattatacat aaagttttat attttcttac 173580

aattaatttt ttgttgatac ctgtgtaaat gggtttctcc tttattcttt catgctgagt 173640
tttaagacga gagaataata gccaacagta ccctttattg ttaaaccaat cctgggttga 173700
tactgccctc agaaacaaat atggaacatc cgtatcatag gagagaagag tttggtcata 173760
actaaggctg tgtggtcctt tgaattatat acaatgttct tctcaacttt gttttatttg 173820
tatcttagga gagaaagaaa cctttgtgag agtttttaca aattaaatca ctaaattgaa 173880
gattcgtcat gcatttctta aaataggtga catgcaactt ttcatcaaac tgtctaccaa 173940
caacagtgtg agataaaact aaattaaaaa aaaaaagttc atctgatttc tactccatct 174000
ctcacaaaaa ttggtcaggc tttgaacatt atacctttgc cataccagcc tctcagtatt 174060
ctggcttgct tattacatca cagttatatg tggtttattt tgtactgtat tactaaaagc 174120
aactatgaac aaaatgtatg acaaagcgaa gtagatacaa ccttctcctt cacttccatc 174180
ctctcacgct cttcaggaca tcccacagat cttccatcag tggttctcaa ctttggttgg 174240
acactagaat catctgtgga gctttaaaga ctaattaatg tttgggtccc acccccagac 174300
tctgttacgt tgtgtgattg gactggagtg tagtctggac gttgaggggt ttaaggctcc 174360
gcgggtcatt ctcatttaca gctgttgctc agtgccatta ttgcctctcc ttgtgagatg 174420
cctgccttac ccagagcaat aaccaggaat cttgtcccag gtctttcagt catttttgca 174480
gagttagtac cacttttgct gtcaaattga cattgtcaca aacttttcat taatatactg 174540
ccatttcgat tccttccaaa tgaaacaata cagaagacgc aaggtgaaag aataactctt 174600
ttaagctaat aattaaccaa ctgtttattg tatttcatgt aaataagaaa cctaattgtg 174660
caatacaatg actgaaatgt gtaaaaatgt agcaaatatg attgtttcat tgcccaagaa 174720
gcagcagcca gaagattctt tataccatct tttactaaat ctacctgctg tcttgctttt 174780
gttaataaag catccatggc atgtttatac catatgctta ttccatagtt tgaaagggga 174840
tttgagttta tcagtcctga aattctacca ttatttctta aggtgtcctc agatgagaaa 174900
agttgtttgt accaatggga aaacttaaat tgtaagacag ttactacagt agttgtgctg 174960
ctcctaagca tcttataacc acaagtctag tatttctttg ctgaatcagg aatgggaagt 175020
gggaactgat tctaataggg taagtcatgg gaagaattca tctggcaatg atggtatttt 175080
ctgcagaaag attgcctaaa taattactaa attataaaat cttagtaata caatacaaaa 175140
tctctgctaa tactgtctct ttctgagtat gtagaggttt ttttcttcag cttaatcagt 175200
tttatttctt tgactattaa gagaattgat tacatattag acaggtgttt taatggtaaa 175260
agcattgctc taggaattat agtaggttgt ttttcagtct ttattcaatt gaagtgaaga 175320
atatttttct ttgtatgttc taacaggcac cagaagtcat cagaatgcaa gataaaaatc 175380
catacagctt tcagtcagat gtatatgcat ttggaattgt tctgtatgaa ttgatgactg 175440
gacagttacc ttattcaaac atcaacaaca gggaccaggt aaatatttac cacgtcttgg 175500

tgtttatttt accgtctata tacaaggctc cagttgtaga aaataagtgt taactcctgg 175560

gtaagcgtga aggatagatt tcttgatttt ttgttaccag ttttagaaat cgtttgtata 175620

cttttggcag taatagcaac acgttaagtc ctttcctcag aatatcagtc atgaatgtta 175680

caatggaata aaattcctga ttttctgact agaaactaca gttacaagaa tggatatttc 175740

ttgaccatcg cacaattaga agaatgagct ctgctactat agcatctggg cattatactt 175800

ttcactgtat cacagattgt gctgtcagaa caggtactca gctatgagat ctttatatta 175860

aattctttta aaattactaa ggtttcttga aatctttaat ttttaaaact aggtaaatgg 175920

aagaaatcat gctttgattt ttctataatg agaatgctag taggagggta attttttact 175980

tcttttcctt cctaagcaac tattccctgt cccttacctt ccaaaaggta ctaacatagc 176040

ctcatggagc tcttagcctt ttctatctct gcctcagtag gctcgcttag acttttaaaa 176100

ttggccaaga aaagtgaatt atctttacta aaattacctt ggataactac attttaaaag 176160

atttgtgatt gtgtgtgtag agagattaca acagttacac tgttaatata ttagaaaaca 176220

ctagttgacc acataaactc tgattaaaga atatttcgac tttatgctgt gatttgcctc 176280

agaatcacct ggaaattttg cttaaaaatg gagaaactgg agcagattgt aattaaatgg 176340

ggagggtcca atcacctgca tttttttctg tttcccaggt gattctaata cagaccaaag 176400

ttgagaatca ccaatctaaa gatttttttt aaaaacagtg caggcttttt aggtatttca 176460

taacttccca acctaatgaa atggtagttt ttgtatataa atgttactgc ttcatttaag 176520

tgatttgtct gttttgtcaa gagctcagct ctatcttttt ggtgctagct ctgagtagct 176580

ttctcacttc acctattgtg atatcggaga aataatctct taagttacca tgtactttgt 176640

gtgtgtactt actgaatcac catatgcccc ttacacaaca gtggtcttga ctgtgtttga 176700

tggtttttaa aaatggctac atttctaccc agttcattat aaaactaaat tttagttggt 176760

gttggccttt ctaactactt catagcttta gaatgttgag tcttagcttt gagtgtgtaa 176820

caacatgact ttggatctga tgttttaagc ctgcaggaaa cttagattca ggatggactg 176880

aaatttcagc taaagaacat aattgaaaca ttgtacttac tgtacttgtg atagatctag 176940

atcactgaaa tgattgattt gacaatggca gttggggtag caaaatggtc tctaatttac 177000

aagcttcatg tcctctgtct tttacagtct tatttattca atcatacagc ataacagcct 177060

gtaccatgtt cacttttcct gtatataata tttttctgga ggaattatgg atttttagtt 177120

tagtttcagt tgatttatca tatagactac aaattaataa aaatttatga acctaagatc 177180

tggatgaaat tcatttgtca gtgaatacgt ttatcttagt acaataataa aattataata 177240

tagaaacaag tattatctcc aaaatacaga taaaagcatc ccagagttct tctctcatcc 177300

acttcttggc attttaggtg ctttgtcctc catgggagta taataaatga tgtggcaagg 177360

gcttactctc catgagagga atgtgtgacc aacagaaggg taaggccttt actagttaat 177420

tctttctaat agtatagtta gaaccttctg gaatttgcta gtctgaaacc aagttaagta 177480

tttaatggag aagaaggagg taaaagaatg atgtcctctt acaactaccc ccattctgct 177540

tcattacccc ctagtctact tacgtcaaat agtactttct atgaaactcc acattttgaa 177600

gggttaactc tggccatcct cagtgaagct gccccaggta ttgctccatt catcctttga 177660

tttttctttt gctatctata atacttggat ctccatagct ctcagattag acttctgttt 177720

aagaatccag gaatattctt tactatgcaa tgtgaatacc attcccctag actctcatac 177780

ccatagtctg aggtggcaga ttttgcctgt aaattcagag cacagctggt aaagcagtgt 177840

gatgtaatgg caggccttgg actcaggtag gtagactagg tctacctacc tctctgggta 177900

tctttttttt ttcttttttc tttttttttt tttccaagac ggagtcttgc tgtgtcgccc 177960

aggctggaat gcagtggcct gatctcagct cactgcaacc tctgcctccc atgttagcga 178020

ttcttctgcc tcagcctccc gagtagctgg gattacaggc acgcagcact acacccagct 178080

aatttttttg tatttttggt agagacaggg gtttcaccat gttggtcagg ctgggctcaa 178140

actcctaacc tgatgatctg cccgcctcag cctgccaaag tgctgggatt acaggcgtga 178200

accactgcac ctggcctttt ttcttttttc aattacctgc aaaataaggg aattcggcta 178260

agaatttctt ccagcttcaa aaatcagatt cttttctaaa atagttctct tagctctttg 178320

caaagtagtg tgccttttta cctttattca ccctagcact aaagtctggg aaatcacttt 178380

gtcatccccc cactctcttt atcattctaa catttttctc tctaatcacc ctgttcttcc 178440

ctccttcagt gcattttctc ttgtaaattg ggattaataa tgtgctcact taagtatatt 178500

gactatacct ttatggcttt cgttccttgg agtagaagtg ccgtgtcttc tttgaagaat 178560

agatagcata tatctgttat ttcaagtgta ttattccagt tataattgct gcataacaaa 178620

ccacccagaa tgtagtagct taaaatatga atctgtaggt tgggcttttc tctgtgagga 178680

agattcttac ctgtttcaca ccgtattgac tagagtagat ttacggggtg ttaaaaggat 178740

ctactcagaa gtggctcatt catgtacctg gcaagttggt gctagctaga agctagaagc 178800

tcagttattc tccacatgag cttttccttg tggacctctt cgcaggtctt caaggacctc 178860

ttcattggtc tttctcacag cgtggttgct aggttctaag cacaagatct gaagaaacag 178920

gaagtaagta ctagtctctt atcagtaggc aaattatttt aatagaaaat gttttcaata 178980

ccaactatat gcaaagcact atggaatata gaaggattca aaatgaataa aacaaaaccc 179040

aggctctgct ctcaatggca tgtgcagttc tgttgaggcc gtgctggggg tgatactgaa 179100

aatgagcaga catgagctcc agtaatacca tggagtgaat tgacagggtc gagacaccag 179160

gagtactgag tgtgtattga aagtatgaaa aatcagtccc agaatttgaa gctggggtat 179220

tggtttcgtt gtgtatgtgc cacatgaatc ttctgtccat gaataacatc caggaaaaac 179280

ttcccttttt tcttctttct gtttcttcat cagtcagcct ctgcacccca ccccagcaga 179340

tactgaaagt ttcctgcttt ctgctctgca ctaactaaat tctgtgggat gtataaaaac 179400

atgagagttg tctttgattt tttttctcac attcctcctg aaagactctt gaaaaactat 179460

gtatctcctt gtatattttt taagttggca tctaaaagtt tttatcttaa gtttaaataa 179520

ttgcaaagaa tgtaatttcc agtatattct attaacattt taaaataaga ctactatatt 179580

agatacatat atatccctct ttttgaaata tatacatcaa aatctaaaaa ccataccgat 179640

tttatgccta ttaaaaaata ctgaagctct tcattaattt ctggaagttt tacatggttc 179700

cttttttctc ttcaaactat ttgtattgtt tctgtcacag aacttcatcc taatgtaaca 179760

tatttgtatg ttttaaagcc ttttattgat tacctaagtt acatctctgc aataaaagta 179820

tgtatataaa tttaaattta gaattttaca aaattgtgac ttcaagctct gattattaat 179880

tttttaattc tgaattatgt ttttgttgca agtaatatat aactgatgaa agtagcatat 179940

aagttttgat atatggaaac atagaatgta aaattatatt ggaaatatgt atcttaatga 180000

atgacatggg gcttttcttt tcccttcaat taatgtatgt atctgtggat aaatacaact 180060

tttttgttag gacactagat actaagactt agtattgatt ctttccatat ttcattttta 180120

aagaggtaaa tactgagaaa ttttatctaa attataagaa gatgacaaag gcaggagttt 180180

tgttataatc acatgcagag tttagtagtc cccaagacca gcttcaggtt tgttaaaagg 180240

acttgctgaa aactcactga aagctgctca agactcactg acaactgtta cattcacagt 180300

tacagtttat tacaatgaaa gaatacagat caaaattaac aagggaagag actcatagga 180360

cagaattcag aagcgttctt gttggagctt cctgtcaccc tctcccaatg gagttgtgga 180420

cagagcttcc agtagcaatg tgtgacaaca cacatggatt attgccaacc agggaagctc 180480

acctgagcct tggagtctag agtttttatt ggggttcagt catatgatta accacctgcg 180540

gggctgacct tagtttccag ctcctgcaga ggtcaagctg atgccacatg acccagagcc 180600

ctctataaat cacattgtta gcacagtctg tctggcatgg cccatggccc ccagataaac 180660

agacactctg atcaggcagg acatttcaag gacttagtga ttacctccca ggagccaaga 180720

gcaaaggcta gctctctctt tgggcaaggt taattcttta ccacatacta tatatcactc 180780

aatcatctga gctcattcct ataatgtacc aaaatttaca tagtaacttg tcattaaaaa 180840

tgttttaaaa gctcagctga catttcaatt aatatttttg aaagtaaaga attggaaacc 180900

aacagactcg ttacccattc gttggagccc agtttgacac cagtatttag aaatgtctct 180960

ttgttgcccc agaggttttt acaccctggg acaatgtata ctatagttag gttacatatg 181020

ggtaaaaggt gtgccttttt ttttttaatt aaagttttag ggtacatgta cacaatgtgc 181080

aggttagtta catatgtata catgtgccat gctggtgtgc tgcacccatt aactcatcat 181140

ttagcattag gtatatctcc taatgctatc cctccccact ccacccaccc cacaacagtc 181200

cccagagtgt gatgttcccc ttcctgtgtc cacgtgttct cattgttcaa ttcccaccta 181260

tgagtgagaa tatgcggtgt ttggtttttt gttcttgaga tagtttacca agaatgatga 181320

tttccagttt catccatgtc cctacaaagg acatgaactc atcatttttt atggctgcat 181380

agtattccat ggtgtatatg tgccacattt tcttaatcca gtctatcatt gatggacatt 181440

tgggttggtt ccaagtcttt gctattgtga atagtgccgc tataaacata cgtgtgcatg 181500

tgtctttata gcagcatgat ttatagtcct ttgggtatat acccagtaat gggatggctg 181560

ggtcaaatgg tatttctagt tctagatccc tgaggaatcg ccacactgac ttccacaaag 181620

gttgaactag tttacagtcc caccaacagt gtaaaagtgt tcctatttct ccacatcctc 181680

tccagcacct gttgtttcct gactttttaa tgattgtgat tctaactggt gtgagatgat 181740

atgtcgttat ggttttgatt tgcatttctc tgatggccag tgatggtgag cattttttca 181800

tgtgtttttt ggctgcataa atgtcttctt ttgagaagtg tctgttcatg tcctttgccc 181860

actttttgat ggggttgttt gtttttttct tgtaaatttg tttgagttca ttgtagattc 181920

tggatattag ccctttgtca gatgagtagg ttgcaaaaat tttctcccat tttgtaggtt 181980

gcctgttcac tctgatggta gtttcttttg ctatgcagaa gctctttagt ttagttagat 182040

cccatttgtc aattttggct tttgttgcca ttgcttttgg tgttttagac atgaagtcct 182100

aaaggtgtgc cttttgtaaa gtggtagaag ggcagttata acagggaaaa tgggaaagca 182160

agataagtgt tacacttcca cttgagtggt tctctggcaa atcagttttt tcaaagggga 182220

taccagtaag ttgatagttg tagaaattaa ttcccttaaa accaccatgt tggctgggcg 182280

tggtggttca cacctgtaac cccagcactt tgggaggctg aggcgggtgg atcacttgag 182340

gtctggagtt cgagaccagc ctggccaaca tggtgaaacc ccatctctac taaaaaaatg 182400

gaaacattag ccaggcatgg tggtgtgcac ctgtaatccc agctacttgg gaggctgagg 182460

cagtagaatc tcttgaaccc aggaagggga ggttgcagtg agccgagatc acaccactac 182520

actctagcct gggcaacaga gcaagactgt ctcaaattaa aaaacaaaaa caaaaaaaac 182580

cccaccatgt ctatacacct ctggcaaagt cttcctgtaa ccccagggat acttgactct 182640

aatttaaaga ctatagacct atgacatggc tgatcaacta gcaaaaagtt atcactcacc 182700

ttaatgaaca gttaacttaa acattgaaaa cctcttgtgt ccacaaggtt atgttaaata 182760

ctggaggtag tggtgttata gatatttttt aagaattttt ttttttttga gacagtctcg 182820

ctctgttggc cagtctggag tatagtgtgg catgatcttg gctcactgca acctctgcct 182880

cctgggttca atcaattcct tgcctcagcc tcccacgtag ctgggactac aggcacatac 182940

cgccacaccc agctaatttt tatattttta gtagagacgg ggtttcatca tcttggccag 183000

gctggtcttg aactcctaga cctcatgatc caccctcctc agcctcccaa agtgctggga 183060

ttacaggcgt gagtcaccac gcctggcttc tttaagaatt tttttaaaat atgtatgtat 183120

gagtcacaat ctctctgcct gagcacccat agtctcattg gagaacttag ataagataca 183180

tatcacaaaa agattaataa ccatacaagg cagtaaatga tcatcacagc tagtggtggg 183240

aagaaggacc attattactt ctaggtgtgt aaagaaaggt atgataagca tggttacctt 183300

tcagttaggc ctgatcatct gggtttcagg tagctagaga agggtgaggg agggcattat 183360
aagcagagta ggagcagcgg caataaaaaa gttaaaagta gctttgtatt gggatagtct 183420
tctctacaaa tcctgttact tacttaactg tattatctcg ggcaagttac ttaatttatt 183480
tgagcattgg attccttatc tgtaaaacag agtcaacacc aaccttgtag aattcttttt 183540
tgagtattag atgatatcta gaatccaagt gtcccaacgt tttttggcct ttcagggcca 183600
ccttttccac tgatagccaa gtaagaatac ctgaattgct gtccataata tatgactata 183660
ggaactccag atcctttctc tcaactttga agacccctgt ttgaagtgct ttacattctc 183720
ctggctgttg cttgtcactt gttgctccaa catattattc caggttgctc ccaaaattct 183780
aagactgctg tcttccatta aaatgatgat gttatgatgg tgtagcttca gtagggggca 183840
ttgtgaaaga aaactgtaat cccctcaatt cagtaactct ttccaaggtt catcctttaa 183900
cctctactac atagaggcca cattgcaatc aaagctttac taatgcctgg cagttaaaca 183960
agggtcatta cagcaccaaa caggaaataa cacaatgtat caagcttggc ggtttctaaa 184020
attcatcagc tctgccctgt agcagggatc ttttatgggc tttctccact ttagcctcta 184080
ctctccccct gttgcaccta ttccctctaa gaccaaaagc ccttctctca ggctgtcctc 184140
cctgagtccc aagactccta acatctatcc ctgcctgccc tcaaaagagg caaaatatta 184200
ggaaattaag aaattagaaa aaattataaa tctctgaatc cagggaagac aagccttttt 184260
atttcgttaa acagatttta gctcctaatg gcttcccctt ctctcctctg gcggaggctc 184320
tggtttgtat aaccaagtgg cccattagcg tttccacaat aaaagggctt aagatgcaga 184380
tgccaaaacg actgctaaaa tgttaacctt tttggttttc cctacacata tatagaggac 184440
ttaacacaac gtccaacaac aagaagtgct gaataaattt tttttttttt taaagtaaag 184500
aagaggcact gtcagatctg ttttagatgg ttaccctgtg agtgggaaaa tggactggat 184560
aaagagagac caaaagcagg ggagcaatta gagaaccttt gccacagtcc aaggatcatg 184620
aagatttgag cttgcactcc ttttgtgggt ttcccaccat ctatgatgtg gcattggttt 184680
tttaaaactt tttattgagg tgtaatacat acatcgtaag ctgtaaatgt acagcttatt 184740
gtgttttcaa tgagtgtgaa gtatattttt caacttatct acaaggttga attatttccc 184800
ttttttttct ctctccagat aatttttatg gtgggacgag gatacctgtc tccagatctc 184860
agtaaggtac ggagtaactg tccaaaagcc atgaagagat taatggcaga gtgcctcaaa 184920
aagaaaagag atgagagacc actctttccc caagtaagta aaagcttcat gctatccaaa 184980
agaacagact aacattcata gacagatttc tgagcacttt tttgggcaca cagtgtgtat 185040
ttcatgagtt tggattctat gtgcagactc cagacaagaa aacacattaa gatggcttca 185100
tgagggttga gcagtggcac acctagaaat tttgggtcct aatacaaaat attcagaaag 185160
actttgcatt tgtccatcag ttctcagact tctcagtctt tgaactcttt tacacattta 185220
aaaattatcg aagacccccc cccccacaaa gagcttttgt ttatataagt tttaattcct 185280

tattagaaat taaaactatt ttttaaaata ttaatgaatt aaacatagta aaccaatatg 185340

ttaaataata tttagaaaaa aacagttcta ttatctaaga gaaaaaaatt agtgaggagt 185400

tgtattgttt tacttttttt gcaaatccct ttgcaaaaag aagacagctg gattctccgc 185460

ttttgcactc acattgcaat atctcacatc atgtcacccc taaaaactct acttaacgct 185520

tgtaaaataa tgacagtaaa aaaggcaaat gatatcttat tgttattaca aaaatagttt 185580

tgacctcgtg gatcccctgg tggtccacgg accacatttt gtgaaccact gctttatttc 185640

ttcttggtgg taaacagatt caagctttcc tttaatactg ggtctttttg aggggcattt 185700

ctgcctgatg cagaaaaagg aaaaggcagt aaggcatttg tcagctcagc ctgcctttac 185760

ctaattcttg ataactcact gctttttttt ttttttttcc atattggaag gataaagcct 185820

taagttaaca aatttcaaaa agaactgtaa ctaaggccag gtgtcgtggc ttacaaatct 185880

caacactttg ggaggccaag gcaggcagat cagttgaggt caggagttcg agaccagcct 185940

ggccaacatg gtgaaaaccg tctctactaa aaatacaaaa attagctggg catagtggca 186000

ggagcctgta atcccagcca ctcaggaggc tgagacatga gaatcgcttg aacccaggag 186060

gtggagattg taatgagctg agatgacacc actgcattcc agccagggca acagaatgag 186120

actctgttta aaacaaaaca aaacaaaaat atctaaatac ctcaactagc ttacagagtt 186180

tagctgtagt agatattata atataaatgc aatgtttctt gatacttagg gagctcctga 186240

tagcactgga taaatatgcc ttgatgaatc agtacagttt caagtgggaa gtgctatttc 186300

ccatagtaac cctgctacca taattactgg agtgttcatg tatgaactct taggcctttg 186360

aatgcccagt cctagctgag taactcagac agaagtcaat gttacaggaa ttagattcac 186420

tcatccttta tttttaaatc taaaaaatgt tattcttctc taaagaatga aagaagataa 186480

aattgatggt tttaacaagt ctgtcagggt gttgcctaag aaaagaaagg gaaagctaag 186540

tggttggctg ggtagatata agtttcattt atcaggtttc aaagttactg ttcctttgag 186600

gaagagtttt tattttttct ttttattttt ttatttgttt ctctaagtca ggaaacctct 186660

gggttgagac tgtcctggtg agagacaagt gctgctgagc agcttcagca tgaccctgtc 186720

ccatgttctc ttttcacgtc atggtgctcg ggttcttact tagaatgttt attgacatta 186780

ataatgcaaa gtacatcctt aggcccactt tttaaatgat attaaaaggg gcaaaatgct 186840

tagctccata cttctaaata taacatttca ttaattacat ggtttcaaaa actgaagtgc 186900

atatgtgaac tttccagatt attggaggct atccctttaa agtgttattt tgaaattttt 186960

gttacaggat atcttttcac accatactgt tatatgccac ataaatttta gatggctgaa 187020

gatctatatg ttttataaaa tatgaaatca tttttatagt tttgaggtag gaaaggcttt 187080

cttaatagac aaaattcaga aggaaaaaat tagcagatgt gagtacatta aaaattttta 187140

aacttctata tagaaaaata acattgaaag ataaaaagaa taattgtagc atatataaca 187200

aaaagtaaat gtttataata tacaaaggac tcctccaaat caataagtaa caaacaatag 187260

aaaatgggca aagggaatat gaacaggtga ttcacataat aaatacaaat ggtgaatgaa 187320

cttaggaaaa ggtgtaaata tttccatggc agtcagtaaa atgcaaagac acaacagtga 187380

aatatatttt aatttcgcct ctcaggttgg taatatccag cactgggaac aatgttggga 187440

agtgaggagg agcattctat gtaaaatttt aaaggtttgg tggaaggcag cattttggaa 187500

gaccttggta gaatccataa attcaaaata cttctaaaaa tctgtgctat gtaacctatt 187560

tcataagtat tcagatatat ataagaatgt ttactataat aagaaaaaga cattaagatt 187620

aagttagttt tgtattgatg acatggatat tggtgagaga aaaagaaaac aagacagaaa 187680

acaaaatgta gtatgatacc tcattttttt tttttatagg ctgggcctgt tctgttgccc 187740

aggctggagt gcagtaatat gatcatagcc cactgcagcc tcaaactcct gggctcaagc 187800

gatcctctca cctcagcctc ctaagtagct gggattgcag gtgcctatca ccacacccag 187860

ctaatttctt gtggtggtgg tggtggtggt tgtagagata gattctcact atgttgccct 187920

ggctggtctt gaactcctgg cctcaagcga tcctctcgcc tcagcctccc aaagtgctgg 187980

gattacaggc gtgagcccca gtgcccagcc tgataaccac tttaaaaggt taaacagagg 188040

ccaggcacgg tggctcacgc ctgtaatccc agcactttgg gaggccgagg caggcggatc 188100

acctgaggtt gggagttcga aaccagcctg accaacatgg agaaactctg actctactaa 188160

gaatacaaaa ttagccaggc gtggtggcac atgcctgtaa tcccagctac tcgggaggct 188220

gaggcaggaa aatcatttga acccaggagg cggaggttgc ggtgagccga gatcgcacca 188280

ttgcactcca gcctgggcaa caagagtgaa attccatctc caaaaaaaaa aagtggtgtt 188340

caggtgggcc ttgttttcat gtatgtattt ttatacataa aaaaaggtac tgaagaggcc 188400

aggcgcagtg actcacacct gtaatcccag cactttggga ggccaaggtg ggtggatcag 188460

ttgaggttag gagttcgaga ccagcctggg caaaatggtg atacccgtct ctactaaaaa 188520

tacaaaatta tccgggcgtg gtggcacacg cctgtgatcc cagctactcg ggaggctgag 188580

gcaggagaat cgcttgaacc tgggaggcgg aggttgcagt gagctgagat cgcgccactg 188640

cactccagcc tggacaatag agtgagactc catctcaaaa aaaaaaaaaa aaggtacaga 188700

agaaagtata gactctaaca gtggttatcc ctggagagca ggatttgaga gccttatact 188760

ctttatacat ttctatagta ttttaatttt tatttgcatg ttatacttgg aatttacaat 188820

tttttgcaac tgcttacttc tttgtcttat actaatcatc ataaagatta ctttttaaaa 188880

aaaatttaac ttttaaaaac aattttcagc caggcatggt ggctcatgtc tgtaatccca 188940

gccctttggg aggccgaggc aggcagatca cctgaggtca ggagtttgag accagcctgg 189000

ccaacgtggt gaaaccctgt ctctgctaaa aatacaaaaa tttagctggg catggtggtg 189060

cgctcctgta atcccagcta ctcaggaggc tgaggcagga gaatcgcttg aacccaggag 189120

ggggaggttg cagtgagctg agattgtgcc actgcactcc agcctggttg acaacagcga 189180

gactccgttt caaaaaaaaa aaaaaattgg tatctcagga caataacaaa agtaataata 189240

atagctgcta aggttttatt gagtgcttat tataggccag gcattatgcc aagcccttta 189300

aacatgtttc atgattatga acatgcatta tcatgctgta tgccttcaag gattataacc 189360

tgtttctttg tgccttaaaa ttgtgaattt ctgcatttta tatattgggg tctatttgtc 189420

gagttctcct atctttgctc ttgggttgtc ccctgtcact tctcatgtgc tactagcact 189480

ctgggtctgt gaggttctgc tttcaattag gtgtatgtaa aacatttccc atggctaggt 189540

ttctttaaag ggcaagtagc tgtgataatt ctgtttagag atagtcataa agtgctttac 189600

ttatttatac tccatcttct tcccaaaaga gacttgtggt ctataacaaa aaggtataaa 189660

attggtttta aatttctatt atttactgtt tcaagactaa caaatgatct aaaatataaa 189720

taaaagctga ctaagaatta ctctccccat ttaatttaca gagagagttt cttcttaaga 189780

aaaaatacca attatttaca aatattttcc caagcattta tgacaatgct gaaaacaatg 189840

taagatttca ggtgctttct tgtaaagtgt gatgggactc ttaaagattt ataccaccca 189900

gattttcatt cttctttctg tttttctttt ttctttcttt cttttttttt ctttttttctt 189960

tttttttctt ttttttttct tttttttttt gtagattctc gcctctattg agctgctggc 190020

ccgctcattg ccaaaaattc accgcagtgc atcagaaccc tccttgaatc gggctggttt 190080

ccaaacagag gattttagtc tatatgcttg tgcttctcca aaaacaccca tccaggcagg 190140

gggatatggt gcgtttcctg tccactgaaa caaatgagtg agagagttca ggagagtagc 190200

aacaaaagga aaataaatga acatatgttt gcttatatgt taaattgaat aaaatactct 190260

cttttttttt aaggtgaacc aaagaacact tgtgtggtta aagactagat ataatttttc 190320

cccaaactaa aatttatact taacattgga tttttaacat ccaagggtta aaatacatag 190380

acattgctaa aaattggcag agcctcttct agaggcttta ctttctgttc cgggtttgta 190440

tcattcactt ggttatttta agtagtaaac ttcagtttct catgcaactt ttgttgccag 190500

ctatcacatg tccactaggg actccagaag aagaccctac ctatgcctgt gtttgcaggt 190560

gagaagttgg cagtcggtta gcctgggtta gataaggcaa actgaacaga tctaatttag 190620

gaagtcagta gaatttaata attctattat tattcttaat aatttttcta taactatttc 190680

tttttataac aatttggaaa atgtggatgt cttttatttc cttgaagcaa taaactaagt 190740

ttctttttat aaa 190753

<210> 4
<211> 13
<212> DNA
<213> Homo sapiens

<400> 4
tacagtgaaa tct 13

<210>  5
<211>  7
<212>  DNA
<213>  Homo sapiens

<400>  5
tatagag                                                                 7


<210>  6
<211>  10
<212>  DNA
<213>  Homo sapiens

<400>  6
tatagctttg                                                              10


<210>  7
<211>  9
<212>  DNA
<213>  Homo sapiens

<400>  7
tatctggag                                                               9


<210>  8
<211>  8
<212>  DNA
<213>  Homo sapiens

<400>  8
tgttacag                                                                8


<210>  9
<211>  8
<212>  DNA
<213>  Homo sapiens

<400>  9
tactacag                                                                8


<210>  10
<211>  7
<212>  DNA
<213>  Homo sapiens

<400>  10
tactgag                                                                 7


<210>  11
<211>  8
<212>  DNA
<213>  Homo sapiens

<400>  11
tacaacag                                                                8


<210>  12
<211>  10

<212>  DNA
<213>  Homo sapiens

<400>  12
tactactacg                                                              10


<210>  13
<211>  12
<212>  DNA
<213>  Homo sapiens

<400>  13
tacagatact cg                                                           12


<210>  14
<211>  15
<212>  DNA
<213>  Homo sapiens

<400>  14
tacagaaaaa tctcg                                                        15


<210>  15
<211>  15
<212>  DNA
<213>  Homo sapiens

<400>  15
tacaaagaaa tctcg                                                        15


<210>  16
<211>  15
<212>  DNA
<213>  Homo sapiens

<400>  16
tacaaggaaa tctcg                                                        15


<210>  17
<211>  13
<212>  DNA
<213>  Homo sapiens

<400>  17
tacagaatct ctg                                                          13


<210>  18
<211>  13
<212>  DNA
<213>  Homo sapiens

<400>  18
tacagaaata tct                                                          13


<210>  19
<211>  13
<212>  DNA
<213>  Homo sapiens

<400>  19
tacagataaa tct                                                          13


<210>  20
<211>  13
<212>  DNA
<213>  Homo sapiens

<400>  20
tacagggaaa tct                                                          13


<210>  21
<211>  13
<212>  DNA
<213>  Homo sapiens

<400>  21
tacagagaaa tct                                                          13


<210>  22
<211>  13
<212>  DNA
<213>  Homo sapiens

<400>  22
tacaatgaaa tct                                                          13


<210>  23
<211>  13
<212>  DNA
<213>  Homo sapiens

<400>  23
tacattgaaa tct                                                          13


<210>  24
<211>  13
<212>  DNA
<213>  Homo sapiens

<400>  24
tacgaggaaa tct                                                          13


<210>  25
<211>  13
<212>  DNA
<213>  Homo sapiens

<400>  25
tacagcgaaa tct                                                          13


<210>  26
<211>  15
<212>  DNA
<213>  Homo sapiens

<400>  26

tacaaagaaa tcgcg                                                    15

<210>  27
<211>  13
<212>  DNA
<213>  Homo sapiens

<400>  27
tacagtggaa tct                                                      13

<210>  28
<211>  13
<212>  DNA
<213>  Homo sapiens

<400>  28
tacagtgaat tct                                                      13

<210>  29
<211>  9
<212>  DNA
<213>  Homo sapiens

<400>  29
tacagtgag                                                            9

<210>  30
<211>  10
<212>  DNA
<213>  Homo sapiens

<400>  30
tacagtgaag                                                          10

<210>  31
<211>  9
<212>  DNA
<213>  Homo sapiens

<400>  31
tacagagag                                                            9

<210>  32
<211>  13
<212>  DNA
<213>  Homo sapiens

<400>  32
tacagatgtt ggg                                                      13

<210>  33
<211>  17
<212>  DNA
<213>  Homo sapiens

<400>  33
tacagatttt ggtctag                                                  17

<210> 34
<211> 10
<212> DNA
<213> Homo sapiens

<400> 34
tacagtgcaa 10


<210> 35
<211> 15
<212> DNA
<213> Homo sapiens

<400> 35
tacagatggg tccca 15


<210> 36
<211> 21
<212> DNA
<213> Artificial Sequence

<220>
<223> forward pyrosequencing frimer U-Braf F

<400> 36
atgcttgctc tgataggaat g 21


<210> 37
<211> 20
<212> DNA
<213> Artificial Sequence

<220>
<223> reverse pyrosequencing primer BRAF-Pyro-R

<400> 37
agcatctcag ggccaaaaat 20


<210> 38
<211> 10
<212> DNA
<213> Artificial Sequence

<220>
<223> wt sequence V600 to S602

<400> 38
agtgaaatct 10


<210> 39
<211> 12
<212> DNA
<213> Artificial Sequence

<220>
<223> wt sequence T599 to S602

<400> 39
tcagtgaaat ct 12

<210>  40
<211>  20
<212>  DNA
<213>  Artificial Sequence

<220>
<223>  pyrosequencing primer U-BRAF-600

<400>  40
ggtgattttg gtctagctac                                                   20


<210>  41
<211>  21
<212>  DNA
<213>  Artificial Sequence

<220>
<223>  sequencing primer U-BRAF-599

<400>  41
aataggtgat tttggtctag c                                                 21


<210>  42
<211>  22
<212>  DNA
<213>  Artificial Sequence

<220>
<223>  Sanger sequencing primer BRAF-15F

<400>  42
tgcttgctct gataggaaaa tg                                                22


<210>  43
<211>  20
<212>  DNA
<213>  Artificial Sequence

<220>
<223>  Sanger sequencing primer BRAF-15R

<400>  43
agcatctcag ggccaaaaat                                                   20

## Description

The present invention relates to a method for differentiating between at least two sequence variants of a polynucleotide by pyrosequencing, wherein at least one sequence variant comprises at least one mutation, said method comprising a) generating a dispensation order based on a nucleotide sequence encompassing the nucleotides affected by said variants, said dispensation order comprising dispensations corresponding to each nucleotide occurring in at least one of the variants, said nucleotide dispensations ordered in a sequence i) generating on average a pyrogram peak for at least every second nucleotide dispensation for each variant and ii) generating the maximal possible number of sequence pyrogram peaks for each variant; b) generating according to the dispensation order of a) a first pyrogram comprising expected intensity values for a first variant and at least a second pyrogram comprising expected intensity values for at least one further variant; c) comparing a sample pyrogram comprising measured intensity values, said sample pyrogram being derived from pyrosequencing analysis of a sample polynucleotide according to the nucleotide dispensation order of a), with the said first and at least the said second pyrogram generated in step b); and d) differentiating between said at least two sequence variants, wherein the sample polynucleotide corresponds to the first variant if the sample pyrogram is essentially identical with the first pyrogram and wherein the sample polynucleotide corresponds to a further variant if the sample pyrogram is essentially identical with a further pyrogram.

Mutations in certain genes are associated with disease, either with hereditary disease or with acquired disease like, e.g. cancer. Over recent years it has been realized that identification of mutations present in a subject or in a subset of cells from a subject, e.g. cancer cells, is of outmost diagnostic value, since such knowledge allows prognosis to be adjusted and suitable therapy measures to be determined. Often, a mutation in a cancer cell confers a selective growth advantage to the cancer cell and thus the presence of such a mutation provides a target for the development of targeted molecular therapeutics.

The human *braf* gene is one example of such a gene, the mutation of which may confer a selective advantage to the cancer cells. Mutations of the *braf* gene are found in the majority of cutaneous malignant melanomas and in subsets of papillary thyroid, serous ovarian, and colorectal carcinomas. More than 80% of the *braf* mutations in cancer are T->A substitutions, leading to a substitution of a glutamate for a valine residue (usually called the "V600E mutation") in the polypeptide chain of the BRAF protein. The added charge appears to mimic a phosphorylation and leads to a constitutive activation of the BRAF protein and thereby to an increased signaling in the mitogen activated protein kinase (MAPK), the mitogen activated protein kinase kinase (MEK), and extracellular signal-related kinase (ERK) pathways.

Specific detection of mutations has been accomplished by a variety of methods, like immunostaining with mutation-specific antibodies, hybridization to mutation-specific oligonucleotide probes, or sequencing. The sequencing methods typically rely on one of the Sanger-methods of sequencing, i.e. chain-termination methods. However, pyrosequencing, i.e. real-time sequencing by synthesis, has also been used, advantages being higher throughput capacity higher diagnostic sensitivity (i.e. the ability to distinguish down to 10% mutant DNA from background noise). The downside of pyrosequencing, a relatively short read length of approximately 100 bp, is hardly of significance in mutation identification, since relevant mutations often are clustered in hot-spots of less than 50 nucleotides in length.

In the case of the human *braf* gene, direct sequencing is widely accepted as the gold standard for mutation detection in biopsies. However, as described above, sequencing can be difficult if a strong background of non-mutated tissue is present in the sample. The current pyrosequencing protocols available for *braf* mutations are adapted to reliably identify the most frequent mutation as described above, but they are unreliable in the identification of other methods, especially at unfavorable signal / background ratios. E.g., Spittle et al. (1) propose a protocol for the identification of the V600E mutation; they also state that the pyrograms obtained for other mutants can be explained once the sequence is known, but they recommend dideoxy cycle sequencing to confirm the specific variant mutation for cases in which the pyrogram shows a pattern that is not consistent with either wild-type sequence or BRAF V600E.

There is, thus, a need in the art to have a method for optimized identification of a mutated sequence by pyrosequencing. The technical problem underlying the present invention, therefore, can be seen as the provision of means and methods for satisfying the aforementioned needs. The technical problem is solved by the embodiments characterized in the claims and herein below.

Therefore, the present invention relates to a method for differentiating between at least two sequence variants of a polynucleotide by pyrosequencing, wherein at least one sequence variant comprises at least one mutation, said method comprising:
a) generating a dispensation order based on a nucleotide sequence encompassing the nucleotides affected by said variants, said dispensation order comprising dispensations corresponding to each nucleotide occurring in at least one of the variants, said nucleotide dispensations ordered in a sequence
   i) generating on average a pyrogram peak for at least every second nucleotide dispensation for each variant and
   ii) generating the maximal possible number of sequence pyrogram peaks for each variant;
b) generating according to the dispensation order of a) a first pyrogram comprising expected intensity values for a first variant and at least a second pyrogram comprising expected intensity values for at least one further variant;
c) comparing a sample pyrogram comprising measured intensity values, said sample pyrogram being derived from pyrosequencing analysis of a sample polynucleotide according to the nucleotide dispensation order of a), with the said first and at least the said second pyrogram generated in step b); and
d) differentiating between said at least two sequence variants, wherein the sample polynucleotide corresponds to the first variant if the sample pyrogram is essentially identical with the first pyrogram and wherein the sample polynucleotide corresponds to a further variant if the sample pyrogram is essentially identical with a further pyrogram.

The method of the present invention, preferably, is an in vitro method. Moreover, it may comprise steps in addition to those explicitly mentioned above. For example, further steps may relate to sample pre-treatments and sample amplification or evaluation of the results obtained by the method. Additionally, internal controls, such as DNA quality controls or performance controls may be used. The method may be carried out manually or assisted by automation. Preferably, steps (a) to (d) may in total or in part be assisted by automation, e.g. by suitable robotic and sensory equipment for the pyrosequencing analysis in step (c).

The term "differentiating", as used herein, means to distinguish between sequence variants. Preferably, at least two, at least five, at least ten, at least 15, at least 20, at least 21, at least 22, at least 23, at least 24, at least 25, at least 26, at least 27,or at least 28 variants are differentiated. More preferably, at least 29, at least 30, at least 31, or at least 32 sequence variants are differentiated by the method of the present invention.

The terms "variant", "sequence variant" or "sequence variant of a polynucleotide" relate to a polynucleotide comprising a specific nucleic acid sequence, i.e. a specific sequence of consecutive nucleotides. Preferably, said variants are naturally occurring variants, e.g. mutations detectable in clinical samples. It is to be understood that, in the context of the present invention, the wild-type sequence of a polynucleotide, i.e. the sequence of the allele with the highest frequency of occurrence in a given population, is also to be regarded as a variant. In order to have two variants that can be differentiated, it is clear for the skilled person, that at least one variant comprises at least one mutation relative to the other variant. Preferably, the at least one mutation is associated with disease. Said disease, preferably, is a hereditary disease. More preferably, the disease an hyperproliferative disorder wherein the cells affected by the disorder comprise a mutation of at least one gene. Even more preferably, the disease is cancer. Most preferably, the cancer is acute myeloid leukemia (AML), acute myeloid leukemia therapy related (AMLTR), astrocytoma, biliary tract carcinoma, breast carcinoma, cholangiocarcinoma, colorectal carcinoma, endometrioid carcinoma, esophageal carcinoma, fibrous histiocytoma-pleomorhic sarcoma, gastrointestinal stromal tumor (GIST), glioma, hepatocellular carcinoma, large intestine adenocarcinoma, small intestine adenocarcinoma, liposarcoma, lung adenocarcinoma, melanoma, malignant melanoma, melanoma metastasis, ocular melanoma, ovarian carcinoma, ovary mucinous carcinoma, ovary serous carcinoma, pancreatic ductal carcinoma, pituitary adenoma, plasma cell myeloma, prostate adenocarcinoma, stomach adenocarcinoma, synovial sarcoma, thyroid anaplastic carcinoma, or thyroid papillary carcinoma.

Preferably, the variants are variants of the human *braf* gene (genomic sequence: Genbank Acc. No: NG_007873.1 GI:188595650 SEQ ID NO: 3, mRNA / cDNA sequence: Genbank Acc. No: NM_004333.4 GI:187608632, SEQ ID NO: 2, the BRAF open reading frame starts at position 62 of this sequence). More preferably, the variants are variants comprised in exon 15 of the *braf* gene. Even more preferably, the variants comprise at least one mutation located at a nucleotide position corresponding to position 1794 to 1804 of the wild-type BRAF open reading frame. Most preferably, the variants are variants selected from Table 1.

**Table 1: variants of the human braf gene**

| change (protein level) | variant (DNA level) | relevant sequence | SEQ ID NO |
|---|---|---|---|
| wt | Wt | TACAGTGAAATCT | 4 |
| T599I;V600E | C1796T;T1799A | TATAGAG | 5 |
| T599_V600>IAL | CAG1796_1798>TAGCTT | TATAGCTTTG | 6 |
| TVKSR599_603>1 | CAGTGAAATCTCGA1796 1809>TC | TATCTGGAG | 7 |
| T599_V600insV | T1794_1795insGTT | TGTTACAG | 8 |
| (A598_T599insV) | | | |
| T599_V600insT (1) | C1796_1797insTAC | TACTACAG | 9 |
| T599T;V600E | A1797T;T1799A | TACTGAG | 10 |
| T599_V600insT (2) | A1797_1798insACA | TACAACAG | 11 |
| T599_V600insTT | A1797insTACTACG | TACTACTACG | 12 |
| VKS600_602>DT | TGAAAT1799_1804>ATA | TACAGATACTCG | 13 |
| V600E (2) | TG1799_1800AA | TACAGAAAAATCTCG | 14 |
| V600K | GT1798_1799AA | TACAAAGAAATCTCG | 15 |
| V600R (1) | GT1798_1799AG | TACAAGGAAATCTCG | 16 |
| VK600_601>E | TGA 17991801 del | TACAGAATCTCTG | 17 |
| V600E;K601I | TG1799_1800AA; A1802T | TACAGAAATATCT | 18 |
| V600D | TG1799_1800AT | TACAGATAAATCT | 19 |
| V600G | T1799G | TACAGGGAAATCT | 20 |
| V600E (1) | T1799A | TACAGAGAAATCT | 21 |
| V600M | G1798A | TACAATGAAATCT | 22 |
| V600L | G1978T | TACATTGAAATCT | 23 |
| V600R (2) | AGT1797_1799GAG | TACGAGGAAATCT | 24 |
| V600A | T 1799C | TACAGCGAAATCT | 25 |
| V600K_S602S | GT1798_1799AA;T1806G | TACAAAGAAATCGCG | 26 |
| K601E | A1801G | TACAGTGGAATCT | 27 |
| K601N | A1803T | TACAGTGAATTCT | 28 |
| K601R | A1802G | TACAGTGAG | 29 |
| K601K | A1802G | TACAGTGAAG | 30 |
| V600E;K601R | T1799A;A1802G | TACAGAGAG | 31 |
| VKSRWS600_605>DV | TGAAATCTCGATGGAG1799_1814>ATGT | TACAGATGTTGGG | 32 |
| T599_V600insDFGLAT | G17981799insATTTTGGTCTAGCTACAG | TACAGATTTTGGTCTAG | 33 |
| K601Q | A1801C | TACAGTGCAA | 34 |
| VKSRWS600_605>D | TGAAATCTCGATGGAG1799_1814>A | TACAGATGGGTCCCA | 35 |

| | | | |
|---|---|---|---|
| All sequences start with the T at position 1794 of the BRAF open reading frame | | | |

The term "pyrosequencing" is understood by the skilled person. The term relates to a sequencing method in which a sequencing primer is annealed to a single-stranded polynucleotide and wherein single nucleotide dispersions are applied to a reaction mixture, such that extension can only take place if the next nucleotide in sequence is the nucleotide present in the reaction mixture. Incorporation of a nucleotide into the growing polynucleotide strand is then translated into a measurable signal. Preferably, the signal is proportional to the amount of nucleotide incorporated.

The term "dispensation order" relates to a sequence of nucleotide dispensations, i.e. additions of a specific nucleotide to the sequencing reaction mixture, applied in a pyrosequencing reaction. The dispensation order of the present invention is not the typical dispensation order used in pyrosequencing, comprising a repetition of all four nucleotides in succession, e.g. in the order (AGTC)ₙ. In contrast, the dispensation order of the present invention is highly optimized to permit differentiation of a large number of variants, as described herein above, while using a small number of elongation cycles. Preferably, the dispensation order comprises dispensations such as to encompass the nucleotides allowing the identification of variants, i.e. encompassing the nucleotides wherein one variant is mutated as compared to another. More preferably, the dispensation order encompasses the nucleotides frequently mutated in polynucleotides comprising exon 15 of the human *braf* gene obtained from tumor samples. Most preferably, the dispensation order encompasses the nucleotide positions 1794 to 1804 of the BRAF open reading frame. It is understood by the skilled artisan that a dispensation order is not a nucleic acid sequence.

The term "generating a dispensation order", as used herein, relates to selecting a dispensation order according to the directions given in this specification. Preferably, the dispensation order is generated with the aid of a computer, comprising the directions of the present invention implemented as algorithms. It is understood by the skilled person that the nucleotide sequence encompassing the nucleotides affected by the variants to be differentiated is the basis for generating the dispensation order. Furthermore, the dispensation order preferably comprises dispensations corresponding to each nucleotide occurring in at least one of the variants.

Preferably, the dispensation order, when applied to a sample comprising at least one variant, on average leads to the generation of a pyrogram peak for at least every second dispensation, i. e. generates on average a pyrogram peak for at least every second nucleotide dispensation for each variant. This means that the ratio of pyrogram peaks obtained to dispensations applied, calculated over the whole dispensation order, is at least 0.5 for each variant. More preferably, said ratio is at least 0.55, at least 0.6, at least 0.65, at least 0.7, or at least 0.75. It is to be understood that the dispensation order may generate pyrogram peaks for two or more dispensations in succession also. The skilled person knows that the area of the pyrogram peak in pyrosequencing is proportional to the amount of nucleotide incorporated in the sequencing reaction, meaning that a pyrogram peak will have n-fold area (and, thus, approximately n-fold height) when a sequence is reached that comprises n identical nucleotides in succession, as compared to a sequence comprising said nucleotide only once.

Preferably, the dispensation order permits differentiation of two variants present in the same sample, as described e.g. in the examples herein below. E.g. when a tumor sample is used, the template may comprise the wild-type variant derived from surrounding, unaffected tissue, and at the same time a mutant variant, derived from tumor cells. More preferably, the dispensation order permits differentiation of more than two variants present in the same sample. Preferably, the dispensation order also comprises simultaneous dispensations of two or more nucleotides for positions irrelevant to the identification of any of the variants.

It is to be understood that the dispensation order of the present invention, preferably, can only be applied with regard to variants known to the skilled person. For new, hitherto unknown variants, the dispensation order may be applicable in the sense that said variant can be identified using said dispensation order, or it may not be applicable, in the sense that said variant can not be differentiated from other variants using said dispensation order.

Preferably, the dispensation order, when applied to a sample comprising at least one variant, leads to the generation of a maximum possible number of pyrogram peaks for each variant, i.e. generates the maximal possible number of sequence pyrogram peaks for each variant. E.g., where variant 1 comprises a sequence GATAT and variant 2 comprises a sequence GTTAT, a dispensation order GATAT would be preferred over GTATA, since the second dispensation order would not generate a pyrogram peak for the last T in variant 1. Preferably, the dispensation order comprises at least one additional dispensation of a nucleotide known not to be present at the specific position in any of the variants; this dispensation serves as a control, since any positive signal obtained for said dispensation is indicative of a contamination or a failure of the detection system.

More preferably, the dispensation order generated is G₁A₂C₃G₄A₅T₆A₇C₈T₉G₁₀A₁₁T₁₂C₁₃T₁₄A₁₅G₁₆, applicable to the 28 variants of the *braf* gene as described in the examples below, or, most preferably, the dispensation order generated is G₁T₂A₃C₄A₅C₆G₇A₈T₉A₁₀C₁₁T₁₂G₁₃A₁₄T₁₅C₁₆T₁₇A₁₈G₁₉, applicable to the 32 variants of the *braf* gene as described in the examples below. It is clear for the skilled artisan that a specific dispensation order will only result in the expected pyrogram if a pyrosequencing primer priming at the same position is used for the generation of all pyrograms. Thus, the preferred dispensation orders are preferably used in conjunction with the sequencing primers related to herein in the examples. It is also clear for the skilled person that the priming position of the pyrosequencing primer may be chosen at a different position, provided that the position is the same for all pyrograms, and that the quality of the sample pyrogram(s) will decrease with increasing distance of the priming site from the site(s) of the expected mutation(s).

The term "pyrogram" is understood by the skilled person. It relates to a graphical representation of the light intensity in the reaction container of a pyrosequencing apparatus over time while a defined sequence of substrate nucleotides according to the dispensation order is applied to the sequencing container. Examples can be found in the Examples herein below. Preferably, in cases where the dispensation order and the sequence comprised as a sample are known, the relative light intensities to be expected over the course of a dispensation order can be calculated, e.g. with the help of a computer. However, preferably, prediction is also possible by assigning an expected relative light intensity of 0 for a dispensation where no reaction will occur, of 1 for a dispensation where one nucleotide will be incorporated, of 2 for a dispensation where two consecutive, identical nucleotides will be incorporated, and so forth.

The first and second, and preferably further, pyrograms of the present invention are reference pyrograms, i.e. they comprise expected peak heights to be compared to sample pyrograms. Preferably, the reference pyrograms are obtained by pyrosequencing a known variant sequence applying the dispensation order of the present invention. Preferably, at least one reference pyrogram is generated for each variant. More preferably, the pyrograms thus generated are compared and at least one property comprised in one pyrogram but not in the other(s), i.e. a unique property, is identified. Preferably, a unique property comprises at least one property selected from the group consisting of (i) the presence or absence of a pyrogram peak for the first variant;(ii) the presence or absence of a pyrogram peak for at least one further variant; (iii) the expected intensity value for a pyrogram peak for the first variant; (iv) the expected intensity value pyrogram peak for at least one further variant; and (v) the ratios of expected intensity values of (iii) and (iv) or (iv) and (iii). Preferably, differentiation between variants relies on the identification of the unique property or unique properties of the variant in the sample pyrogram. It is to be understood that a unique property, as used herein, preferably, is not necessarily a property occurring exclusively in one variant. However, the unique property, alone or in combination with other unique properties, shall allow the unambiguous identification of a variant.

The term "sample pyrogram" as used herein, relates to a pyrogram obtained performing a pyrosequencing reaction with the dispensation order using the sequence variant of a polynucleotide to be differentiated as a template. Said sample pyrogram, thus, is a true pyrogram generated during sequencing. "Comparing a sample pyrogram" with other pyrograms relates to observing if the measured light intensities correspond to the predicted light intensities of said other pyrograms. Preferably, peak area, or peak height, are used as surrogate measures of light intensities. It is understood by the skilled person that relative peak areas or peak heights are preferably to be compared, not absolute values.

"A sample polynucleotide corresponding to a variant", as used herein, relates to a sample polynucleotide having the same sequence as the said variant, i.e. comprising the same mutation(s). A sample polynucleotide corresponds to a variant if the pyrogram obtained for said variant is essentially identical to the pyrogram predicted (or obtained) for said variant. Preferably, at least one unique property is identified for each variant and the fact that a sample polynucleotide corresponds to a variant is established by identifying said unique property in the sample pyrogram. More preferably, at least one unique property is comprised in a unique dispensation order pattern.

As used herein, a "unique dispensation order pattern" is a specific pattern within a dispensation order, wherein a certain order of peaks or of unique properties allows the differentiation of a variant or the assignment of a variant to a group. Within such a group, the specific variant preferably is identified by a unique property as described herein above. Preferably, the unique dispensation order patterns are A₁₀C₁₁T₁₂ in combination with A₁₈G₁₉ within the dispensation pattern G₁T₂A₃C₄A₅C₆G₇A₈T₉A₁₀C₁₁T₁₂G₁₃A₁₄T₁₅C₁₆T₁₇A₁₈G₁₉, or A₇C₈T₉ in combination with A₁₅G₁₆ within the dispensation pattern G₁A₂C₃G₄A₅T₆A₇C₈T₉G₁₀A₁₁T₁₂C₁₃T₁₄A₁₅G₁₆.

### Figure Legends

Fig. 1: BRAF mutation analysis by (A) Sanger sequencing and (B) pyrosequencing. wt - wild type; mt - mutant; recognition patterns are indicated in black boxes; (+) indication of positive peaks within recognition patterns.
Fig. 2: Locations of primers within *braf* exon 15.
   The G residue at position 1800 of the BRAF open reading frame corresponds to the G residue at position 132 of this figure. The corresponding amino acid sequence of the BRAF protein is depicted below the corresponding DNA sequence.
Fig. 3: Predicted pyrogram peaks for *braf* mutations according to the dispensation pattern * dispensation order's nucleotides, which are involved into mt:wt ratio, are bolded; ** recognition patterns' nucleotides are indicated in black boxes; I intensity of correspondent dispensation order's nucleotide

### Examples

### Example 1: Patients' characteristics

A total of formalin fixed paraffin embedded (FFPE) tissue samples of cutaneous metastasis from 28 patients were enrolled into this study. Diagnoses were established and controlled in each tumor sample according to histopathological standards by two experienced dermopathologists independently. All patients signed informed consent at initial clinical investigation. The study was approved by local ethic committees.

### Example 2: DNA Extraction and Pyrosequencing

For the analysis of tumor samples, haemoltoxylin- and eosin-stained slides were reviewed by an experienced pathologist to ensure sufficient viable tumor content (60-90% tumor cells). Total genomic DNA was extracted from seven 10 µm thick unstained sections of FFPE tissue blocks according to manufacturer's instructions using an automated DNA Extractor (Qiasymphony, Qiagen). The total DNA was eluted in 50 µl and immediately stored at -20°C for later usage. The eluted DNA was quantified using Qubit dsDNA BR Assay (Invitrogen).

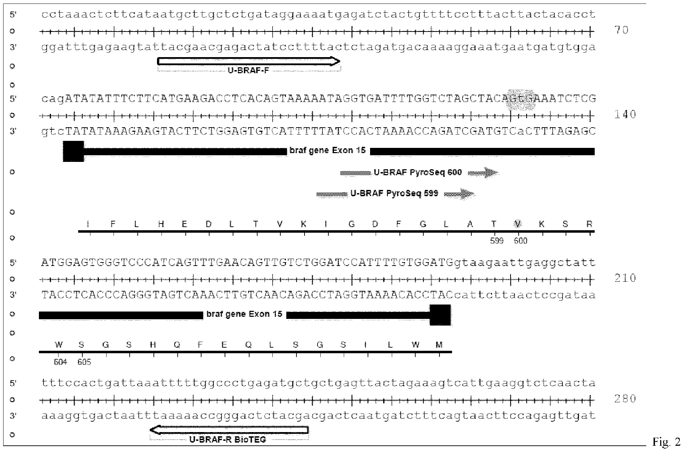

For pyrosequencing assay, the region of human *braf* spanning mutation sites within exon 15 was amplified using forward primer U-BRAF F 5'-ATGCTTGCTCTGATAGGAATG-3' (SEQ ID NO: 36) and biotinylated reverse primer BRAF-Pyro-R Biotin-TEG-5'-AGCATCTCAGGGCCAAAAAT-3' (Eurofins MWG Operon, SEQ ID NO: 37, Fig. 2). Each PCR reaction mixture was prepared with 10-20 ng genomic DNA, 10 pmol each primer, 2.5 mM dNTPs and 5 units Biotherm Taq polymerase (Genecraft) in a total volume of 50 µl. Amplification of BRAF fragment was performed in PCR cycler Flexcycler (Analytik Jena) as follows: 95°C for 5 minutes, 45 cycles of 95°C for 30 seconds, 57°C for 30 seconds and 72°C for 45 seconds followed by final extension at 72°C for 5 minutes. Specific amplification of the 229-bp fragment was verified by visualizing 5 µl PCR product on a 1.5% agarose TBE gel using SubCell electrophoresis unit (Bio-RAD), followed by 30-minutes incubation in 1^{X} SybrGreen TBE solution (Biozym).

Pyrosequencing procedure was performed identifying variant mutations either at codons V600 to S602 (5'-AGTGAAATCT-3', SEQ NO: 38) or at codons T599 to S602 (5'-TCAGTGAAATCT-3', SEQ ID NO 39) with sequencing primer U-BRAF-600 5'-GGTGATTTTGGTCTAGCTAC-3' (SEQ NO: 40) or with U-BRAF-599 5'-AATAGGTGATTTTGGTCTAGC-3' (Eurofins MWG Operon, SEQ NO: 41, Fig. 2), respectively. 20 µl PCR product were used in each dispensation order protocols of Pyromark Q24 according to manufacturer's instructions (Qiagen). Sequence pyrograms were manually analyzed using Pyromark Q24 software version 2.0.6 (Qiagen). Raw pyrosequencing data were normalized against negative A₁₅/C₁₆/A₁₈/G₁₉, followed by automatic analysis using simple operators of MS Excel 2003 (personal communication).

### Example 3: Sanger Sequencing

Sanger sequencing was performed bidirectionally with 1 µl PCR product amplified for pyrosequencing as described above, using BRAF-15F 5'-TGCTTGCTCTGATAGGAAAATG-3' (SEQ NO: 42) and BRAF-15R 5'-AGCATCTCAGGGCCAAAAAT-3' (Eurofins MWG, SEQ NO: 43) with Big Dye Terminator V1.1 cycle sequencing reagents under following PCR conditions: 25 cycles at 95°C for 20 seconds, 55°C for 15 seconds, and 60°C for 1 min (Life Technologies). DNA sequences were finally determined on 3500 Gene Analyzer (Life Technologies) and each sample was visually analyzed on the presence of mutation within Exon 15.

### Example 4: Results

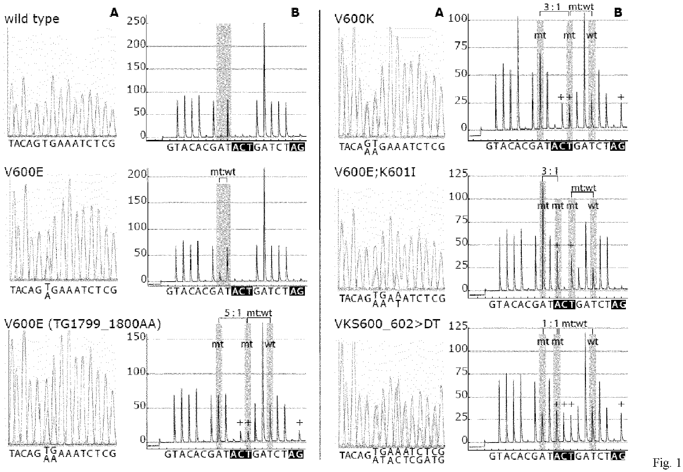

Here we present a universal approach U-BRAF that enables the detection of BRAF mutation status within exon 15 by a single pyrosequencing-based assay. We performed analysis of 75 samples, direct sequencing of which resulted in 46 (61.3 %) of mutants consisting of 28/46 (60.9%) samples with V600E mutation, 2/46 (4.3%) samples with alternative tandem mutation V600E (TG>AA), 9/46 (19.6%) samples with V600K tandem mutation, 2/46 (4.3%) samples with V600E;K601I tandem mutation, and 5/46 (10.9%) samples with complex in-frame deletion-insertion (delins) VKS600_602>DT (Fig. 1, Table 2).

Direct sequencing is widely accepted as a gold standard routinely used to detect BRAF mutations in biopsy specimens. Although restriction fragment length polymorphism analysis (RFLP) and single strand conformation polymorphism analysis (SSCP) detection methods are simpler and easy to apply, they are still less sensitive and less specific than direct DNA sequencing.¹ Alternative methods like cobas® BRAF 4800 (Roche) or BRAF RGQ PCR (Qiagen) claim to detect mutations down to a 1% level, but being the quantitative PCR-based, both detection methods have limited precision and present difficulties in reliably detecting low-copy-number templates due to nonspecific amplification and competitive side reactions.² Despite the alternative approaches based on Shifted Termination Assay (STA) and Amplification Refractory Mutations System allele-specific PCR (ARMS AS-PCR) give comparably sensitive results, detecting down to 5% mutation level in a wild type background, they are designed for detection of few hotspot BRAF mutations only.

In contrast, pyrosequencing, a real-time sequencing by synthesis, has a high throughput and capable to detect minor sequencing variants with the greater diagnostic sensitivity than direct sequencing, distinguishing up to 10% mutant DNA from background noise.³ It shows high accuracy and precision of pyrosequencing in quantitative identification of BRAF mutations in melanoma cell lines and formalin-fixed paraffin-embedded (FFPE) tumors.⁴

Commercially available pyrosequencing approach for BRAF status detection (therascreen® BRAF Pyro® Kit) is designed to analyze complementary strand of DNA starting directly at codon V600. Unfortunately, due to mismatching of sequencing primer, any sample with mutations downstream from codon V600 could be identified as a false wild type. Moreover, V600K or V600R mutants may be interpreted as a false V600E mutation at mutant-to-wild-type ratio equal to 25% or less (Qiagen, personal communication). In general, for all known BRAF status detection approaches in case of variant mutations beyond V600E/K/D/R/A, the direct DNA sequencing is additionally required.

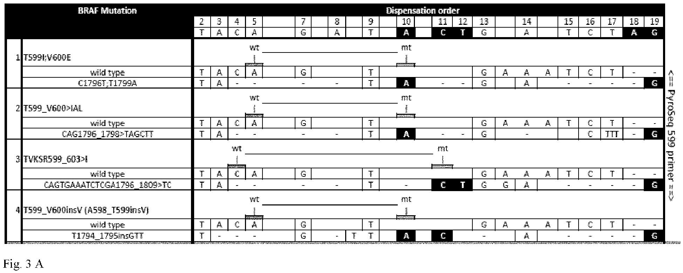

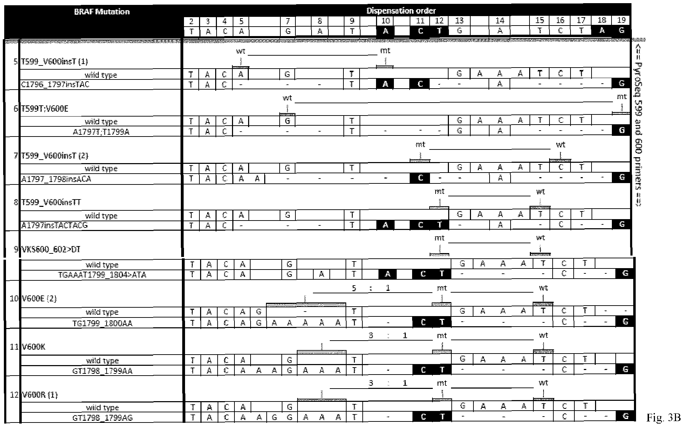

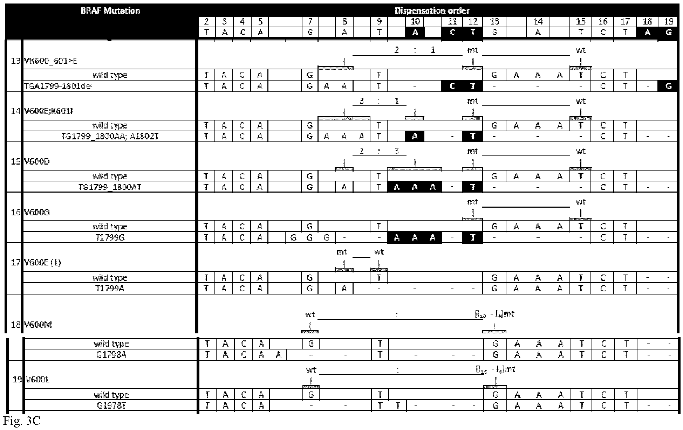

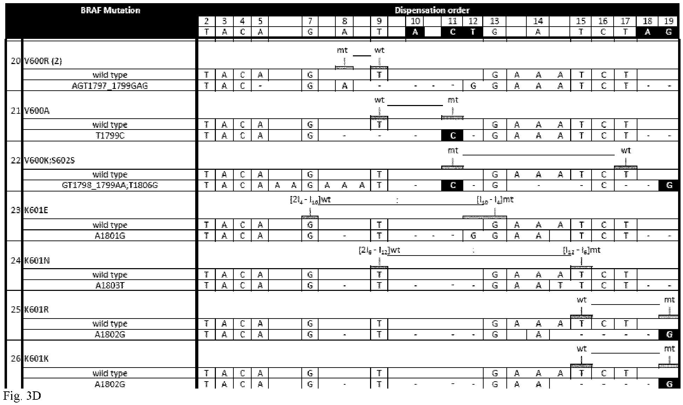

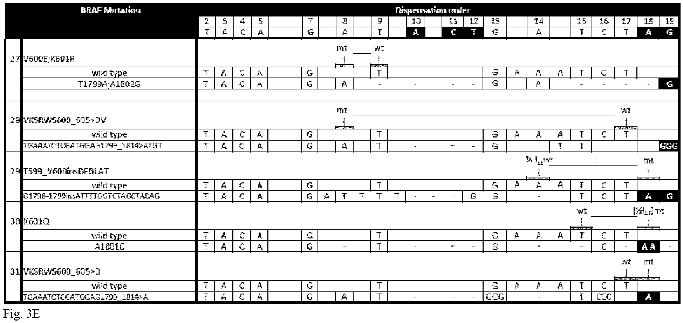

To overcome these limitations, we designed our approach analyzing the sense strand of *braf* gene (exon 15) towards mutations, deletions and/or insertions affecting V600 and/or codons downstream. We started the pyrosequencing analysis with the conventional dispensation order G₁A₂C₃G₄[A₅T₆]G₇A₈T₉ created by Pyromark Q24 software Version 2.0.6 (Qiagen) flanking the T1799A hotspot mutation at codon 600 and ending with the first nucleotide of codon 601. Negative nucleotide dispensations G₁ and C₃ were included by software as internal controls for possible nucleotide misincorporations. Although T1799A mutation was definitely determined by this dispensation order, the variant tandem mutations resulted in aberrant sequencing pyrograms including high peak height deviation at different dispensations, uncertain surrounding reference sequence patterns or high sum deviation in variable position. Because the knowledge of specific variant in each case could explain the altered pyrogram tracing created by a change in order and/or quantity of incorporation of each nucleotide, we embedded internal recognition patterns A₇C₈T₉ and A₁₅G₁₆ into dispensation order U-BRAF-V600 G₁A₂C₃G₄A₅T₆[A₇C₈T₉]G₁₀A₁₁T₁₂C₁₃T₁₄[A₁₅G₁₆] facilitating the identification of tandem mutations V600E (TG1799_1800AA), V600K and V600E;K601I. The presence of variant mutations beyond V600E affects the pyrogram sequence pattern by re-ordering of nucleotide incorporation in mutant DNA sequence resulting in unique pyrogram for each BRAF mutation (Table 3, Fig. 1). In this case, two recognition patterns of dispensation order serve to distinguish between individual mutations (Fig. 3). Furthermore, the ratio A₈:T₁₂ (in means of peak heights) distinguishes between tandem mutation V600E (5:1) and V600K (3:1) respectively (Fig. 3, Table 4).

Importantly, we found novel complex in-frame mutation p.VKS600_602delinsDT (c.TGAAAT1799_1804delinsATA, Table 3, Fig. 3). In concordance with previous study, we suppose that this mutation could activate BRAF kinase by charging aspartic acid in place of valine,⁵ although interpretation of the clinical significance of this variant can be challenging. We also identified tandem mutation V600E;K601I, which was incorrectly defined as V600E;K600M mutation by Indsto et al.⁶

Comparing with direct sequencing data, five additional samples were identified as BRAF mutants increasing the percentage of mutants to 70.7% convincing in better sensitivity of pyrosequencing (Table 1).

We next asked whether our approach could be suitable for detection of other BRAF mutations within exon 15. To test this idea, we performed the literature search for published BRAF mutations in different human tumors using Pubmed (http://www.ncbi.nlm.nih.gov/pubmed). To cover 31 BRAF mutations affecting codons from T599 to S605, we designed novel dispensation order U-BRAF^{V600}, G₁T₂A₃C₄A₅C₆G₇A₈T₉A₁₀C₁₁T₁₂G₁₃A₁₄T₁₅C₁₆T₁₇A₁₈G₁₉ (Fig. 3). G₁ and C₄ nucleotides were included as negative controls. Dispensations T₂A₃C₄ and T₁₇ were embedded for detection of BRAF mutations affecting either codon T599⁷⁻¹⁰ or within complex in-frame insertion T599_V600insDFLAGT,⁵ respectively.

Comparing our review of articles with Catalogue of Somatic Mutations in Cancer (COSMIC) database¹¹ we revealed a number of entries either representing one mutation as two independent entries or a complex mutation is divided into two independent cases. K601I, S602S and K601del mutations, which are described as individual mutations by COSMIC database, are indeed the parts of complex mutations V600E;K601I, V600E;S602S and V600E;K601del respectively. Although the mutation p.K601del is defined as a deletion of AAA triplet at position 1801 to 1803 (c.1801_1803delAAA),¹¹ this mutation is indeed created by deletion of triplet TGA at 1799-1801 (c.1799_1801delTGA) resulting in complex mutation V600_K601delinsE.⁵ Mutation c.1794_1795insGTT⁷ is represented both as p.A598_T599insV and as p.T599_V600insV.¹¹ Advisably, to distinguish tandem mutations from other type of BRAF mutants, it might be necessary to annotate these mutations into a separate group of complex mutations in the COSMIC database.
Due to the absence of correspondent nucleotide sequences in the original publication, the unique mutations K601E_W604 and T599T_V600R published by Edlundh-Rose et al.¹² not included into U-BRAF^{V600} method. Additionally, unpublished DNA sequencing data by Sadow et al. made impossible to prove the misspelled "VKWRV600-604E" mutation,¹³ which is annotated as V600_W604del in the COSMIC database.¹¹

**Table 2: BRAF mutations within exon 15 in cutaneous melanoma metastases**

| **Case¹** | **Sample¹** | **Age/Sex²** | **Sanger sequencing** | **Pyrosequencin g** | **mt/wt ratio³ (range in %)** | **cobas** |
|---|---|---|---|---|---|---|
| 1 | A,B | 53/f | V600E | V600E | 36;38 | |
| 2 | A,B | 47/f | V600E | V600E | 30;33 | |
| 3 | A,B,C | 40/m | V600E | V600E | 17-38 | |
| 4 | A,B | 79/m | V600E | V600E | 54;55 | |
| 5 | A | 55/m | wt | wt | - | |
| 6 | A,B,C | 69/m | wt | wt | - | 0 |
| 7 | A | 80/m | V600E | V600E | 37 | |
| 8 | A,B,C,D,E | 53/f | V600E | V600E | 18-38 | |
| 9 | A,B | 87/f | wt | wt | - | |
| 10 | A,B,C | 80/m | V600E | V600E | 59-60 | |
| 11 | A | 82/f | V600E | V600E | 16 | |
| | B,C | | wt | wt | - | 0 |
| 12 | A | 83/m | wt | wt | - | |
| 13 | A,B,C,D | 56/m | wt | wt | - | |
| 14 | A,B,C,D,E | 57/m | VKS600_602> DT | VKS600_602> DT | 23-42 | |
| 15 | A, | 74/f | wt | wt | 8 | 0 |
| | B | | wt | V600E | 16 | - |
| 16 | A,B | 65/m | V600E | V600E | 33;34 | |
| 17 | A,B | 52/m | V600K | V600K | 12;19 | -; |
| 18 | A | 30/m | V600E | V600E | 33 | |
| 19 | A | 75/f | wt | wt | - | 0 |
| 20 | A,B,D | 66/m | V600E | V600E | 22-41 | |
| | C,E | | wt | V600E | 16;11 | 0 |
| 21 | A,B | 73/m | V600E | V600E | 31-33 | 0 |
| | | | (TG>AA) | (TG>AA) | | |
| 22 | A,B | 37/m | V600K | V600K | 43;49 | |
| 23 | A | 71/f | wt | wt | - | |
| 24 | A,B | 52/m | wt | wt | - | |
| 25 | A | 54/f | V600E | V600E | 14 | |
| 26 | A,B,C,D,E,F ,G,H | 66/m | wt | wt | - | |
| 27 | A,B,C,D,E | 54/m | V600K | V600K | 43-61 | + |
| 28 | A,B | 78/f | V600E | V600E | 32;33 | |
| 29 | A,B | 44/m | V600E;K601I | V600E;K601I | 29;59 | 0 |

| | | | | | | |
|---|---|---|---|---|---|---|
| ¹different samples of the same tumor are specified by 1, 2 etc., different tumors of the same patient specified by A, B etc.; ²age in years, f = female, m = male; ³wt - wild type, mt - mutant. | | | | | | |

**Table 3: Recognition patterns for 31 BRAF mutations by U-BRAF-V600;**

| | Mutation | Dispensation Order Patterns | | | | | | Unique properties of each mutation within one group | mutant to wild type ratio* | Ref | Identification COSMIC v57 |
|---|---|---|---|---|---|---|---|---|---|---|---|
| | | A₁₀ | C₁₁ | T₁₂ | | A₁₈ | G₁₉ | | | | |
| **I** | V600E | - | - | - | | - | - | A₈=Aₘₜ; T₉= T_{wt} | A₈^{mt} : T₉^{wt} | 16 | COSM476 |
| | V600L | - | - | - | | - | - | G₇=G_{wt}; absence of A₈; T₉=[2Tₘₜ+ T_{wt}] | [I₁₀-I₄] G₇^{wt} | 15 | COSM33808 |
| | V600M | - | - | - | | - | - | G₇=G_{wt}; absence of A₈; T₉= [T_{wt}+ Tₘₜ] | [I₁₀ - I₄]^{mt} : G7^{wt} | 14 | COSM1130 |
| | V600R (AGT>GAG) | - | - | - | | - | - | A₅=A_{wt}; G₁₃=[G_{wt}+ 2Gₘₜ] | A₈^{mt} : T₉^{wt} | 16 | COSM1127 |
| | K601E | - | - | - | | - | - | absence of A₈; G₁₃=[G_{wt}+2Gₘₜ]; A₁₄=[13A_{wt}+2Aₘₜ] | [I₁₀ - I₄]^{mt} : [21₄ -I₁₀] | 17 | COSM478 |
| | K601N | - | - | - | | - | - | absence of A₈; T₉=[T_{wt}+ Tₘₜ]; 14=[3A_{wt}+2Aₘₜ] | [I₁₂ - I₆]^{mt} : [21₆ -I₁₂]^{wt} | 18 | COSM1132 |
| **II** | V600E;K601I | + | - | + | | - | - | A₈ : A₁₀ = 3 : 1 | T₁₂^{mt} T₁₅^{wt} | 6 | COSM475; COSM26491 |
| | V600D | + | - | + | | - | - | A₈ : A₁₀ = 1 : 3 | T₁₂^{mt}: T₁₅^{wt} | 16 | COSM477 |
| | V600G | + | - | + | | - | - | G₇=G_{wt}+3Gₘₜ; absence of A₈; A₁₀₌3Aₘₜ; G₁₃=G_{wt} | T₁₂^{mt}: T₁₅^{wt} | 15 | COSM6137 |
| **III** | V600E (TG>AA) | - | + | + | | - | + | A₈:T₁₂=5:1 | T₁₂^{mt} :T₁₅^{wt} | 6 | COSM475 |
| | V600K | - | + | + | | - | + | A₅=_{[}A_{wt}+3Aₘₜ]; (A₈ : T₁₂)=3: 1 | T₁₂^{mt}: T₁₅^{wt} | 16 | COSM473 |
| | V600R | - | + | + | | - | + | A₅=[A_{wt}+2A_{mt]} G₇=[Gwt+2G_{mtL} (A₈ : T₁₂)=3: 1 | T₁₂^{mt}: T₁₅^{wt} | 16 | COSM474 |
| | VK600_601>E | - | + | + | | - | + | A₈: T₁₂= 2:1 | T₁₂^{mt}: T₁₅^{wt} | 5 | COSM1133 |

| | Mutation | Dispensation Order Patterns | | | | | | Unique properties of each mutation within one group | mutant to wild type ratio* | Ref | SEQ_ID COSMIC v57** |
|---|---|---|---|---|---|---|---|---|---|---|---|
| | | **A₁₀** | **C₁₁** | **T₁₂** | | **A₁₈** | **G₁₉** | | | | |
| **III** | TVKSR599_603>1 | - | + | + | | - | + | absence of mutant C₄, A₅, G₇; absence of A₈ G₁₃=[G_{wt}+2Gₘₜ] | C₁₁^{mt} : C₄^{wt} | 8 | COSM30605 |
| **IV** | T599T;V600E | - | - | - | | - | + | absence of mutant A₅, G₇; absence of A₈ | G₁₉^{mt} : G₇^{wt} | 19 | COSM24963 COSM476 |
| | K601R | - | - | - | | - | + | absence of A₈; A₁₄=[3A_{wt}+Aₘₜ] | G₁₉^{mt} : T₁₅^{wt} | 15 | COSM13625 |
| | K601K | - | - | - | | - | + | absence of A₈; A₁₄=[3A_{wt}+2Aₘₜ] | G₁₉^{mt} : T₁₅^{wt} | 25 | COSM28507 |
| | V600E;K601R | - | - | - | | - | + | A₈=Aₘₜ; T₉=T_{wt} | A₈^{mt} :T₉^{wt} | 14 | COSM475 COSM13625 |
| **IV** | VKSRWS600_605>DV | - | - | - | | - | + | G₁₉= 3Gₘₜ; absence of mutant A₁₄; T₁₅=[T_{wt}+2Tₘₜ] | A₈^{mt} : T₁₇^{wt} | 20 | COSM33764 |
| **V** | K601Q | - | - | - | | + | - | absence of A₈; T₉=[T_{wt}+ Tₘₜ]; A₁₈=2Aₘₜ | [½ I₁₈] ^{mt}:T₁₅^{wt} | 21 | COSM1066665 |
| | VKSRWS600_605>D | - | - | - | | + | - | G₁₃=[G_{wt}+ 3Gₘₜ] C₁₆=[C_{wt}+3Cₘₜ] | A₁₈^{mt} : T₁₇^{wt} | 22 | COSM1129 |

| | Mutation | Dispensation Order Patterns | | | | | | Unique properties of each mutation within one group | mutant to wild type ratio* | Ref | SEQ ID COSMIC v57** |
|---|---|---|---|---|---|---|---|---|---|---|---|
| | | **A₁₀** | **C₁₁** | **T₁₂** | | **A₁₈** | **G₁₉** | | | | |
| **VI** | V600K;S602S | - | + | - | | - | + | A₈=3Aₘₜ | T₁₇^{mt}: C₁₁^{wt} | 19 | COSM473; COSM21611 |
| | T599_V600insT (2) | - | + | - | | - | + | absence of A₈ | C₁₁^{mt}: C₁₆^{wt} | 23 | COSM36245 |
| **VII** | T599_V600msT (1) | + | + | - | | - | + | absence of mutant G_{7;} T9=[T_{wt}+Tₘₜ] | C₁₁^{mt} : C₁₆^{wt} | 24 | COSM30730 |
| | T599_V600msV | + | + | - | | - | + | absence of mutant A₃, C₄; T₉=[T_{wt}+2Tₘₜ] | A₁₀^{mt} : A₅^{wt} | 7 | COSM26625 |
| **VIII** | VKS600_602>DT | + | + | + | | - | + | A₈=Aₘₜ; absence of mutant A₁₄ | T₁₂^{mt} T₁₅^{wt} | - | unpublished |
| | T599_V600insTT | + | + | + | | - | + | absence of A₈; absence of mutant A₅, G₇ | T₁₂^{mt} : T₁₅^{wt} | 16 | COSM26459 |
| **IX** | T5991;V600E | + | - | - | | - | + | A₁₄=3A_{wt}+Aₘₜ; T₁₇=T_{wt} | A₁₀^{mt}: A₅^{wt} | 9 | COSM472; COSM476 |
| | TV599_600>IAL | + | - | - | | - | + | absence of mutant A₁₄;T₁₇=[T_{wt}+3Tₘₜ] | A₁₀^{mt} : A₅^{wt} | 10 | COSM33780 |
| | T599_V600insDFLAGT | - | - | - | | + | + | unique; T₉=[T_{wt} + 4Tₘₜ] | A₁₈^{mt}:[⅓I₁₁]^{wt} | 5 | COSM26504 |
| | V600A | - | + | - | | - | - | unique; absence of A₈ | C₁₁^{mt} : T₉^{wt} | 14 | COSM18443 |

| | | | | | | | | | | | |
|---|---|---|---|---|---|---|---|---|---|---|---|
| * I - intensity value of correspondent nucleotide dispensation; A-peak reduction factor 0.9 should be taken into consideration; **COSMIC - Catalogue of Somatic Mutations in Cancer, Version 57, Sanger Institute | | | | | | | | | | | |

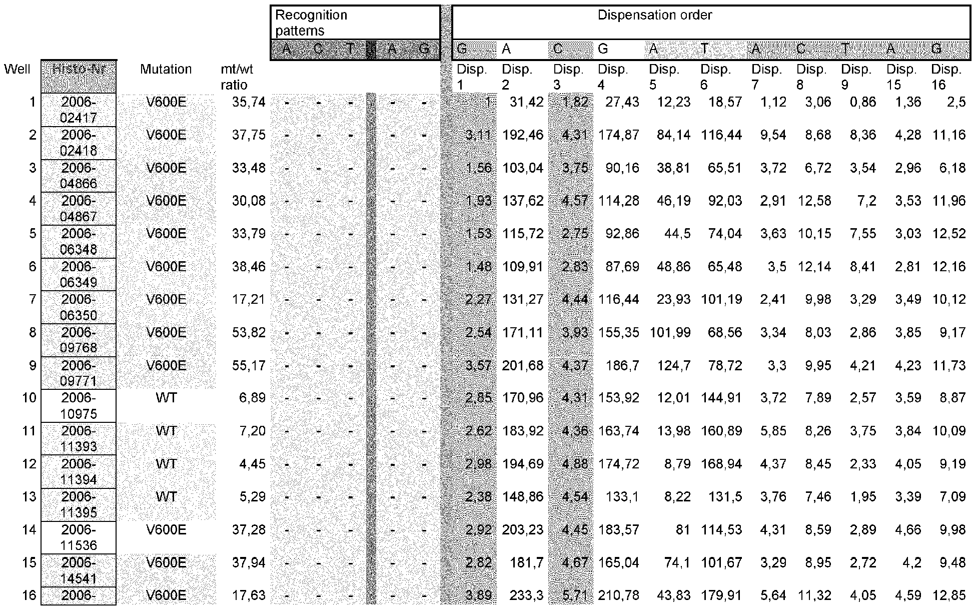

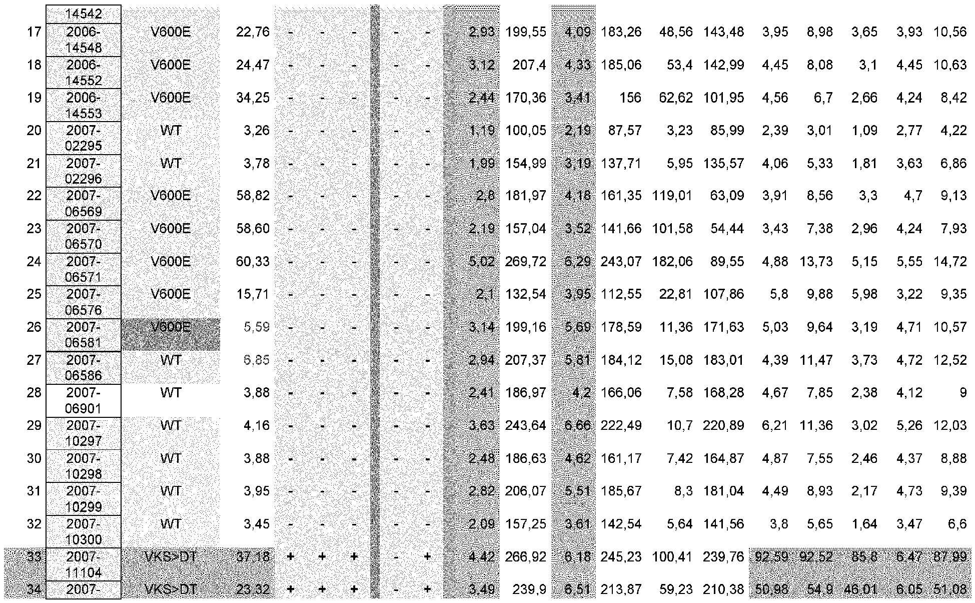

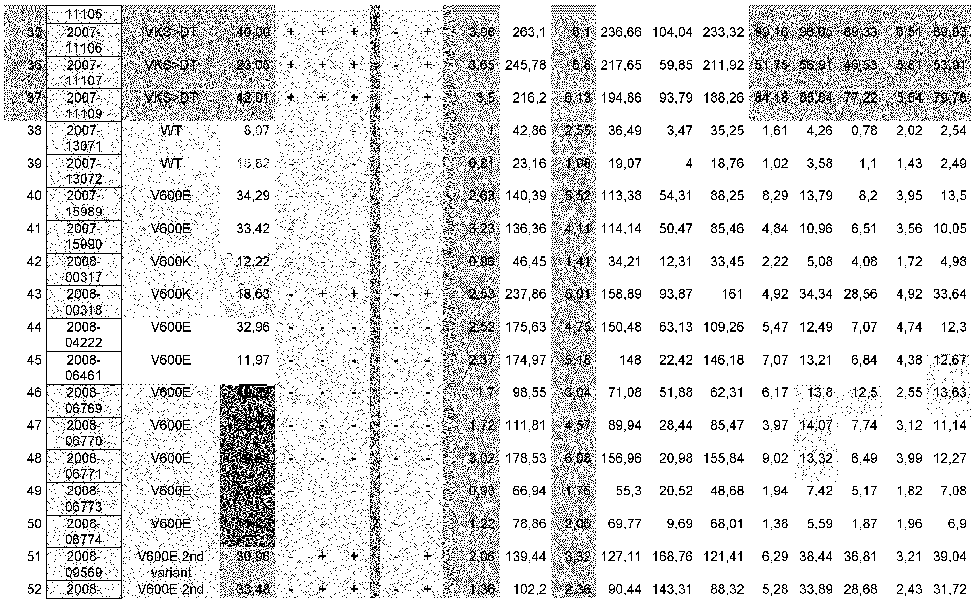

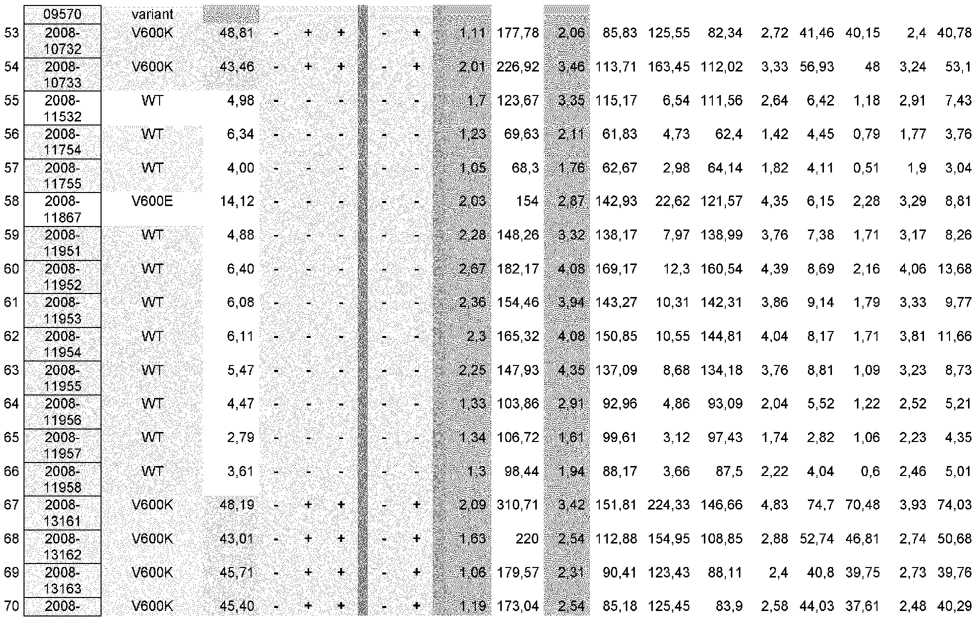

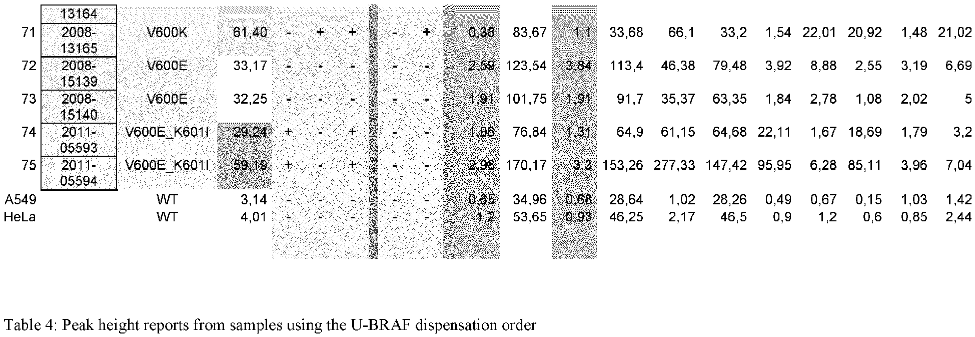

### Reference List

1. Chung, K. W. et al. Clin. Endocrinol. (Oxf) 65, 660-666 (2006).
2. Heyries, K. A. et al. Nat. Methods 8, 649-651 (2011).
3. Jo, Y. S. et al. Clin. Endocrinol. (Oxf) 70, 139-144 (2009).
4. Spittle, C. et al. J. Mol. Diagn. 9, 464-471 (2007).
5. Hou, P., Liu, D., Xing, M. Cell Cycle 6, 377-379 (2007).
6. Indsto, J. O. et al. J. Cutan. Pathol. 34, 448-455 (2007).
7. Carta, C. et al. Clin. Endocrinol. (Oxf) 64, 105-109 (2006).
8. Lupi, C. et al. J. Clin. Endocrinol. Metab 92, 4085-4090 (2007).
9. Gill, M., Renwick, N., Silvers, D. N., Celebi, J. T. J. Invest Dermatol. 122, 1325-1326 (2004).
10. Chiosea, S. et al. Endocr. Pathol. 20, 122-126 (2009).
11. Forbes, S. A. et al. Curr. Protoc. Hum. Genet. Chapter 10, Unit 11.1-26 (2008).
12. Edlundh-Rose, E. et al. Melanoma Res. 16, 471-478 (2006).
13. Sadow, P. M., Heinrich, M. C., Corless, C. L., Fletcher, J. A., Nose, V. Endocr. Pathol. 21, 73-79 (2010).
14. Lin, J. et al. Br. J. Cancer 104, 464-468 (2011).
15. Boulalas, I., Zaravinos, A., Delakas, D., Spandidos, D. A. Int. J. Biol. Markers 24, 17-21 (2009).
16. Omholt, K., Platz, A., Kanter, L., Ringborg, U., Hansson, J. Clin. Cancer Res. 9, 6483-6488 (2003).
17. Trovisco, V. et al. Hum. Pathol. 36, 694-697 (2005).
18. Helmke, B. M. et al. Gastroenterology 127, 1815-1820 (2004).
19. Deichmann, M. et al. BMC. Cancer 5, 58 (2005).
20. Barzon, L. et al. Eur. J. Endocrinol. 159, 77-80 (2008).
21. Sarkozy, A. et al. Hum. Mutat. 30, 695-702 (2009).
22. Cruz, F., III et al. Cancer Res. 63, 5761-5766 (2003).
23. Schindler, G. et al. Acta Neuropathol. 121, 397-405 (2011).
24. Eisenhardt, A. E. et al. Int. J. Cancer (2010).
25. Deichmann, M. et al. Int. J. Oncol. 29, 139-145 (2006).

## Claims

1. A method for differentiating between at least two sequence variants of a polynucleotide by pyrosequencing, wherein at least one sequence variant comprises at least one mutation, said method comprising:
a) generating a dispensation order based on a nucleotide sequence encompassing the nucleotides affected by said variants, said dispensation order comprising dispensations corresponding to each nucleotide occurring in at least one of the variants, said nucleotide dispensations ordered in a sequence
i) generating on average a pyrogram peak for at least every second nucleotide dispensation for each variant and
ii) generating the maximal possible number of sequence pyrogram peaks for each variant;
b) generating according to the dispensation order of a) a first pyrogram comprising expected intensity values for a first variant and at least a second pyrogram comprising expected intensity values for at least one further variant;
c) comparing a sample pyrogram comprising measured intensity values, said sample pyrogram being derived from pyrosequencing analysis of a sample polynucleotide according to the nucleotide dispensation order of a), with the said first and at least the said second pyrogram generated in step b); and
d) differentiating between said at least two sequence variants, wherein the sample polynucleotide corresponds to the first variant if the sample pyrogram is essentially identical with the first pyrogram and wherein the sample polynucleotide corresponds to a further variant if the sample pyrogram is essentially identical with a further pyrogram.

2. The method of claim 1, wherein step b) further comprises comparing said first and at least one further pyrogram and by this means identifying at least one unique property for a first variant compared to at least one second variant based on said first and at least one second pyrogram, wherein in step c) the sample pyrogram comprises for at least each nucleotide of the at least one unique property a measured intensity value, and wherein in step d) the sample polynucleotide corresponds to the first variant if the sample pyrogram comprises the at least one unique property corresponding to the first variant and wherein the sample polynucleotide corresponds to a further variant if the sample pyrogram comprises the at least one unique property corresponding to a further variant.

3. The method of claim 2, wherein at least one of the at least one unique properties is comprised in a unique dispensation order pattern.

4. The method of claim 2 or 3, wherein said at least one unique property comprises at least one property selected from the group consisting of:
(i) the presence or absence of a pyrogram peak for the first variant;
(ii) the presence or absence of a pyrogram peak for at least one further variant;
(iii) the expected intensity value for a pyrogram peak for the first variant;
(iv) the expected intensity value pyrogram peak for at least one further variant; and
(v) the ratios of expected intensity values of (iii) and (iv) or (iv) and (iii).

5. The method of any one of claims 1 to 4, wherein steps a) to d) of said method are carried out computer-implemented.

6. The method of any one of claims 1 to 5, wherein said at least two variants comprise at least one mutation in exon 15 of the *braf* gene.

7. The method of claim 6, wherein the at least one mutation is located at a nucleotide position corresponding to nucleotide positions 1794 to 1804 of the wild-type *braf* open reading frame.

8. The method of any one of claims 1 to 7, wherein said mutation is a mutation associated with a disease.

9. The method of claim 8, wherein said disease is cancer.

10. The method of claim 9, wherein said cancer is acute myeloid leukemia (AML), acute myeloid leukemia therapy related (AMLTR), astrocytoma, biliary tract carcinoma, breast carcinoma, cholangiocarcinoma, colorectal carcinoma, endometrioid carcinoma, esophageal carcinoma, fibrous histiocytoma-pleomorhic sarcoma, gastrointestinal stromal tumor (GIST), glioma, hepatocellular carcinoma, large intestine adenocarcinoma, small intestine adenocarcinoma, liposarcoma, lung adenocarcinoma, melanoma, malignant melanoma, melanoma metastasis, ocular melanoma, ovarian carcinoma, ovary mucinous carcinoma, ovary serous carcinoma, pancreatic ductal carcinoma, pituitary adenoma, plasma cell myeloma, prostate adenocarcinoma, stomach adenocarcinoma, synovial sarcoma, thyroid anaplastic carcinoma, or thyroid papillary carcinoma.

11. The method of any one of claims 1 to 9, wherein said dispensation order is G₁T₂A₃C₄A₄C₆G₇A₈T₉A₁₀C₁₁T₁₂G₁₃A₁₄T₁₅C₁₆T₁₇A₁₈G₁₉ or G₁A₂C₃G₄A₅T₆A₇C₈T₉G₁₀A₁₁T₁₂C₁₃T₁₄A₁₅G₁₆.

12. The method of claim 3, wherein said unique dispensation order patterns are A₁₀C₁₁T₁₂ in combination with A₁₈G₁₉ or A₇C₈T₉ in combination with A₁₅G₁₆.

13. The method of any one of claims 1 to 12, wherein said variants are variants selected from the list provided in Table 1.

14. The method of claim 13, wherein said at least one unique property is a unique property as shown in Table 1.

15. The method of any one of claims 1 to 14, wherein at least two variants present in the same sample are differentiated from at least one further variant.
